# EUROPEAN PATENT SPECIFICATION

(11) **EP 3 080 611 B1**
(45) Date of publication and mention of the grant of the patent: **14.11.2018**
(21) Application number: 14870444.8
(22) Date of filing: 12.12.2014
(51) Int. Cl.: G01N 33/68, C07K 16/18, C07K 14/47, G01N 33/50

(54) **SOLUBLE HIGH MOLECULAR WEIGHT (HMW) TAU SPECIES AND APPLICATIONS THEREOF**
LÖSLICHE HOCHMOLEKULARE (HMW) TAU-SPEZIES UND ANWENDUNGEN DAVON
ESPÈCE TAU SOLUBLE DE POIDS MOLÉCULAIRE ÉLEVÉ (HMW) ET SES APPLICATIONS

(30) Priority: 13.12.2013 US 201361915762 P; 30.07.2014 US 201462030984 P; 03.09.2014 US 201462045313 P
(43) Date of publication of application: 19.10.2016
(73) Proprietor: The General Hospital Corporation, Boston, MA 02114 (US)
(72) Inventor: HYMAN, Bradley, Swampscott, Massachusetts 01907 (US); TAKEDA, Shuko, Boston, Massachusetts 02114 (US)
(74) Representative: Crease, Devanand John
(86) International application number: PCT/US2014/069967
(87) International publication number: WO 2015/089375

(56) References cited:
- WO-A1-2009/033151
- WO-A1-2009/033151
- WO-A1-2013/103964
- WO-A2-2010/144711
- WO-A2-2010/144711
- WO-A2-2012/054008
- US-A1- 2009 176 728
- US-A1- 2013 028 914
- US-A1- 2013 028 914
- US-A1- 2013 150 560
- SHUKO TAKEDA ET AL: "Neuronal uptake and propagation of a rare phosphorylated high-molecular-weight tau derived from Alzheimer's disease brain", NATURE COMMUNICATIONS, vol. 6, 13 October 2015 (2015-10-13), page 8490, XP055363852, DOI: 10.1038/ncomms9490
- SHUKO TAKEDA ET AL: "Seed-competent high-molecular-weight tau species accumulates in the cerebrospinal fluid of Alzheimer's disease mouse model and human patients", ANNALS OF NEUROLOGY., vol. 80, no. 3, 3 August 2016 (2016-08-03) , pages 355-367, XP055363979, BOSTON, US ISSN: 0364-5134, DOI: 10.1002/ana.24716
- TAKEDA SHUKO ET AL: "A unique high-molecular-weight tau species is involved in propagation and accumulates in the cerebrospinal fluid of Alzheimer's disease patients", ALZHEIMER'S & DEMENTIA: THE JOURNAL OF THE ALZHEIMER'SASSOCIATION, vol. 11, no. 7, July 2015 (2015-07), XP029354208, ISSN: 1552-5260, DOI: 10.1016/J.JALZ.2015.06.938
- WARD ET AL.: 'Tau oligomers and tau toxicity in neurodegenerative disease' BIOCHEMICAL SOCIETY TRANSACTIONS vol. 40, August 2012, pages 667 - 671, XP055294513
- COWAN ET AL.: 'Are tau aggregates toxic or protective in tauopathies?' FRONTIERS IN NEUROLOGY vol. 4, 13 August 2013, pages 1 - 13, XP055294516
- GEORGIEFF ET AL.: 'Expression of high molecular weight tau in the central and peripheral' JOURNAL OF CEIII SCIENCE vol. 105, July 1993, pages 729 - 737, XP055294520
- POOLER ET AL.: 'Propagation of tau pathology in Alzheimer's disease: identification of novel therapeutic targets' ALZHEIMER'S RESEARCH & THERAPY vol. 5, 23 October 2013, pages 1 - 8, XP055294534

## Description

### TECHNICAL FIELD

The technology described herein relates generally to novel forms of tau species and applications thereof as well as methods of treating and/or diagnosing tau-associated neurodegeneration in a subject.

### BACKGROUND

Accumulation and aggregation of microtubule-associated protein tau (Mandelkow et al. (1995) Neurobiology of aging, 16(3):355-362; discussion 362-353), as intracellular inclusions known as neurofibrillary tangles (NFTs), is a pathological hallmark of neurodegenerative diseases including Alzheimer's disease (AD) (Iqbal et al. (2010) Current Alzheimer research, 7(8): 656-664). Cognitive deficits in AD are most closely linked with progression of NFTs in a hierarchical pattern, starting in the entorhinal cortex (EC) and marching throughout the brain during disease progression (Hyman et al. (1984) Science, 225 (4667): 1168-1170). One theory posits a "prion-like" spreading of tau: misfolded tau travels between neurons and provides a template for aggregation of naive endogenous tau in recipient neurons, which becomes neurotoxic.

WO 2010/144711 A2 describes methods and compositions for treating, preventing and diagnosing Alzheimer's Disease or other taupathies in a subject by administering an immunogenic tau peptide or an antibody recognizing the immunogenic tau epitope.

Although the precise mechanisms for this characteristic tau pathology spread remain unknown, it has been previously discussed that the tau pathology spread can occur by a trans-synaptic transfer of tau proteins between neurons (Pooler et al. (2013) Alzheimer's research & therapy, 5(5): 49; Walker et al. (2013) JAMA neurology, 70(3): 304-310). However, the tau species involved in inter-neuron propagation remains unclear.

Better understanding of the molecular basis of tau propagation can allow preventing progression from early mild memory impairment to full cognitive deterioration and dementia. Accordingly, there is a need to identify specific tau species responsible for inter-neuron propagation, which can be used as a more effective target for therapeutic intervention and biomarker development.

### SUMMARY

Various aspects described herein stem from, at least in part, discovery of soluble high molecular weight (HMW) tau species (including phosphorylated form) present at a low level in the brain of subjects with Alzheimer's disease (AD) or frontotemporal dementia (FTD), and abilities of the soluble HMW tau species to be taken up by a neuron and propagate between neurons. In particular, to identify the soluble HMW tau species responsible for propagation, the inventors have developed a novel 3-chamber microfluidic device to form dual-layered neurons and examine neuronal tau uptake, axonal transport, and synaptic transmission. By characterizing uptake and propagation properties of different tau species derived from, for example, the interstitial fluid of awake, behaving tau-transgenic mice, and cortical extracts from the mice as well as human AD postmortem cortices, the inventors have discovered, that PBS-soluble phosphorylated high-molecular-weight (HMW) tau species, though very low in abundance, are taken up, axonally transported, and passed-on to synaptically connected neurons in a time- and concentration-dependent manner. In contrast, low molecular weight (LMW) tau species (e.g., monomer/dimer size tau), though much higher in abundance, are not efficiently taken up by neurons. Thus, the discovery of the rare species of soluble HMW phosphorylated tau involved in inter-neuron-propagation provides a more effective target for therapeutic intervention and biomarker development.

Further, the inventors have discovered that tau propagation between neurons does not require endogenous, intracellular tau for template misfolding, but tau-null mice had substantially less pathological misfolding and gliosis, and thus, suitably, complete removal of endogenous or host tau produces a neuroprotective effect. For example, the inventors have shown that tau-null neurons lacked activation of Aβ-induced mitochondrial intrinsic caspase cascades in the neurons and were subsequently protected from Aβ-induced dendritic spine loss and neurodegeneration. Accordingly, described herein are compositions comprising soluble HMW tau species that is responsible for inter-neuron propagation and applications thereof. Methods of treating and diagnosing tau-associated neurodegeneration in a subject are also described herein.

A composition comprising soluble high molecular weight (HMW) tau species is described herein. The soluble HMW tau species in the composition is non-fibrillar, with a molecular weight of at least about 500 kDa, and the composition is substantially free of soluble low molecular weight (LMW) tau species.

The soluble HMW tau species can have a molecular weight of at least about 669 kDa. In some embodiments, the soluble HMW tau species can have a molecular weight of about 669 kDa to about 1000 kDa.

The non-fibrillar soluble HMW tau species can be in a form of particles. The particle size can vary with the molecule weight of the tau species. The particle size can range from about 10 nm to about 30 nm.

The inventors have discovered that the AD brain extract contained significantly higher levels of phosphorylated, soluble HMW tau species, when compared to a control brain without AD. Thus, the soluble HMW tau species in the composition can be phosphorylated. The soluble HMW tau species in the composition can be hyper-phosphorylated.

The soluble HMW tau species can be soluble in an aqueous and/or buffered solution. For example, the soluble HMW tau species can be soluble in phosphate-buffered saline. The soluble HMW tau species can be soluble in a biological fluid, e.g., a brain interstitial fluid or cerebrospinal fluid.

The soluble HMW tau species can be preferentially taken up by a neuron and axonally transported from the neuron to a synaptically-connected neuron, as compared to neuron uptake and neuron-to-neuron transport of the soluble LMW tau species. The soluble LMW tau species has a lower molecular weight than that of the soluble HMW tau species. The soluble LMW tau species can have a molecular weight of no more than 200 kDa.

The compositions described herein can comprise an agent to suit the need of a selected application. For example, where the HMW tau is to be used as an antigen to raise an antibody, purified soluble HMW tau can be combined with saline or phosphate-buffered saline. Alternatively, or in addition, the HMW tau antigen can be admixed with or conjugated to an adjuvant or carrier to enhance its antigenicity.

Accordingly, one aspect described herein provides an isolated antibody or antigen-binding portion thereof that specifically binds soluble high molecular weight (HMW) tau species and does not bind soluble low molecular weight (LMW) tau species. The HMW tau species is non-fibrillar, with a molecular weight of at least about 500 kDa, and the LMW tau species has a molecular weight of no more than 200 kDa. The soluble HMW tau species can have a molecular weight of at least about 669 kDa or more. In some embodiments, the soluble HMW tau species can have a molecular weight of about 669 kDa to about 1000 kDa.

The non-fibrillar soluble HMW tau species can be in a form of particles. The particle size can vary with the molecule weight of the tau species. The particle size can range from about 10 nm to about 30 nm.

In some embodiments, the isolated antibody or antigen-binding portion thereof can specifically bind a phosphorylated form of the soluble HMW tau species.

The isolated antibody or antigen-binding portion thereof can specifically bind the soluble HMW tau species soluble in an aqueous solution and/or a buffered solution. For example, the isolated antibody or antigen-binding portion thereof can specifically bind the soluble HMW tau species soluble in phosphate-buffered saline. The isolated antibody or antigen-binding portion thereof can specifically bind the soluble HMW tau species soluble in a biological fluid, e.g., a brain interstitial fluid or cerebrospinal fluid.

The isolated antibody or antigen-binding portion thereof can be designed to reduce the soluble HMW tau species being taken up by a neuron by at least about 10% or more.

The isolated antibody or antigen-binding portion thereof can be designed to reduce the soluble HMW tau species being axonally transported from a neuron to a synaptically-connected neuron by at least about 10% or more.

The inventors have shown that a relatively low level of soluble HMW tau species was released from the neurons and found in brain interstitial fluid, and that the soluble HMW tau species, which accounts for only a small fraction of all tau in the samples, was robustly taken up by neurons and was involved in inter-neuron propagation, whereas uptake of soluble LMW tau species (e.g., monomer/dimer size) tau was very inefficient. Thus, a method of preventing propagation of pathological tau protein between synaptically-connected neurons is also described herein. One aspect provided described herein provides an antagonist of a soluble HMW tau species for use in the treatment of neurodegenerative diseases by preventing propagation of pathological tau protein between synaptically-connected neurons is also provided herein. The method or treatment comprises selectively reducing the extracellular level of soluble HMW tau species in contact with a synaptically-connected neuron, wherein the soluble HMW tau species is non-fibrillar, with a molecular weight of at least about 500 kDa. A reduced level of the soluble HMW tau species results in reduced propagation of pathological tau protein between synaptically-connected neurons.

In some embodiments, the extracellular level of soluble LMW tau species is not substantially reduced during said selective reduction.

Methods or treatments for selectively reducing the extracellular level of soluble HMW tau species can be based on physical removal and/or molecular interactions between the soluble HMW tau species and a proper antagonist. The soluble HMW tau species can be selectively reduced by microdialysis. In some embodiments, the soluble HMW tau species can be selectively reduced by contacting the extracellular space or fluid in contact with the synaptically-connected neurons with an antagonist of the soluble HMW tau species. Examples of an antagonist of the soluble HMW tau species include, without limitations, an antibody, a nuclease (e.g., but not limited to, a zinc finger nuclease (ZFN), transcription activator-like effector nuclease (TALEN), a CRISPR/Cas system, a transcriptional repressor, a nucleic acid inhibitor (e.g., RNAi, siRNA, anti-miR, antisense oligonucleotides, ribozymes, and a combination of two or more thereof), a small molecule, an aptamer, and a combination of two or more thereof.

Tau pathology is known to spread in a hierarchical pattern in Alzheimer's disease (AD) brain during disease progression, e.g., by trans-synaptic tau transfer between neurons. Since the soluble HMW tau species is identified herein to be involved in neuron-to-neuron propagation, intervention to deplete such HMW tau species can inhibit tau propagation and hence disease progression in tauopathies. Accordingly, a method of reducing tau-associated neurodegeneration in a subject is described herein. Examples of tau-associated neurodegeneration include, but are not limited to, Alzheimer's disease, Parkinson's disease, or frontotemporal dementia. The method of treatment comprises selectively reducing the level of soluble HMW tau species in the brain or cerebrospinal fluid (CSF) of a subject determined to have, or be at risk for, tau-associated neurodegeneration, wherein the soluble HMW tau species is non-fibrillar, with a molecular weight of at least about 500 kDa, wherein a reduced level of the soluble HMW tau species results in reduced tau- associated neurodegeneration.

Suitably, the level of soluble LMW tau species in the subject is not substantially reduced during the treatment.

Suitably, at least a portion of the soluble HMW tau species present in brain interstitial fluid of the subject is removed or rendered inactive. Suitably, at least a portion of the soluble HMW tau species present in cerebrospinal fluid of the subject is removed or rendered inactive.

Methods for selectively reducing the extracellular level of soluble HMW tau species in the brain of a subject can be based on physical removal and/or molecular interactions between the soluble HMW tau species and a proper antagonist. The soluble HMW tau species present in the brain interstitial fluid and/or cerebrospinal fluid of the subject can be selectively reduced by brain microdialysis. The soluble HMW tau species present in the brain interstitial fluid and/or cerebrospinal fluid of the subject can be selectively reduced by administering to the brain or CSF of the subject an antagonist of soluble HMW tau species. Examples of an antagonist of the soluble HMW tau species include, without limitations, an antibody, a nuclease (e.g., but not limited to, a zinc finger nuclease (ZFN), transcription activator-like effector nuclease (TALEN), a CRISPR/Cas system, a transcriptional repressor, a nucleic acid inhibitor (e.g., RNAi, siRNA, anti-miR, antisense oligonucleotides, ribozymes, and a combination of two or more thereof), a small molecule, an aptamer, and a combination of two or more thereof.

The method can further comprise selecting a subject determined to have soluble HMW tau species present in the brain or CSF at a level above a reference level. A reference level can represent a level of soluble HMW tau species present in healthy subject(s).

In a further aspect, a method of diagnosing tau-associated neurodegeneration based on the presence and/or levels of the soluble HMW tau species is also provided herein. Exemplary tau-associated neurodegeneration includes, but is not limited to, Alzheimer's disease, Parkinson's disease, or frontotemporal dementia. The inventors have shown that while the total tau levels in brain extracts from AD and control brains were not significantly different, the AB brain extract contained significantly higher levels of soluble HMW tau species or phosphorylated, soluble HMW tau species, when compared to the control brain. Therefore, the method of diagnosing tau-associated neurodegeneration can comprise (a) fractionating a sample of brain interstitial fluid or cerebrospinal fluid from a subject; and (b) detecting soluble HMW tau species in the sample such that the presence and amount of the soluble HMW tau species is determined, wherein the soluble HMW tau species is non-fibrillar, with a molecular weight of at least about 500 kDa; and (c) identifying the subject to have, or be at risk for tau-associated neurodegeneration when the level of the soluble HMW tau species in the sample is the same as or above a reference level; or identifying the subject to be less likely to have tau-associated neurodegeneration when the level of the soluble HMW tau species is below a reference level. A reference level can represent a level of soluble HMW tau species present in healthy subject(s).

In some embodiments, the sample, prior to the fractionating of (a), can be substantially free of soluble LMW tau species, wherein the soluble LMW tau species has a molecular weight of no more than 200 kDa. For example, a sample of brain interstitial fluid or cerebrospinal fluid can be obtained from a subject to be diagnosed by microdialysis, e.g., using a proper filter molecular-weight cut-off, which would allow only molecules with a molecular weight of at least about 600 kDa to be collected.

The sample, prior to the fractionating of (a), can comprise soluble LMW tau species, wherein the soluble LMW tau species has a molecular weight of no more than 200 kDa. By fractionating the sample, one can isolate the soluble HMW tau species from the rest of the sample (e.g., a portion including soluble LMW tau species) to determine a diagnostic level. Without limitations, fractionation can be based on size exclusion and/or antibody-based methods.

In some embodiments where soluble LMW tau species is present in the sample, the method can further comprise detecting the amount of the soluble LMW tau species in the sample. In these embodiments, the subject can be identified to have, or be at risk for tau-associated neurodegeneration if a ratio of the soluble HMW tau species to the soluble LMW tau species is the same as or above a reference level ratio; or the subject is identified to be less likely to have tau-associated neurodegeneration if the ratio of the soluble HMW tau species to the soluble LMW tau species is below the reference level ratio. A reference level ratio can represent a level ratio of soluble HMW tau species to soluble LMW tau species present in healthy subject(s).

In some embodiments, the detecting can further comprise detecting phosphorylation of the soluble HMW tau species.

Not only does the discovery of soluble HMW tau species provide a therapeutic target and a biomarker for tau-associated neurodegeneration as described herein, the soluble HMW tau species can also be used in vitro to induce inter-neuron propagation, a phenotypic feature of progression in neurodegeneration, and thus develop an in vitro model to screen for effective agents that reduce cross-synaptic spread of misfolded tau proteins and thus treat tau-associated neurodegeneration. Accordingly, a further aspect provided herein relates to a method of identifying an agent that is effective to reduce cross-synaptic spread of misfolded tau proteins. The method comprises (a) contacting a first neuron in a first chamber of a neuron culture device with soluble HMW tau species, wherein the first neuron is axonally connected with a second neuron in a second chamber of the neuron culture device, and wherein the second neuron is not contacted with the soluble HMW tau species; (b) contacting the first neuron from (a) in the first chamber with a candidate agent; and (c) detecting transport of the soluble HMW tau species from the first neuron to the second neuron. An effective agent for reducing cross-synaptic spread of misfolded tau proteins can be identified based on detection of the presence of the soluble HMW tau species in an axon and/or soma of the second neuron.

While any neuron culture device suitable for monitoring axonal extension and/or transport can be used in the methods described herein, in some embodiments, the neuron culture device is a microfluidic device. In some embodiments, the microfluidic device can comprise a first chamber for placing a first neuron and a second chamber for placing a second neuron, wherein the first chamber and the second chamber are interconnected by at least one microchannel exclusively sized to permit axon growth.

The soluble HMW tau species described herein can also be used in screening assays to identify agents that modulate the formation or activity of the HMW tau species itself (e.g., by blocking the formation or stability of the HMW tau species, or, for example, by blocking post-translational modifications or by destabilizing the HMW tau structure). For example, aptamers, small molecules or other agents can be applied to neuronal cell cultures and the presence or amount of soluble HMW tau monitored. An agent so identified that blocks the formation or accumulation of HMW tau would be of interest as a potential therapeutic.

The inventors have shown that as compared to tau-expressing animals, tau-null animals have displayed less pathological tau misfolding and gliosis, even in the presence of neurofibrillary tangles (NFTs), and have also displayed a reduced activation of Aβ-induced mitochondrial intrinsic caspase cascades in the neurons. Thus, removing endogenous, intracellular tau proteins can provide a neuroprotective effect, e.g., reducing neurotoxicity and/or increasing neuron survival. While previous reports have discussed that a reduction of tau proteins can improve neurodegeneration, one of skill in the art would not have expected that removing a significant amount of endogenous, intracellular tau proteins, i.e., essential proteins that stabilize microtubules, would not produce any other adverse effects to the neurons. However, the inventors here have discovered that a tau-null human subject has normal neuron phenotypes as a healthy human subject expressing tau proteins. As such, methods of reducing neural damage or neurodegeneration induced by tauopathy are also described herein. Exemplary tauopathy can be Alzheimer's disease, Parkinson's disease, or frontotemporal dementia.

The method of reducing neural damage or neurodegeneration induced by tauopathy comprises administering to the brain of a subject determined to have tauopathy an agent that inhibits at least about 50% expression level of endogenous, intracellular tau protein in the subject, thereby reducing neurotoxicity (and/or increasing neuron survival) in the presence of neurofibrillary tangles.

The agent can inhibit at least about 70% expression level of the tau protein in the subject. The agent can inhibit at least about 90% expression level of the tau protein in the subject. The agent can inhibit at least about 95% expression level of the tau protein in the subject. The agent can inhibit 100% expression level of the tau protein in the subject.

The agent selected to inhibit the expression levels of endogenous, intracellular tau protein can disrupt expression of the MAPT (microtubule-associated protein tau) gene and/or inhibit transcription of the MAPT gene. Such agent can include, but is not limited to, an antibody, a nuclease (e.g., but not limited to, a zinc finger nuclease (ZFN), transcription activator-like effector nuclease (TALEN), a CRISPR/Cas system, a transcriptional repressor, a nucleic acid inhibitor (e.g., RNAi, siRNA, anti-miR, antisense oligonucleotides, ribozymes, and a combination of two or more thereof), a small molecule, an aptamer, and a combination of two or more thereof.

Methods known for effectively delivering an agent to the brain of a subject can be used to perform the methods described herein. The agent can be administered to the brain via a carrier. An exemplary carrier can be an adeno-associated virus.

The brain of the subject can be further determined to have an amyloid beta plaque and the administration can reduce neurotoxicity (and/or increases neuron survival) in the presence of amyloid beta.

### BRIEF DESCRIPTION OF THE DRAWINGS

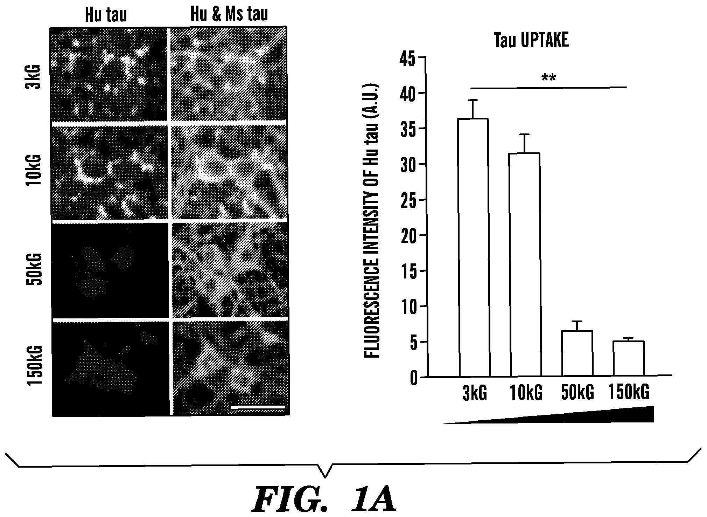

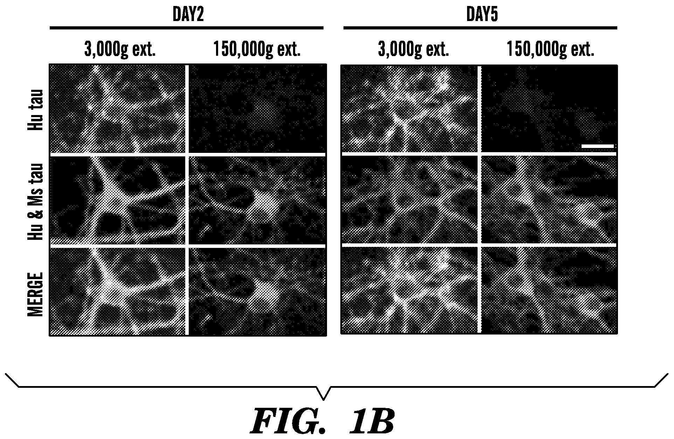

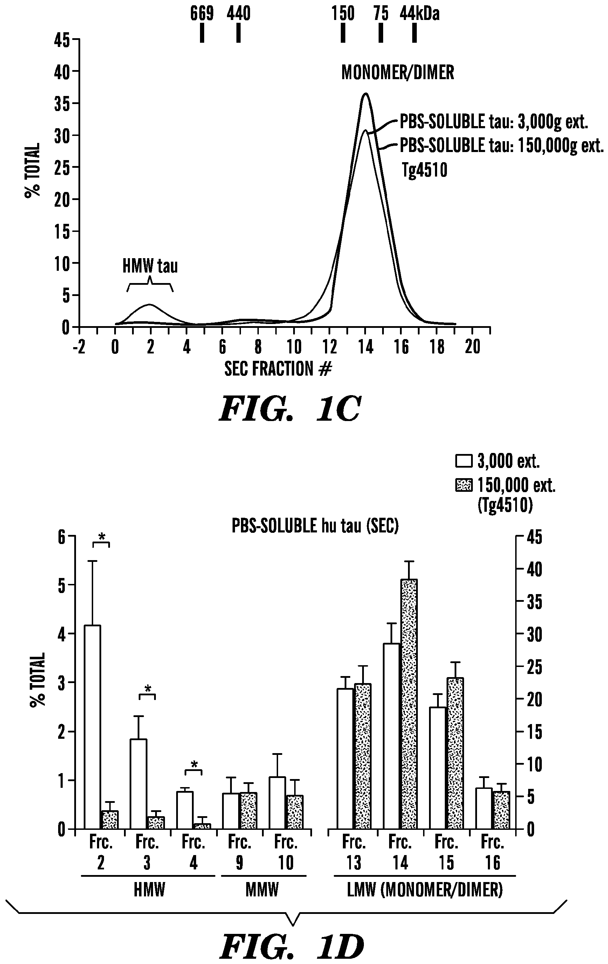

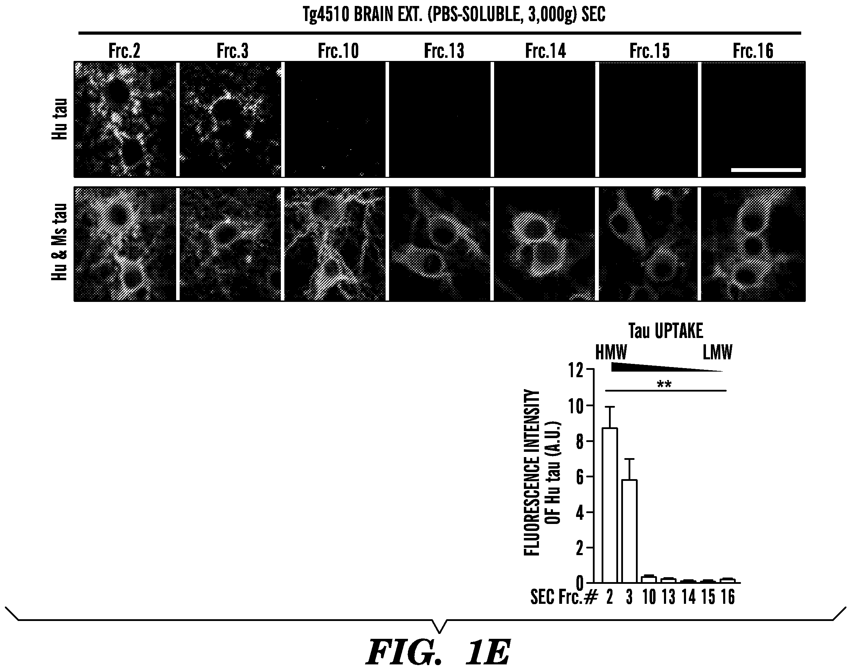

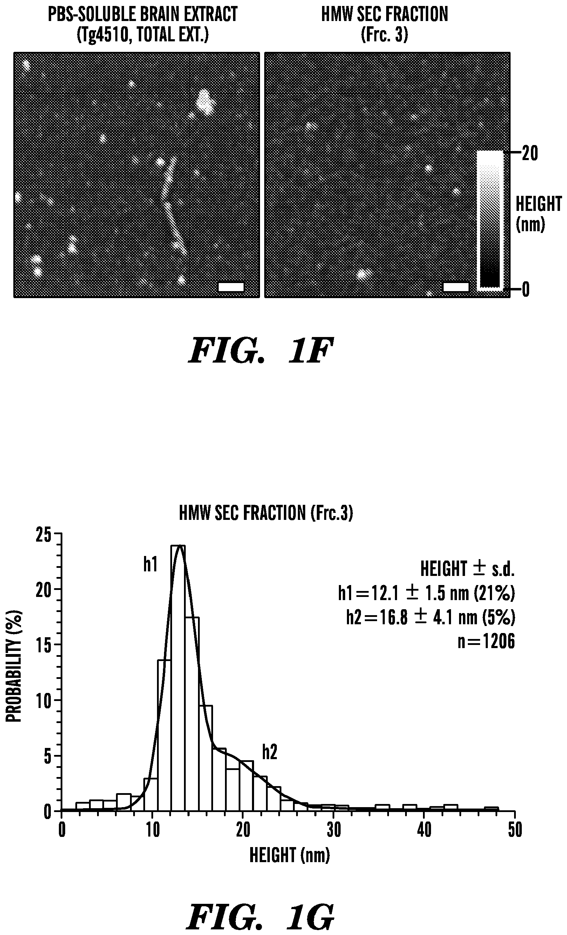

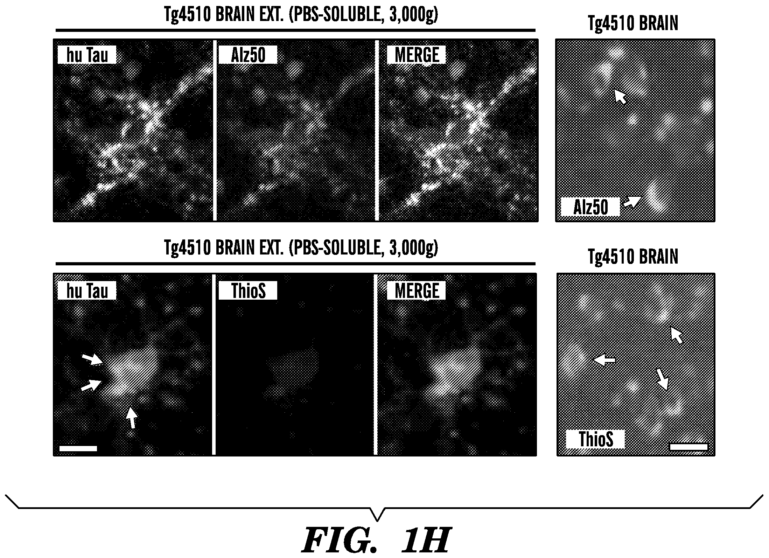

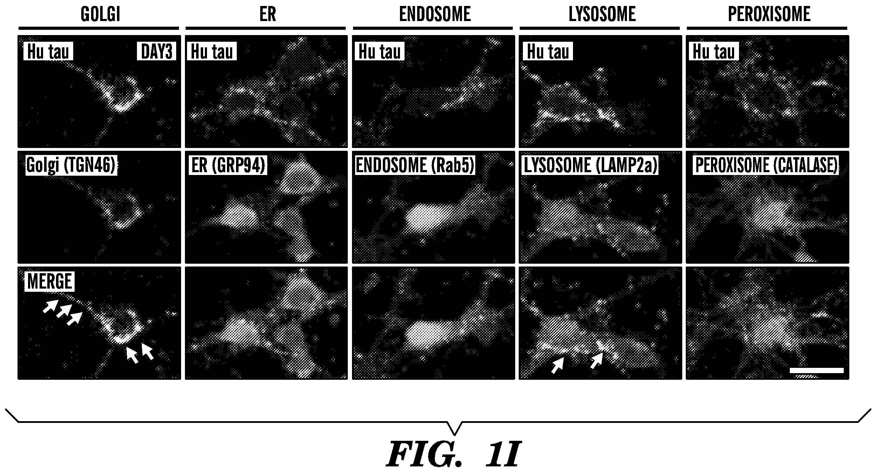

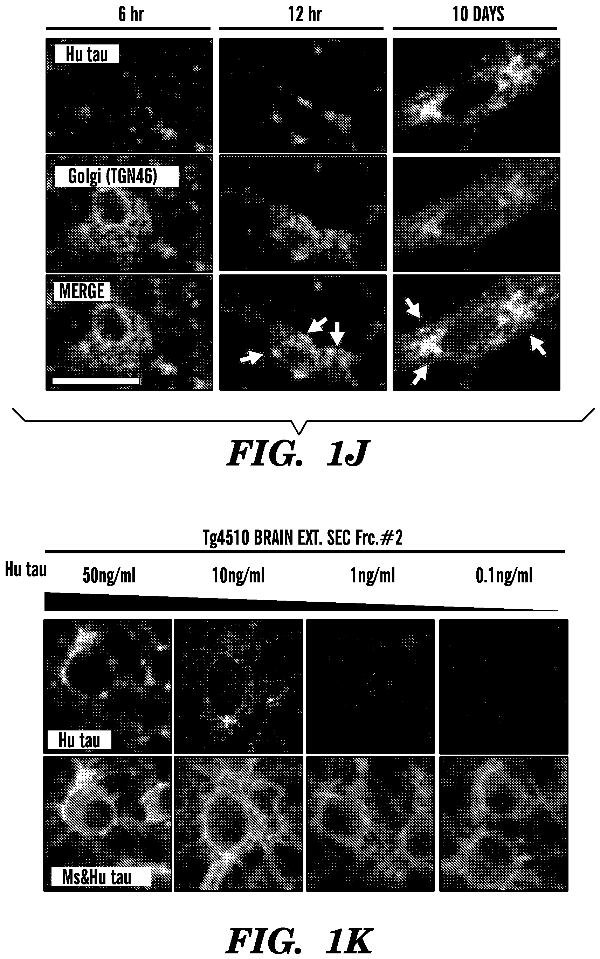

**Figs. 1A-1K** are fluorescent images and data graphs showing neuronal uptake of HMW oligomer tau from brain extract of rTg4510 tau-transgenic mouse. **(****Fig. 1A****)** Mouse primary neurons were incubated with PBS-soluble brain extracts (3,000g, 10,000g, 50,000g, or 150,000g centrifugation supernatant) from a 12-month-old rTg4510 mouse for 24 hours. Each brain extract was diluted with culture medium to a final concentration of 500 ng/ml human tau. (**Fig. 1A****,** left) Neurons were immunostained with human tau specific antibody ("Hu tau,") and total (human and mouse) tau antibody ("Hu & Ms tau," as a neuronal marker). (**Fig. 1A****,** right) Quantification of human tau uptake (fluorescence intensity). n = 9-12 / group; **P < 0.01. (**Fig. 1B**) Primary neurons were incubated with 3,000g or 150,000g brain extracts (12-month-old rTg4510, PBS-soluble, 500 ng/ml human tau) for 2 and 5 days, and immunostained with human tau specific antibody ("Hu tau") and total ("Hu & Ms tau") tau antibody. (**Figs. 1C** and **1D**) Size exclusion chromatography (SEC) of PBS-soluble brain extracts from rTg4510 mouse. Human tau levels in each SEC-separated sample were measured by ELISA. **(****Fig. 1C****)** Representative graph of human tau levels in SEC-separated samples from 3,000gand 150,000g brain extracts. **(****Fig. 1D****)** Mean human tau levels of HMW (Frc.2-4), MMW (Frc.9-10), and LMW (Frc.13-16) SEC fractions. (n = 3 / group); *P < 0.05. HMW, high molecular weight; MMW, middle molecular weight; LMW, low molecular weight. (**Fig. 1E****,** left) Primary neurons were incubated with SEC fractions from PBS-soluble rTg4510 brain extract (3,000g) for 2 days and immunostained for human tau ("Hu tau") and total ("Hu & Ms tau") tau. Each SEC fraction was diluted to a final concentration of 100 ng/ml human tau. (**Fig. 1E****,** right) Quantification of human tau uptake (fluorescence intensity), n = 3-5 / group; **P < 0.01. (**Figs. 1F** and **1G****)** Atomic force microscopy (AFM) analysis of HMW tau isolated from rTg4510 mouse brain. (**Fig. 1F**) Tau was isolated by immunoprecipitation (IP) from PBS-soluble brain extract (10,000g total extract (left) or SEC Frc. 3 (right)) of rTg4510 (12 months old) by using human tau-specific antibody Tau13. Full color range corresponds to a vertical scale of 20 nm. Scale bar: 100 nm. **(****Fig. 1G****)** Size (AFM heights) distribution histogram of HMW tau oligomers (SEC Frc. 3). n = 1206 particles from seven randomly-picked images (1.5 µm × 1.5 µm). **(****Fig. 1H****)** Human tau taken up from rTg4510 brain extract by primary neuron was co-stained with Alz50 antibody (early indicator of pathological tau misfolding) or ThioS (highly fibrilized tau). Brain sections from rTg4510 mouse (12 months old) were used as positive controls for each staining (right panels). **(****Fig. 1I****)** Subcellular localization of human tau taken up by primary neurons (PBS-soluble brain extract, 3,000g, 500 ng/ml human tau, day 3). Human tau ("Hu tau") taken up by primary neurons was co-immunostained with subcellular organelle markers for the Golgi apparatus (TGN46), endoplasmic reticulum (ER)(GRP94), endosomes (Rab5), lysosomes (LAMP2), and peroxisomes (catalase). Human tau taken up into neurons was colocalized with Golgi and lysosome markers at day 3 (white arrows). **(****Fig. 1J****)** Time course of human tau uptake into primary neurons. Human tau ("Hu tau") was co-immunostained with a Golgi marker (TGN46) at 6 hr, 12 hr, and 10 days after starting the incubation with rTg4510 brain extract. **(****Fig. 1K****)** Concentration dependency of human tau uptake. Mouse primary neurons were incubated for 2 days with four different concentrations of human tau (0.1, 1.0, 10, and 50 ng/ml in culture medium) from SEC Frc.2 of rTg4510 brain extract (PBS-soluble, 3,000g). Scale bar: 25 µm, except for **(****Fig. 1F****)**.

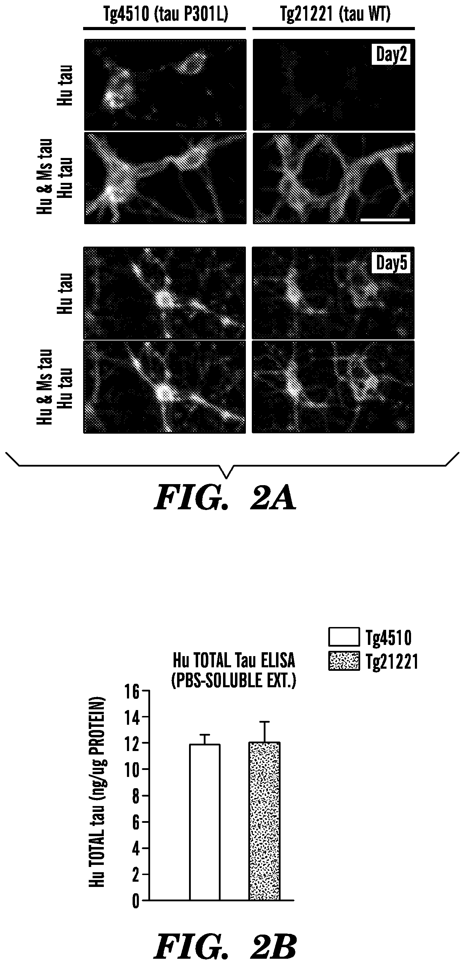

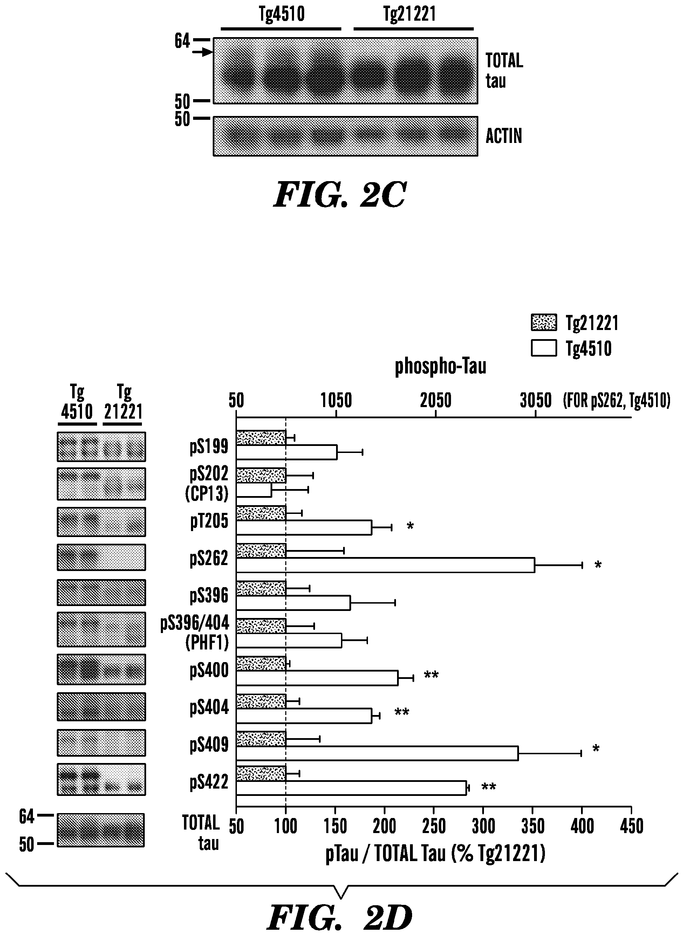

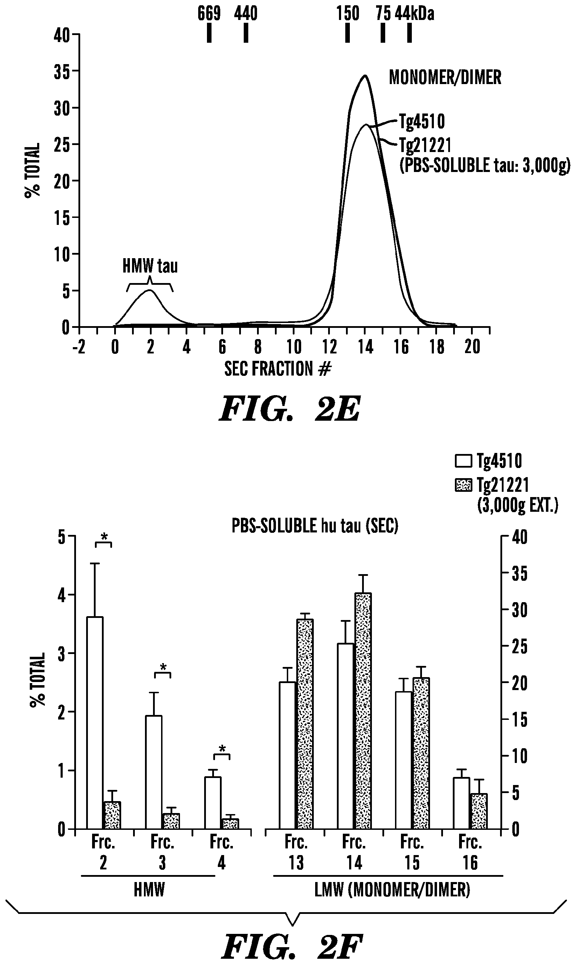

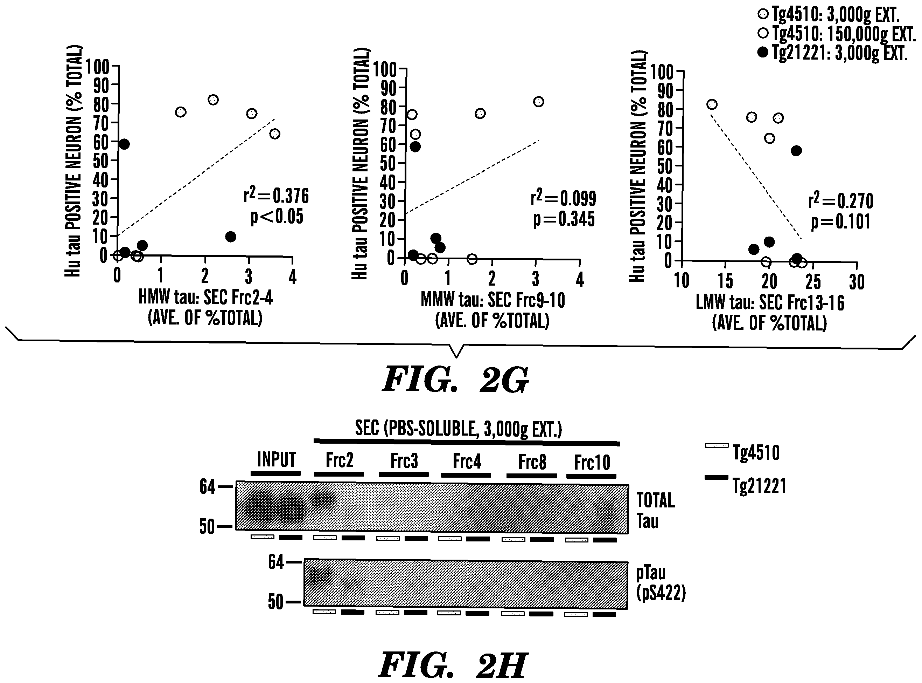

**Figs. 2A-2H** are fluorescent images, data graphs, and immunoblot pictures showing that lack of PBS-soluble phosphorylated HMW tau species is associated with low tau uptake in primary neurons. **(****Fig. 2A****)** Uptake of human tau from brain extracts from rTg4510 (overexpressing P301L mutant tau) and rTg21221 (overexpressing wild-type human tau) mice by mouse primary neurons (PBS-soluble, 3,000g ext., final concentration of 500 ng/ml human tau in culture medium). Neurons were immunostained with human tau specific antibody ("Hu tau") and total (human and mouse, "Hu & Ms tau") tau antibody at day 2 and 5. Scale bar: 50 µm. **(****Fig. 2B****)** Human tau levels in PBS-soluble brain extracts (3,000g) from rTg4510 and rTg21221 mice measured by human tau specific ELISA. **(****Fig. 2C****)** Immunoblot analysis of PBS-soluble brain extracts (3,000g) with total tau antibody (DA9). Up-shifted bands in rTg4510 brain suggest phosphorylation of tau (arrow). **(****Fig. 2D****)** Phospho-tau levels in rTg4510 and rTg21221 mouse brain extracts (PBS-soluble, 3,000g). Brain extracts were immunoblotted with ten different phospho-tau specific antibodies recognizing different epitopes. Representative immunoblot (left panels) and quantification of phospho-tau levels (right) at each epitope. (n = 3-4); *P < 0.05, **P < 0.01. (e, f) SEC analysis of PBS-soluble tau species from rTg4510 and rTg21221 mouse brain extracts. Human tau levels in each SEC-separated sample were measured by human tau specific ELISA. **(****Fig. 2E****)** Representative graph of human tau levels in SEC-separated samples from rTg4510 and rTg21221 brain extracts. **(****Fig. 2F****)** Mean human tau levels of HMW (Frc.2-4) and LMW (Frc.13-16) SEC fractions. (n = 3-6); *P < 0.05. **(****Fig. 2G****)** Correlations of human tau uptake by primary neurons with human tau levels in each SEC-separated fraction. (n = 11) Spearman rank test. **(****Fig. 2H****)** Immunoblot analysis of SEC-separated fractions from PBS-soluble brain extracts. Each SEC fraction was immunoblotted with total tau antibody (DA9) and phospho-tau (pS422) specific antibody. 11-13 months old animals were used.

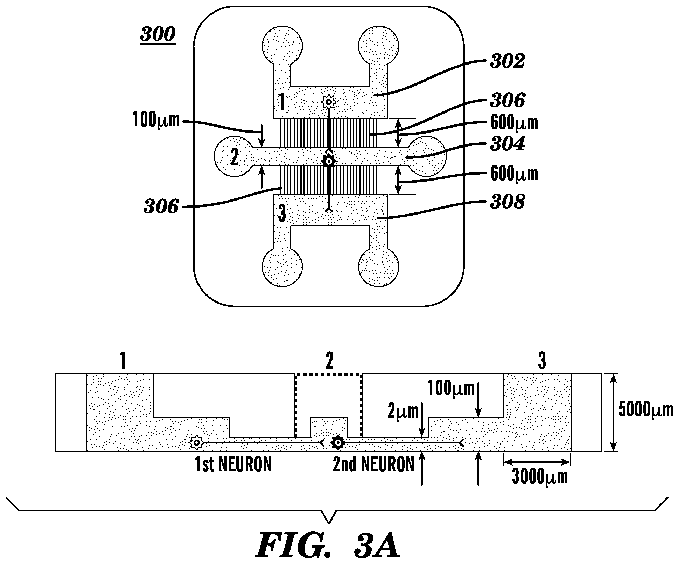

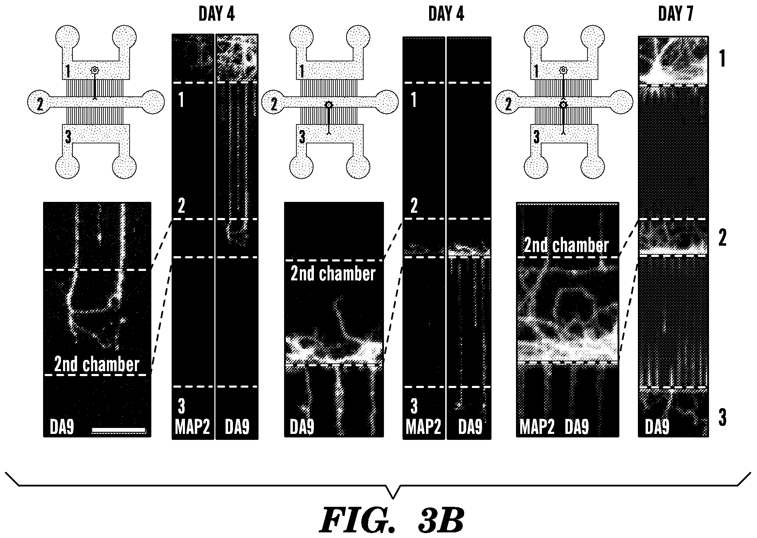

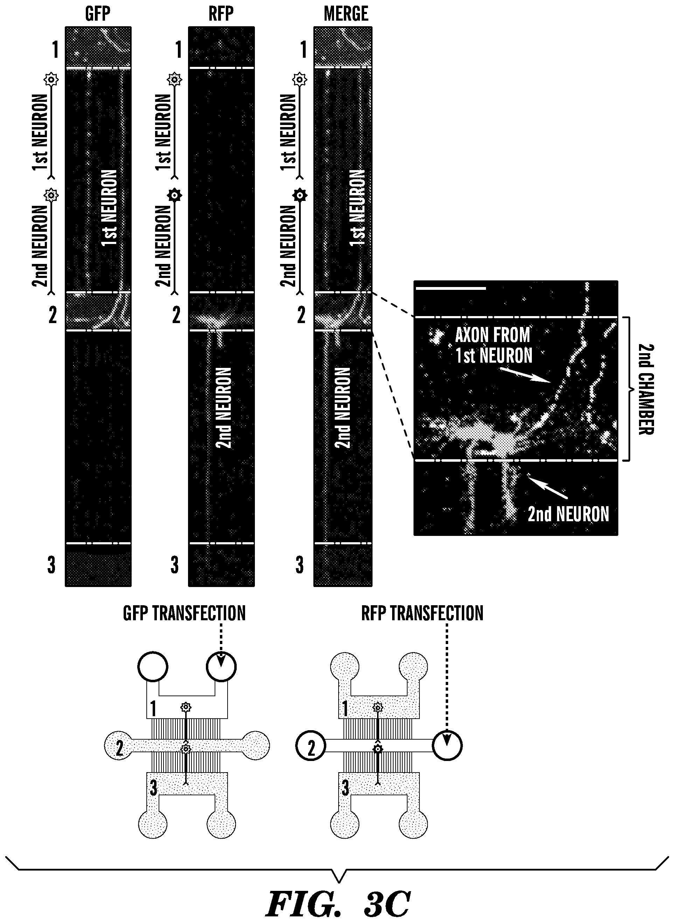

**Figs. 3A-3C** are schematic diagrams and fluorescent images showing three-chambered microfluidic device for modeling dual-layered neurons. **(****Fig. 3A****)** Schematics of a microfluidic device for culturing neurons in three distinct chambers. Mouse primary neurons are plated into the 1st and 2nd chambers (100 µm in thickness) and axon growth is guided through microgrooves (3 µm in thickness, 600 µm in length) connecting each chamber. (**Fig. 3B****,** left) Axons from the 1st chamber neuron (DA9 as axonal marker) extend into the 2nd chamber within 4 days (neurons were plated only in the 2nd chamber). No MAP2 positive dendrites were found in the 2nd chamber, indicating that a 600 µm microgroove is sufficiently long to isolate axon terminals from soma and dendrites. (**Fig. 3B****,** middle) Most axons from the 2nd chamber neuron extend into the 3rd chamber (neurons were plated only in the 2nd chamber). (**Fig. 3B****,** right) Two sets of neurons were plated into the 1st and 2nd chamber and established synaptic contact in the 2nd chamber. **(****Fig. 3C****)** Neurons in the 1st and 2nd chambers were transfected with green fluorescent protein (GFP) and red fluorescent protein (RFP), respectively. GFP positive axon from the 1st chamber neuron extended into the 2nd chamber, connecting to RFP positive 2nd chamber neuron, which projected its axon into the 3rd chamber. Scale bar: 50 µm.

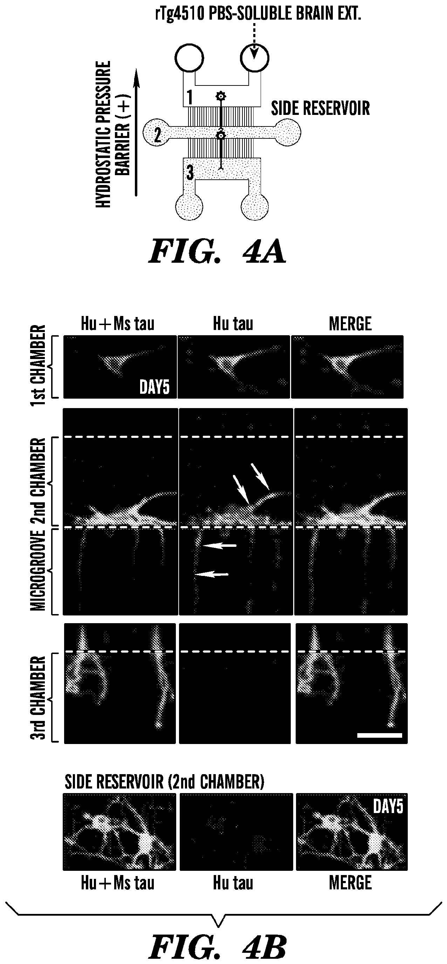

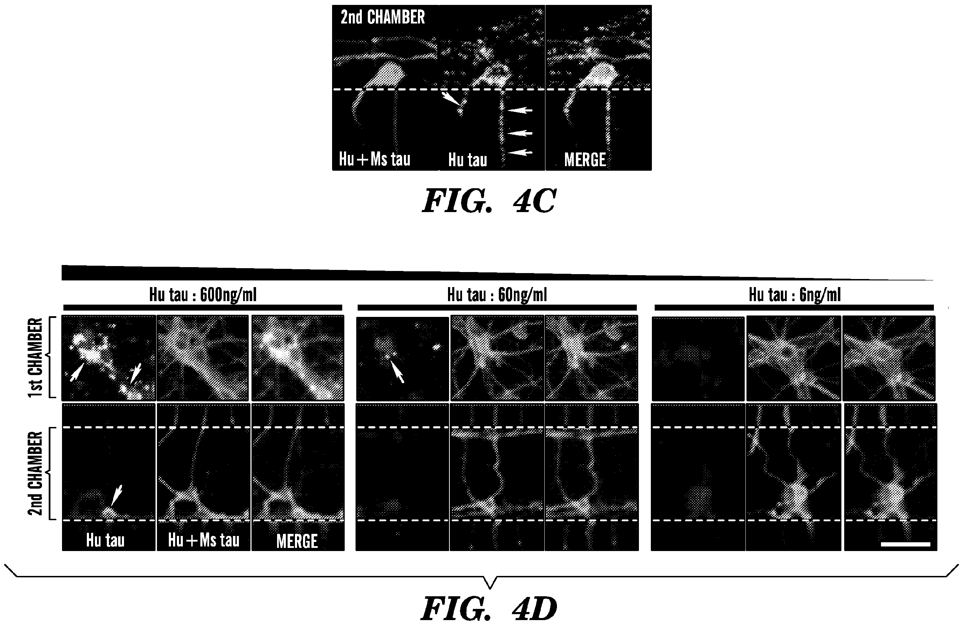

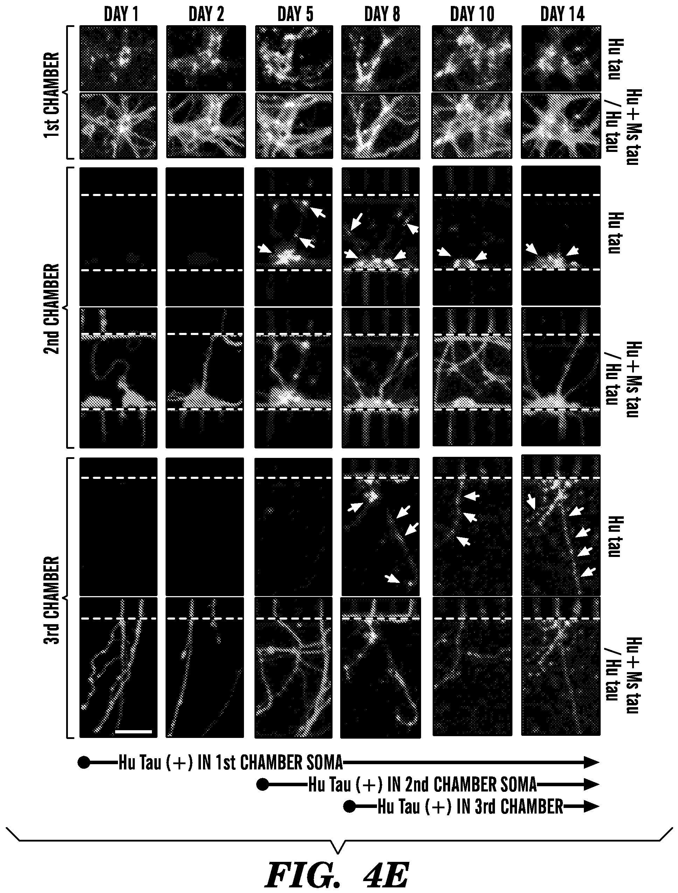

**Figs. 4A-4E** contain fluorescent images showing neuron-to-neuron transfer of rTg4510 mouse brain-derived human tau species in a three-chambered microfluidic device. **(****Fig. 4A****)** PBS-soluble extract from rTg4510 mouse brain (500 ng/ml human tau) was added to the 1st chamber of a 3-chamber microfluidic neuron device. Diffusion of brain extract from the 1st to the 2nd chamber was blocked by a hydrostatic pressure barrier during incubation. **(****Fig. 4B****)** Immunostaining for human tau ("Hu tau") and total (human and mouse; "Hu & Ms tau") tau at day 5. Human tau positive neurons were detected in the 2nd chamber (white arrow). Neurons in the side reservoir of the 2nd chamber were negative for human tau staining (bottom). **(****Fig. 4C****)** A human tau positive axon (arrow) and dendrite (arrow head) extending from the 2nd chamber neuron. **(****Fig. 4D****)** Concentration dependency of tau uptake and propagation. rTg4510 brain extract (PBS-soluble, 3,000g) was diluted in culture medium to obtain three different concentrations (6, 60, and 600 ng/ml) of human tau and added into the 1st chamber. Neurons were immunostained for human tau ("Hu tau") and total (human and mouse; "Hu & Ms tau") tau (red) at day 5. **(****Fig. 4E****)** Time course of neuron-to-neuron transfer of rTg4510 brain derived human tau. The rTg4510 brain extract (PBS-soluble, 3,000g, 500 ng/ml human tau) was added to the 1st chamber and incubated for up to 14 days. Neurons were immunostained at different time points. Human tau positive 2nd chamber neurons (arrow head) and axons from the 1st chamber neuron (arrow head) were detected after 5 days of incubation. Human tau positive axons were detected in the 3rd chamber after 8 days of incubation (arrow). Scale bar: 50 µm.

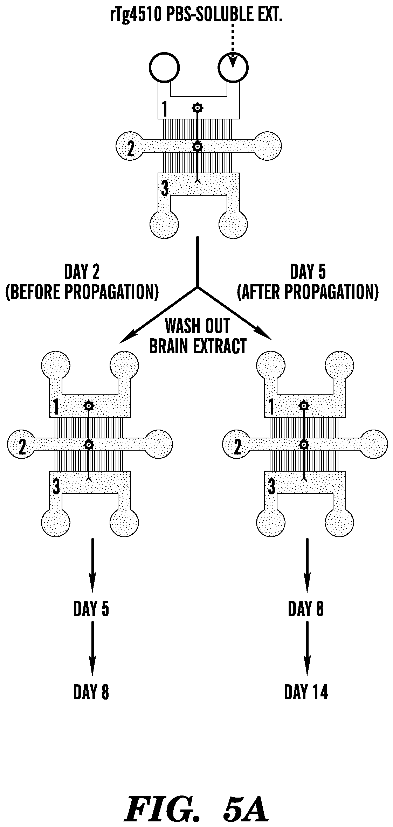

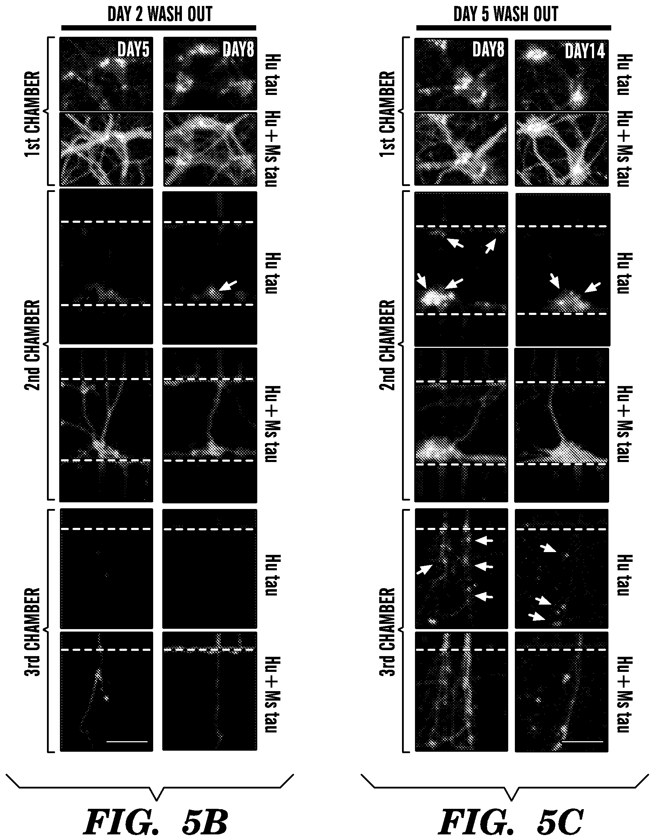

**Figs. 5A-5C** contain fluorescent images showing that rTg4510 brain derived human tau was stable and propagated even after removal of brain extract from the chamber. **(****Fig. 5A****)** rTg4510 brain extract (PBS-soluble, 3,000g) was diluted in culture medium (500 ng/ml human tau in final concentration) and added to the 1st chamber of 3-chamber microfluidic neuron device. After 2 days (before tau propagation occurs) or 5 days (after tau propagation occurred, but not yet progressed to the 3rd chamber) of incubation, brain extract was washed out from the 1st chamber and replaced with fresh culture medium. **(****Figs. 5B** and **5C****)** Neurons were immunostained for human tau ("Hu tau") and total (human and mouse, "Hu & Ms tau") tau at designated time points. **(****Fig. 5B****)** Human tau positive neuron was detected in the 2nd chamber (day 8, arrow) even after Tg brain extract was washed out from the 1st chamber at day 2. **(****Fig. 5C****)** Human tau was detected in the 3rd chamber axons (arrow) even after Tg brain extract was washed out from the 1st chamber at day 5. Human tau taken up by the 1st chamber neuron was still detectable at day 14 (9 days after removal of Tg brain extract). Scale bar: 50 µm.

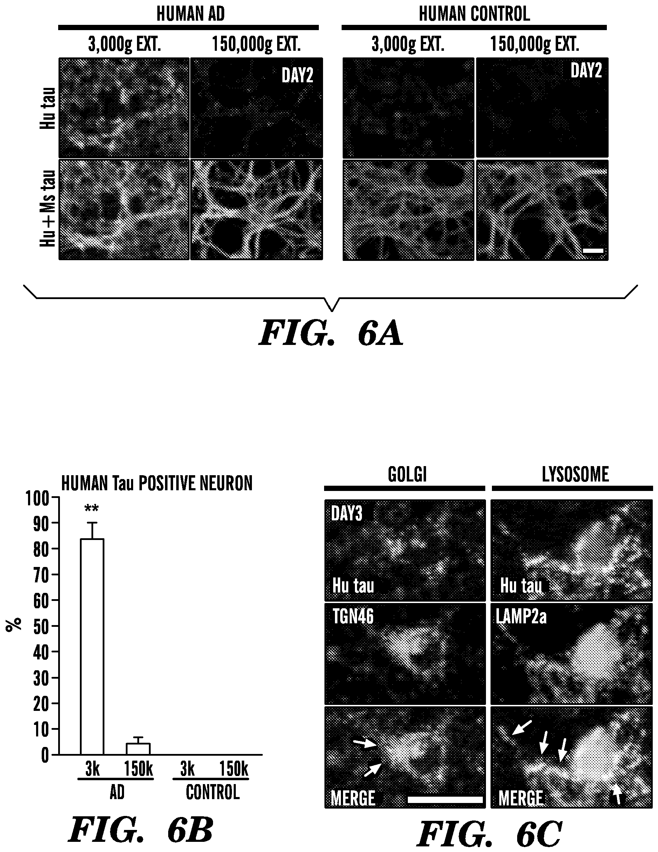

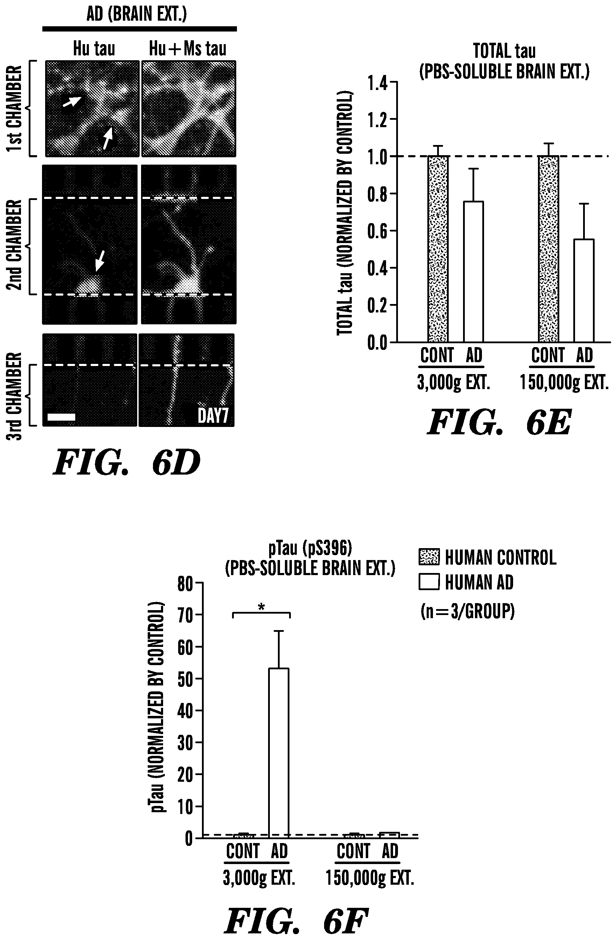

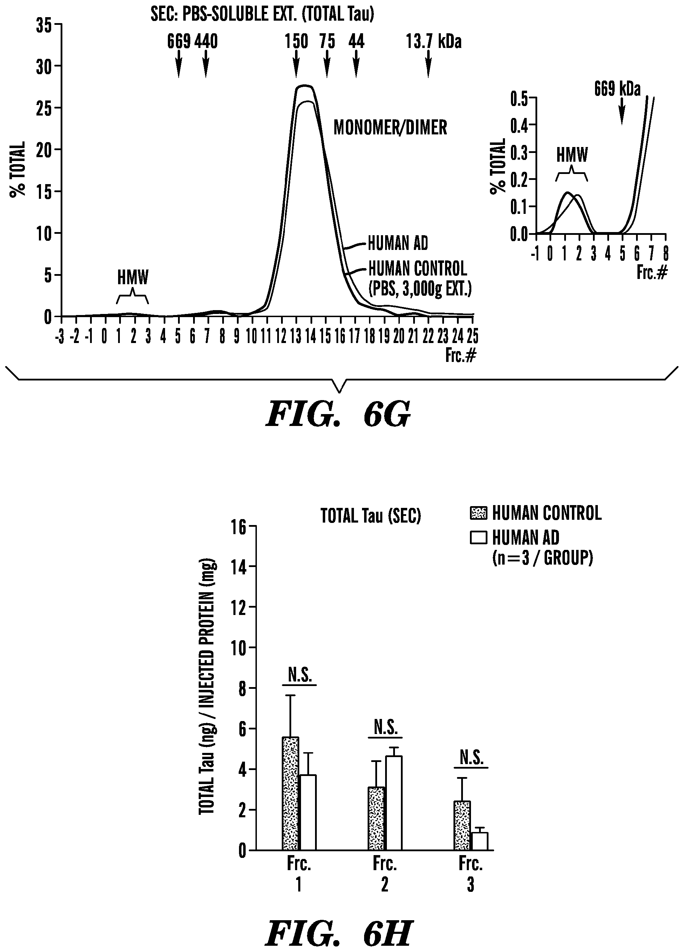

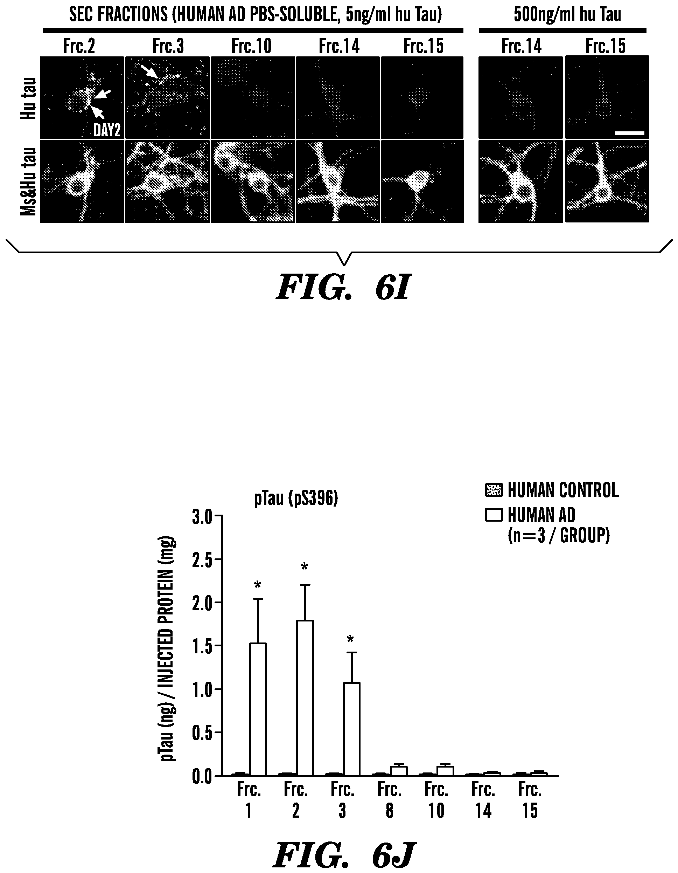

**Figs. 6A-6J** are fluorescent images and data graphs showing neuronal uptake of PBS-soluble HMW oligomer tau derived from human AD brain. **(****Figs. 6A** and **6B****)** Mouse primary neurons were incubated with PBS-soluble extracts (3,000g or 150,00g centrifugation supernatant) from human AD or control brain tissue. Each brain extract was diluted with culture medium to a final concentration of 500 ng/ml human tau. **(****Fig. 6A****)** Neurons were immunostained for human tau ("Hu tau") and total (human and mouse, "Hu & Ms tau") tau at day 2. **(****Fig. 6B****)** Quantification of the number of human tau positive neurons at day 2 (n = 3 / group); **P < 0.01. **(****Fig. 6C****)** Subcellular localization of human tau taken up by mouse primary neurons (PBS-soluble brain extract, 3,000g, 500 ng/ml human tau). Human tau ("Hu tau") taken up into primary neurons was co-immunostained with Golgi (TGN46) and lysosomes (LAMP2) markers . **(****Fig. 6D**) Neuron-to-neuron transfer of human AD brain derived tau in a 3-chamber microfluidic neuron culture device. Human AD brain extract (PBS-soluble, 3,000g, 500 ng/ml human tau) was added to the 1st chamber of a 3-chamber microfluidic neuron device. Human tau ("Hu tau") positive neurons were detected in both the 1st and 2nd chamber at day 7 (arrow). **(****Figs. 6E** and **6F**) Quantification of total tau **(****Fig. 6E****)** and phospho-tau (pS396, **Fig. 6F****)** levels in each extract from human AD and control brain (ELISA). n = 3 / group; *P < 0.05. **(****Figs. 6G and 6H****)** SEC analysis of PBS-soluble tau species from human AD and control brain. Total tau levels in each SEC-separated sample were measured by ELISA. **(****Fig. 6G****)** Representative graph of total tau levels in SEC-separated samples from AD and control brain extracts. Small peaks for HMW fractions were detected in both groups (right panel). **(****Fig. 6H****)** Mean total tau levels of HMW SEC fractions (Frc.1-3). n = 3 / group. **(****Fig. 6I****)** Tau uptake from each SEC fraction by mouse primary neurons. Each SEC fraction was diluted to a final concentration of 5 or 500 ng/ml human tau and incubated with mouse primary neurons. Neurons were immunostained for human tau ("Hu tau") and total (human and mouse, "Hu & Ms tau") tau at day 2. **(****Fig. 6J****)** Quantification of phospho-tau (pS396) levels in each SEC fraction from human AD and control brain (ELISA). n = 3 / group; *P < 0.05. Scale bar: 25 µm. HMW, high molecular weight.

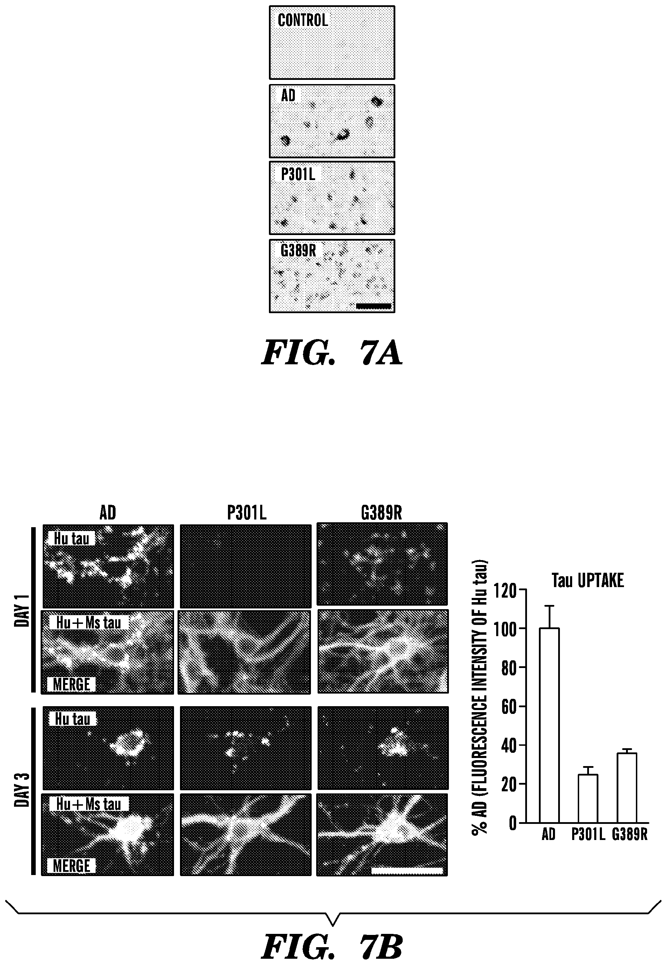

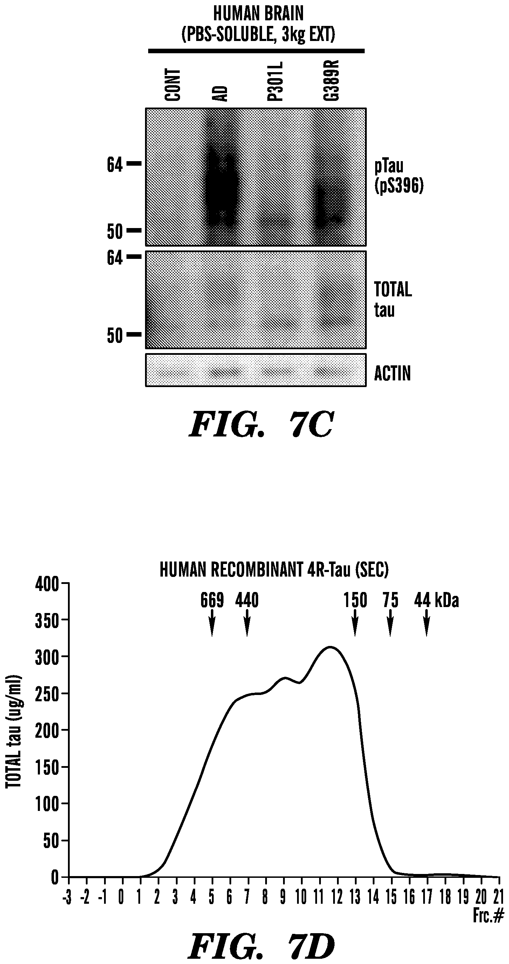

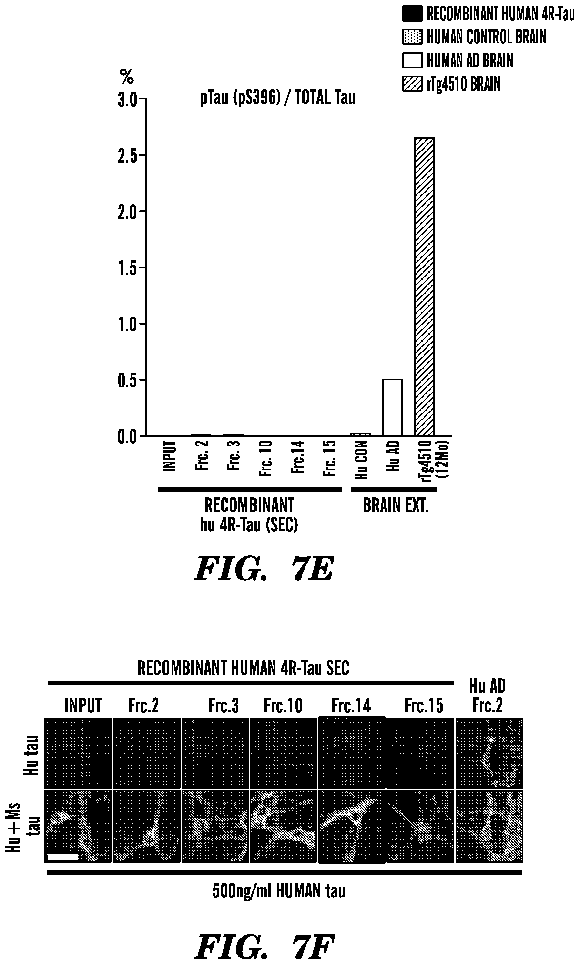

**Figs. 7A-7F** are experimental data showing neuronal uptake of phosphorylated tau species from human familial frontotemporal dementia brains, but not from non-phosphorylated synthetic human tau. **(****Fig. 7A****)** Tau immunostained brain sections from control, AD, and two different familial frontotemporal dementia (FTD) cases (P301L and G389R tau mutation). AD and P301L brains showed frequent NFTs, although G389R brain had only tau-positive neuropil threads without NFTs. **(****Fig. 7B****)** Mouse primary neurons were incubated with PBS-soluble extracts (3,000g, 500 ng/ml human tau) of frontal cortex from AD and two FTD cases. **(****Fig. 7B****,** left) Neurons were immunostained for human tau ("Hu tau") and total (human and mouse, "Hu & Ms tau") tau at day 1 and 3. **(****Fig. 7B****,** right) Fluorescence intensity of human tau staining (day 1). Results were obtained from 6-7 independent culture wells for each case. **(****Fig. 7C****)** Immunoblot analysis of phospho-tau (pS396) and total tau levels (DA9) in PBS-soluble brain extracts. **(****Figs. 7D-7F****)** Non-phosphorylated HMW tau species were not taken up by primary neurons. **(****Fig. 7D****)** Tau oligomer mixture solution was prepared by incubating recombinant human tau (hTau-441, 3.35 mg/ml) with 2mM DTT for 2 days at 37°C, followed by SEC separation and ELISA measurement of tau levels in each fraction. **(****Fig. 7E****)** ELISA measurement of phospho-tau (pS396) levels (normalized by total tau levels) in SEC fractions of recombinant tau oligomer mixture and brain extracts (human control and AD, and rTg4510 mouse; PBS-soluble, 3,000g). **(****Fig. 7F****)** Each SEC fraction was diluted to a final concentration of 500 ng/ml tau with culture medium and incubated with mouse primary neurons. Neurons were immunostained for human tau ("Hu tau") and total (human and mouse, "Hu & Ms tau") tau at day 2. SEC Frac.2 from human AD brain extract (PBS-soluble, 3,000g) was also incubated at the same concentration of tau (500 ng/ml) to serve as a positive control. Scale bar: 50 µm. HMW, high molecular weight.

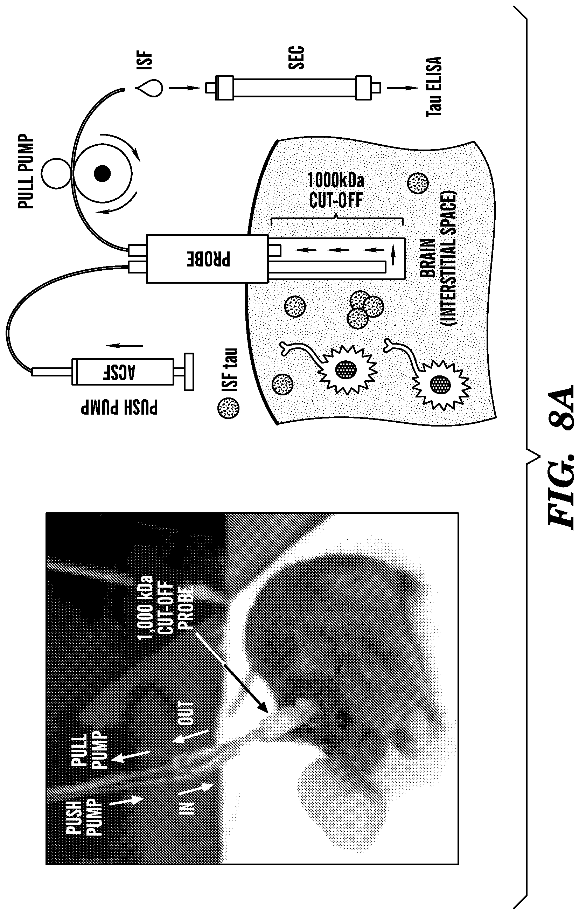

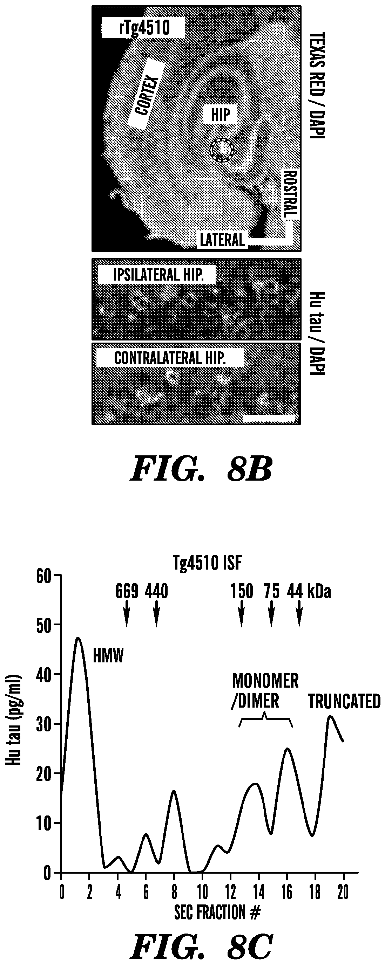

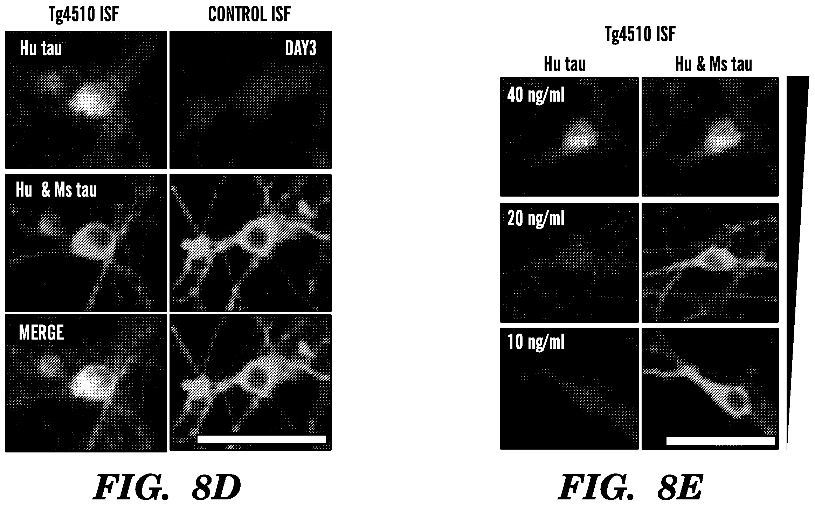

**Figs. 8A-8E** are experimental data showing that extracellular tau species from rTg4510 mouse brain can be taken up by mouse primary neurons. **(****Fig. 8A****)** A large-pore probe in vivo microdialysis technique with push-pull perfusion system. ISF samples were collected from awake, freely-moving rTg4510 and control mice (seven months old) using a 1,000 kDa cut-off microdialysis probe (flow rate = 0.5 µl/ml, from hippocampus). **(****Fig. 8B****)** Representative probe placement in hippocampus. Horizontal brain sections were obtained from rTg4510 mouse after ISF collection (24 hours after probe insertion) and stained for human tau (Tau13, "Hu tau") and DAPI. Dotted line depicts microdialysis probe location **(****Fig. 8B****,** top). The probe was briefly perfused with Texas red dye (70 kDa, 1 mg/ml) to locate the site of microdialysis. There was no morphological evidence of substantial neuronal loss within hippocampal tissue surrounding the microdialysis probe tract. There was no apparent difference in the number of human tau positive neurons between ipsilateral (probe-implanted side) and contralateral hippocampal sections (**Fig. 8B****,** bottom). Hip, hippocampus. **(****Fig. 8C****)** Representative graph of human tau levels in SEC-separated ISF sample from rTg4510 mouse. 400 µl of microdialysate was loaded on SEC column and tau levels in each fraction were measured by ELISA. **(****Fig. 8D****)** ISF collected from rTg4510 and control mice were incubated with mouse primary neurons, which were then immunostained for human tau ("Hu tau") and total (human and mouse, "Hu & Ms tau") tau at day 3. ISF from rTg4510 was diluted with culture medium to a final concentration of 40 ng/ml human tau and the same volume of ISF from a control mouse was used for incubation. **(****Fig. 8E****)** Concentration dependency of ISF tau uptake by primary neurons. rTg4510 brain ISF was diluted in culture medium to obtain three different concentrations (10, 20, and 40 ng/ml) of human tau. Neurons were immunostained at day 3. HMW, high molecular weight; MMW, middle molecular weight. Scale bar: 50 µm.

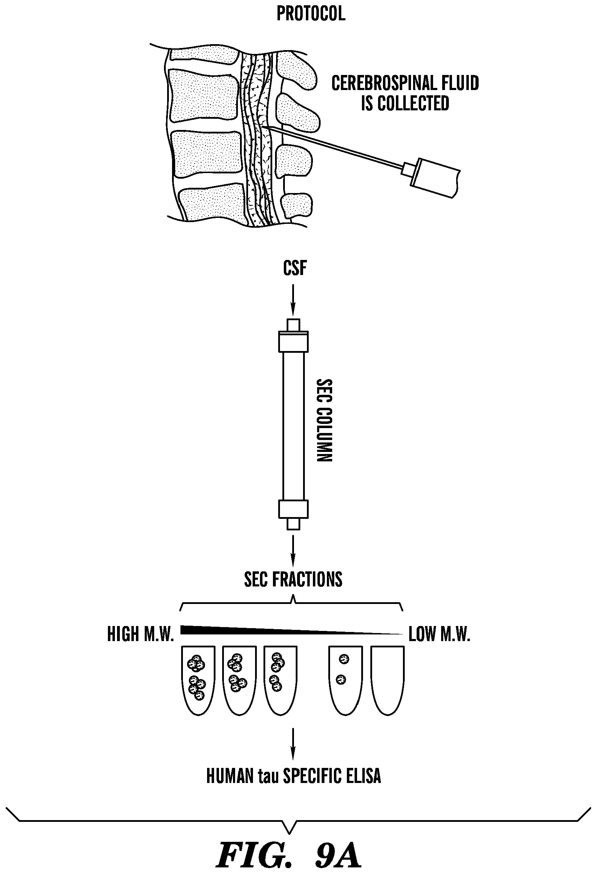

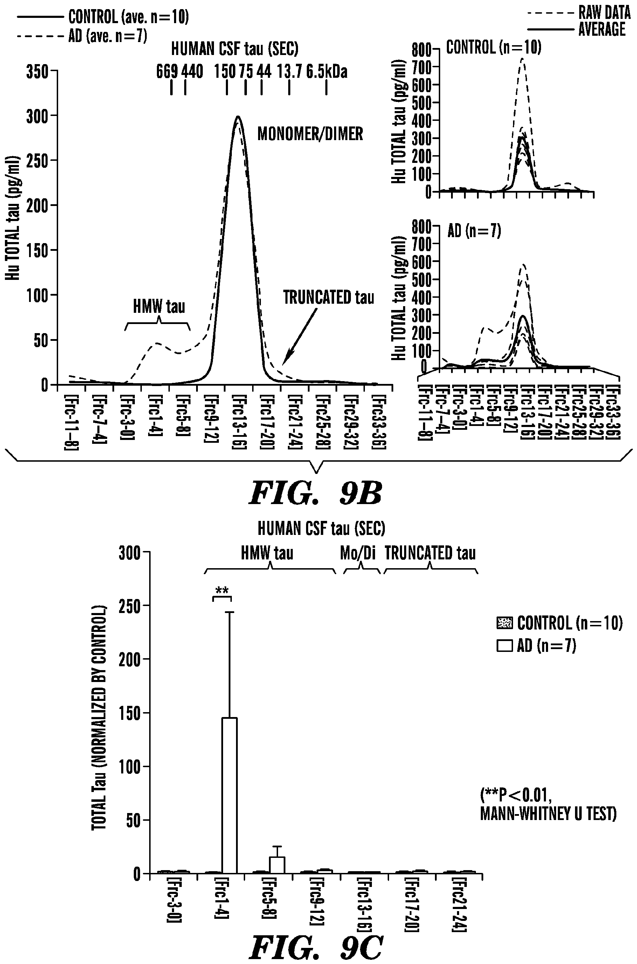

**Figs. 9A-9C** are experimental data showing that HMW oligomer tau is released into the extracellular space of the brain and accumulates in the CSF of Alzheimer's disease patients. The cerebrospinal fluid (CSF) of AD and control subjects was examined to isolate the specific tau species and its diagnostic potentials were evaluated. **(****Figs. 9A-9B**) To detect and characterize HMW tau species in human CSF, the molecular-weight size distribution of tau species contained in human CSF samples was assessed using SEC. CSF samples from 7 AD and 10 control cases were analyzed. **(****Fig. 9C****)** The total tau levels in each SEC fraction were measured using the human tau-specific ELISA. Notably, the HMW tau species was detected in the CSF of the AD patients. Concentrations of CSF HMW tau were significantly higher in the AD patients than the control subjects (p < 0.01, Mann-Whitney U-test), although monomer/dimer-sized tau levels were comparable between the groups.

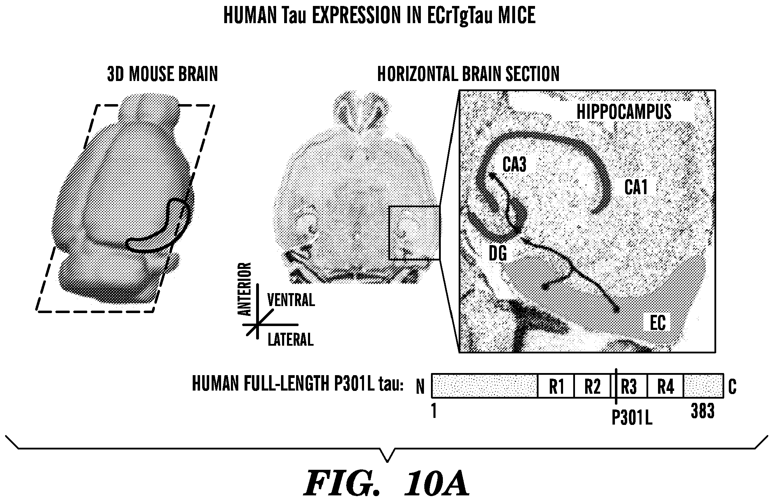

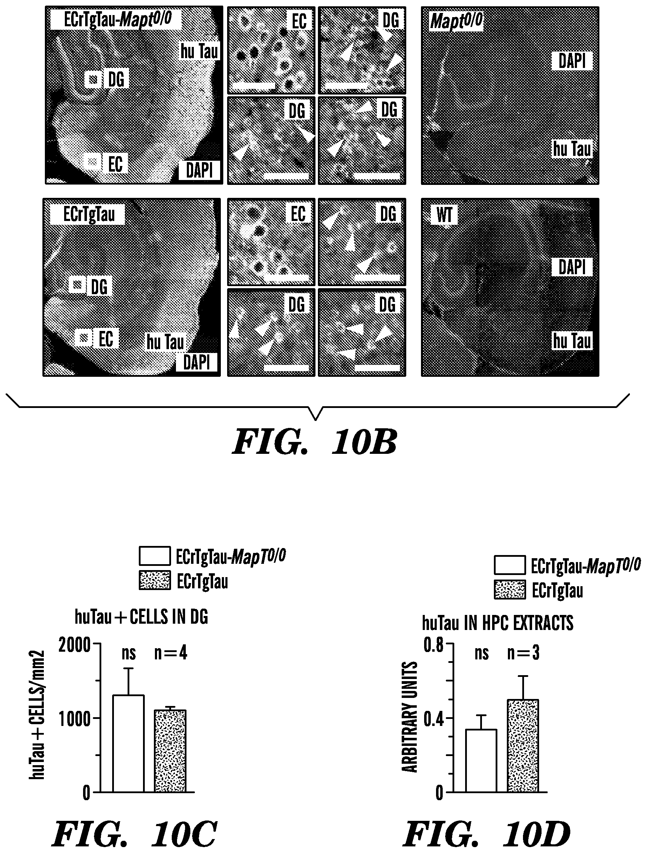

**Figs. 10A-10D** show trans-synaptic propagation of transgenic human tau in absence of endogenous mouse tau. **(****Fig. 10A****),** 3D-brain model and horizontal brain section of ECrTgTau mice illustrating the transgenic human mutant P301L tau expression in layer 2/3 of entorhinal cortex (EC) and the propagation of transgenic tau to dentate gyrus (DG) and hippocampal regions CA1 and CA3. **(****Fig. 10B**) Immunostained horizontal sections reveal robust propagation of human tau from expressing neurons in EC to neurons in the DG (white arrows) both in presence (ECrTgTau) and absence (ECrTgTau-Mapt0/0) of endogenous mouse tau. (**Figs. 10C-10D**) The number of human tau-positive (huTau+) cell bodies in the DG (C; n=4 mice per group, p=0.58) and the levels of human tau in hippocampal extracts (D; n= 3 mice per group, p=0.14) were similar in ECrTgTau-Mapt0/0 and ECrTgTau and mice. Two-tailed Student's T-test, data displayed as mean±SEM. ns, not significant, scale bars 50 µm. 3D-brain model was generated using Allen's 3D-mouse brain atlas (accessible online at http://www.brain-map.org/).

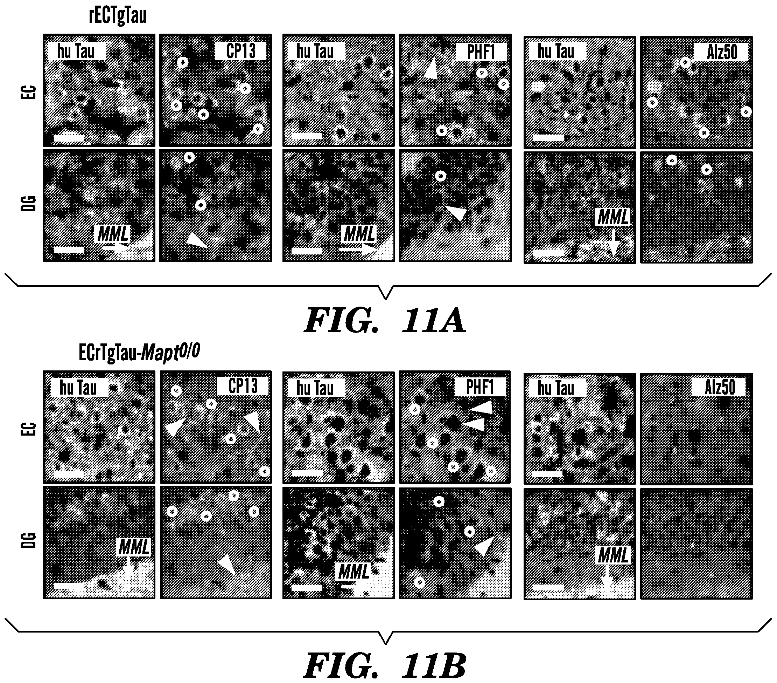

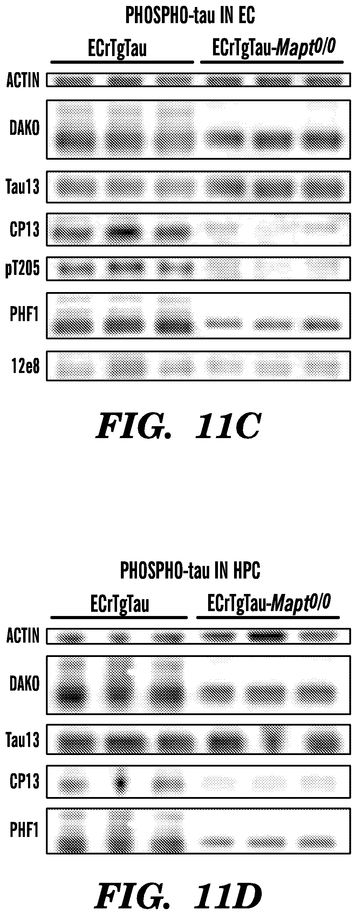

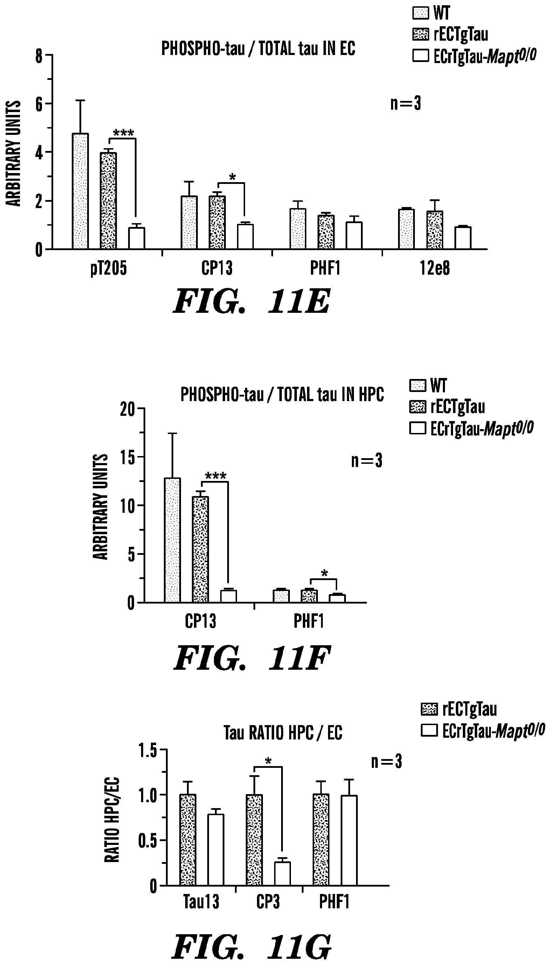

**Figs. 11A-11G** show dissociation of tau propagation, phosphorylation and misfolding. Co-immunostaining of human tau (TauY9) with phosphorylation site-specific antibodies CP13 (pS202/pT205) am PHF1 (pS394/pS404) show phospho-tau in the soma (white circles) of EC neurons and a subset of human tau receiving DG neurons and their projections (white arrow heads) both in ECrTgTau **(****Fig. 11A****)** and ECrTgTau-Mapt0/0 mice **(****Fig. 11B****).** Human tau in the middle molecular layer (MML, white arrows) stained with PHF1 but not CP13. "Misfolded" (Alz50) tau was present in somata of EC and DG neurons and in the MML in ECrTgTau **(****Fig. 11A****)** but not in ECrTgTau-MaptO/O **(****Fig. 11B****)** mice. (**Figs. 11C-11F**) Phospho-tau levels in PBS extracts from EC **(****Figs. 11C** and **11E**) revealed higher pT205 (p=0.0005), CP13 (pS202/pT205, p=0.026), PHF1 (pS396/pS404, p=0.37), and 12E8 (pS262/pS356, p=0.31, not significant) in ECrTgTau compared to ECrTgTau-MaptO/O mice. CP13 (p=0.0003) and PHF1 (p=0.03) were also significantly elevated in HPC extracts **(****Figs. 11D** and **11F****)** from ECrTgTau mice. **(****Fig. 11G****)** Propagation of tau estimated from hippocampal:entorhinal (HPC:EC) tau ratios was similar for human tau (Tau13, p=0.26) and PHF1 (p=0.98) but significant lower for CP13 (p=0.034) in ECrTgTau-MaptO/O compared to ECrTgTau mice. n=3 mice per group, two-tailed Student's T-test, data displayed as mean±SEM. ns, not significant, scale bars 50 µm.

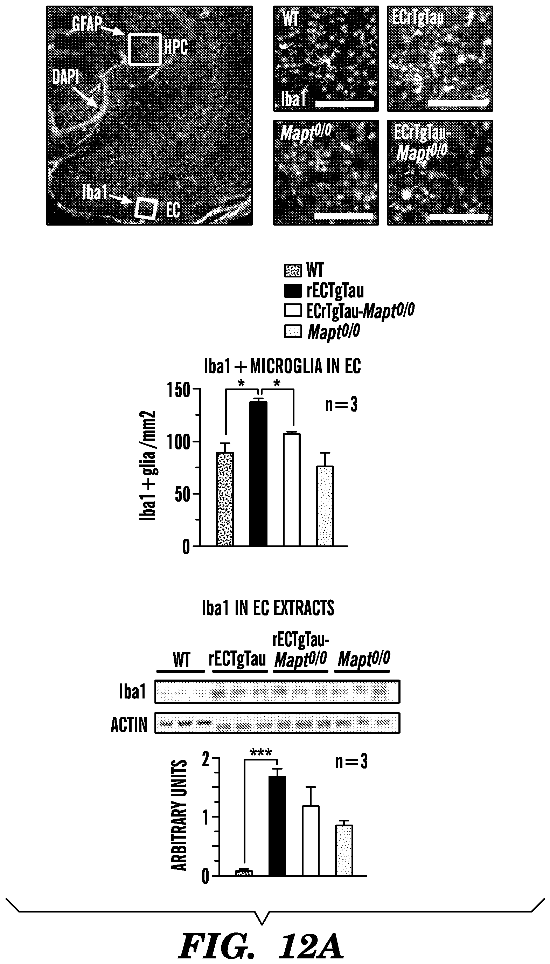

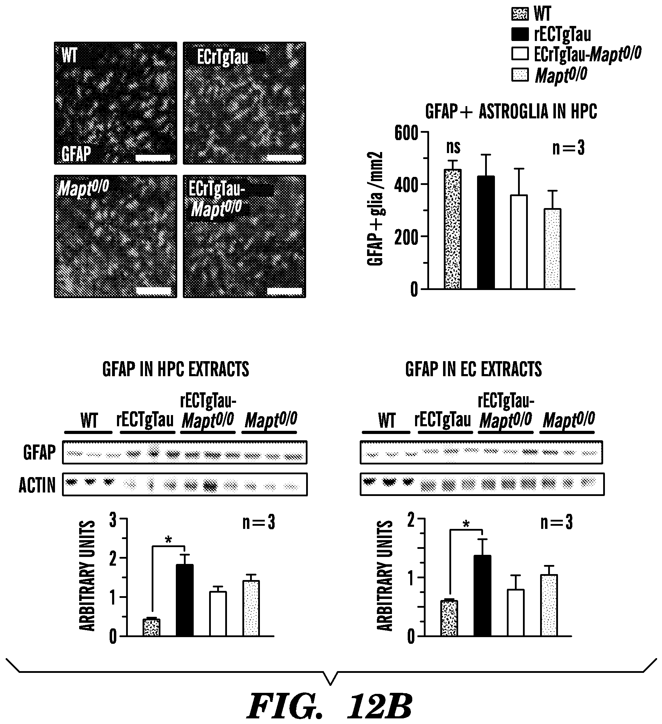

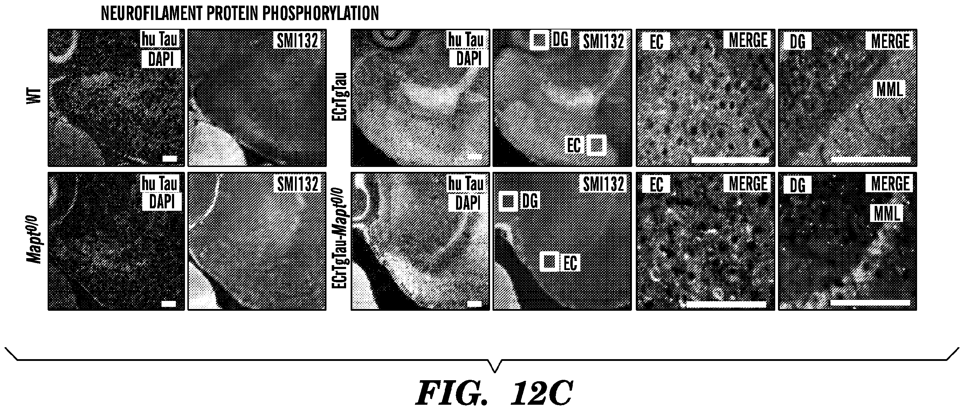

**Figs. 12A-12C** show reduced gliosis and neurofilament phosphorylation in ECrTgTau-MaptO/O mice. **(****Fig. 12A****)** Immunofluorescence labeling revealed a significantly increased number of microglia (Iba1+) in the EC (layer1 and 2/3) in ECrTgTau compared to WT (p=0.008), ECrTgTau-MaptO/O (p=0.0025), and Mapt0/0 mice (p=0.012). In EC extracts, the amount of Iba in ECrTgTau mice was ≈20-fold increased compared to WT (p=0.0006), ≈1.4-fold compared to ECrTgTau-MaptO/O (p=0.24), and ≈2-fold compared to Mapt0/0 mice (p=0.01). **(****Fig. 12B****)** The number of activated astrocytes (GFAP+) in the hippocampus was similar in WT, ECrTgTau, ECrTgTau-MaptO/O, and Mapt0/0 mice (not significant). The amounts of GFAP in extracts of HPC and EC, however, were higher in ECrTgTau compared to WT (HPC: p=0.004; EC: p=0.03), ECrTgTau-Mapt0/0 (HPC: p=0.047; EC: p=0.1), and TauKO mice (HPC: 0.13; EC: p=0.38). n=3 mice per group, two-tailed Student's T-test, data displayed as mean±SEM. **(****Fig. 12C****)** Immunostaining of phosphorylated neurofilament proteins using SMI132 shows intense local labeling of human tau (TauY9) containing EC neurons and their axonal projections throughout the HPC of ECrTgTau but not ECrTgTau-MaptO/O or control WT and Mapt0/0 animals. Higher magnification images of DG and EC areas reveal intense SMI132 labeling of many processes in ECrTgTau and only few in ECrTgTau-MaptO/O mice. ns, not significant, scale bars 100 µm.

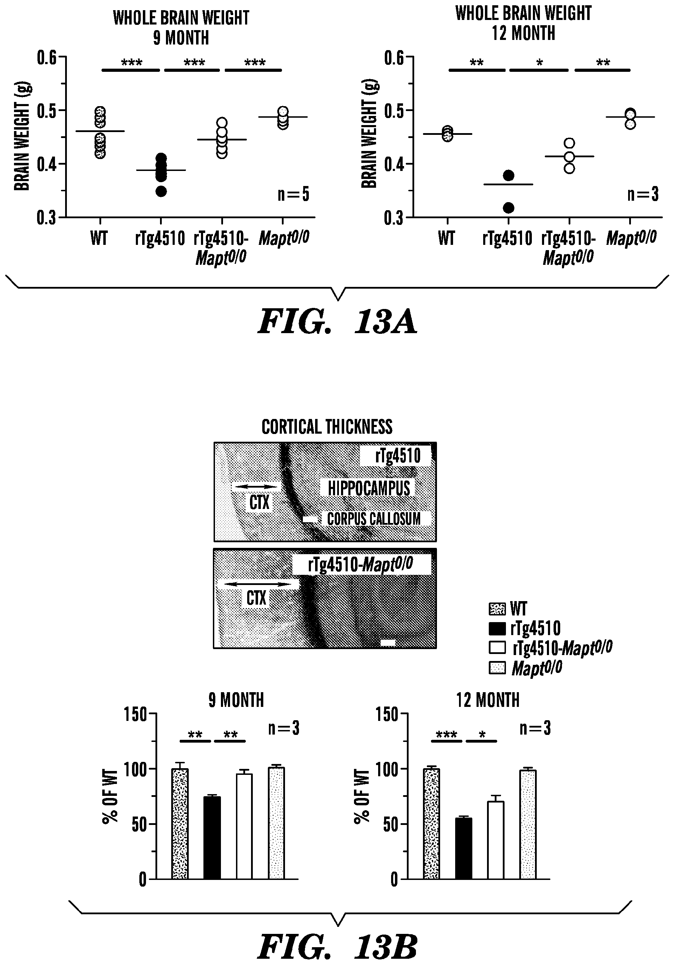

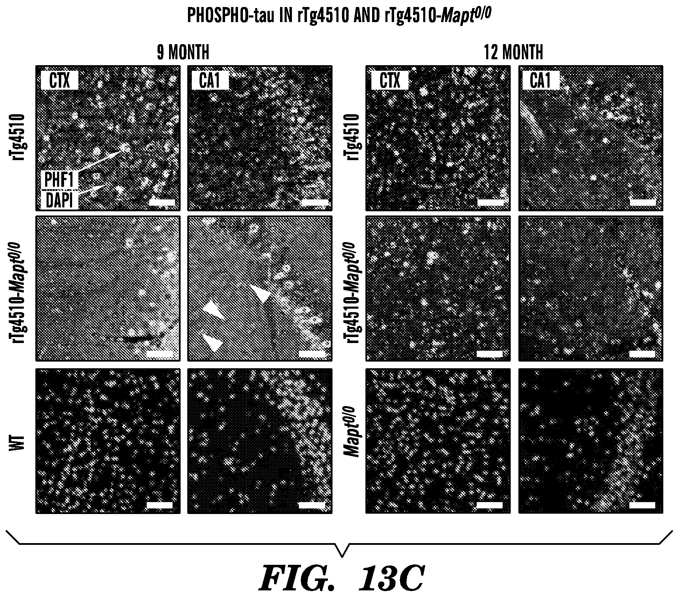

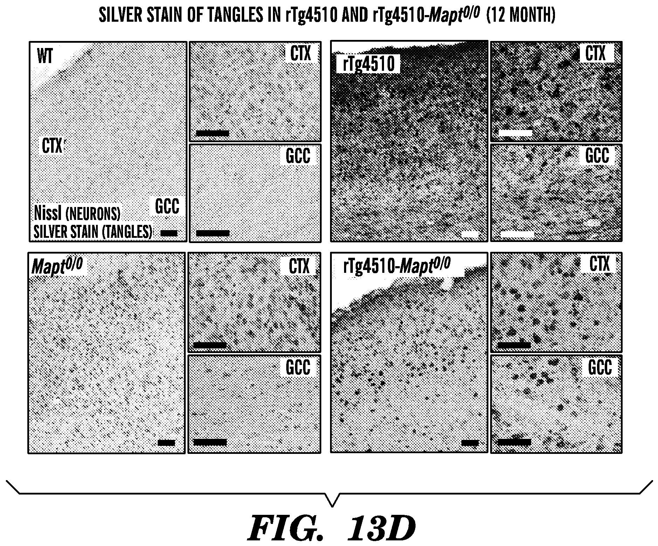

**Figs. 13A-13D** show absence of endogenous mouse tau rescues P301L tau induced atrophy and delays tangle pathology in rTg4510 mice. **(****Fig. 13A****)** Comparing whole brain wet weights of 9 and 12 month old rTg4510 and rTg4510-Mapt0/0 animals revealed that the pronounced brain matter loss observed in rTg4510compared to WT mice (weight loss >16% at 9month, p=0.0002; >22% at 12 month, p=0.0087) is rescued to >96% in 9month old and to ≈89% in 12 month old rTg4510-Mapt0/0 mice (9 month: p=0001, n=5 mixed gender mice per group; 12 month: p=0.042, n=3 mixed gender mice per group). **(****Fig. 13B****)** Cortical thickness, measured adjacent to hippocampus from CTX surface to corpus callosum in horizontal (9 month) or coronal (12 month) brain sections, decreased by ≈23-26% at 9 months and by ≈46% at 12 month of age in rTg4510 compared to WT (p=0.009, p=0.0002), rTg4510-Mapt0/0 (p=0.005, p=0.0223), and Mapt0/0 mice (p=0.0008, p=0.0006; n=3 mice per group). Cortex thickness of rTg4510-Mapt0/0 and Mapt0/0 was similar at 9 months (p=0.13) and 12 months (p=0.975). **(****Fig. 13C****)** Cortical and hippocampal CA1 neurons of 9 and 12 month old rTg4510 mice were filled with hyperphosphorylated tau (immunolabeled with PHF1) forming condensed tangle-like inclusions. At 9 month of age, rTg4510-Mapt0/0 mice had fewer PHF1 filled cell bodies iSn the cortex and PHF1 staining was still present in axons of CA1 neurons (white arrows). At 12 month, the number and appearance of PHF1-filled somata in CTX and CA1 were similar in both rTg4510 and rTg4510-Mapt0/0 mice. **(****Fig. 13D****)** By Gallyas silver stain, 12 month old rTg4510 mice appeared to have a similar number of cortical (CTX) tangles but less aggregated tau in axons and neurites of CTX as well as corpus callosum (GCC) compared to rTg4510-Mapt0/0 mice. WT and Mapt0/0 mice showed neither PHF1 **(****Fig. 13C****)** nor silver staining **(****Fig. 13D**). ns, not significant, scale bars 100 µm.

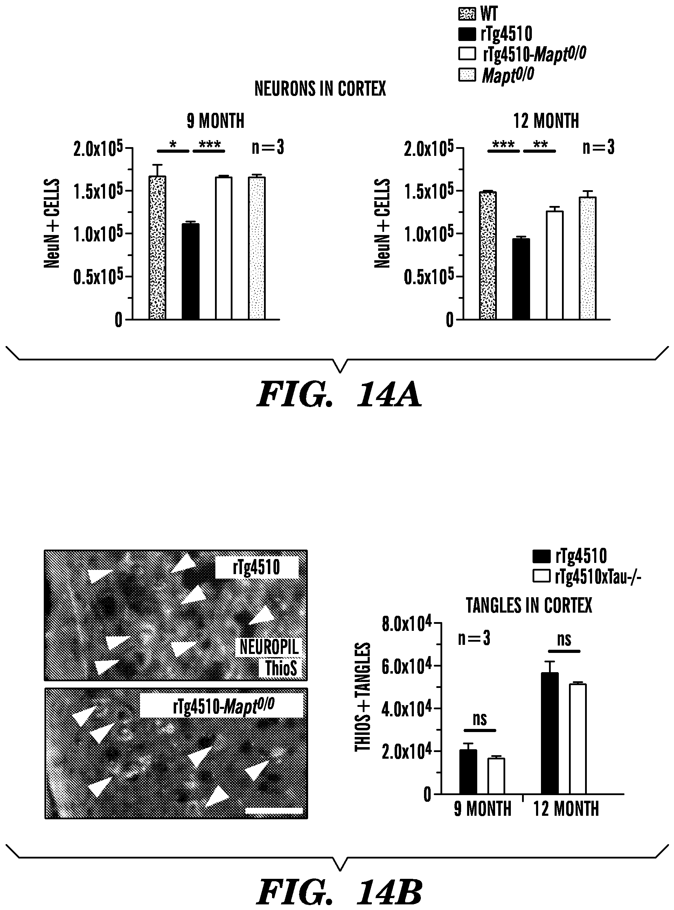

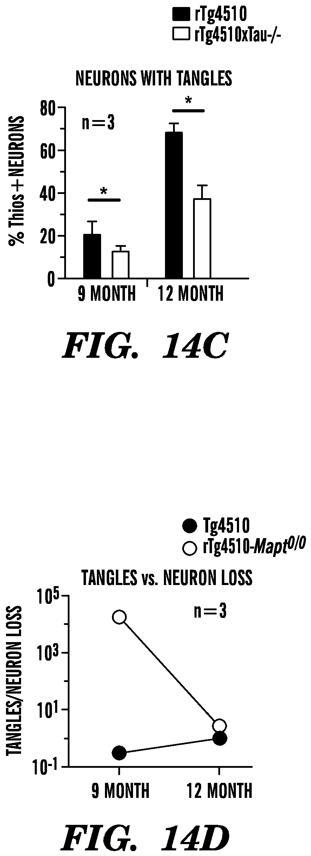

**Figs. 14A-14D** show diminished neuronal loss and tangle toxicity in absence of endogenous tau. **(****Fig. 14A****)** The number of neurons (NeuN+ cells) in the cortex of rTg4510 mice was significantly reduced to ≈67% compared to both WT (p=0.0223) and rTg4510-Mapt0/0 (p<0.0001) at 9 month. At 12 month, the neuronal loss in rTg4510 mice (≈63% of WT) remained similar to 9 month. The number of neurons in rTg4510-Mapt0/0 slightly decreased to ≈88% of Mapt0/0 mice (not significant). **(****Fig. 14B****)** The number of cortical tangles stained with Thioflavine-S (ThioS, white arrows) was similar in rTg4510 and rTg4510-Mapt0/0 mice (9 month: p=0.32; 12 month: p=0.51) and increased significantly from 9 to 12 month of age (rTg4510: p=0.0074; rTg4510-Mapt0/0: p=0.0002). **(****Fig. 14C****)** The percentage of tangle bearing neurons (=number tangles/number neurons) in the cortex was ≈1.6 to 1.8-fold higher in rTg4510 mice at 9 (p=0.03) and at 12 month (p=0.0087; Student's T-test, n=3 mice per group). **(****Fig. 14D****)** Comparing the ratios of tangle number: neuron loss (=tangles/([number of neurons in WT or Mapt0/0 controls]-[number neurons in rTg4510 or rTg4510-Mapt0/0]) at 9 and 12 month of age, highlights the improved neuronal survival at given tangle load in rTg4510-Mapt0/0 mice. ns, not significant, scale bars 100 µm.

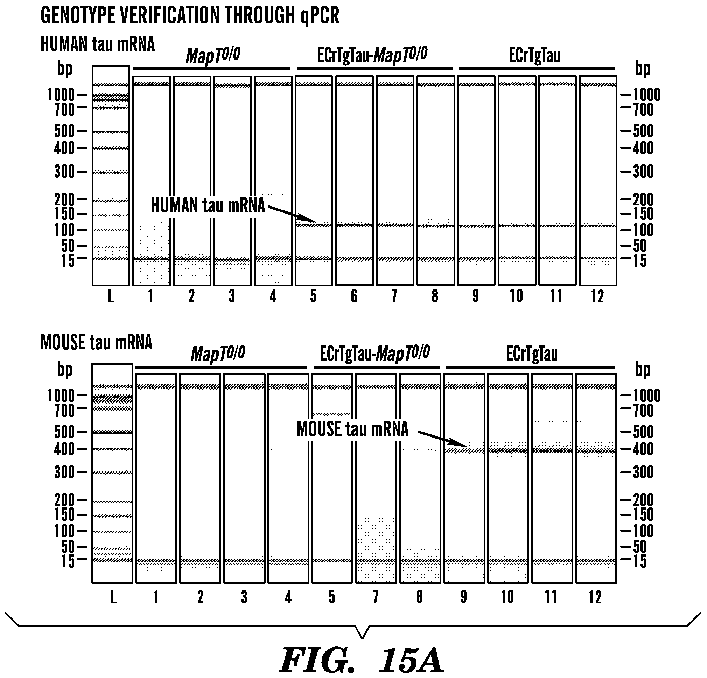

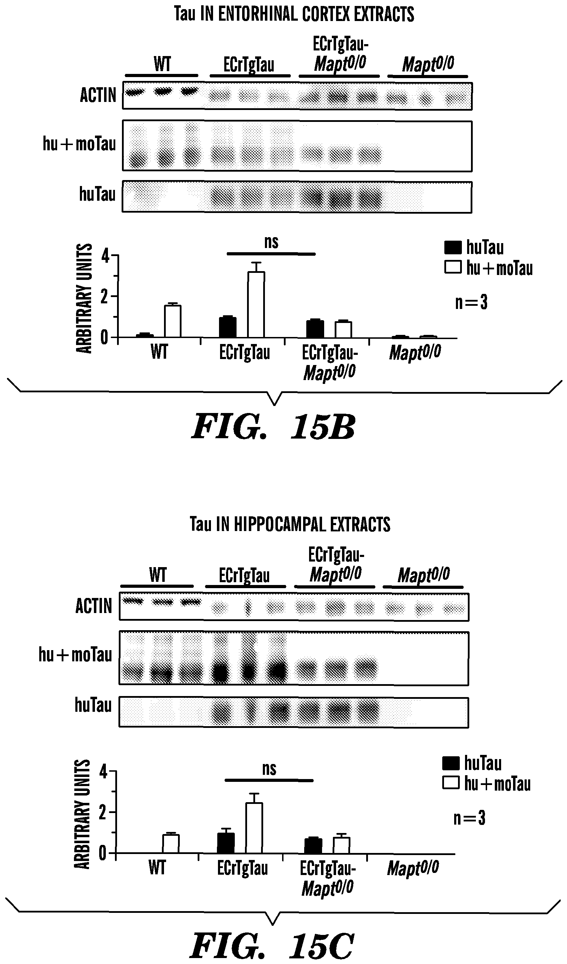

**Figs. 15A-15C** show lack of mouse tau in Mapt0/0 mice and presence of human tau in HPC by Western Blot. **(****Fig. 15A****)** qPCR using RNA extracted from fresh frozen brain tissue of 18 moth old Mapt0/0, ECrTgTau-Mapt0/0, and ECrTgTau mice (n= 4 mice per group) was transcribed into cDNA using primers recognizing human tau transgene transcript (top) or genomic mouse tau (MapT) transcript (bottom). GAPDH mRNA was co-transcribed as amplification control. ECrTgTau-Mapt0/0 and ECrTgTau mice showed human tau expression, whereas Mapt0/0 and ECrTgTau-Mapt0/0 mice lacked mouse tau expression. **(****Figs. 15B-15C****)** Human (huTau) and total tau (hu+moTau) levels in brain extracts from 18 month old wildtype (WT), ECrTgTau, ECrTgTau crossed to tau-knockout mice (ECrTgTau-MaptO/O), and Mapt0/0 mice (n=3 mice per group). ECrTgTau and ECrTgTau-MaptO/O animals show equal transgenic huTau expression in the EC (**Fig. 15B****,** n=3 mice, p=0.29) and similar amounts of huTau in hippocampus (HPC) (**Fig. 15C****,** n=3 mice, p=0.14). ns, non-significant.

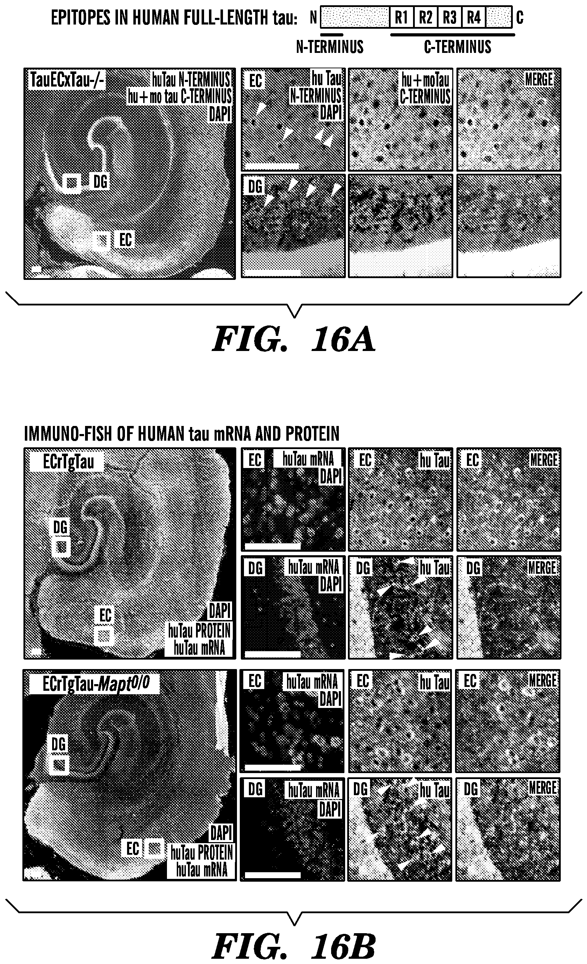

**Figs. 16A-16B** show full-length tau protein propagation from EC into DG in ECrTgTau-MaptO/O mice. **(****Fig. 16A****)** Horizontal brain section co-immunolabeled with Tau13, a monoclonal mouse antibody recognizing the N-terminal end of human (not mouse) tau (epitope = amino acids 20-35) and DAKO, a polyclonal rabbit antibody recognizing the C-terminal half (epitope = multiple sites between amino acids 243-441) of all (mouse and human) tau. Human tau in cell bodies (white circles) and processes (white arrows) was recognized by both the N- and C-terminal antibodies in both EC and DG neurons, suggesting the trans-synaptic propagation of full-length tau. **(****Fig. 16B****)** Restriction of transgenic human P301L tau (huTau) expression to the EC in 18 month old ECrTgTau and ECrTgTau-MaptO/O mice was verified using in situ fluorescence hybridization (FISH) of human tau mRNA ("hu Tau mRNA") combined with immunofluorescent labeling (Immuno-FISH) of human tau (TauY9, "hu Tau"). In both mouse lines, EC neurons were positive for both human tau mRNA and huTau protein, whereas DG neurons had huTau protein but no human tau mRNA. Scale bars 100 µm.

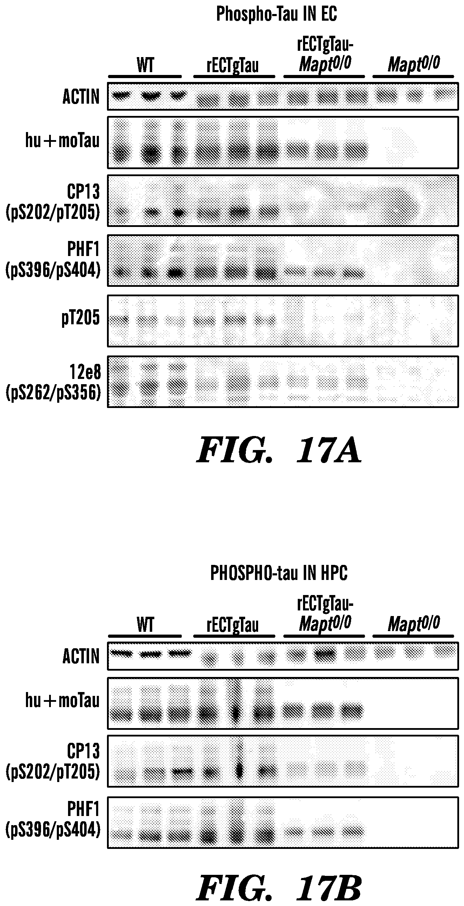

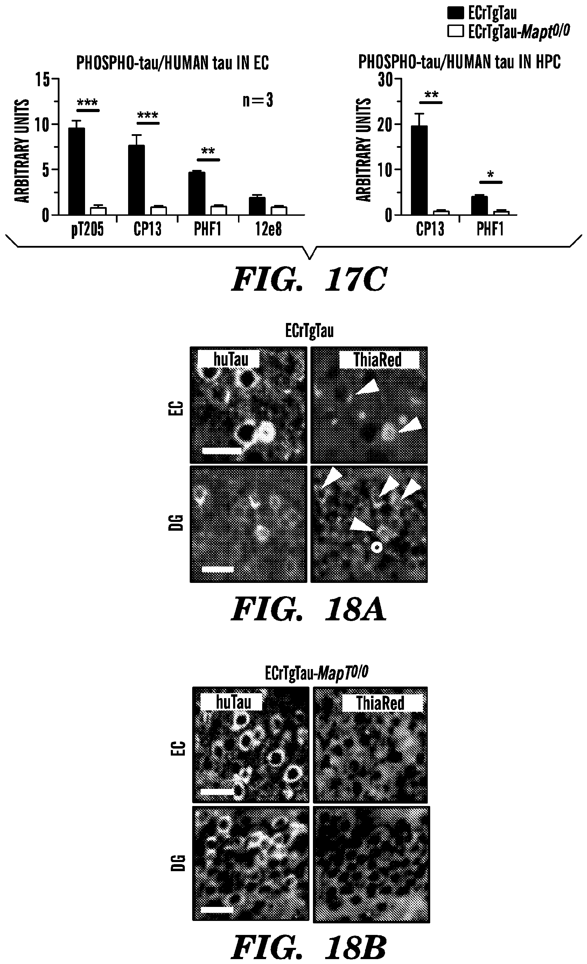

**Figs. 17A-17C** show phospho-tau in EC and HPC extracts of ECrTgTau and ECrTgTau-MaptO/O mice. **(****Figs. 17A-17B****)** Western blot analysis of phospho-tau in homogenates from **(****Fig. 17A****)** entorhinal cortex (EC) and **(****Fig. 17B****)** hippocampus (HPC) in all four genotypes (WT, ECrTgTau, ECrTgTau-MaptO/O, Mapt0/0). Note that WT and ECrTgTau mice show similar phosphorylation levels, and these levels are higher than those observed in ECrTgTau-MaptO/O mice. **(****Fig. 17C****)** The levels of different phospho-tau forms in relation to human tau protein content are ≈2 to 10-fold higher in ECrTgTau compared to ECrTgTau-MaptO/O mice in both EC and HPC (n=3 mice per group, Student's T-test). These results indicate that mouse tau more than human tau provides a major contribution to the phospho-tau pool in ECrTgTau mice.
**Figs. 18A-18B** show that aggregated tau appears in cell bodies of 18 month old ECrTgTau but not ECrTgTau-MaptO/O mice. **(****Fig. 18A****)** In brain sections immunolabeled for human tau (huTau) and co-stained with the red tangle dye Thiazine Red (ThiaRed), aggregated tau was present in ECrTgTau mice in a subset of cell bodies having human tau in the EC and DG (white circles). **(****Fig. 18B****)** No Thiazine Red could be found in any brain region of ECrTgTau-Mapt0/0 mice (n=4 mice). Scale bars 50 µm.

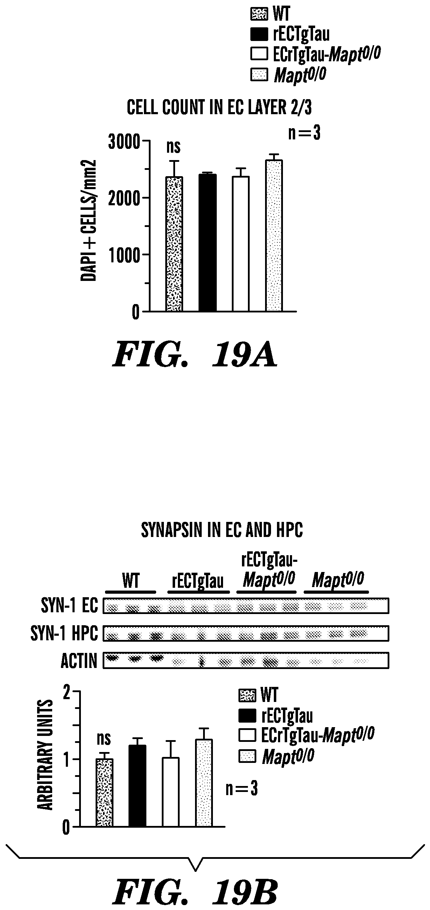

**Figs. 19A-19B** show no changes in EC cell number and synapsin-1 levels in ECrTgTau mice at 18 months. **(****Fig. 19A****)** The number of DAPI-positive cell nuclei in layer 2/3 of entorhinal cortex was the same in ECrTgTau, ECrTgTau-MaptO/O, WT, and Mapt0/0 mice (n=3 mice per group), indicating the lack of human tau induced neuronal death in these mouse lines at 18 month of age. **(****Fig. 19B****)** Levels of the pre-synaptic protein synapsin-1 in entorhinal cortex (EC) and hippocampal (HPC) extracts were similar in WT, ECrTgTau, ECrTgTau-MaptO/O, and Mapt0/0 mice (n=3 mice per group, not significant), indicating the absence of synapse loss in ECrTgTau mice at 18 month of age. ns, not significant.

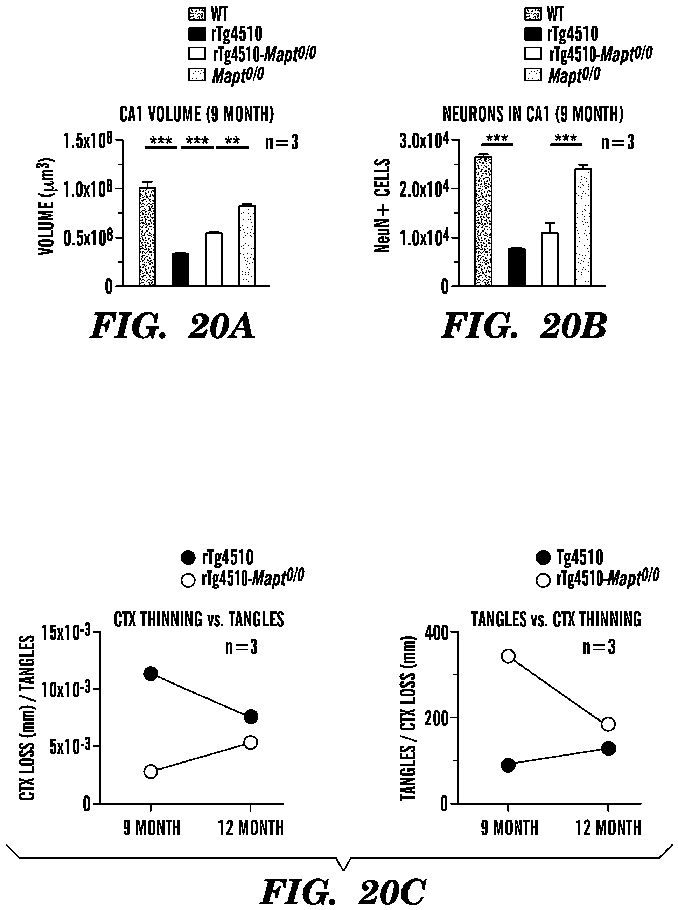

**Figs. 20A-20C** show rescued neurodegeneration in CA1 and reduced tangle toxicity in CTX of rTg4510-Mapt0/0 mice. **(****Figs. 20A-20B****)** In 9 month old animals, volume **(****Fig. 20A****)** and neuron number **(****Fig. 20B****)** of hippocampal area CA1 were significantly decreased in rTg4510 compared to WT (CA1 volume: p=0.0004; CA1 neurons: p<0.0001) mice. In rTg4510-Mapt0/0 mice this CA1 degeneration was partially rescued (CA1 volume: p<0.0001; CA1 neurons: p=0.06). **(****Fig. 20C****)** rTg4510 mice show substantially more cortex thinning (decrease in CTX thickness compared to WT or MapT0/0 mice) per tangle than rTg4510-MapT0/0 at both 9 and 12 months, indicating a higher neuropil toxicity of tangles in presence of endogenous mouse tau. Tangle toxicity only slightly increases in rTg4510-MapT0/0 between 9 and 12 month of age, and slightly decreased in rTg4510 mice.

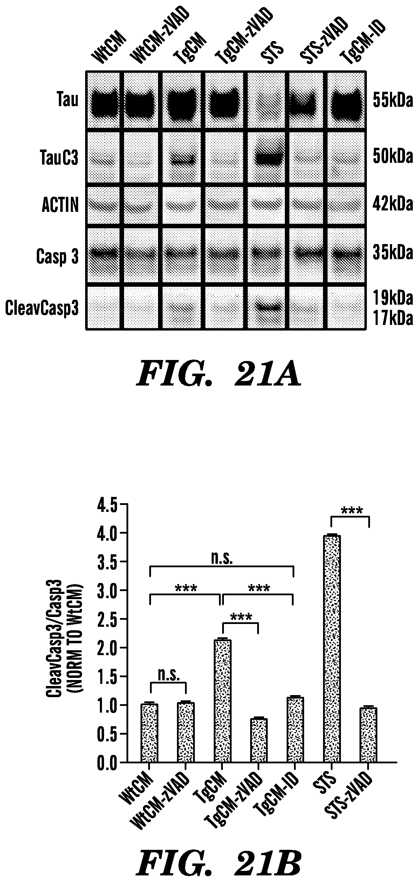

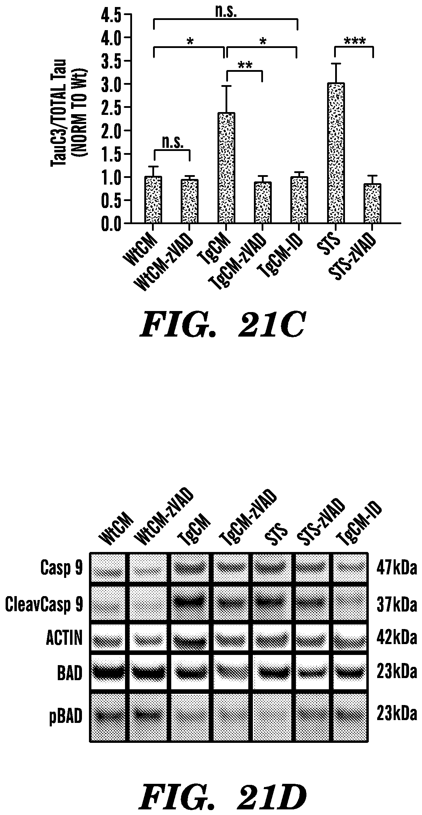

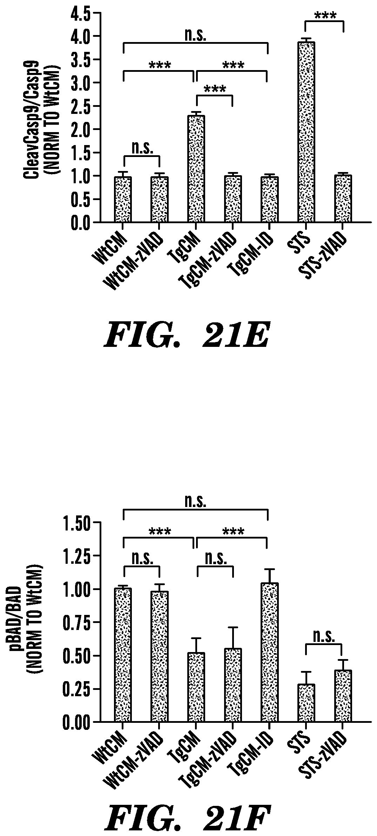

**Figs. 21A-21F** show that Aβ induces activation of caspases 3 and 9, tau cleavage and BAD dephosphorylation. **(****Figs. 21A-21F****)** Western blot analysis of wild-type primary cortical neuron (from CD1 mice) treated for 24 hrs with WtCM, TgCM (containing 4nM of Aβ), Aβ-immunodepleted TgCM (TgCM-ID), 20 µM of zVAD-FMK (a pan caspase inhibitor) or staurosporine (STS, 1 µM for 6 hours). **(****Fig. 21A****)** Representative immunoblots of total cell lysates probed for tau, tau cleaved at Asp421 (TauC3), caspase 3, cleaved caspase 3, and actin (loading control). Quantification of **(****Fig. 21B****)** cleaved caspase 3 levels to total caspase 3 and of **(****Fig. 21C****)** TauC3 to total tau. **(****Fig. 21D****)** Western blots were also performed on whole cell lysates for caspase 9, cleaved caspase 9, BAD, pBAD and actin. **(****Fig. 21E****)** Quantification of the ratio of cleaved caspase 9 (active caspase 9) and total caspase 9 levels as well as **(****Fig. 21F****)** phosphorylated BAD to total BAD. For the representative blots, the same membrane was probed for BAD and pBAD, a second membrane was probed for cleaved caspase 9, caspase 9 and actin. (Bars represent the means +/- SEM of n=3 independent experiments. One-way ANOVAs with Bonferroni's multiple comparisons test *p<0.05, **p<0.01, ***p<0.001, n.s.=not significant

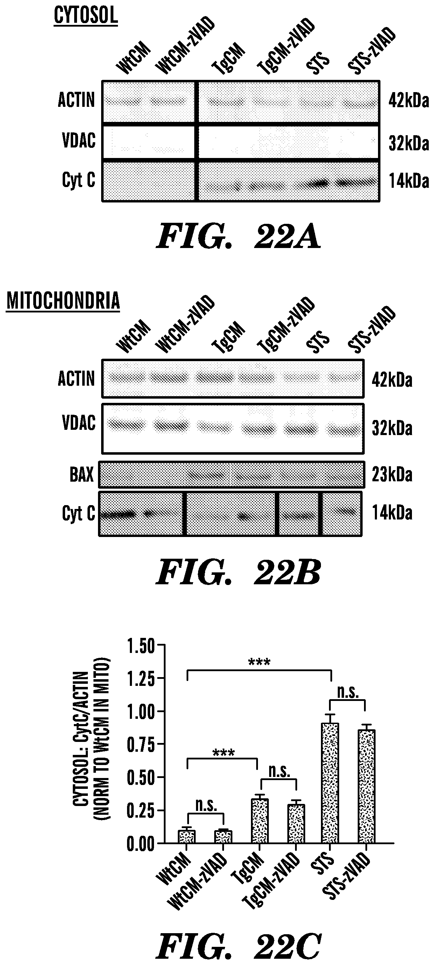

**Figs. 22A-22E** show that Aβ promotes caspase activation via the mitochondrial intrinsic pathway. **(****Figs. 22A-22B****)** Western blot analysis of wild-type primary cortical neurons treated for 24 hrs with TgCM, WtCM, TgCM-ID, zVAD-FMK and STS. After pelleting large cellular debris and insoluble material, cell lysates were further separated into fractions containing a cytosolic supernatant **(****Fig. 22A****)** and a pellet **(****Fig. 22B****)** enriched for mitochondria, which were analyzed for cytochrome c (cyt c), VDAC, BAX, and actin **(****Figs. 22C-22E****).** For the representative blots shown in **Fig. 22B****,** the same membrane was probed for VDAC and actin, a second membrane was probed for BAX and cyt c. (Bars represent the means +/- SEM of n=3 independent experiments. One-way ANOVAs with Bonferroni's multiple comparisons test *p<0.05, **p<0.01, ***p<0.001, n.s.=not significant)

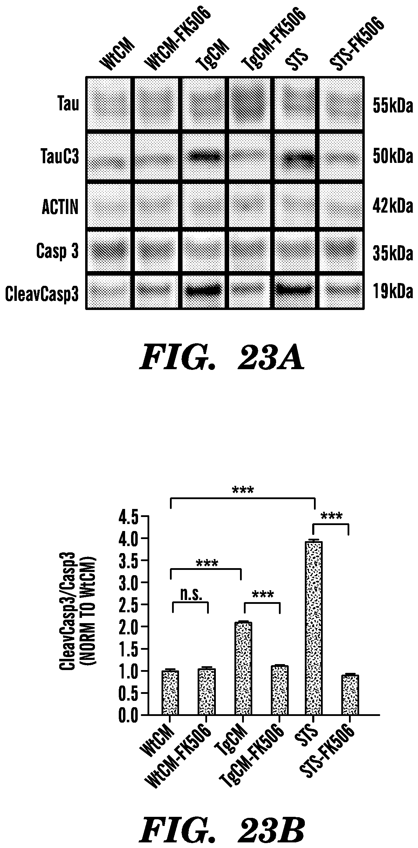

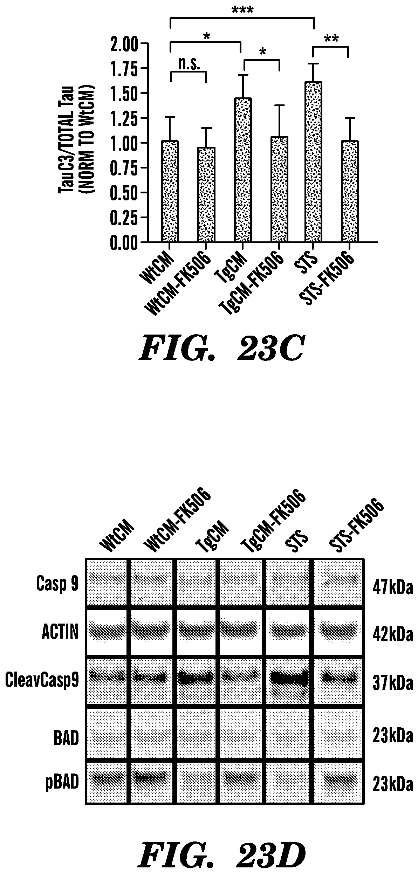

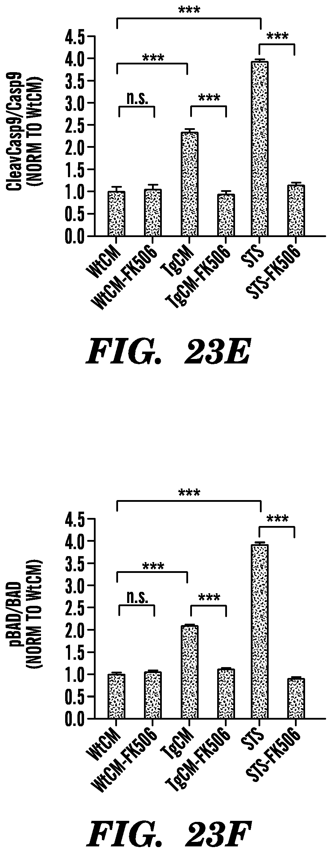

**Figs. 23A-23F** show that Aβ promotes activation of caspases via calcineurin activation. **(****Figs. 23A-23F****)** Western blot analysis of whole cell lysates from neurons treated for 24 hrs with TgCM, WtCM, FK506 and STS showing **(****Figs. 23A** and **23B****)** levels of cleaved caspase 3 and total caspase 3; **(****Figs. 23A** and **23C****)** cleaved tau and total tau; **(****Figs. 23D** and **23E****)** cleaved caspase 9 and total caspase 9; **(****Figs. 23D** and **23F****)** and BAD phosphorylation. For the representative blots: **(****Fig. 23A****)** the same membrane was probed for tau, tauC3 and actin, a second membrane was probed for cleaved caspase 3 and caspase 3; **(****Fig. 23D****)** the same membrane was probed for BAD and pBAD and actin, a second membrane was probed for cleaved caspase 9 and caspase 9. (Bars represent the means +/- SEM of n=3 independent experiments. One-way ANOVAs with Bonferroni's multiple comparisons test **p<0.01, ***p<0.001, n.s.=not significant).

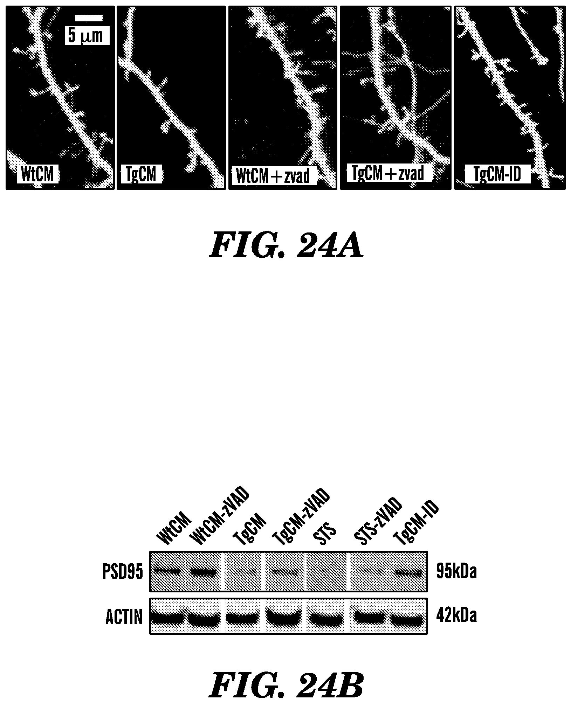

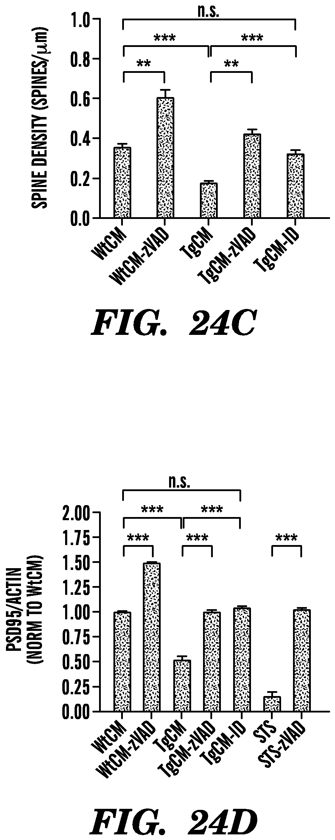

**Figs. 24A-24D** show that Aβ-induced spine loss is reduced by caspase inhibition. **(****Fig. 24A****)** Confocal images of spines from GFP transfected neurons treated for 24 hrs with WtCM, TgCM, TgCM-ID, or zVAD-FMK. **(****Fig. 24B****)** Western blot of cell lysates from treated neurons, proved for PSD95, a synaptic marker. **(****Fig. 24C****)** Quantification of spine density from confocal images (Kruskal-Wallis ANOVA, Dunn's multiple comparisons test, n=30-40 neurons per group). **(****Fig. 24D****)** Quantification of PSD95 levels from Western blot (Bars represent the means +/- SEM of n=3 independent experiments. One-way ANOVAs with Bonferroni's multiplecomparisons test **p<0.01, ***p<0.001, n.s.=not significant).

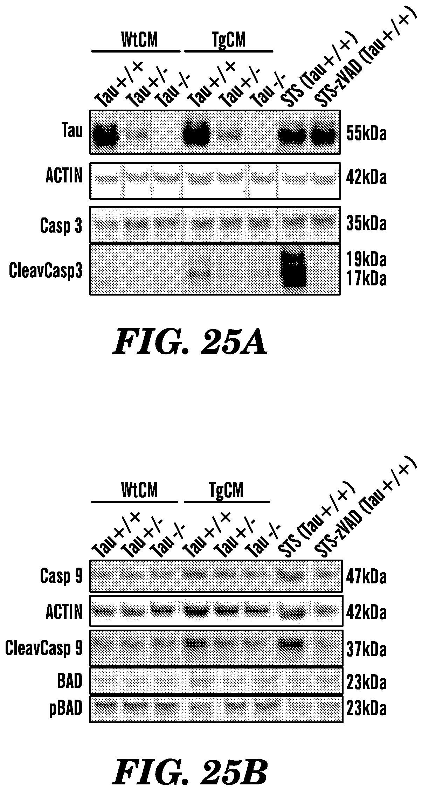

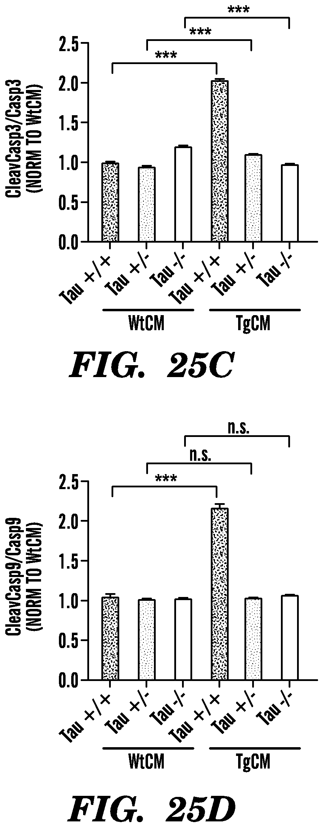

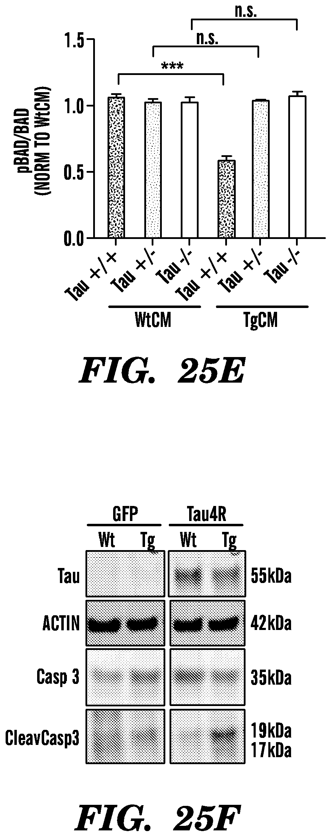

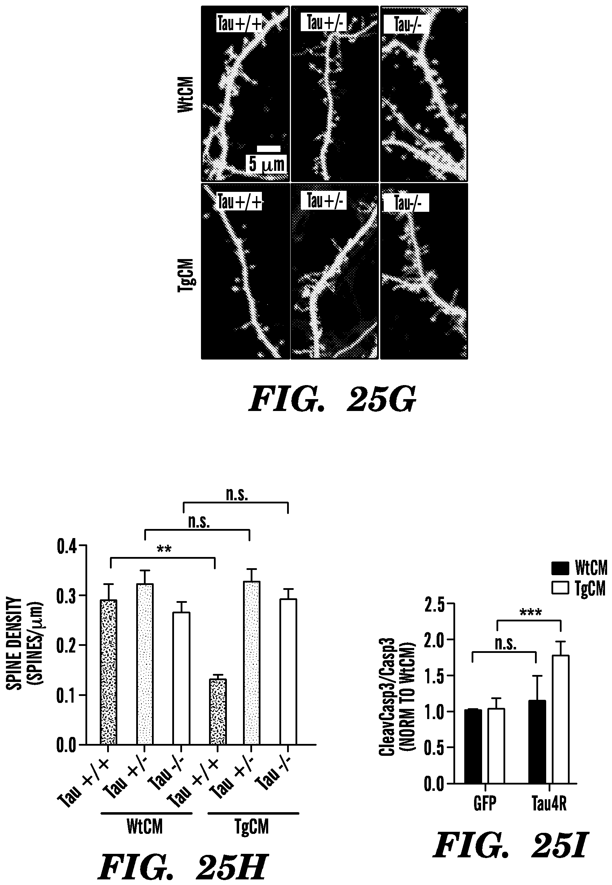

**Figs. 25A-25I** show that tau mediates Aβ-induced apoptic cascade activation and spine loss. **(****Figs. 25A** and **25C****)** cleaved caspase 3 and total caspase 3 cultures from Tau-/-, Tau+/- and Tau+/+ mice treated with WtCM or TgCM (n=3 independent experiments per group). **(****Figs. 25B** and **25D****)** Levels of cleaved caspase 9 and total caspase 9 and **(****Figs. 25B** and **25E****)** levels of pBAD and BAD in the same cultures. For the representative blots: **(****Fig. 25A****)** the same membrane was probed for BAD and pBAD, a second membrane was probed for cleaved caspase 9, caspase 9; and actin **(****Fig. 25D****)** the same membrane was probed for tau and actin, a second membrane was probed for cleaved caspase 3 and caspase 3. **(****Figs. 25F** and **25I****)** Tau-/- neurons transduced with GFP (control), human full-length tau (Tau4R), using AAV (adeno-associated virus) mediated gene delivery. Three days after transduction, neuronal cultures were exposed to TgCM (Tg) or WtCM (Wt) for 24 hrs. Western blot analysis of neuronal lysates using antibodies against tau (Tau), cleaved caspase 3, total caspase 3 and β-actin are shown. For the representative blots, the same membrane was probed for tau, a second membrane was probed for cleaved caspase 3, caspase 3, and actin (n=3 independent experiments per group). Results are normalized to control (GFP transduced neurons treated with WtCM). **(****Fig. 25G****)** Confocal images of GFP expressing Tau +/+, +/- and -/- neurons treated with WtCM or TgCM for 24 hrs. **(****Fig. 25H****)** Spine density of GFP transfected neurons (n=30-40 neurons per group). (Bars represent the means +/-SEM. Two-way ANOVAs with Bonferroni's multiple comparisons test **p<0.01, ***p<0.001, n.s.=not significant).

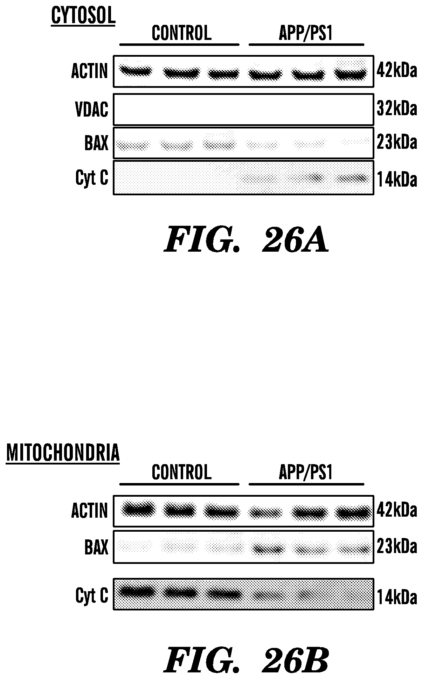

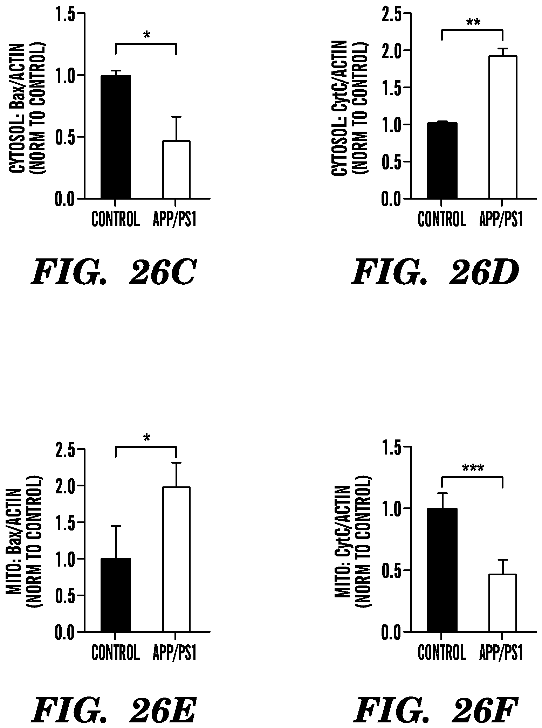

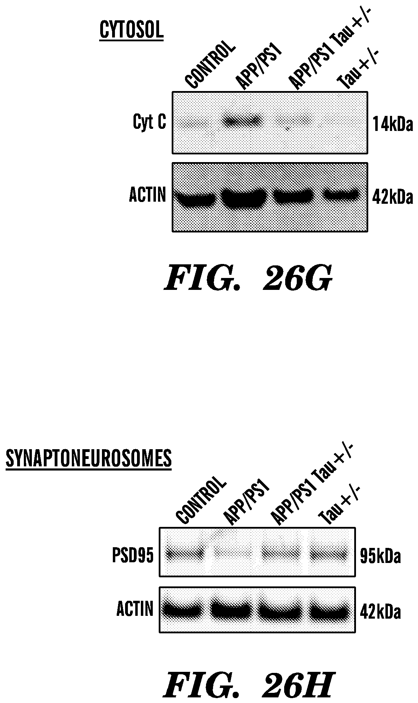

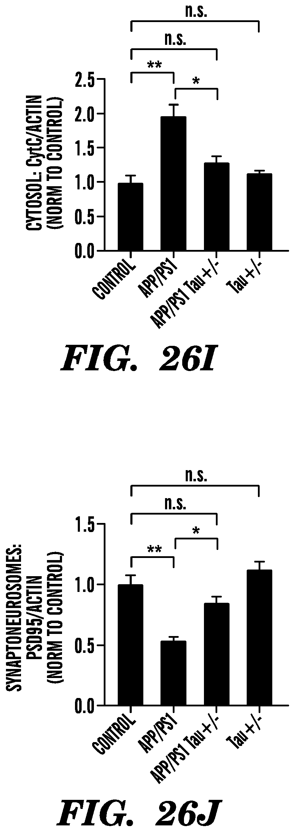

**Figs. 26A-26J** show that tau reduction is protective against Aβ-induced changes in vivo. **(****Fig. 26A****)** Western blot of BAX, cyt c, in the brain cytosolic fraction and in **(****Fig. 26B****)** the mitochondrial pellet obtained from 6 month old APP/PS1 mice. **(****Figs. 26C** and **26D****)** Quantification of BAX and cyt c in cytosol. **(****Figs. 26E** and **26F****)** Quantification of BAX and cyt c in the mitochondrial pellet. **(****Fig. 26G****)** Western blot and **(****Fig. 26I****)** quantification of cyt c in cytosol fraction from control (wild-type), APP/PS1, APP/PS1 Tau+/- or Tau+/- mice at 4 months of age. **(****Fig. 26H****)** Western blot and **(****Fig. 26J****)** quantification of PSD95 in synaptoneurosomes prepared from 4 month old APP/PS1, APP/PS1 Tau+/-, and Tau+/- mice. (Student's t test in **Figs. 26C-26F****.** One-way ANOVA with Bonferroni's multiple comparisons test in I, J. Bars represent the means +/- SEM, n=3 animals per group. *p<0.05, **p<0.01, ***p<0.001, n.s.=not significant)

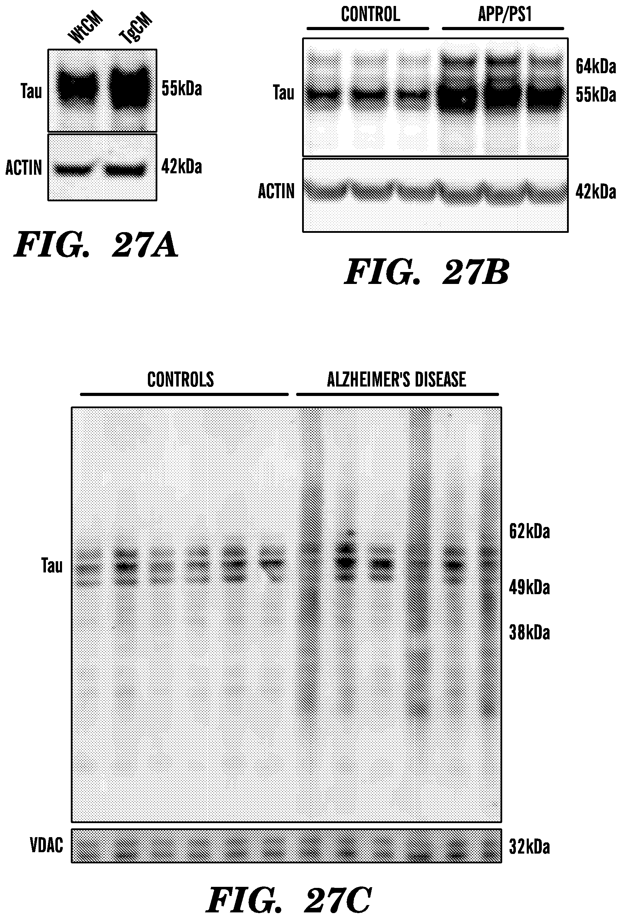

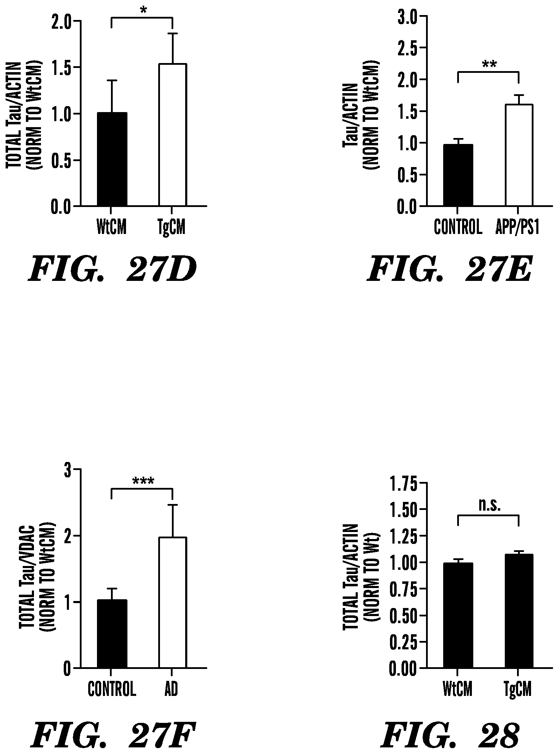

**Figs. 27A-27F** show that tau associates with mitochondria in vitro and in vivo. **(****Figs. 27A****,** **27D****)** 24 hr incubation of primary neurons with TgCM increases tau the mitochondria (n=8 wells per condition). **(****Figs. 27B****,** **27E****)** Mitochondrial extracts from 6-month-old APP/PS1 mice have increased tau compared to control mice (n=3 animals per group). **(****Figs. 27C****,** **27F****)** Mitochondrial extracts from human Alzheimer's disease brain have increased tau compared to age-matched controls (n=9 samples per group). (Bars represent means +/- SEM. Student's t tests. *p<0.05, **p<0.01, ***p<0.001).
**Fig. 28** shows that total tau is not altered in whole cell lysates from primary neurons. Quantification of western blot levels of tau (from **Fig. 21A**) from cultures treated with WtCM or TgCM for 24 hrs. (Bars represent the means +/- SEM, n=5 wells per group. Student's t test. n.s.= not significant).

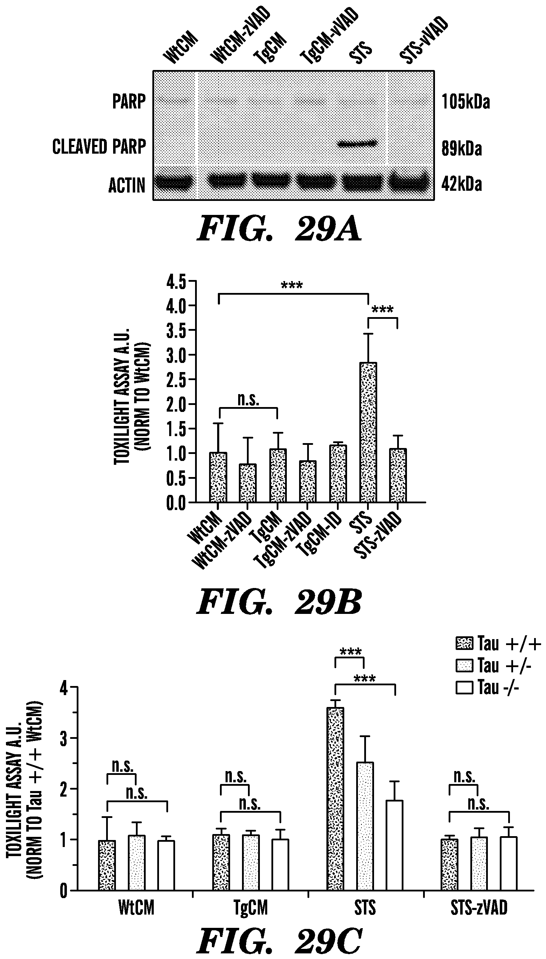

**Figs. 29A-29C** show that application of TgCM for 24 hrs does not induce cell death in primary neuronal cultures. **(****Fig. 29A****)** Western blot of cleaved PARP and total PARP in cultures treated with WtCM, TgCM, STS, and zVAD for 24 hrs (n=3 per group). **(****Fig. 29B****)** Toxilight assay for cell death in cultures treated for 24 hrs with WtCM, TgCM, TgCM-ID, and zVAD (a.u.=arbitrary units, One-way ANOVA, n=3-8 per group). **(****Fig. 29C****)** Toxilight assay for cell death in cultures from Tau+/+ , Tau+/-, and Tau-/-mice (Two-way ANOVA, n=4 per group). (Bars represent the means +/- SEM. Bonferroni's multiple comparisons test ***p<0.0001, n.s.= not significant).

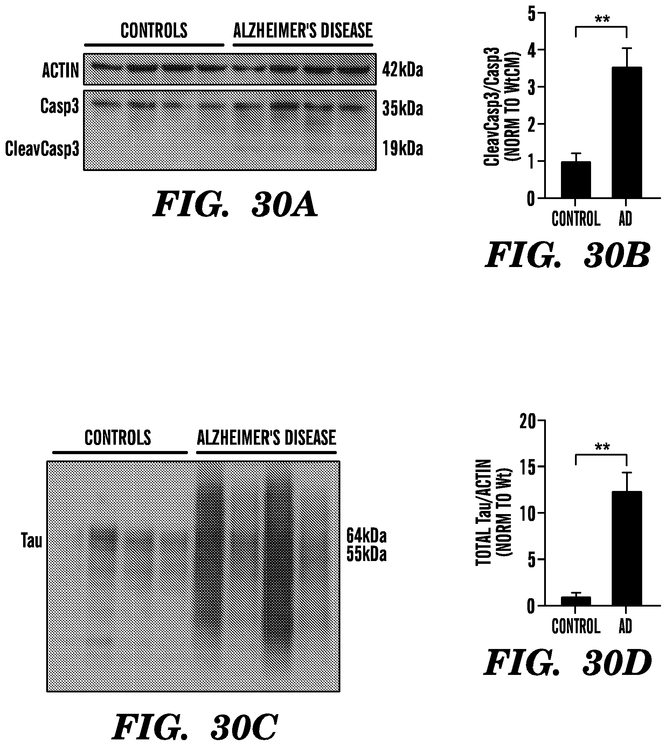

**Figs. 30A-30D** show that increased activation of caspase 3 and localization of tau in human synaptoneurosomes. Synaptoneurosomes were prepared from AD or non-demented human tissue. **(****Figs. 30A, 30B****)** Western blot analysis of the synaptoneurosomes probed for cleaved caspase 3 and total caspase 3 in the AD samples versus non-demented controls. **(****Figs. 30C, 30D****)** Western blot analysis of synaptoneurosomes probed for total tau in the AD samples versus non-demented controls. (Bars represent the means +/- SEM, n=4 per group. Student's t test **p<0.01).

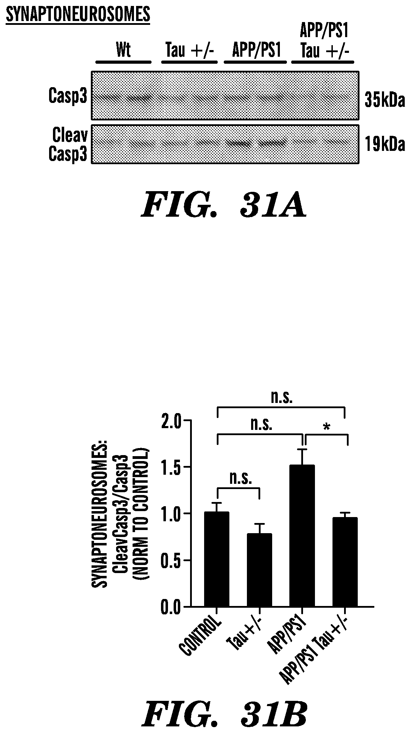

**Figs. 31A-31B** show that tau reduction is protective against Aβ-induced caspase 3 activation in vivo. Western blot **(****Fig. 31A****)** of synaptoneurosomes prepared from non-transgenic controls, APP/PS1, APP/PS1 Tau+/-, and Tau+/- mice at 4 months of age shows increased cleaved caspase 3 in synaptoneurosomes from APP/PS1 mice compared to APP/PS1 mice carrying only one copy of the tau allele **(****Fig. 31B****)** . (One-way ANOVA with Bonferroni's multiple comparisons test, n=3 per group. Bars represent the means +/- SEM. *p<0.05, **p<0.01, ***p<0.001, n.s.=not significant).

### DETAILED DESCRIPTION OF THE INVENTION

Various aspects described herein stem from, at least in part, discovery of soluble high molecular weight (HMW) tau species (including phosphorylated form) present at a low extracellular level in the brain of subjects with Alzheimer's disease (AD) or frontotemporal dementia (FTD), and abilities of the soluble HMW tau species to be taken up by a neuron and propagate between neurons. In particular, to identify the soluble HMW tau species responsible for propagation, the inventors have developed a novel 3-chamber microfluidic device to form dual-layered neurons and examine neuronal tau uptake, axonal transport, and synaptic transmission. By characterizing uptake and propagation properties of different tau species derived from, for example, the interstitial fluid of awake, behaving tau-transgenic mice, and cortical extracts from the mice as well as human AD postmortem cortices, the inventors have discovered, that PBS-soluble phosphorylated high molecular-weight (HMW) tau species, though very low in abundance, are taken up, axonally transported, and passed-on to synaptically connected neurons in a time- and concentration-dependent manner. In contrast, low molecular weight (LMW) tau species (e.g., monomer/dimer size tau), though much higher in abundance, are not inefficiently taken up by neurons. Thus, the discovery of the rare species of soluble HMW phosphorylated tau involved in inter-neuron-propagation provides a more effective target for therapeutic intervention and biomarker development.

Further, the inventors have discovered that tau propagation between neurons does not require endogenous, intracellular tau for template misfolding, but tau-null mice had substantially less pathological misfolding and gliosis, and thus, suitably, complete removal of endogenous, intracellular host tau produces a neuroprotective effect. For example, the inventors have shown that tau-null neurons lacked activation of Aβ-induced mitochondrial intrinsic caspase cascades in the neurons and were subsequently protected from Aβ-induced dendritic spine loss and neurodegeneration.

Accordingly, described herein are compositions comprising soluble HMW tau species that is responsible for inter-neuron propagation and applications thereof. Methods of treating and diagnosing tau-associated neurodegeneration in a subject are also described herein.

### Compositions comprising soluble high molecular weight (HMW) tau species

A composition comprising soluble high molecular weight (HMW) tau species is described herein. The soluble HMW tau species in the composition is non-fibrillar, with a molecular weight of at least about 500 kDa.

Suitably, the composition is substantially free of soluble low molecular weight (LMW) tau species. As used herein and throughout the specification, the term "substantially free of soluble low molecular weight (LMW) tau species" includes the complete absence (i.e., 0%) of LMW tau species and a trace amount of LMW tau species that is not readily detectable by known methods in the art, e.g., but not limited to size exclusion chromatogram, ELISA, microscopy, atomic force microscopy, or any combinations thereof. Suitably, the proportion of HMW to LMW tau in the compositions described herein are substantially different from the proportions in which these protein forms occur *in vivo.* Thus, where HMW tau makes up only a small proportion of the total tau occurring normally *in vivo,* e.g., in humans, described herein are preparations in which at least 50% of the total tau protein is the HMW form, e.g., preparations with at least 50% HMW and 50% or less LMW tau, at least 60% HMW tau and 40% LMW tau or less, at least 70% HMW tau and 30% LMW tau or less, at least 80% HMW tau and 20% or less LMW tau, at least 90% HMW tau and 10% or less LMW tau, or 95% HMW Tau and less than 5% LMW tau.
Suitably, the composition can comprise no more than 5% (w/w) soluble LMW tau species, including, e.g., no more than 4% , no more than 3%, no more than 2%, no more than 1%, no more than 0.5% (w/w) soluble LMW tau species. As used herein, the term "substantially lacking LMW tau" means that a given HMW tau preparation has less than 1% LMW tau, and preferably less than 0.1% LMW tau, less than 0.01% tau or lower, by weight.

As used herein and throughout the specification, the term "high molecular weight tau species" or "HMW tau species" refers to a population of tau species molecules that are non-fibrillar and oligomeric, wherein the tau species molecules each have a molecular weight of at least about 500 kDa. The HMW tau species has a pathologically misfolded conformation (e.g., positive for Alz 50 antibody staining) and appears as oligomeric structures (e.g., in atomic force microscopy). The HMW tau species can be intracellular (e.g., inside neurons) or extracellular (e.g., in the brain interstitial fluid and/or cerebrospinal fluid). The HMW tau species can be produced by fractionating brain extracts and/or brain interstitial fluid from tau-transgenic animals (e.g., tau-transgenic mice), e.g., by centrifugation (e.g., x 3000 g or less) and/or size exclusion chromatography as described in the Examples, and selecting the fraction(s) with a molecular weight of at least about 500 kDa or more. The HMW tau species can be produced by multimerizing recombinant tau proteins. The HMW tau species can be phosphorylated or hyper-phosphorylated as described in further details below.

As used herein, the term "non-fibrillar" refers to HMW tau species that are not aggregated into neurofibrillary tangles (NFTs). NFTs are generally formed inside neurons from hyperphosphorylation of tau proteins assembled into insoluble filaments.

As used herein in reference to the soluble HMW tau species described herein, the term "oligomeric" or "oligomers" means a complex or an aggregate comprising a finite number of tau monomer or dimer subunits. The soluble HMW tau species described herein can comprise at least 2 or more tau monomer or dimer subunits, including, e.g., at least 3, at least 4, at least 5, at least 6, at least 7, at least 8, at least 9, at least 10, at least 15, at least 20, at least 30, at least 40 or more, tau monomer or dimer subunits. The HMW tau species described herein can comprise about 3-100 tau monomer or dimer subunits, about 4-90 tau monomer or dimer subunits, about 5-80 tau monomer or dimer subunits, about 5-70 tau monomer or dimer subunits, about 5-60 tau monomer or dimer subunits, about 5-50 tau monomer or dimer subunits, about 5-40 tau monomer or dimer subunits, about 5-30 tau monomer or dimer subunits, or about 5-20 tau monomer or dimer subunits.

*Tau proteins or microtubule associated protein tau (MAPT):* Tau proteins belong to the family of microtubule-associated proteins (MAP). They are mainly expressed in neurons where they play an important role in the assembly of tubulin monomers into microtubules to constitute the neuronal microtubules network, although non-neuronal cells (e.g., heart, kidney, lung, muscle, or pancreas cells) can have trace amounts. Microtubules are involved in maintaining the cell shape and serve as tracks for axonal transport. Tau proteins are translated from a single gene located on chromosome 17. Their expression is developmentally regulated by an alternative splicing mechanism and six different isoforms exist in the human adult brain. Buee et al., Brain Research Reviews (2000) 33: 95-130.

Tau can be subdivided into four regions: an N-terminal projection region, a proline-rich domain, a microtubule-binding domain (MBD), and a C-terminal region. Morris et al., Neuron (2011) 70: 410-426. Alternative splicing around the N-terminal region and MBD generates six main isoforms in adult human brain (Goedert et al., Neuron (1989) 3: 519-526), with the range from 352-441 amino acids. They differ in either zero, one or two inserts of 29 amino acids at the N-terminal part (exon 2 and 3), and three or four repeat-regions at the C-terminal part exon 10 missing. Therefore, the longest isoform in the CNS has four repeats (R1, R2, R3 and R4) and two inserts (441 amino acids total), while the shortest isoform has three repeats (R1, R3 and R4) and no insert (352 amino acids total). Tau isoforms are named by how many microtubule binding repeat sequences are expressed (termed R) and by which N-terminal exons are included (termed N). For example, 3R tau has three microtubule binding repeat sequences, while 4R tau has four due to inclusion of exon 10. 0N tau includes no N-terminal exons, IN tau exon 2, and 2N tau exons 2 and 3 (Lee et al., Annu. Rev. Neurosci. (2001) 24: 1121-1159). Tau mutations are numbered by their location in 4R2N human tau (Lee et al., 2001). Six additional isoforms are formed by alternative splicing around exon 6, resulting in a total of 12 tau isoforms expressed in brain (Wei and Andreadis, J. Neurochem (1998) 70: 1346-1356). The references cited herein are incorporated herein by reference.

The soluble HMW tau species can be an oligomer enriched in at least one or more (e.g., at least two or more) of the tau isoforms selected from the group consisting of tau isoform 1, tau isoform 2, tau isoform 3, tau isoform 4, tau isoform 5, and tau isoform 6. The soluble HMW tau species can be an oligomer enriched in at least one or more (e.g., at least two or more) of the tau isoforms selected from the group consisting of (2-3-10-); (2+3-10-); (2+3+10-); (2-3-10+); (2+3-10+); (2+3+10+). All MAP (microtubule-associated protein) tau protein isoforms are known in the art and their nucleotide and protein sequences are available on the world wide web from the NCBI, including, e.g., human. Table 1 below shows exemplary Accession Nos of the nucleotide and amino acid sequences of different human tau isoforms that are available at NCBI.

**Table 1. Amino acid sequences of different human tau isoforms**

| Various human MAPT (microtubule associated protein tau) isoforms | Nucleotide sequence (NCBI Accession No.) | Amino acid sequence (NCBI Accession No.) |
|---|---|---|
| MAPT isoform 1 | NM_016835 | NP_058519 |
| MAPT isoform 2 | NM_005910 | NP_005901 |
| MAPT isoform 3 | NM_016834 | NP_058518 |
| MAPT isoform 4 | NM_016841 | NP_058525 |
| MAPT isoform 5 | NM_001123067 | NP_ 001116539 |
| MAPT isoform 6 | NM_001123066 | NP_ 001116538 |
| MAPT isoform 7 | NM_001203251 | NP_ 001190180 |
| MAPT isoform 8 | NM_001203252 | NP_ 001190181 |

As used herein and throughout the specification, the term "soluble" when referring to the HMW or LMW tau species, means the HMW or LMW tau species dissolves and forms a substantially homogeneous olution in a biological flued, e.g., CSF, brain interstitial fluid, plasma etc. The HMW tau soluble species described herein also dissolves and forms a substantially homogeneous solution in phosphate-buffered saline.

As used herein, the term "molecular weight" refers to the mass of a given molecule (e.g., a HMW or LMW tau species molecule) or the average mass of a population (e.g., two or more) of given molecules (e.g., a population of HMW tau species molecules or LMW tau species molecules). Different average values can be defined depending on the statistical method that is applied. Suitably, the molecular weight is number average molecular weight. Alternatively, the molecular weight is mass average molecular weight. The molecular weights of HMW or LMW tau species can be generally measured by any methods known in the art, e.g., but not limited to, gel electrophoresis, gel chromatography, size exclusion chromatography, light scattering, and/or mass spectrometry. Suitably, the molecular weight of the HMW tau species or LMW tau species is measured by size exclusion chromatography.

In the compositions described herein, the soluble HMW tau species has a molecular weight of at least about 500 kDa or more. The soluble HMW tau species can have a molecular weight of at least about 550 kDa, at least about 600 kDa, at least about 650 kDa, at least about 700 kDa, at least about 750 kDa, at least about 800 kDa, at least about 850 kDa, at least about 900 kDa, at least about 950 kDa, or more. The soluble HMW tau species can have a molecular weight of at least about 669 kDa or more. The soluble HMW tau species can have a molecular weight of about 500 kDa to about 2000 kDa, about 550 kDa to about 1500 kDa, about 600 kDa to about 1000 kDa, about 650 kDa to about 1000 kDa or about 669 kDa to about 1000 kDa. The soluble HMW tau species can form a molecular weight distribution. The soluble HMW tau species can all have substantially the same molecular weight.

The non-fibrillar soluble HMW tau species can be present in an aggregate of particles. The particles can be of any shape, and are not limited to spherical or globular particles. The particle size of the soluble HMW tau species can vary with their molecule weights. The particle size of the soluble HMW tau species can range from about 1 nm to about 50 nm, about 5 nm to about 40 nm, about 10 nm to about 30 nm, or about 15 nm to about 25 nm. The soluble HMW tau species can form a particle size distribution. The soluble HMW tau species can all have substantially the same particle size.

The soluble HMW tau species can consist essentially of or consists of, tau monomer and/or dimer subunits.

The soluble HMW tau species can comprise other constituents such as other proteins and lipids.

The inventors have discovered that an AD brain extract contained significantly higher levels of phosphorylated, soluble HMW tau species, when compared to a control brain without AD. Thus, the soluble HMW tau species in the composition can be phosphorylated. The soluble HMW tau species in the composition can be hyper-phosphorylated. As used herein, the term "hyper-phosphorylated" or "hyperphosphorylation" refers to the circumstance where the number of phosphorylated sites (i.e., the number of phosphate moieties) on the HMW tau species is greater than that on the LMW tau species or non-aggregating normal tau proteins; or where the phosphorylation sites on the HMW tau species are phosphorylated at levels higher than that in the LMW tau species or non-aggregating normal tau proteins. Suitably, at least one or more (including, e.g., 1, 2, 3, 4, 5, 6, 7, 8, 9, 10, or all) phosphorylation sites on the HMW tau species are phosphorylated at levels at least about 50% (including, at least about 60%, at least about 70%, at least about 80%, at least about 90%, at least about 95% or up to 100%) higher than that in the LMW tau species or non-aggregating normal tau proteins. Suitably, at least one or more (including, e.g., 1, 2, 3, 4, 5, 6, 7, 8, 9, 10, or all) phosphorylation sites on the HMW tau species are phosphorylated at levels at least about 1.5-fold (including, at least about 2-fold , at least about 2.5-fold , at least about 3-fold , at least about 3.5-fold, at least about 4-fold, at least about 4.5-fold, at least about 5-fold or higher) higher than that in the LMW tau species or non-aggregating normal tau proteins. Examples of phosphorylation sites that can be hyperphosphorylated in the HMW tau species include, without limitations, pS199, pT205, pS262, pS396, pS396/404, pS400, pS409, pS422, or a combination of two or more thereof. The HMW tau species can be hyper-phosphorylated at least one or more (e.g., at least two, at least three, at least four, at least five, at least six) of the following phosphorylation sites: pT205, pS262, pS400, pS404, pS409, and pS422. Full length tau isoform is represented by, e.g., NCBI Accession No. NP_005901.2, the information at which is incorporated herein by reference.

The soluble HMW tau species can be preferentially taken up by a neuron. As used herein, the term "preferentially taken up by a neuron" refers to a neuron having a higher likelihood to take up the soluble HMW tau species than to take up the soluble LMW tau species. Suitably, a neuron has a higher likelihood, by at least about 30% or more (including, e.g., at least about 40%, at least about 50%, at least about 60%, at least about 70%, at least about 80%, at least about 90%, at least about 95% or higher), to take up the soluble HMW tau species than to take up the soluble LMW tau species. Suitably, a neuron has a higher likelihood, by at least about 1.5-fold or more (including, e.g., at least about 2-fold, at least about 2.5-fold, at least about 3-fold, at least about 3.5-fold, at least about 4-fold, at least about 5-fold, at least about 10-fold, or higher), to take up the soluble HMW tau species than to take up the soluble LMW tau species.

Once the soluble HMW tau species is taken up by a neuron, the soluble HMW tau species can be axonally transported from the neuron to a synaptically-connected neuron. Thus, a neuron can preferentially axonally transport soluble HMW tau species from its cell body to a synaptically-connected neuron. As used herein, the term "preferentially axonally transport soluble HMW tau species from its cell body to a synaptically-connected neuron" refers to a neuron having a higher likelihood to axonally transport soluble HMW tau species from its cell body to a synaptically-connected neuron, as compared to axonal transport of soluble LMW tau species between synaptically connected neurons. A neuron can have a higher likelihood to axonally transport soluble HMW tau species from its cell body to a synaptically-connected neuron, by at least about 30% or more (including, e.g., at least about 40%, at least about 50%, at least about 60%, at least about 70%, at least about 80%, at least about 90%, at least about 95% or higher), as compared to axonal transport of soluble LMW tau species between synaptically connected neurons. A neuron can have a higher likelihood to axonally transport soluble HMW tau species from its cell body to a synaptically-connected neuron, by at least about 1.5-fold or more (including, e.g., at least about 2-fold, at least about 2.5-fold, at least about 3-fold, at least about 3.5-fold, at least about 4-fold, at least about 5-fold, at least about 10-fold, or higher), as compared to axonal transport of soluble LMW tau species between synaptically connected neurons.

The term "axonally transport" or "axonal transport" is used herein to refer to directed transport of molecules and/or organelles along an axon of a neuron. The "axonal transport" can be "anterograde" (outward from the cell body) or "retrograde" (back toward the cell body). Thus, anterograde transport of soluble HMW tau species delivers the soluble HMW tau species taken up a neuron from its cell body outwards to distant synapses.

As used herein and throughout the specification, the term "synaptically connected" refers to neurons which are in communication with one another via a synapse. A synapse is a zone of a neuron specialized for a signal transfer. Synapses can be characterized by their ability to act as a region of signal transfer as well as by the physical proximity at the synapse between two neurons. Signaling can be by electrical or chemical means.

As used herein and throughout the specification, the term "low molecular weight tau species" or "LMW tau species" refers to a population of tau species molecules that are substantially tau monomer subunits and/or tau dimer subunits. The LMW tau species can be intracellular (e.g., inside neurons) or extracellular (e.g., in the brain interstitial fluid and/or cerebrospinal fluid). The LMW tau species can be produced by fractionating brain extracts and/or brain interstitial fluid from tau-transgenic animals (e.g., tau-transgenic mice), e.g., by centrifugation at higher speed (e.g., x 50000 g or more) and/or size exclusion chromatography as described in the Examples, and selecting the fraction(s) with a molecular weight of no more than 200 kDa, or less. The soluble LMW tau species can have a molecular weight of no more than 200 kDa, or less, including, e.g., no more than 150 kDa, no more than 100 kDa, no more than 50 kDa, or less.

The compositions described herein can comprise an agent to suit the need of a selected application. For example, the compositions described herein can be adapted to raise an antibody against the soluble HMW tau species. Accordingly, the composition can further comprise an adjuvant for raising an antibody against the soluble HMW tau species. The term "adjuvant," as used herein, refers to molecule(s), compound(s), and/or material(s) which, when administered to an individual or an animal (e.g., mice) in vitro, increase(s) the immune response of the individual or the animal to an antigen (e.g., soluble HMW tau species) administered. Some antigens are weakly immunogenic when administered alone or are toxic to the individual at concentrations which evoke immune responses in the individual or animal. An adjuvant can enhance the immune response of the individual or animal to the antigen by making the antigen more strongly immunogenic, thus enhancing antibody production. The adjuvant effect can also lower the dose of the antigen necessary to achieve an immune response in the individual or animal. Commonly used adjuvants include, but are not limited to, Incomplete Freund's Adjuvant, which consists of a water in oil emulsion, Freund's Complete Adjuvant, which comprises the components of Incomplete Freund's Adjuvant, with the addition of Mycobacterium tuberculosis, and alum. The HMW tau compositions described herein can be conjugated to an adjuvant. Conjugation to an antigenic carrier such as keyhole limpet hemocyanin can also be used to increase the antigenicity of the HMW tau complexes described herein. Other antigenic carrier proteins that can be conjugated to HMW tau include, e.g., Concholepas concholepas hemocyanin ("Blue Carrier"), bovine serum albumin, cationized bovine serum, and ovalbumin. It is further contemplated that treatments that stabilize the HMW tau complexes in the HMW form that is preferentially transmitted from neuron to neuron will render the HMW tau more likely to serve as an antigen to raise antibodies specific for the HMW as opposed to either the HMW or LMW forms of the tau protein. Various approaches can be used to effect stabilization of the HMW tau structures. For example, HMW tau can be stabilized in the HMW/synaptically transmissible form of tau by cross linking isolated HMW tau. A number of chemical cross-linking reagents are known and available commercially, including homobifunctional and heterobifunctional cross linkers that react, e.g., with amines (e.g., N-hydrosuccinimide (NHS) ester cross linkers, including disuccinimidyl glutarate, disuccinimidyl suberate, bis[sulfosuccinimidyl] suberate, dithiobis[succinimidyl] propionate, among others, imidoester including dimethyl adipimidate-2HCl, dimethyl suberimidate, etc.) or with sulfhydryls (e.g., maleimide-based cross linkers, e.g., bismaleimidoethane, bismaleimidohexane, dithiobismaleimidoethane, etc.) The cross-linkers can be modulated by tailoring reaction conditions as known in the art. A relatively small degree of cross-linking can provide substantial stabilization relative to non-cross linked HMW, and thereby provide an enhanced activity as, e.g., as an antigen or as a target for screening assays. HMW tau can also be stabilized, e.g., by interacting with a surface, e.g., plastic, nitrocellulose, or nylon membrane. In this form, stabilized HMW tau can serve as a substrate or target for screening for agents that specifically bind the HMW complex form of the tau protein. As but one example, a surface funtionalized with HMW tau can be used to pan for, e.g., bacteriophage displaying a binding polypeptide. Libraries of bacteriophage display constructs are well-known in the art. One can enhance the likelihood of obtaining a phage-displayed HMW tau binding protein that does not substantially also bind LMW tau by first contacting the phage library with LMW tau immobilized on a surface to subtract out those library members including LMW-tau binding polypeptides. After subtraction in this manner, the library is then contacted with HMW tau immobilized on a separate surface, followed by isolation and propagation of those phages that stick to the HMW tau. Solid supports/surfaces can include, without limitation, nitrocellulose or nylon membranes, affinity column chromatography matrices, nylon or other polymer beads, among others.

### Anti-soluble HMW tau species antagonist agents or antagonists of soluble HMW tau species

Unlike insoluble neurofibrillary tangles as intracellular proteins in neurons, the soluble HMW tau species described herein is a novel soluble, non-fibrillar tau aggregate, which can be found in the extracellular space, e.g., soluble in brain interstitial fluid and/or cerebrospinal fluid. As described earlier, the inventors have shown that the soluble HMW tau species are preferentially taken up by neurons and axonally transported to synaptically-connected neurons, thus progressing tau spreading between neurons. By reducing or blocking neuron uptake of the soluble HMW tau species described herein, tau spreading can be prevented. Accordingly, described herein, are compositions comprising soluble HMW tau species antagonist agents, such as antibodies or antigen-binding fragments thereof, nucleic acids, and small molecules, for inhibiting or reducing soluble HMW tau species being taken up by a neuron and/or axonally transported from the neuron to a synaptically-connected neuron, and methods of use thereof for inhibition or reduction of neuron uptake of soluble HMW tau species and pathologies associated with tau propagation.

As used interchangeably herein, the terms "tau propagation" and "tau spreading" refers to transport of misfolded tau protein between neurons.

As used herein, a "soluble HMW tau species antagonist agent" or "an antagonist of soluble HMW tau species" refer to an agent, such as a small molecule, inhibitory nucleic acid, or soluble HMW tau species-specific antibody or antigen-binding fragment thereof, that inhibits or causes or facilitates a qualitative or quantitative inhibition, decrease, or reduction in one or more processes, mechanisms, effects, responses, functions, activities or pathways mediated by soluble HMW tau species. Thus, the term "soluble HMW tau species antagonist agent" refers to an agent that inhibits formation of the soluble HMW tau species, or one that binds to, partially or totally blocks, decreases, or prevents neuron uptake of soluble HMW tau species, e.g., by at least about 10% or more (including, e.g., at least about 20%, at least about 30%, at least about 40%, at least about 50%, at least about 60%, at least about 70%, at least about 80%, at least about 90% or more, up to 100%) and/or blocks, decreases, or prevents inter-neuron propagation of the soluble HMW tau species upon the neuron uptake, e.g., by at least about 10% or more (including, e.g., at least about 20%, at least about 30%, at least about 40%, at least about 50%, at least about 60%, at least about 70%, at least about 80%, at least about 90% or more, up to 100%).

In some examples of these aspects and all such aspects described herein, soluble HMW tau species antagonist agents do not bind soluble low molecular weight (LMW) tau species. For example, in some examples, soluble HMW tau species antagonist agents do not bind soluble LMW tau species that have a molecular weight of no more than 200 kDa, including, e.g., no more than 150 kDa, no more than 100 kDa, or lower.

The term "agent" as used herein in reference to a soluble HMW tau species antagonist means any compound or substance such as, but not limited to, a small molecule, nucleic acid, polypeptide, peptide, drug, ion, etc. An "agent" can be any chemical, entity, or moiety, including, without limitation, synthetic and naturally-occurring proteinaceous and non-proteinaceous entities. Suitably, an agent is a nucleic acid, a nucleic acid analogue, a protein, an antibody, a peptide, an aptamer, an oligomer of nucleic acids, an amino acid, or a carbohydrate, and includes, without limitation, proteins, oligonucleotides, ribozymes, DNAzymes, glycoproteins, siRNAs, lipoproteins, aptamers, and modifications and combinations thereof etc. Suitably, agents are small molecules having a chemical moiety. For example, chemical moieties include unsubstituted or substituted alkyl, aromatic, or heterocyclyl moieties. Compounds can be known to have a desired activity and/or property, e.g., inhibit neuron uptake of soluble HMW tau species and/or optional inter-neuron propagation of the soluble HMW tau species, or can be selected from a library of diverse compounds, using, for example, screening methods.

The soluble HMW tau species antagonist agents can specifically bind, and reduce or inhibit neuron uptake of, non-fibrillar, soluble HMW tau species described herein, for example, with a molecular weight of at least about 500 kDa. The soluble HMW tau species can have a molecular weight of at least about 669 kDa or more. The soluble HMW tau species can have a molecular weight of about 669 kDa to about 1000 kDa. The non-fibrillar soluble HMW tau species can be in a form of particles. The particle size can vary with the molecular weight of the tau species. The particle size can range from about 10 nm to about 30 nm.

The soluble HMW tau species antagonist agents can specifically bind, and reduce or inhibit neuron uptake of, at least one or more phosphorylated forms of the soluble HMW tau species described herein.

The soluble HMW tau species antagonist agents can specifically bind, and reduce or inhibit neuron uptake of, the HMW tau species described herein soluble in an aqueous solution and/or a buffered solution. For example, the soluble HMW tau species antagonist agents can specifically bind, and reduce or inhibit neuron uptake of, the HMW tau species described herein soluble in phosphate-buffered saline. The soluble HMW tau species antagonist agents can specifically bind, and reduce or inhibit neuron uptake of, the HMW tau species described herein soluble in a biological fluid, e.g., a brain interstitial fluid or cerebrospinal fluid.

***Soluble HMW tau species antagonist antibodies and antigen-binding fragments thereof*:** Also described herein, are compositions comprising soluble HMW tau species antagonist antibodies that specifically bind soluble HMW tau species and does not bind soluble low molecular weight (LMW) tau species.

Soluble HMW tau species antibody antagonists for use in the composition and methods described herein include complete immunoglobulins, antigen binding fragments of immunoglobulins, as well as antigen-binding fragments that comprise antigen binding domains of immunoglobulins. As used herein, "antigen-binding fragments" of immunoglobulins include, for example, Fab, Fab', F(ab')2, scFv and dAbs. Modified antibody formats have been developed which retain binding specificity, but have other characteristics that can be desirable, including for example, bispecificity, multivalence (more than two binding sites), and compact size (*e.g.*, binding domains alone).

As antibodies can be modified in a number of ways, the term "antibody" should be construed as covering any specific binding member or substance having an antibody binding domain with the required specificity for soluble HMW tau species. Thus, this term covers antibody fragments, derivatives, functional equivalents and homologues of antibodies, including any polypeptide comprising an HMW tau-specific immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023 and U.S. Patent Nos. 4,816,397 and 4,816,567.

Accordingly, in some aspects, provided herein are soluble HMW tau species antagonist antibodies or antibody fragments thereof that are specific for soluble HMW tau species, wherein the soluble HMW tau species antagonist antibodies or antibody fragments thereof specifically binds to soluble HMW tau species and reduces or inhibits the biological activity of soluble HMW tau species, e.g., being taken up by neuron(s) and/or inducing inter-neuron propagation. Suitably, soluble HMW tau species is human soluble HMW tau species.

As used herein, a "soluble HMW tau species antibody" is an antibody that binds to soluble HMW tau species with sufficient affinity and specificity that does not bind substantially soluble LMW tau species. The antibody selected will normally have a binding affinity for soluble HMW tau species, for example, the antibody can bind human soluble HMW tau species with a K_{D} value between 10⁻⁵ M to 10⁻¹⁰M or lower. Antibody affinities can be determined, for example, by a surface plasmon resonance based assay (such as the BIAcore assay described in PCT Application Publication No. WO2005/012359); enzyme-linked immunoabsorbent assay (ELISA); and competition assays (*e.g.* RIA's). An HMW tau-specific antibody as described herein will bind soluble HMW tau protein with a K_{D} at least 100-fold lower than its K_{D} for soluble LMW tau protein, preferably at least 10³ -fold lower, 10⁴ - fold lower or even 10⁵ fold lower. Relative affinities can also be evaluated, e.g. by competition assays.

In certain aspects described herein, a soluble HMW tau species antibody can be used as a therapeutic agent in targeting and interfering with diseases or conditions where soluble HMW tau species activity is involved. Also, a soluble HMW tau species antibody can be subjected to other biological activity assays, *e.g.,* in order to evaluate its effectiveness as a therapeutic, or its effectiveness as a diagnostic aid, *etc.* Such assays are known in the art and depend on the target antigen and intended use for the antibody. Examples include measurements of soluble HMW tau species being taken up by neuron(s) as described in Example 1; antibody-dependent cellular cytotoxicity (ADCC) and complement-mediated cytotoxicity (CDC) assays (U.S. Pat. No. 5,500,362); and agonistic activity or hematopoiesis assays (see WO 95/27062). Other biological activity assays that can be used to assess a soluble HMW tau species antibody are described in the Examples section such as measuring inter-neuron propagation of soluble HMW tau species using a 3-chamber microfluidic device.

As used herein, a "blocking" antibody or an antibody "antagonist" is one which inhibits or reduces biological activity of the antigen it binds. In this context, "reduces" refers to at least a 50% reduction in the relevant biological activity (e.g., interneuron transmission of HMW tau), e.g., at least 60%, at least 70%, at least 80%, at least 90% or more. For example, a soluble HMW tau species antagonist antibody binds soluble HMW tau species and inhibits the ability of soluble HMW tau species to, for example, to be taken up by neuron(s) and/or to get involved in inter-neuron propagation. While 100% inhibition is not necessarily required to achieve a therapeutic benefit, blocking antibodies or antagonist antibodies completely inhibit the biological activity of soluble HMW tau species described herein.

Thus, soluble HMW tau species antibodies or antibody fragments thereof that are useful in the compositions and methods described herein include any antibodies or antibody fragments thereof that bind with sufficient affinity and specificity to soluble HMW tau species, *i.e.,* are specific for soluble HMW tau species, and can reduce or inhibit the biological activity of soluble HMW tau species, specifically ability of soluble HMW tau species being taken up by neuron(s) and/or inducing inter-neuron propagation.

As described herein, an "antigen" is a molecule that is bound by a hypervariable region binding site of an antibody or antigen-binding fragment thereof. Typically, antigens are bound by antibody ligands and are capable of raising an antibody response *in vivo.* An antigen can be a polypeptide, protein, nucleic acid or other molecule. In the case of conventional antibodies and fragments thereof, the antigen binding site as defined by the hypervariable loops (L1, L2, L3 and HI, H2, H3) is capable of binding to the antigen.

As used herein, an "epitope" can be formed both from contiguous amino acids, or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, and more usually, at least 5, about 9, or about 8-10 amino acids in a unique spatial conformation. An "epitope" includes the unit of structure conventionally bound by an immunoglobulin V_{H}/N_{L} pair. Epitopes define the minimum binding site for an antibody, and thus represent the target of specificity of an antibody. In the case of a single domain antibody, an epitope represents the unit of structure bound by a variable domain in isolation. The terms "antigenic determinant" and "epitope" can also be used interchangeably herein.

In some examples of the aspects described herein, a soluble HMW tau species antagonist antibody is a monoclonal antibody. The term "monoclonal antibody" as used herein refers to an antibody obtained from a population of substantially homogeneous antibodies, *i.e.,* the individual antibodies comprising the population are identical except for possible naturally occurring mutations that can be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The modifier "monoclonal" is not to be construed as requiring production of the antibody by any particular method. For example, the monoclonal antibodies to be used in accordance with various aspects described herein can be made by the hybridoma method first described by Kohler et al., Nature 256:495 (1975), or can be made by recombinant DNA methods (see, *e.g.,* U.S. Pat. No. 4,816,567). The "monoclonal antibodies" can also be isolated from phage antibody libraries using the techniques described in Clackson et al., Nature 352:624-628 (1991) or Marks et al., J. Mol. Biol. 222:581-597 (1991), for example.

The soluble HMW tau species antagonist monoclonal antibodies described herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)).

"Humanized" forms of non-human (*e.g.,* murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies can comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

As used herein, a "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. The human antibody is selected from a phage library, where that phage library expresses human antibodies (Vaughan et al. Nature Biotechnology 14:309-314 (1996): Sheets et al. Proc. Natl. Acad. Sci. 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, *e.g.,* mice in which the endogenous mouse immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody can be prepared via immortalization of human B lymphocytes producing an antibody directed against a target antigen (such B lymphocytes can be recovered from an individual or can have been immunized *in vitro*). See, *e.g.,* Cole et al., Monoclonal Antibodies and Cancer Therapy, Alan R. Liss, p. 77 (1985); Boerner et al., J. Immunol., 147 (1):86-95 (1991); and U.S. Pat. No. 5,750,373.

In other examples of these aspects, the soluble HMW tau species antagonist antibody is a soluble HMW tau species-specific antibody fragment. The term "antibody fragment," as used herein, refer to a protein fragment that comprises only a portion of an intact antibody, generally including an antigen binding site of the intact antibody and thus retaining the ability to bind antigen. Examples of antibody fragments encompassed by the present definition include: (i) the Fab fragment, having V_{L}, C_{L}, V_{H} and C_{H}1 domains; (ii) the Fab' fragment, which is a Fab fragment having one or more cysteine residues at the C-terminus of the C_{H}1 domain; (iii) the Fd fragment having V_{H} and C_{H}1 domains; (iv) the Fd' fragment having V_{H} and C_{H}1 domains and one or more cysteine residues at the C-terminus of the CH1 domain; (v) the Fv fragment having the V_{L} and V_{H} domains of a single arm of an antibody; (vi) the dAb fragment (Ward et al., Nature 341, 544-546 (1989)) which consists of a V_{H} domain; (vii) isolated CDR regions; (viii) F(ab')₂ fragments, a bivalent fragment including two Fab' fragments linked by a disulfide bridge at the hinge region; (ix) single chain antibody molecules (*e.g.,* single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (V_{H}) connected to a light chain variable domain (V_{L}) in the same polypeptide chain (see, *e.g.,* EP 404,097; WO 93/11161; and Hollinger et al., Proc. Natl. Acad. Sci. USA, 90:6444-6448 (1993)); (xi) "linear antibodies" comprising a pair of tandem Fd segments (V_{H}-C_{H}1-V_{H}-C_{H}1) which, together with complementary light chain polypeptides, form a pair of antigen binding regions (Zapata et al. Protein Eng. 8(10):1057-1062 (1995); and U.S. Pat. No. 5,641,870).

Accordingly, the soluble HMW tau species antagonist antibody fragment may suitably be a Fab fragment comprising V_{L}, C_{L}, V_{H} and C_{H}1 domains. The soluble HMW tau species antagonist antibody fragment may suitably be a Fab' fragment, which is a Fab fragment having one or more cysteine residues at the C-terminus of the C_{H}1 domain. The soluble HMW tau species antagonist antibody fragment may suitably be a Fd fragment comprising V_{H} and C_{H}1 domains. The soluble HMW tau species antagonist antibody may suitably be a Fd' fragment comprising V_{H} and C_{H}1 domains and one or more cysteine residues at the C-terminus of the C_{H}1 domain. The soluble HMW tau species antagonist antibody fragment may suitably be a Fv fragment comprising the V_{L} and V_{H} domains of a single arm of an antibody. The soluble HMW tau species antagonist antibody fragment may suitably be a dAb fragment comprising a V_{H} domain. The soluble HMW tau species antagonist antibody fragment may suitably comprise isolated CDR regions. The human soluble HMW tau species antagonist antibody fragment may suitably be a F(ab')₂ fragment, which comprises a bivalent fragment comprising two Fab' fragments linked by a disulfide bridge at the hinge region. The soluble HMW tau species antagonist antibody fragment may suitably be a single chain antibody molecule, such as a single chain Fv. The soluble HMW tau species antagonist antibody fragment may suitably be a diabody comprising two antigen binding sites, comprising a heavy chain variable domain (V_{H}) connected to a light chain variable domain (V_{L}) in the same polypeptide chain. The soluble HMW tau species antagonist antibody fragment may suitably be a linear antibody comprising a pair of tandem Fd segments (V_{H}-C_{H}1-V_{H}-C_{H}1) which, together with complementary light chain polypeptides, form a pair of antigen binding regions.

Antibodies to soluble HMW tau species can be raised by one of skill in the art using well known methods. Antibodies are readily raised in animals such as rabbits or mice by immunization with an antigen (e.g., soluble HMW tau species) or a fragment thereof. Immunized mice are particularly useful for providing sources of B cells for the manufacture of hybridomas, which in turn are cultured to produce large quantities of monoclonal antibodies. Antibody manufacture methods are described in detail, for example, in Harlow et al., Eds., Antibodies: A Laboratory Manual, Cold Spring Harbor Laboratory, New York (1988), which is hereby incorporated by reference in its entirety. Both polyclonal and monoclonal antagonistic antibody of soluble HMW tau species can be used in the methods described herein. Suitably, a monoclonal antagonistic antibody of soluble HMW tau species is used where conditions require increased specificity for a particular protein.

*Other Antibody Modifications.* In some examples of these aspects, amino acid sequence modification(s) of the antibodies or antibody fragments thereof specific for soluble HMW tau species described herein are contemplated. For example, it can be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid changes also can alter post-translational processes of the antibody, such as changing the number or position of glycosylation sites.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include antibody with an N-terminal methionyl residue or the antibody fused to a cytotoxic polypeptide. Other insertional variants of the antibody molecule include the fusion to the N- or C-terminus of the antibody to an enzyme (*e.g.* for antibody-directed enzyme prodrug therapy (ADEPT)) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated for use in the soluble HMW tau species antagonist antibodies or antibody fragments thereof described herein.

Substantial modifications in the biological properties of the antibodies or antibody fragments thereof specific for soluble HMW tau species are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids can be grouped according to similarities in the properties of their side chains (in A. L. Lehninger, in Biochemistry, second ed., pp. 73-75, Worth Publishers, New York (1975)): (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); (4) basic: Lys (K), Arg (R), His (H).

Alternatively, naturally occurring residues can be divided into groups based on common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; (6) aromatic: Trp, Tyr, Phe. Non-conservative substitutions will entail exchanging a member of one of these classes for another class.

Any cysteine residue not involved in maintaining the proper conformation of the soluble HMW tau species antibodies or antibody fragments thereof can be substituted, generally with serine, to improve the oxidative stability of the molecule and prevent aberrant crosslinking Conversely, cysteine bond(s) can be added to the antibody to improve its stability (particularly where the antibody is an antibody fragment such as an Fv fragment).

A particularly preferred type of substitutional variant involves substituting one or more hypervariable region residues of a parent antibody. Generally, the resulting variant(s) selected for further development will have improved biological properties relative to the parent antibody from which they are generated. A convenient way for generating such substitutional variants involves affinity maturation using phage display.

Another type of amino acid variant of the antibody alters the original glycosylation pattern of the antibody. By altering is meant deleting one or more carbohydrate moieties found in the antibody, and/or adding one or more glycosylation sites that are not present in the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine can also be used.

Addition of glycosylation sites to the soluble HMW tau species antibodies or antibody fragments thereof is accomplished by altering the amino acid sequence such that it contains one or more of the above-described tripeptide sequences (for N-linked glycosylation sites). The alteration can also be made by the addition of, or substitution by, one or more serine or threonine residues to the sequence of the original antibody (for O-linked glycosylation sites).

Where the antibody comprises an Fc region, the carbohydrate attached thereto can be altered. For example, antibodies with a mature carbohydrate structure that lacks fucose attached to an Fc region of the antibody are described in US Pat Appl No US 2003/0157108 A1, Presta, L. See also US 2004/0093621 A1 (Kyowa Hakko Kogyo Co., Ltd). Antibodies with a bisecting N-acetylglucosamine (GlcNAc) in the carbohydrate attached to an Fc region of the antibody are referenced in WO03/011878, Jean-Mairet et al. and U.S. Pat. No. 6,602,684, Umana et al. Antibodies with at least one galactose residue in the oligosaccharide attached to an Fc region of the antibody are reported in WO97/30087, Patel et al. See, also, WO98/58964 (Raju, S.) and WO99/22764 (Raju, S.) concerning antibodies with altered carbohydrate attached to the Fc region thereof.

To increase the serum half-life of soluble HMW tau species antibodies described herein, one can incorporate a salvage receptor binding epitope into the antibody (especially an antibody fragment) as described in U.S. Pat. No. 5,739,277, for example. As used herein, the term "salvage receptor binding epitope" refers to an epitope of the Fc region of an IgG molecule (*e.g.,* IgG1, IgG2, IgG3, or IgG4) that is responsible for increasing the *in vivo* serum half-life of the IgG molecule.

Antibodies with improved binding to the neonatal Fc receptor (FcRn), and increased half-lives, are described in WO00/42072 (Presta, L.) and US2005/0014934A1 (Hinton et al.). These antibodies comprise an Fc region with one or more substitutions therein which improve binding of the Fc region to FcRn.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

The soluble HMW tau species antibodies and antibody fragments thereof described herein can also be formulated as immunoliposomes. Liposomes containing the antibody are prepared by methods known in the art, such as described in Epstein et al., Proc. Natl. Acad. Sci. USA, 82:3688 (1985); Hwang et al., Proc. Natl. Acad. Sci. USA, 77:4030 (1980); and U.S. Pat. Nos. 4,485,045 and 4,544,545. Liposomes with enhanced circulation time are disclosed in U.S. Pat. No. 5,013,556.

Particularly useful liposomes can be generated, for example, by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the soluble HMW tau species antibodies described herein can be conjugated to the liposomes as described in Martin et al. J. Biol. Chem. 257: 286-288 (1982) via a disulfide interchange reaction. A therapeutic agent, e.g., for treatment of tauopathy is optionally contained within the liposome. See Gabizon et al. J. National Cancer Inst. 81(19)1484 (1989).

***Nucleic acid inhibitors of soluble HMW tau species.*** In some examples of the compositions and methods described herein, a soluble HMW tau species antagonist agent is an RNA interference agent that specifically targets (microtubule-associated protein tau (MAPT) and can be used for the inhibition of expression of MAPT in vivo. RNA interference (RNAi) uses small interfering RNA (siRNA) duplexes that target the messenger RNA encoding a target polypeptide for selective degradation and is a powerful approach for inhibiting the expression of selected target polypeptides. siRNA-dependent post-transcriptional silencing of gene expression involves cleaving the target messenger RNA molecule at a site guided by the siRNA. "RNA interference (RNAi)," as used herein, refers to the evolutionally conserved process whereby the expression or introduction of RNA of a sequence that is identical or highly similar to a target gene results in the sequence specific degradation or specific post-transcriptional gene silencing (PTGS) of messenger RNA (mRNA) transcribed from that targeted gene (see Coburn, G. and Cullen, B. (2002) J. of Virology 76(18):9225), thereby inhibiting expression of the target gene. Suitably, the RNA interference agent or siRNA is a double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex (termed "RNA induced silencing complex," or "RISC") that recognizes and cleaves target mRNAs. RNAi can also be initiated by introducing nucleic acid molecules, e.g., synthetic siRNAs or RNA interfering agents, to inhibit or silence the expression of target genes. As used herein, "inhibition of target gene expression" includes any decrease in expression or protein activity or level of the target gene or protein encoded by the target gene as compared to a situation wherein no RNA interference has been induced. The decrease will be of at least 10%, at least 20%, at least 30%, at least 40%, at least 50%, at least 60%, at least 70%, at least 80%, at least 90%, at least 95%, or at least 99% or more as compared to the expression of a target gene or the activity or level of the protein encoded by a target gene which has not been targeted by an RNA interfering agent.

As used herein, siRNAs also include small hairpin (also called stem loop) RNAs (shRNAs). Suitably, these shRNAs are composed of a short (*e.g.,* about 19 to about 25 nucleotide) antisense strand, followed by a nucleotide loop of about 5 to about 9 nucleotides, and the analogous sense strand. Alternatively, the sense strand can precede the nucleotide loop structure and the antisense strand can follow. These shRNAs can be contained in plasmids, retroviruses, and lentiviruses and expressed from, for example, the pol III U6 promoter, or another promoter (see, *e.g.* , Stewart, et al. (2003) RNA Apr;9(4):493-501, incorporated by reference herein in its entirety). The target gene or sequence of the RNA interfering agent can be a cellular gene or genomic sequence, *e.g.*, the human MAPT genomic sequence. An siRNA can be substantially homologous to the target gene or genomic sequence, or a fragment thereof, *i.e.,* the MAPT gene or mRNA. As used in this context, the term "homologous" is defined as being substantially identical, sufficiently complementary, or similar to the target MAPT mRNA, or a fragment thereof, to effect RNA interference of the target MAPT. In addition to native RNA molecules, RNA suitable for inhibiting or interfering with the expression of a target sequence includes RNA derivatives and analogs. Preferably, the siRNA is identical to its target. The siRNA preferably targets only one sequence.

Each of the RNA interfering agents, such as siRNAs, can be screened for potential off-target effects by, for example, expression profiling. Such methods are known to one skilled in the art and are described, for example, in Jackson et al. Nature Biotechnology 6:635-637, 2003. In addition to expression profiling, one can also screen the potential target sequences for similar sequences in the sequence databases to identify potential sequences which may have off-target effects. For example, according to Jackson et al. (Id.), 15, or perhaps as few as 11 contiguous nucleotides, of sequence identity are sufficient to direct silencing of non-targeted transcripts. Therefore, one can initially screen the proposed siRNAs to avoid potential off-target silencing using the sequence identity analysis by any known sequence comparison methods, such as BLAST. siRNA sequences are chosen to maximize the uptake of the antisense (guide) strand of the siRNA into RISC and thereby maximize the ability of RISC to target human GGT mRNA for degradation. This can be accomplished by scanning for sequences that have the lowest free energy of binding at the 5'-terminus of the antisense strand. The lower free energy leads to an enhancement of the unwinding of the 5'- end of the antisense strand of the siRNA duplex, thereby ensuring that the antisense strand will be taken up by RISC and direct the sequence-specific cleavage of the human MAPT mRNA.

siRNA molecules need not be limited to those molecules containing only RNA, but, for example, further encompasses chemically modified nucleotides and non-nucleotides, and also include molecules wherein a ribose sugar molecule is substituted for another sugar molecule or a molecule which performs a similar function. Moreover, a non-natural linkage between nucleotide residues can be used, such as a phosphorothioate linkage. The RNA strand can be derivatized with a reactive functional group of a reporter group, such as a fluorophore. Particularly useful derivatives are modified at a terminus or termini of an RNA strand, typically the 3' terminus of the sense strand. For example, the 2'-hydroxyl at the 3' terminus can be readily and selectively derivatized with a variety of groups. Other useful RNA derivatives incorporate nucleotides having modified carbohydrate moieties, such as 2'O-alkylated residues or 2'-O-methyl ribosyl derivatives and 2'-O-fluoro ribosyl derivatives. The RNA bases can also be modified. Any modified base useful for inhibiting or interfering with the expression of a target sequence may be used. For example, halogenated bases, such as 5-bromouracil and 5-iodouracil can be incorporated. The bases can also be alkylated, for example, 7-methylguanosine can be incorporated in place of a guanosine residue. Non-natural bases that yield successful inhibition can also be incorporated. The most preferred siRNA modifications include 2'-deoxy-2'-fluorouridine or locked nucleic acid (LAN) nucleotides and RNA duplexes containing either phosphodiester or varying numbers of phosphorothioate linkages. Such modifications are known to one skilled in the art and are described, for example, in Braasch et al., Biochemistry, 42: 7967-7975, 2003. Most of the useful modifications to the siRNA molecules can be introduced using chemistries established for antisense oligonucleotide technology. Preferably, the modifications involve minimal 2'-O-methyl modification, preferably excluding such modification.
Modifications also preferably exclude modifications of the free 5'-hydroxyl groups of the siRNA.

Suitably, the RNA interference agent targeting MAPT is delivered or administered in a pharmaceutically acceptable carrier. Additional carrier agents, such as liposomes, can be added to the pharmaceutically acceptable carrier. Suitably, the RNA interference agent is delivered by a vector encoding the small hairpin RNA (shRNA) in a pharmaceutically acceptable carrier to the cells in an organ of an individual. The shRNA is converted by the cells after transcription into siRNA capable of targeting MAPT.

Suitably, the vector is a regulatable vector, such as tetracycline inducible vector. Methods described, for example, in Wang et al. Proc. Natl. Acad. Sci. 100: 5103-5106, using pTet-On vectors (BD Biosciences Clontech, Palo Alto, CA) can be used. Suitably, the RNA interference agents used in the methods described herein are taken up actively by neurons in vivo following intracranial injection, *e.g.,* hydrodynamic injection, without the use of a vector, illustrating efficient in vivo delivery of the RNA interfering agents. One method to deliver the siRNAs is catheterization of the blood supply vessel of the target organ. Other strategies for delivery of the RNA interference agents, *e.g.* , the siRNAs or shRNAs used in the methods described herein, can also be employed, such as, for example, delivery by a vector, *e.g.* , a plasmid or viral vector, *e.g.* , a lentiviral vector and/or adeno-associated viral (AAV) vector. Such vectors can be used as described, for example, in Xiao-Feng Qin et al. Proc. Natl. Acad. Sci. U.S.A., 100: 183-188. Other delivery methods include delivery of the RNA interfering agents, *e.g.,* the siRNAs targeting MAPT described herein, using a basic peptide by conjugating or mixing the RNA interfering agent with a basic peptide, *e.g.,* a fragment of a TAT peptide, mixing with cationic lipids or formulating into particles. The RNA interference agents, *e.g.,* the siRNAs targeting MAPT mRNA, can be delivered singularly, or in combination with other RNA interference agents, *e.g.,* siRNAs, such as, for example siRNAs directed to other cellular genes.

Synthetic siRNA molecules, including shRNA molecules, can be generated using a number of techniques known to those of skill in the art. For example, the siRNA molecule can be chemically synthesized or recombinantly produced using methods known in the art, such as using appropriately protected ribonucleoside phosphoramidites and a conventional DNA/RNA synthesizer (see, *e.g.* , Elbashir, S.M. et al. (2001) Nature 411:494-498; Elbashir, S.M., W. Lendeckel and T. Tuschl (2001) Genes & Development 15:188-200; Harborth, J. et al. (2001) J. Cell Science 114:4557-4565; Masters, J.R. et al. (2001) Proc. Natl. Acad. Sci., USA 98:8012-8017; and Tuschl, T. et al. (1999) Genes & Development 13:3191-3197). Alternatively, several commercial RNA synthesis suppliers are available including, but not limited to, Proligo (Hamburg, Germany), Dharmacon Research (Lafayette, CO, USA), Pierce Chemical (part of Perbio Science , Rockford, IL , USA), Glen Research (Sterling, VA, USA), ChemGenes (Ashland, MA, USA), and Cruachem (Glasgow, UK). As such, siRNA molecules are not overly difficult to synthesize and are readily provided in a quality suitable for RNAi. In addition, dsRNAs can be expressed as stem loop structures encoded by plasmid vectors, retroviruses and lentiviruses (Paddison, P.J. et al. (2002) Genes Dev. 16:948-958; McManus, M.T. et al. (2002) RNA 8:842-850; Paul, C.P. et al. (2002) Nat. Biotechnol. 20:505-508; Miyagishi, M. et al. (2002) Nat. Biotechnol. 20:497-500; Sui, G. et al. (2002) Proc. Natl. Acad. Sci., USA 99:5515-5520; Brummelkamp, T. et al. (2002) Cancer Cell 2:243; Lee, N.S., et al. (2002) Nat. Biotechnol. 20:500-505; Yu, J.Y., et al. (2002) Proc. Natl. Acad. Sci., USA 99:6047-6052; Zeng, Y., et al. (2002) Mol. Cell 9:1327-1333; Rubinson, D.A., et al. (2003) Nat. Genet. 33:401-406; Stewart, S.A., et al. (2003) RNA 9:493-501). These vectors generally have a polIII promoter upstream of the dsRNA and can express sense and antisense RNA strands separately and/or as a hairpin structures. Within cells, Dicer processes the short hairpin RNA (shRNA) into effective siRNA.

The targeted region of the siRNA molecule for use in the compositions and methods described herein can be selected from a given target gene sequence, *e.g.,* a MAPT coding sequence, beginning from about 25 to 50 nucleotides, from about 50 to 75 nucleotides, or from about 75 to 100 nucleotides downstream of the start codon. Nucleotide sequences can contain 5' or 3' UTRs and regions nearby the start codon. Analysis of sequence databases, including but not limited to the NCBI, BLAST, Derwent and GenSeq as well as commercially available oligosynthesis companies such as OLIGOENGINE®, can also be used to select siRNA sequences against EST libraries to ensure that only one gene is targeted.

*Delivery of RNA interfering agents.* Methods of delivering RNA interference agents, *e.g.,* an siRNA, or vectors containing an RNA interference agent, to the target cells, *e.g.,* lymphocytes or other desired target cells, for uptake include injection of a composition containing the RNA interference agent, *e.g.,* an siRNA targeting MAPT, or directly contacting the cell, *e.g.,* a lymphocyte, with a composition comprising an RNA interference agent, *e.g.,* an siRNA targeting MAPT. Alternatively, an RNA interference agent, *e.g.,* an siRNA targeting MAPT, can be injected directly into any neuron or the brain of a subject, via, *e.g.,* hydrodynamic injection or catheterization. Administration can be by a single injection or by two or more injections. The RNA interference agent is delivered in a pharmaceutically acceptable carrier. One or more RNA interference agents can be used simultaneously.

Suitably, specific cells are targeted with RNA interference, limiting potential side effects of RNA interference caused by non-specific targeting of RNA interference. The method can use, for example, a complex or a fusion molecule comprising a cell targeting moiety and an RNA interference binding moiety that is used to deliver RNA interference effectively into cells. For example, an antibody-protamine fusion protein when mixed with siRNA, binds siRNA and selectively delivers the siRNA into cells expressing an antigen recognized by the antibody, resulting in silencing of gene expression only in those cells that express the antigen. The siRNA or RNA interference-inducing molecule binding moiety is a protein or a nucleic acid binding domain or fragment of a protein, and the binding moiety is fused to a portion of the targeting moiety. The location of the targeting moiety can be either in the carboxyl-terminal or amino-terminal end of the construct or in the middle of the fusion protein. A viral-mediated delivery mechanism can also be employed to deliver siRNAs to cells *in vitro* and in vivo as described in Xia, H. et al. (2002) Nat Biotechnol 20(10): 1006). Plasmid- or viral-mediated delivery mechanisms of shRNA can also be employed to deliver shRNAs to cells *in vitro* and in vivo as described in Rubinson, D.A., et al. ((2003) Nat. Genet. 33:401-406) and Stewart, S.A., et al. ((2003) RNA 9:493-501). The RNA interference agents targeting MAPT, *e.g.,* the siRNAs or shRNAs, can be introduced along with components that perform one or more of the following activities: enhance uptake of the RNA interfering agents, *e.g.,* siRNA, by neurons; inhibit annealing of single strands; stabilize single strands; or otherwise facilitate delivery to the target neuron and increase inhibition of the target MAPT. The dose of the particular RNA interfering agent will be in an amount necessary to effect RNA interference, *e.g.* , post translational gene silencing (PTGS), of the particular target gene, thereby leading to inhibition of target gene expression or inhibition of activity or level of the protein encoded by the target gene.

***Small molecule inhibitors of soluble HMW tau species.*** In some examples of the compositions and methods described herein, a soluble HMW tau species antagonist agent is a small molecule antagonist or agent that specifically targets soluble HMW tau species and can be used for the inhibition of soluble HMW tau species being taken up by neuron(s) and/or inducing inter-neuron propagation.

As used herein, the term "small molecule" refers to a chemical agent which can include, but is not limited to, a peptide, a peptidomimetic, an amino acid, an amino acid analog, a polynucleotide, a polynucleotide analog, an aptamer, a nucleotide, a nucleotide analog, an organic or inorganic compound (*e.g.,* including heterorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Suitably, a small molecule antagonist of soluble HMW tau species selectively binds to soluble HMW tau species, and does not substantially bind soluble low molecular weight (LMW) tau species. As used herein, "selectively binds" or "specifically binds" refers to the ability of a soluble HMW tau species antagonist described herein to bind to the soluble HMW tau species polypeptide, with a K_{D} 10⁻⁵ M (10000 nM) or less, *e.g.,* 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less. For example, if an antagonist (small molecule, antibody or other) described herein binds to the soluble HMW tau species polypeptide with a K_{D} of 10⁻⁵ M or lower, but not substantially to other molecules, or a related homologue, then the agent is said to specifically bind the soluble HMW tau species polypeptide. An agent that specifically binds tau, whether HMW or LMW, is contemplated for its effects on blocking propagation of tau pathology. However, it is preferred, as described herein, that the agent or antagonist (whether small molecule, antibody or other) binds to HMW tau and not substantially to LMW tau. By "not substantially" is meant that the K_{D} for HMW tau, as determined, e.g., by competition ally or by other means known in the art, is at least 10² -fold lower than that for LMW tau, and preferably at least 10³-fold lower, at least 10⁴-fold lower, 10⁵-fold lower or less. Specific binding can be influenced by, for example, the affinity and avidity of the antagonist and the concentration of the antagonist used. A person of ordinary skill in the art can determine appropriate conditions under which the antagonists described herein selectively bind using any suitable methods, such as titration of a soluble HMW tau species antagonist in a suitable assay measuring neuron uptake of soluble HMW tau species, such as those described herein in the Examples.

***MAPT-specific nucleases:*** In some examples of the compositions and methods described herein, a soluble HMW tau species antagonist agent is a nuclease that specifically targets MAPT gene and can be used for the inhibition of soluble HMW tau species being taken up by neuron(s) and/or inducing inter-neuron propagation.

As used herein, the term "nuclease" refers to an agent that induces a break in a nucleic acid sequence, e.g., a single or a double strand break in a double-stranded DNA sequence. Nucleases include those which bind a preselected or specific sequence and cut at or near the preselected or specific sequence, e.g., engineered zinc finger nucleases (ZFNs) and engineered TAL effector nucleases. Nucleases are not limited to ZFNs and TAL (transcription activator-like) effector nuclease, but can be any nuclease suitable for use with a targeting vector to achieve improved targeting efficiency. Non-limiting examples include other zinc finger-based nucleases and engineered meganucleases that cut at preselected or specific sequences (e.g., MAPT).

Specifically contemplated herein are active zinc finger nuclease proteins specific for MAPT and fusion proteins, including zinc finger protein transcription factors (ZFP-TFs) or zinc finger nucleases (ZFNs), comprising these MAPT-specific zinc finger proteins. The proteins comprising MAPT-specific zinc finger proteins can be used for therapeutic purposes, including for treatment of tau-associated neurodegeneration or tauopathy. For example, zinc finger nuclease targeting of the MAPT locus in neurons can be used to disrupt or delete the MAPT sequence. Zinc finger nucleases have been used to target different genes, e.g., as described in International Patent Application Nos. WO 2010/076939, WO2010/107493, and WO2011/139336; U.S. Patent Application No. US 2011/0158957; and U.S. Patent 8,563,314 (each hereby incorporated by reference), and can be adapted to disrupt or inhibit expression and/or activity of MAPT gene.

TAL effector nucleases suitable for use in the methods of various aspects described herein include any TAL nucleases known in the art. Examples of suitable TAL nucleases, and methods for preparing suitable TAL nucleases, are disclosed, e.g., in US Patent Application No. 2011/0239315; 2011/0269234 ; 2011/0145940; 2003/0232410; 2005/0208489; 2005/0026157; 2005/0064474; 2006/0188987 ; and 2006/0063231 (each hereby incorporated by reference). Suitably, TAL effector nucleases are engineered that cut in or near a target nucleic acid sequence (e.g., MAPT) in, e.g., a genome of interest, wherein the target nucleic acid sequence is at or near a sequence to be modified by a targeting vector. TAL effector nucleases are proteins that comprise an endonuclease domain and one or more TAL effector DNA binding domains, wherein the one or more TAL effector DNA binding domains comprise a sequence that recognizes a preselected or specific nucleic acid sequence (e.g., MAPT).

CRISPR (Clustered Regularly Interspaced Short Palindromic Repeats)/Cas system can be used to induce single or double strand breaks in target nucleic acid sequences (e.g., MAPT). It is based on an adaptive defense mechanism evolved by bacteria and archaea to protect them from invading viruses and plasmids, which relies on small RNAs for sequence-specific detection and silencing of foreign nucleic acids. Methods of using CRISPR/Cas system for gene editing and/or altering expression of gene products are known in the art, e.g., as described in U.S. Patent No. 8697359, and in International Patent Application Nos. WO 2014/131833 and WO 2013/176772 (each incorporated herein by reference), and can be adapted to disrupt and/or inhibit expression level and/or activity of MAPT gene.

### Methods of treatment based on selective reduction in the extracellular level of soluble HMW tau species

The inventors have shown that a relatively low level of soluble HMW tau species was released from neurons and found in brain interstitial fluid, and that the soluble HMW tau species, which accounted for only a small fraction of all tau in the samples, was robustly taken up by neurons and was involved in inter-neuron propagation, whereas uptake of soluble LMW tau species (e.g., monomer/dimer size) tau was very inefficient. Thus, a method of preventing propagation of pathological tau protein between synaptically-connected neurons is also provided herein. The method comprises selectively reducing the extracellular level of soluble HMW tau species described herein in contact with a synaptically-connected neuron. A reduced extracellular level of the soluble HMW tau species results in reduced neuron uptake of the soluble HMW tau species, thereby reducing propagation of pathological tau protein between synaptically-connected neurons.

As used herein in this aspect and other aspect described herein, the term "selectively reducing" means a greater ability to reduce extracellular level of soluble HMW tau species described herein than to reduce extracellular level of soluble LMW tau species described herein. Suitably, "selectively reducing" refers to reducing at least about 30% or more (including, e.g., at least about 40%, at least about 50%, at least about 60%, at least about 70%, at least about 80%, at least about 90%, at least 95% or more) of the extracellular level of soluble HMW tau species, while the extracellular level of soluble LMW tau species is reduced by no more than 30% or less (including, e.g., no more than 20%, no more than 10%, no more than 9%, no more than 8%, no more than 6%, no more than 5%, no more than 4%, no more than 2%, no more than 1% or lower). Suitably, "selectively reducing" refers to reducing at least about 30% or more (including, e.g., at least about 40%, at least about 50%, at least about 60%, at least about 70%, at least about 80%, at least about 90%, at least 95% or more) of the extracellular level of soluble HMW tau species, while the extracellular level of soluble LMW tau species is not substantially reduced during the selective reduction. For example, no more than 10% or less (including, e.g., no more than 9%, no more than 8%, no more than 6%, no more than 5%, no more than 4%, no more than 2%, no more than 1% or lower) of the extracellular level of soluble LMW tau species is reduced during the selective reduction.

As used herein, the term "extracellular level" refers to the level of a soluble molecule (e.g., HMW tau species or LMW tau species) outside of a neuron. Depending on the context of each application, the extracellular level may suitably refer to the level in a cell culture medium. The extracellular level may suitably refer to the level in brain interstitial fluid. The extracellular level may suitably refer to the level in cerebrospinal fluid.

The extracellular level of the soluble HMW tau species can be selectively reduced to a concentration of no more than 250 ng/mL, no more than 200 ng/mL, no more than 150 ng/mL, no more than 100 ng/mL, no more than 75 ng/mL, no more than 50 ng/mL, no more than 25 ng/mL, no more than 20 ng/mL, no more than 10 ng/mL, no more than 5 ng/mL, no more than 1 ng/mL or lower.

Methods for selectively reducing the extracellular level of soluble HMW tau species can be based on physical removal and/or molecular interactions between the soluble HMW tau species and an anti-soluble HMW tau species antagonist described herein. The soluble HMW tau species can be selectively reduced by microdialysis. The term "microdialysis" as used herein and throughout the specification generally denotes a method of collecting a molecule or substance of interest from a microenvironment to be analyzed, e.g., from a human or animal tissue or fluid, to a collector device (e.g., an interior part of a micro-dialysis probe) through a semi-permeable membrane or a selectively-permeable membrane. For example, a molecule or substance of interest diffuses through the membrane and collected by a perfusion fluid flowing in an interior part of a micro-dialysis probe.

The soluble HMW tau species can be selectively reduced by contacting the extracellular space or fluid in contact with the synaptically-connected neurons with at least one or more antagonist of the soluble HMW tau species, e.g., as described in the section "*Anti-soluble HMW tau species antagonist agents or antagonists of soluble HMW tau species"* herein. Examples of an antagonist of the soluble HMW tau species include, without limitations, an antibody, a nuclease (e.g., but not limited to, a zinc finger nuclease (ZFN), transcription activator-like effector nuclease (TALEN), a CRISPR/Cas system, a transcriptional repressor, a nucleic acid inhibitor (e.g., RNAi, siRNA, anti-miR, antisense oligonucleotides, ribozymes, and a combination of two or more thereof), a small molecule, an aptamer, and a combination of two or more thereof. Where the contact is in vitro, the soluble HMW tau species can be selectively reduced by adding at least one or more antagonist of the soluble HMW tau species described herein into the cell culture medium in which synaptically-connected neurons are cultured. Where the contact is in vivo, the soluble HMW tau species can be selectively reduced by introducing at least one or more antagonist of the soluble HMW tau species described herein into brain interstitial fluid or cerebrospinal fluid.

A reduced extracellular level of the soluble HMW tau species using the methods described herein can result in reduced neuron uptake of the soluble HMW tau species by at least by about 10% or more (including, e.g., at least about 20%, at least about 30%, at least about 40%, at least about 50%, at least about 60%, at least about 70%, at least about 80%, at least about 90%, or more), thereby reducing propagation of pathological tau protein between synaptically-connected neurons by at least by about 10% or more (including, e.g., at least about 20%, at least about 30%, at least about 40%, at least about 50%, at least about 60%, at least about 70%, at least about 80%, at least about 90%, or more), as compared to without the selective reduction of soluble HMW tau species.

The methods described herein can be used for therapeutic treatment of tau-associated neurodegeneration or tauopathy. Tau pathology is known to spread in a hierarchical pattern in Alzheimer's disease (AD) brain during disease progression, e.g., by trans-synaptic tau transfer between neurons. Since the soluble HMW tau species is identified herein to be involved in neuron-to-neuron propagation, intervention to deplete such low HMW tau species can inhibit tau propagation and hence disease progression in tauopathies. Accordingly, a method of reducing tau-associated neurodegeneration in a subject is provided herein. Examples of tau-associated neurodegeneration include, but are not limited to, Alzheimer's disease, Parkinson's disease, or frontotemporal dementia. The method of treatment comprises selectively reducing the level of soluble HMW tau species in the brain of a subject determined to have, or be at risk for, tau-associated neurodegeneration, wherein the soluble HMW tau species is non-fibrillar, with a molecular weight of at least about 500 kDa, wherein a reduced level of the soluble HMW tau species results in reduced tau- associated neurodegeneration. Suitably, the level of soluble LMW tau species in the subject is not substantially reduced during the treatment.

Suitably, at least a portion of the soluble HMW tau species present in brain interstitial fluid of the subject is removed. Suitably, at least a portion of the soluble HMW tau species present in cerebrospinal fluid of the subject is removed.

Methods for selectively reducing the extracellular level of soluble HMW tau species has been described above and can be applied to selectively reduce the level of HMW tau species in the brain of a subject. For example, the soluble HMW tau species present in the brain interstitial fluid and/or cerebrospinal fluid of the subject can be selectively reduced by brain microdialysis. The soluble HMW tau species present in the brain interstitial fluid and/or cerebrospinal fluid of the subject can be selectively reduced by administering to the brain of the subject an antagonist of soluble HMW tau species, e.g., by intracranial injection, intracortical injection, or intracerebroventricular injection, or via peripheral administration of a molecule that crosses the blood brain barrier in sufficient quantities.
The method can further comprise selecting a subject determined to have soluble HMW tau species present in the brain (e.g., in brain interstitial fluid or cerebrospinal fluid) at a level above a reference level, or determined to be at risk for, or have tau-associated neurodegeneration or tauopathy. A reference level can represent an extracellular level of soluble HMW tau species present in the brain (e.g., in brain interstitial fluid or cerebrospinal fluid) of healthy subject(s). The reference level can be at least about 25 ng/mL or higher (including, e.g., at least about 50 ng/mL, at least about 100 ng/mL, at least about 150 ng/mL, at least about 200 ng/mL or higher). Methods to diagnose or determine a subject for tau-associated neurodegeneration or tauopathy are known in the art and can be used herein to select a subject amenable to the methods of treatment described herein. Methods of diagnosing tau-associated neurodegeneration as described below and as described in the section *"Selection of Subjects in Need Thereof for the Methods of Treatment Described herein"* below can also be used to select a subject amenable to the methods of treatment described herein.

### Methods of diagnosing tau-associated neurodegeneration

In a further aspect, a method of diagnosing tau-associated neurodegeneration based on the presence and/or levels (e.g., extracellular levels) of the soluble HMW tau species is also provided herein. Exemplary tau-associated neurodegeneration includes, but is not limited to, Alzheimer's disease, Parkinson's disease, or frontotemporal dementia. The inventors have shown that while the total tau levels in brain extracts from AD and control brains were not significantly different, the AB brain extract contained significantly higher levels of soluble HMW tau species or phosphorylated, soluble HMW tau species, when compared to the control brain. Therefore, the method of diagnosing tau-associated neurodegeneration can comprise (a) fractionating a sample of brain interstitial fluid or cerebrospinal fluid from a subject; and (b) detecting soluble HMW tau species in the sample such that the presence and amount of the soluble HMW tau species is determined, wherein the soluble HMW tau species is non-fibrillar, with a molecular weight of at least about 500 kDa; and (c) identifying the subject to have, or be at risk for tau-associated neurodegeneration when the level of the soluble HMW tau species in the sample is the same as or above a reference level; or identifying the subject to be less likely to have tau-associated neurodegeneration when the level of the soluble HMW tau species is below a reference level.

The total level of soluble HMW tau species in the sample can be detected for diagnostic purpose. The level of phosphorylated HMW tau species in the sample can be detected for diagnostic purpose. The phosphorylation levels of at least one or more phosphorylation sites present in the soluble HMW tau species in the sample can be detected for diagnostic purpose. Examples of phosphorylation sites that can be phosphorylated or hyperphosphorylated in the HMW tau species include, without limitations, pS199, pT205, pS262, pS396, pS396/404, pS400, pS409, pS422, a combination of two or more thereof. The HMW tau species can be phosphorylated or hyper-phosphorylated at least one or more (e.g., at least two, at least three, at least four, at least five, at least six) of the following phosphorylation sites: pT205, pS262, pS400, pS404, pS409, and pS422. Antibodies to these phosphorylation sites are commercially available, e.g., from Life Technologies.

Accordingly, a reference level can represent the total level of soluble HMW tau species present in the brain of healthy subject(s). In this case, a reference level can represent the total level of soluble HMW tau species present in brain interstitial fluid or cerebrospinal fluid of healthy subject(s). The reference level can be at least about 25 ng/mL or higher (including, e.g., at least about 50 ng/mL, at least about 100 ng/mL, at least about 150 ng/mL, at least about 200 ng/mL or higher).

A reference level can represent the level of phosphorylated HMW tau species present in the brain of healthy subject(s). In this case, a reference level can represent the level of phosphorylated HMW tau species present in brain interstitial fluid or cerebrospinal fluid of healthy subject(s). The reference level can be at least about 25 ng/mL or higher (including, e.g., at least about 50 ng/mL, at least about 100 ng/mL, at least about 150 ng/mL, at least about 200 ng/mL or higher).

A reference level can represent the phosphorylation level of at least one or more phosphorylation sites present in the soluble HMW tau species in the sample.

As used herein, the term "fractionating" refers to separating a sample into a plurality of fractions based on a certain parameter, e.g., molecular sizes or molecular weights. In the context of various aspects described herein, the term "fractionating" refers to separating soluble HMW tau species from a sample of brain interstitial fluid or cerebrospinal fluid or enriching the sample with soluble HMW tau species. Suitably, the fractionation is based on molecular size and/or molecular weight of molecules present in the sample. Such size or weight exclusion methods are known in the art, e.g., but not limited to size exclusion chromatography, centrifugation, gel electrophoresis, sucrose density, affinity chromatography, dialysis, or a combination of two or more thereof.

The sample, prior to the fractionating of (a), can be substantially free of soluble LMW tau species, wherein the soluble LMW tau species has a molecular weight of no more than 200 kDa or lower. For example, a sample of brain interstitial fluid or cerebrospinal fluid can be obtained from a subject to be diagnosed by microdialysis, e.g., using a permeable membrane with a proper molecular-weight cut-off, e.g., which would allow only molecules with a molecular weight of at least about 600 kDa to be collected.

Alternatively, the sample, prior to the fractionating of (a), can comprise soluble LMW tau species, wherein the soluble LMW tau species has a molecular weight of no more than 200 kDa. By fractionating the sample, one can isolate the soluble HMW tau species from other low MW molecules in the sample (e.g., soluble LMW tau species) and enrich the sample with the soluble HMW tau species for diagnostic purposes.

After fractionation, the soluble HMW tau species in the sample can be detected by any methods typically used to detect tau protein, including, e.g., but not limited to, ELISA, western blot, immunoassay, size exclusion chromatography, a combination of two or more thereof.

Where soluble LMW tau species is present in the sample, the method can further comprise detecting the amount of the soluble LMW tau species in the sample. In this case, the subject can be identified to have, or be at risk for tau-associated neurodegeneration if a ratio of the soluble HMW tau species to the soluble LMW tau species is the same as or above a reference level ratio; or the subject is identified to be less likely to have tau-associated neurodegeneration if the ratio of the soluble HMW tau species to the soluble LMW tau species is below the reference level ratio.

A reference level ratio can represent an extracellular level ratio of soluble HMW tau species to soluble LMW tau species present in the brain of healthy subject(s). The level of LMW tau greatly exceeds that of HMW tau, even in individuals with AD. Thus, HMW tau generally makes up only ∼1-5% (or less) of total tau protein. A reference level ratio can represent a level ratio of soluble HMW tau species to soluble LMW tau species present in brain interstitial fluid or cerebrospinal fluid of healthy subject(s). The reference level ratio can range from about 10000:1 to about 20:1, about 1000:1 to about 50:1, or about 500: 1 to about 100:1. The method can further comprise administering to a subject identified to have, or be at risk for tau-associated neurodegeneration a therapeutic treatment, e.g., a pharmaceutical composition comprising one or more anti-soluble HMW tau species antagonists described herein.

### Methods of screening for agents that reduce cross-synaptic spread misfolded tau proteins

Not only does the discovery of soluble HMW tau species provide a therapeutic target and a biomarker for tau-associated neurodegeneration as described herein, the soluble HMW tau species can also be used *in vitro* to induce inter-neuron propagation, a phenotypic feature of progression in neurodegeneration, and thus develop an *in vitro* model to screen for effective agents that reduce cross-synaptic spread misfolded tau proteins and thus treat tau-associated neurodegeneration. Accordingly, a further aspect provided herein relates to a method of identifying an agent that is effective to reduce cross-synaptic spread of misfolded tau proteins or soluble HMW tau species described herein. The method comprises (a) contacting a first neuron in a first chamber of a neuron culture device with soluble HMW tau species, wherein the first neuron is axonally connected with a second neuron in a second chamber of the neuron culture device, and wherein the second neuron is not contacted with the soluble HMW tau species; (b) contacting the first neuron in the first chamber with a candidate agent; and (c) detecting transport of the soluble HMW tau species from the first neuron to the second neuron.

The first neuron can be contacted with the soluble HMW tau species and the candidate agent concurrently. The first neuron can be contacted with the soluble HMW tau species prior to contact with the candidate agent. The first neuron can be contacted with the soluble HMW tau species after contact with the candidate agent.

As used herein, the term "candidate agent" refers to any compound or substance such as, but not limited to, a small molecule, nucleic acid, polypeptide, peptide, drug, ion, etc, which is desired to be tested for its ability to reduce or inhibit neuron uptake of soluble HMW tau species and/or to reduce inter-neuron propagation of the soluble HMW tau species. A "candidate agent" can be any chemical, entity, or moiety, including, without limitation, synthetic and naturally-occurring proteinaceous and non-proteinaceous entities. Suitably, a candidate agent is a nucleic acid, a nucleic acid analogue, a protein, an antibody, a peptide, an aptamer, an oligomer of nucleic acids, an amino acid, or a carbohydrate, and includes, without limitation, proteins, oligonucleotides, ribozymes, DNAzymes, glycoproteins, siRNAs, lipoproteins, aptamers, and modifications and combinations thereof etc. Suitably, agents are small molecules having a chemical moiety. For example, chemical moieties include unsubstituted or substituted alkyl, aromatic, or heterocyclyl moieties.

An effective agent for reducing cross-synaptic spread of misfolded tau proteins can be identified based on detection of the presence or level of the soluble HMW tau species in an axon and/or soma of the second neuron. If the soluble HMW tau species in an axon and/or soma of the second neuron is reduced by at least about 30% or more (including, e.g., at least about 40%, at least about 50%, at least about 60%, at least about 70%, at least about 80%, at least about 90%, at least about 95%, or more, and up to 100%), as compared to when the first neuron in the first chamber is not contacted with the candidate agent, the candidate agent is identified as an effective agent for reducing cross-synaptic spread of misfolded tau proteins or soluble HMW tau species described herein.

As used herein, the term "axonally connected" refers to neurons are connected by an axon. As used herein, the term "axon" refers to a long cellular protrusion from a neuron, whereby efferent (outgoing) action potentials are conducted from the cell body towards target cells.

While any neuron culture device suitable for monitoring axonal extension and/or transport can be used in the methods described herein, suitably, the neuron culture device is a microfluidic device. The microfluidic device can comprise a first chamber for placing at least a first neuron and a second chamber for placing at least a second neuron, wherein the first chamber and the second chamber are interconnected by at least one microchannel exclusively sized to permit axon growth. The microfluidic device can comprise a first chamber for placing a first population (e.g., at least 2 or more) of neurons and a second chamber for placing a second population (e.g., at least 2 or more) of neurons, wherein the first chamber and the second chamber are interconnected by at least two or more microchannels, each exclusively sized to permit axon growth.

As used herein, the term "exclusively sized to permit axon growth" refers to the dimensions of the interconnecting microchannel(s) being sized to exclusively allow an extension of an axon, originating from the cell body of neuron(s) in the first chamber to enter the second chamber. For example, the length of the microchannel(s) interconnecting the first chamber and the second chamber is optimized such that no MAP2-positive dendrites can enter the second chamber, thus isolating axon terminals from soma and dendrites. The length of the microchannel(s) can be at least about 400 µm or more, including, e.g., at least about 500 µm, at least about 600 µm, at least about 700 µm, at least about 800 µm, at least about 900 µm, at least about 1000 µm. The length of the microchannel(s) can be at least about 450 µm or more. The length of the microchannel(s) can be at least about 600 µm or more.

The width of the microchannels can be exclusively sized to permit axon growth. The width of the microchannels can range from about 3 µm to about 15 µm, or from about 5 µm to about 10 µm, or from about 6 µm to about 10 µm.

The microfluidic device can further comprise a third chamber for placing at least a third neuron, wherein the second chamber and the third chamber are interconnected by at least one microchannel exclusively sized to permit axon growth as described herein.

By way of example, **Fig. 3A** shows a schematic diagram of an exemplary neuron culture device. **Fig. 3A** shows a microfluidic device 300, which comprises a first chamber 302 for placing at least a first neuron and a second chamber 304 for placing at least a second neuron, wherein the first chamber 302 and the second chamber 304 are interconnected by at least one microchannel 306 exclusively sized to permit axon growth. More than one microchannel 306 (e.g., at least two or more microchannels) interconnecting the two chambers can be desirable so that multiple axons can be monitored simultaneously. The microfluidic device 300 can further comprise a third chamber 308 for placing at least a third neuron, wherein the second chamber 304 and the third chamber 308 are interconnected by at least one microchannel 306 exclusively sized to permit axon growth.

To prevent diffusion of the soluble HMW tau species and candidate agent from the first chamber into other chambers (e.g., the second chamber and/or the optional third chamber), the second chamber and/or the optional third chamber can be added with a greater amount of cell culture medium than what is added in the first chamber such that the volume difference between the chambers can result in continuous convection ("hydrostatic pressure barrier"). The amount of the cell culture medium added into the second and/or the optional third chamber can be greater than that in the first chamber by at least about 1.5-fold, at least about 2-fold, at least about 3-fold, at least about 4-fold, at least about 5-fold, at least about 10-fold or higher. The amount of the cell culture medium added into the second and/or the optional third chamber can be greater than that in the first chamber by at least about 4-fold or higher.

To detect the transport of soluble HMW tau species from a first neuron to a second neuron, the neurons can be fixed, immunostained for presence of soluble HMW tau species using anti-tau antibodies as described in the Examples or any commercially-available anti-tau antibodies, and examined under a microscope. In order to distinguish the first neuron from the second neuron, the first neuron and the second neurons can be labeled with a different fluorescent molecule.

### Methods of treatment based on reducing endogenous, intracellular tau proteins

In one aspect, the inventors have shown that as compared to tau-expressing animals, tau-null animals have displayed less pathological tau misfolding and gliosis, even in the presence of neurofibrillary tangles (NFTs), and have also displayed a reduced activation of Aβ-induced mitochondrial intrinsic caspase cascades in the neurons. Thus, removing endogenous, intracellular tau proteins can provide a neuroprotective effect, e.g., reducing neurotoxicity and/or increasing neuron survival. While previous reports have discussed that a reduction of tau proteins can improve neurodegeneration, one of skill in the art would not have expected that removing a significant amount of endogenous, intracellular tau proteins, i.e., essential proteins that stabilize microtubules, would not produce any adverse effects to the neurons. However, the inventors here have discovered that a tau-null human subject has normal neuron phenotypes or no abnormalities in cognitive function as a healthy human subject expressing non-aggregating tau proteins. As such, methods of reducing neural damage or neurodegeneration induced by tauopathy based on reducing endogenous, intracellular tau proteins are also provided herein. Exemplary tauopathy can be Alzheimer's disease, Parkinson's disease, or frontotemporal dementia.

The method of reducing neural damage or neurodegeneration induced by tauopathy comprises administering to the brain of a subject determined to have tauopathy a tau antagonist agent (e.g., an agent that inhibits at least about 50% expression level of endogenous, intracellular tau protein) in the subject, thereby reducing neurotoxicity (and/or increasing neuron survival) in the presence of neurofibrillary tangles and/or amyloid beta.

The term "neurotoxicity" refers to the toxic effect of amyloid-derived-diffusable ligands (ADDLs) as a multimeric neurotoxic form of Aβ and/or neurofibrillary tangles on neurons either in vitro and/or in vivo. For example, ADDLs bind to specific neuronal receptors triggering aberrant neuronal signaling, which compromises long term potentiation and causes memory deficits. Thus, ADDLs alter the function of a neuron in such a manner that, while still viable, the neuron does not properly function. Such altered functionality is referred to herein as "neuronal dysfunction," which is a subclass of neurotoxicity. Persistent ADDL signaling causes aberrant transcription and the progressive loss of synapses, and very long term persistent ADDL signaling and accumulated structural pathology leads to eventual neuron death and gross brain dystrophy.

As used herein, a "tau antagonist agent" or "tau antagonist" refers to an agent, such as a small molecule, antagonist polypeptide, inhibitory nucleic acid, or MAPT-specific antibody or antigen-binding fragment thereof, that inhibits or causes or facilitates a qualitative or quantitative inhibition, decrease, or reduction in one or more processes, mechanisms, effects, responses, functions, activities or pathways mediated by MAPT. Thus, the term "tau antagonist agent" refers to an agent that inhibits expression of the MAPT polypeptide or polynucleotide encoding MAPT, or one that binds to, partially or totally blocks stimulation, decreases, prevents, delays activation, inactivates, desensitizes, or down regulates the activity of the MAPT polypeptide or polynucleotide encoding MAPT. Such MAPT antagonists can e.g., inhibit MAPT expression, e.g., MAPT translation, post-translational processing of MAPT, stability, degradation, or nuclear or cytoplasmic localization of the MAPT polypeptide, or transcription, post transcriptional processing, stability or degradation of a polynucleotide encoding MAPT. A tau antagonist agent can be known to have a desired activity and/or property, e.g., inhibition of MAPT activity or expression, or can be selected from a library of diverse compounds, using, for example, screening methods known in the art or as described herein.

As used herein and throughout the specification, the term "MAPT" or "microtubule-associated protein tau" generally refers to a MAPT polypeptide or a MAPT polynucleotide that is similar or identical to the sequence of a wild-type MAPT.

Suitably, the term "MAPT" refers to a MAPT polypeptide having an amino acid sequence that is at least 70% or more (including at least 75%, at least 80%, at least 85%, at least 90%, at least 95%, at least 97%, at least 99%, or 100%) identical to that of a wild-type MAPT, and is capable of modulating the stability of axonal microtubules.

Suitably, the term "MAPT" refers to a MAPT polynucleotide having a nucleotide sequence that is at least 70% or more (including at least 75%, at least 80%, at least 85%, at least 90%, at least 95%, at least 97%, at least 99%, or 100%) identical to that of a wild-type MAPT or a portion thereof, and encodes a MAPT polypeptide as described herein.

The wild-type MAPT sequences of various isoforms and of different species are available on the world wide web from the NCBI, including human, mouse, rat, and dog. For example, the nucleotide sequences encoding different isoforms of human MAPT and their corresponding amino acid sequences are available at NCBI and their Accession Nos are included in Table 1 shown herein.

Where the term "MAPT" refers to a MAPT polypeptide, a "variant" of a MAPT polypeptide encompasses a portion or fragment of such a MAPT polypeptide that retains at least about 70% or more (including at least 75%, at least 80%, at least 85%, at least 90%, at least 95%, at least 97%, at least 99%, or 100%) of the axonal microtubule-stabilizing activity of the wild-type MAPT polypeptide. The variant also encompasses conservative substitution variants of a MAPT polypeptide that retain at least about 70% or more (including at least 75%, at least 80%, at least 85%, at least 90%, at least 95%, at least 97%, at least 99%, or 100%) of the axonal microtubule-stabilizing activity of the wild-type MAPT polypeptide.

The amino acid identity between two polypeptides can be determined, for example, by first aligning the two polypeptide sequences using an alignment algorithm, such as BLAST® or by other methods well-known in the art.

As described herein, methods for measuring MAPT or a fragment thereof from a sample are known in the art, including, but not limited to mRNA expression using PCR or real-time PCR, protein analysis using western blot, immunoassay, and/or ELISA, and/or sequencing analysis. Thus, nucleic acid molecules can be isolated from a patient's sample (e.g., a biopsy) to measure MAPT mRNA expression, or proteins can be isolated to measure MAPT protein expression.

Suitably, the tau antagonist agent selected for the method described herein inhibits at least about 50% expression level of endogenous, intracellular tau protein. Suitably, the tau antagonist agent selected for the method described herein inhibits at least about 70% expression level of endogenous, intracellular tau protein. Suitably, the tau antagonist agent selected for the method described herein inhibits at least about 90% expression level of endogenous, intracellular tau protein. Suitably, the tau antagonist agent selected for the method described herein inhibits 95% expression level of endogenous, intracellular tau protein. Suitably, the tau antagonist agent selected for the method described herein inhibits 99% expression level of endogenous, intracellular tau protein.

The agent selected to inhibit the expression levels of endogenous, intracellular tau protein can disrupt expression of the MAPT (microtubule-associated protein tau) gene and/or inhibit transcription of the MAPT gene. Such agent can include, but is not limited to, an antibody, a nuclease (e.g., but not limited to, a zinc finger nuclease (ZFN), transcription activator-like effector nuclease (TALEN), a CRISPR/Cas system, a transcriptional repressor, a nucleic acid inhibitor (e.g., RNAi, siRNA, anti-miR, antisense oligonucleotides, ribozymes, and a combination of two or more thereof), a small molecule, an aptamer, and a combination of two or more thereof. MAPT-specific siRNAs are available commercially, e.g., SC-36614 from Santa Cruz Biotechnology, and/or the Accell SMART POOL™ MAPT siRNA or individual Accell™ MAPT siRNAs, available from Dharmacon Inc.

Methods known for effectively delivering an agent to the brain of a subject can be used to perform the methods described herein. The agent can be administered to the brain via a carrier, e.g., for enhanced delivery of the agent. An exemplary carrier can be a virus or viral vector (e.g., but not limited to, retrovirus, adenovirus, adeno-associated virus (AAV), recombinant AAV expression vector), a nanoparticle, and/or a liposome.

The brain of the subject can be further determined to have an amyloid beta plaque (e.g., by magnetic resonance imaging (MRI), which is further described in Baltes et al., Methods Mol Biol (2011) 711: 511-33) and the administration can reduce neurotoxicity (and/or increases neuron survival) in the presence of amyloid beta.

The term "amyloid beta" is used herein to refer to a family of peptides that are the principal chemical constituent of the senile plaques and vascular amyloid deposits (amyloid angiopathy) found in the brain, e.g., in patients of Alzheimer's disease (AD), Down's Syndrome, and Hereditary Cerebral Hemorrhage with Amyloidosis of the Dutch-Type (HCHWA-D). Amyloid beta peptides are fragments of beta-amyloid precursor protein (APP) which comprises a variable number of amino acids, typically 38-43 amino acids.

### Selection of subjects in need thereof for the methods of treatment of various aspects described herein

The terms "treatment" and "treating" as used herein, with respect to treatment of a disease, means preventing the progression of the disease, or altering the course of the disorder (for example, but are not limited to, slowing the progression of the disorder), or reversing a symptom of the disorder or reducing one or more symptoms and/or one or more biochemical markers in a subject, preventing one or more symptoms from worsening or progressing, promoting recovery or improving prognosis. For example, in the case of treating a tau-associated neurodegeneration or tauopathy, e.g., AD, therapeutic treatment refers to reduced neurodegenerative morphologies, e.g., reduced inter-neuron propagation after administration of a soluble HMW tau species antagonist agent described herein. Alternatively, the therapeutic treatment refers to alleviation of at least one symptom associated with a tau-associated neurodegeneration or tauopathy, e.g., AD. Measurable lessening includes any statistically significant decline in a measurable marker or symptom, such as assessing the cognitive improvement with neuropsychological tests such as verbal and perception after treatment. Suitably, at least one symptom of a tau-associated neurodegeneration or tauopathy, e.g., AD, is alleviated by at least about 10%, at least about 15%, at least about 20%, at least about 30%, at least about 40%, or at least about 50%. More suitably, at least one symptom is alleviated by more than 50%, e.g., at least about 60%, or at least about 70%. Even more suitably, at least one symptom is alleviated by at least about 80%, at least about 90% or greater, as compared to a control (e.g. in the absence of a soluble HMW tau species antagonist agent described herein).

The methods of treatment described herein can further comprise a step of diagnosing a subject with a tau-associated neurodegeneration or tauopathy AD prior to the treatment. Subjects amenable to methods of treatment are subjects that have been diagnosed with a tau-associated neurodegeneration or tauopathy. Exemplary tau associated neurodegeneration or tauopathy includes, but is not limited to Alzheimer's disease, Parkinson's disease, or frontotemporal dementia.

Suitably, subjects amenable to methods of treatment are subjects that have been diagnosed with Alzheimer's disease. Methods for diagnosing Alzheimer's disease are known in the art. For example, the stage of Alzheimer's disease can be assessed using the Functional Assessment Staging (FAST) scale, which divides the progression of Alzheimer's disease into 16 successive stages under 7 major headings of functional abilities and losses: Stage 1 is defined as a normal adult with no decline in function or memory. Stage 2 is defined as a normal older adult who has some personal awareness of functional decline, typically complaining of memory deficit and forgetting the names of familiar people and places. Stage 3 (early Alzheimer's disease) manifests symptoms in demanding job situation, and is characterized by disorientation when traveling to an unfamiliar location; reports by colleagues of decreased performance; name- and word-finding deficits; reduced ability to recall information from a passage in a book or to remember a name of a person newly introduced to them; misplacing of valuable objects; decreased concentration. In stage 4 (mild Alzheimer's Disease), the patient may require assistance in complicated tasks such as planning a party or handling finances, exhibits problems remembering life events, and has difficulty concentrating and traveling. In stage 5 (moderate Alzheimer's disease), the patient requires assistance to perform everyday tasks such as choosing proper attire. Disorientation in time, and inability to recall important information of their current lives, occur, but patient can still remember major information about themselves, their family and others. In stage 6 (moderately severe Alzheimer's disease), the patient begins to forget significant amounts of information about themselves and their surroundings and require assistance dressing, bathing, and toileting. Urinary incontinence and disturbed patterns of sleep occur. Personality and emotional changes become quite apparent, and cognitive abulia is observed. In stage 7 (severe Alzheimer's disease), speech ability becomes limited to just a few words and intelligible vocabulary may be limited to a single word. A patient can lose the ability to walk, sit up, or smile, and eventually cannot hold up the head.

Other alternative diagnostic methods for AD include, but not limited to, cellular and molecular testing methods disclosed in US Patent No.: U.S. Pat. No. 7,771,937, U.S. Pat. No. 7,595,167, US 55580748, and PCT Application No.: WO2009/009457, the content of which is incorporated by reference in its entirety. Additionally, protein-based biomarkers for AD, some of which can be detected by non-invasive imaging, e.g., PET, are disclosed in U.S. Pat. No. 7,794,948, the content of which is incorporated by reference in its entirety.

Genes involved in AD risk can be used for diagnosis of AD. One example of other AD risk genes is apolipoprotein E-ε4 (APOE-ε4). APOE-ε4 is one of three common forms, or alleles, of the APOE gene; the others are APOE-e2 and APOE-e3. APOE provides the blueprint for one of the proteins that carries cholesterol in the bloodstream. Everyone inherits a copy of some form of APOE from each parent. Those who inherit one copy of APOE-ε4 have an increased risk of developing AD. Those who inherit two copies have an even higher risk, but not a certainty of developing AD. In addition to raising risk, APOE-ε4 may tend to make symptoms appear at a younger age than usual. Other AD risk genes in addition to APOE-e4 are well established in the art. Some of them are disclosed in US Pat. App. No.: US 2010/0249107, US 2008/0318220, US 2003/0170678 and PCT Application No.: WO 2010/048497, the content of which is incorporated by reference in its entirety. Genetic tests are well established in the art and are available, for example for APOE-e4. A subject carrying the APOE-ε4 allele can, therefore, be identified as a subject at risk of developing AD.

Suitably, subjects with Aβ burden are amenable to the methods of treatment described herein. Such subjects include, but not limited to, the ones with Down syndrome, the unaffected carriers of APP or presenilin gene mutations, and the late onset AD risk factor, apolipoprotein E-ε4.

Suitably, subjects with a tau-associated neurodegeneration or tauopathy (e.g., AD) who are currently receiving a therapeutic treatment for the tau-associated neurodegeneration or tauopathy can also be subjected to the methods of treatment as described herein.

A subject who has been diagnosed with an increased risk for developing a tau-associated neurodegeneration or tauopathy (e.g., AD), e.g., using the diagnostic methods described herein or any diagnostic methods (e.g., for AD) known in the art, can be subjected to the methods of treatment as described herein.

As used herein, a "subject" can mean a human or an animal. Examples of subjects include primates (e.g., humans, and monkeys). Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. A patient or a subject includes any subset of the foregoing, e.g., all of the above, or includes one or more groups or species such as humans, primates or rodents. In certain examples of the aspects described herein, the subject is a mammal, e.g., a primate, e.g., a human. The terms, "patient" and "subject" are used interchangeably herein. A subject can be male or female.

Suitably, the subject is a mammal. The mammal can be a human, non-human primate, mouse, rat, dog, cat, horse, or cow, but are not limited to these examples. Mammals other than humans can be advantageously used as subjects that represent animal models of neurodegeneration. In addition, the methods and compositions described herein can be employed in domesticated animals and/or pets. Suitably, the subject is a human subject. A human subject can be of any age, gender, race or ethnic group, e.g., Caucasian (white), Asian, African, black, African American, African European, Hispanic, Mideastern, etc.

### Pharmaceutical compositions and modes of administration for methods of treatment described herein

Also described herein are pharmaceutical compositions comprising an effective amount of at least one or more (e.g., 1, 2, 3, or more) soluble HMW tau species antagonist agent described herein and/or at least one or more (e.g., 1, 2, 3, or more) tau antagonist described herein. The composition further comprises at least one additional therapeutic agent that inhibits neurodegeneration, e.g., an AKAP79 peptide, or FK506 or a NFAT antagonist described in U.S. Patent App. No. 2013/0195866, the content of which is incorporated herein by reference.

A vector can be used to express and deliver a soluble HMW tau species antagonist agent and/or a tau antagonist into neurons. For example, a viral vector as described herein with an expression cassette can encode a MAPT antagonist sequence. The precise determination of an effective dose can be based on individual factors, including their plaque size, age, and amount of time since neurodegeneration. Therefore, dosages can be readily adjusted for each individual patient by those skilled in the art.

Any expression vector known in the art can be used to express the sensor systems described herein. The term "vectors" used interchangeably with "plasmid" refer to a nucleic acid molecule capable of transporting another nucleic acid to which it has been linked. Vectors capable of directing the expression of genes and/or nucleic acid sequence to which they are operatively linked are referred to herein as "expression vectors." In general, expression vectors of utility in recombinant DNA techniques are often in the form of "plasmids" which refer to circular double stranded DNA loops which, in their vector form are not bound to the chromosome. Other expression vectors can be used, for example, but are not limited to, plasmids, episomes, bacteriophages or viral vectors, and such vectors may integrate into the host's genome or replicate autonomously in the particular cell. Other forms of expression vectors known by those skilled in the art which serve the equivalent functions can also be used. Expression vectors comprise expression vectors for stable or transient expression encoding the DNA.

Suitably, the expression vector further comprises a promoter. As used herein, a "promoter" or "promoter region" or "promoter element" used interchangeably herein refers to a segment of a nucleic acid sequence, typically but not limited to DNA or RNA or analogues thereof, that controls the transcription of the nucleic acid sequence to which it is operatively linked. The promoter region includes specific sequences that are sufficient for RNA polymerase recognition, binding and transcription initiation. This portion of the promoter region is referred to as the promoter. In addition, the promoter region includes sequences which modulate this recognition, binding and transcription initiation activity of RNA polymerase. These sequences may be cis-acting or may be responsive to trans-acting factors. Promoters, depending upon the nature of the regulation may be constitutive or regulated.

Suitably, the expression vector further comprises a regulatory sequence. The term "regulatory sequences" is used interchangeably with "regulatory elements" herein refers element to a segment of nucleic acid, typically but not limited to DNA or RNA or analogues thereof, that modulates the transcription of the nucleic acid sequence to which it is operatively linked, and thus act as transcriptional modulators. Regulatory sequences modulate the expression of gene and/or nucleic acid sequence to which they are operatively linked. Regulatory sequence often comprise "regulatory elements" which are nucleic acid sequences that are transcription binding domains and are recognized by the nucleic acid-binding domains of transcriptional proteins and/or transcription factors, repressors or enhancers etc. Typical regulatory sequences include, but are not limited to, transcriptional promoters, an optional operate sequence to control transcription, a sequence encoding suitable mRNA ribosomal binding sites, and sequences to control the termination of transcription and/or translation. Regulatory sequences are selected for the assay to control the expression of split-biomolecular conjugate in a cell-type in which expression is intended.

Regulatory sequences can be a single regulatory sequence or multiple regulatory sequences, or modified regulatory sequences or fragments thereof. Modified regulatory sequences are regulatory sequences where the nucleic acid sequence has been changed or modified by some means, for example, but not limited to, mutation, methylation etc.

The term "operatively linked" or "operatively associated" are used interchangeably herein, and refer to the functional relationship of the nucleic acid sequences with regulatory sequences of nucleotides, such as promoters, enhancers, transcriptional and translational stop sites, and other signal sequences. For example, operative linkage of nucleic acid sequences, typically DNA, to a regulatory sequence or promoter region refers to the physical and functional relationship between the DNA and the regulatory sequence or promoter such that the transcription of such DNA is initiated from the regulatory sequence or promoter, by an RNA polymerase that specifically recognizes, binds and transcribes the DNA. In order to optimize expression and/or in vitro transcription, it may be necessary to modify the regulatory sequence for the expression of the nucleic acid or DNA in the cell type for which it is expressed. The desirability of, or need of, such modification may be empirically determined.

Suitably, an expression vector is a viral vector. As used herein, the term "viral vector" refers to any form of a nucleic acid derived from a virus and used to transfer genetic material into a cell via transduction. The term encompasses viral vector nucleic acids, such as DNA and RNA, encapsidated forms of these nucleic acids, and viral particles in which the viral vector nucleic acids have been packaged. Examples of a viral vector include, but are not limited to, retroviruses, lentiviruses, adenoviruses, adeno-associated viruses, and combinations thereof.

For administration to a subject in need thereof, e.g., a subject diagnosed with or predisposed to tau-associated neurodegeneration or tauopathy (e.g., AD), a soluble HMW tau species antagonist and/or a tau antagonist can be provided in a pharmaceutically acceptable composition. As used herein, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The pharmaceutically acceptable composition can further comprise one or more pharmaceutically carriers (additives) and/or diluents. As used herein, the term "pharmaceutically-acceptable carrier" means a pharmaceutically-acceptable material, composition or vehicle, such as a liquid, diluent, excipient, manufacturing aid or encapsulating material, for administration of a soluble HMW tau species antagonist described herein or a tau antagonist described herein. Each carrier must be "acceptable" in the sense of being compatible with the other ingredients of the formulation and not injurious to the patient. Pharmaceutically acceptable carriers include any and all solvents, dispersion media, coatings, antibacterial and antifungal agents, isotonic and absorption delaying agents, and the like which are compatible with the activity of the soluble HMW tau species antagonist and/or the tau antagonist and are physiologically acceptable to the subject. Some examples of materials which can serve as pharmaceutically-acceptable carriers include: (i) sugars, such as lactose, glucose and sucrose; (ii) starches, such as corn starch and potato starch; (iii) cellulose, and its derivatives, such as sodium carboxymethyl cellulose, methylcellulose, ethyl cellulose, microcrystalline cellulose and cellulose acetate; (iv) powdered tragacanth; (v) malt; (vi) gelatin; (vii) lubricating agents, such as magnesium stearate, sodium lauryl sulfate and talc; (viii) excipients, such as cocoa butter and suppository waxes; (ix) oils, such as peanut oil, cottonseed oil, safflower oil, sesame oil, olive oil, corn oil and soybean oil; (x) glycols, such as propylene glycol; (xi) polyols, such as glycerin, sorbitol, mannitol and polyethylene glycol (PEG); (xii) esters, such as ethyl oleate and ethyl laurate; (xiii) agar; (xiv) buffering agents, such as magnesium hydroxide and aluminum hydroxide; (xv) alginic acid; (xvi) pyrogen-free water; (xvii) isotonic saline; (xviii) Ringer's solution; (xix) ethyl alcohol; (xx) pH buffered solutions; (xxi) polyesters, polycarbonates and/or polyanhydrides; (xxii) bulking agents, such as polypeptides and amino acids (xxiii) serum component, such as serum albumin, HDL and LDL; (xxiv) C2-C12 alcohols, such as ethanol; and (xxv) other non-toxic compatible substances employed in pharmaceutical formulations. Wetting agents, coloring agents, release agents, coating agents, sweetening agents, flavoring agents, perfuming agents, preservative and antioxidants can also be present in the formulation.

For compositions or preparations described herein to be administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Pharmaceutically acceptable carriers can vary in the pharmaceutical compositions described herein, depending on the administration route and formulation. For example, the pharmaceutically acceptable composition described herein can be delivered via injection. These routes for administration (delivery) include, but are not limited to, subcutaneous or parenteral including intravenous, intracortical, intracranial, intracerebroventricular, intramuscular, intraperitoneal, and infusion techniques. Suitably, the pharmaceutical acceptable composition is in a form that is suitable for intracortical injection. Alternatively, the pharmaceutical composition is formulated for intracranial injection. Other forms of administration can be also be employed, e.g., oral, systemic, or parenteral administration.

The pharmaceutical compositions described herein can be specially formulated for administration in solid or liquid form. Additionally, the pharmaceutical compositions can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

When administering a pharmaceutical composition described herein parenterally, it will be generally formulated in a unit dosage injectable form (solution, suspension, emulsion). The pharmaceutical formulations suitable for injection include sterile aqueous solutions or dispersions. The carrier can be a solvent or dispersing medium containing, for example, water, cell culture medium, buffers (e.g., phosphate buffered saline), polyol (for example, glycerol, propylene glycol, liquid polyethylene glycol, and the like), and suitable mixtures thereof. The pharmaceutical carrier can be a buffered solution (e.g. PBS).

The pharmaceutical composition can be formulated in an emulsion or a gel. In this case, at least one soluble HMW tau species antagonist described herein and/or at least one tau antagonist described herein can be encapsulated within a biocompatible gel, e.g., hydrogel and a peptide gel. The gel pharmaceutical composition can be implanted to the brain near the degenerating neuronal cells, e.g., the cells in proximity to the amyloid plaque or neurofibrillary tangles, or in the interstitial space of the brain.

Additionally, various additives which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. In many cases, it may be desirable to include isotonic agents, for example, sugars, sodium chloride, and the like.

The compositions can also contain auxiliary substances such as wetting or emulsifying agents, pH buffering agents, gelling or viscosity enhancing additives, preservatives, colors, and the like, depending upon the route of administration and the preparation desired. Standard texts, such as "REMINGTON'S PHARMACEUTICAL SCIENCE", 17th edition, 1985, incorporated herein by reference, may be consulted to prepare suitable preparations, without undue experimentation. With respect to the pharmaceutical compositions described herein, however, any vehicle, diluent, or additive used should have to be biocompatible or inert with the soluble HMW tau species antagonists described herein and/or tau antagonists described herein.

The compositions can be isotonic, i.e., they can have the same osmotic pressure as blood and lacrimal fluid. The desired isotonicity of the pharmaceutical compositions described herein can be accomplished using sodium chloride, or other pharmaceutically acceptable agents such as dextrose, boric acid, sodium tartrate, propylene glycol or other inorganic or organic solutes. Suitably, sodium chloride is used in buffers containing sodium ions.

Viscosity of the compositions can be maintained at the selected level using a pharmaceutically acceptable thickening agent. Suitably, methylcellulose is used because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The important point is to use an amount which will achieve the selected viscosity. Viscous compositions are normally prepared from solutions by the addition of such thickening agents.

Neurons transduced with a vector encoding a soluble HMW tau species antagonist described herein and/or a tau antagonist described herein can be included in the pharmaceutical compositions and stored frozen. In this case, an additive or preservative known for freezing cells can be included in the compositions. A suitable concentration of the preservative can vary from 0.02% to 2% based on the total weight although there may be appreciable variation depending upon the preservative or additive selected. One example of such additive or preservative can be dimethyl sulfoxide (DMSO) or any other cell-freezing agent known to a skilled artisan. In this case, the composition will be thawed before use or administration to a subject, e.g., neuronal stem cell therapy.

Typically, any additives (in addition to the soluble HMW tau species antagonists described herein and/or tau antagonists described herein) can be present in an amount of 0.001 to 50 wt % solution in phosphate buffered saline, and the active ingredient is present in the order of micrograms to milligrams, such as about 0.0001 to about 5 wt %, about 0.0001 to about 1 wt %, about 0.0001 to about 0.05 wt % or about 0.001 to about 20 wt %, about 0.01 to about 10 wt %, and about 0.05 to about 5 wt %. For any therapeutic composition to be administered to a subject in need thereof, and for any particular method of administration, it is preferred to determine toxicity, such as by determining the lethal dose (LD) and LD50 in a suitable animal model e.g., rodent such as mouse; and, the dosage of the composition(s), concentration of components therein and timing of administering the composition(s), which elicit a suitable response. Such determinations do not require undue experimentation from the knowledge of the skilled artisan.

The pharmaceutical compositions described herein can be prepared by mixing the ingredients following generally-accepted procedures. For example, an effective amount of at least one soluble HMW tau species antagonist described herein and/or at least one tau antagonist described herein can be re-suspended in an appropriate pharmaceutically acceptable carrier and the mixture can be adjusted to the final concentration and viscosity by the addition of water or thickening agent and possibly a buffer to control, pH or an additional solute to control tonicity. An effective amount of at least one soluble HMW tau species antagonist described herein and/or at least one tau antagonist described herein and any other additional agent, e.g., for inhibiting neurodegeneration, can be mixed with the cell mixture. Generally the pH can vary from about 3 to about 7.5. The pH of the composition can be about 6.5 to about 7.5. Compositions can be administered in dosages and by techniques well known to those skilled in the medical and veterinary arts taking into consideration such factors as the age, sex, weight, and condition of the particular patient, and the composition form used for administration (e.g., liquid). Dosages for humans or other mammals can be determined without undue experimentation by a skilled artisan.

Suitable regimes for initial administration and further doses or for sequential administrations can be varied. Suitably, a therapeutic regimen includes an initial administration followed by subsequent administrations, if necessary. Multiple administrations of at least one soluble HMW tau species antagonist described herein and/or at least one tau antagonist described herein can be injected to the subject's brain. For example, at least one soluble HMW tau species antagonist described herein and/or at least one tau antagonist described herein can be administered in two or more, three or more, four or more, five or more, or six or more injections. The same soluble HMW tau species antagonist described herein and/or the same tau antagonist described herein can be administered in each subsequent administration. A different soluble HMW tau species antagonist described herein and/or a different tau antagonist described herein can be administered in each subsequent administration. Injections can be made in cortex, e.g., somatosensory cortex. Injections can be administered in proximity to a plaque, e.g., amyloid-beta plaque or neurofibrillary tangles.

The subsequent injection can be administered immediately after the previous injection, or after at least about 1 minute, after at least about 2 minute, at least about 5 minutes, at least about 15 minutes, at least about 30 minutes, at least about 1 hour, at least about 2 hours, at least about 3 hours, at least about 6 hours, at least about 12 hours, at least about 24 hours, at least about 2 days, at least about 3 days, at least about 4 days, at least about 5 days, at least about 6 days or at least about 7 days. The subsequent injection can be administered after at least about 1 week, at least about 2 weeks, at least about 1 month, at least about 2 years, at least about 3 years, at least about 6 years, or at least about 10 years.

Suitably, a dosage comprising a pharmaceutical composition described herein is considered to be pharmaceutically effective if the dosage reduce degree of neurodegeneration, e.g., indicated by an increased neuron survival or reduced neurotoxicity or improvement in brain or cognitive function, by at least about 5%, at least about 10%, at least about 15%, at least about 20%, at least about 30%, at least about 40%, or at least about 50%. Suitably, the brain or cognitive function is improved by more than 50%, e.g., at least about 60%, or at least about 70%. More suitably, the brain or cognitive function is improved by at least about 80%, at least about 90% or greater, as compared to a control (e.g. in the absence of the composition described herein).

Alternative expressions of the inventive concept *described herein can be defined in any of the following numbered paragraphs:*
1. A composition comprising soluble high molecular weight (HMW) tau species, wherein the soluble HMW tau species is non-fibrillar, with a molecular weight of at least about 500 kDa, and wherein the composition is substantially free of soluble low molecular weight (LMW) tau species.
2. The composition of paragraph 1, wherein the soluble HMW tau species has a molecular weight of at least about 669 kDa.
3. The composition of paragraph 1, wherein the soluble HMW tau species has a molecular weight of about 669 kDa to about 1000 kDa.
4. The composition of any of paragraphs 1-3, wherein the soluble HMW tau species is in a form of particles.
5. The composition of paragraph 4, wherein the particle size ranges from about 10 nm to about 30 nm.
6. The composition of any of paragraphs 1-5, wherein the soluble HMW tau species is phosphorylated.
7. The composition of any of paragraphs 1-6, wherein the soluble HMW tau species is soluble in phosphate-buffered saline.
8. The composition of any of paragraphs 1-7, wherein the soluble HMW tau species is preferentially taken up by a neuron and axonally transported from the neuron to a synaptically-connected neuron, as compared to neuron uptake and neuron-to-neuron transport of the soluble LMW tau species.
9. The composition of paragraph 8, wherein the soluble LMW tau species has a molecular weight of no more than 200 kDa.
10. The composition of paragraph 9, further comprising an adjuvant for raising an antibody against the soluble HMW tau species.
11. An isolated antibody or antigen-binding portion thereof that specifically binds soluble high molecular weight (HMW) tau species and does not bind soluble low molecular weight (LMW) tau species, wherein the HMW tau species is non-fibrillar, with a molecular weight of at least about 500 kDa, and wherein the LMW tau species has a molecular weight of no more than 200 kDa.
12. The isolated antibody or antigen-binding portion thereof of paragraph 11, which reduces the soluble HMW tau species being taken up by a neuron.
13. The isolated antibody or antigen-binding portion thereof of paragraph 11 or 12, which reduces the soluble HMW tau species being axonally transported from a neuron to a synaptically-connected neuron.
14. The isolated antibody or antigen-binding portion thereof of any of paragraphs 11-13, wherein the soluble HMW tau species has a molecular weight of at least about 669 kDa.
15. The isolated antibody or antigen-binding portion thereof of paragraph 14, wherein the soluble HMW tau species has a molecular weight of about 669 kDa to about 1000 kDa.
16. The isolated antibody or antigen-binding portion thereof of any of paragraphs 11-15, wherein the soluble HMW tau species is in a form of particles.
17. The isolated antibody or antigen-binding portion thereof of paragraph 16, wherein the particle size ranges from about 10 nm to about 30 nm.
18. The isolated antibody or antigen-binding portion thereof of any of paragraphs 11-17, wherein the soluble HMW tau species is phosphorylated.
19. The isolated antibody or antigen-binding portion thereof of any of paragraphs 11-18, wherein the soluble HMW tau species is soluble in phosphate-buffered saline.
20. A method of preventing propagation of pathological tau protein between synaptically-connected neurons comprising selectively reducing the extracellular level of soluble HMW tau species in contact with a synaptically-connected neuron, wherein the soluble HMW tau species is non-fibrillar, with a molecular weight of at least about 500 kDa, wherein a reduced level of the soluble HMW tau species results in reduced propagation of pathological tau protein between synaptically-connected neurons.
21. The method of paragraph 20, wherein the extracellular level of soluble LMW tau species is not substantially reduced during said selective reduction.
22. The method of any of paragraph 20 or 21, wherein the soluble HMW tau species is selectively reduced by microdialysis.
23. The method of any of paragraphs 20-22, wherein the soluble HMW tau species is selectively reduced by contacting the extracellular space or fluid in contact with the synaptically-connected neurons with an antagonist of the soluble HMW tau species.
24. The method of paragraph 23, wherein the antagonist of the HMW tau species is selected from the group consisting of an antibody, a zinc finger nuclease, a transcriptional repressor, a nucleic acid inhibitor, a small molecule, an aptamer, a gene-editing composition, and a combination thereof.
25. A method of reducing tau-associated neurodegeneration in a subject comprising selectively reducing the level of soluble HMW tau species in the brain of the subject determined to have, or be at risk for, tau-associated neurodegeneration, wherein the soluble HMW tau species is non-fibrillar, with a molecular weight of at least about 500 kDa, wherein a reduced level of the soluble HMW tau species results in reduced tau-associated neurodegeneration.
26. The method of paragraph 25, wherein the level of soluble LMW tau species in the subject is not substantially reduced during the treatment.
27. The method of paragraph 25 or 26, wherein at least a portion of the soluble HMW tau species is present in brain interstitial fluid of the subject.
28. The method of any of paragraphs 25-27, wherein at least a portion of the soluble HMW tau species is present in cerebrospinal fluid of the subject.
29. The method of any of paragraphs 25-28, wherein the soluble HMW tau species in the brain of the subject is selectively reduced by brain microdialysis.
30. The method of any of paragraphs 25-29 wherein the soluble HMW tau species is selectively reduced by administering to the brain of the subject an antagonist of soluble HMW tau species.
31. The method of paragraph 30, wherein the antagonist of the HMW tau species is selected from the group consisting of an antibody, a zinc finger nuclease, a transcriptional repressor, a nucleic acid inhibitor, a small molecule, an aptamer, a gene-editing composition, and a combination thereof.
32. The method of any of paragraphs 25-31, further comprising selecting a subject determined to have soluble HMW tau species present in the brain at a level above a reference level.
33. The method of any of paragraphs 25-32, wherein the tau-associated neurodegeneration is Alzheimer's disease, Parkinson's disease, or frontotemporal dementia.
34. A method of diagnosing tau-associated neurodegeneration comprising
   a. fractionating a sample of brain interstitial fluid or cerebrospinal fluid from a subject;
   b. detecting soluble HMW tau species in the sample such that the presence and amount of the soluble HMW tau species is determined, wherein the soluble HMW tau species is non-fibrillar, with a molecular weight of at least about 500 kDa; and
   c. identifying the subject to have, or be at risk for tau-associated neurodegeneration when the level of the soluble HMW tau species in the sample is the same as or above a reference level; or
      identifying the subject to be less likely to have tau-associated neurodegeneration when the level of the soluble HMW tau species is below a reference level.
35. The method of paragraph 34, wherein the sample is substantially free of soluble LMW tau species, wherein the soluble LMW tau species has a molecular weight of no more than 200 kDa.
36. The method of paragraph 34 or 35, wherein the sample comprises soluble LMW tau species, wherein the soluble LMW tau species has a molecular weight of no more than 200 kDa.
37. The method of paragraph 36, further comprising detecting the amount of the soluble LMW tau species in the sample.
38. The method of any of paragraphs 34-37, wherein the subject is identified to have, or be at risk for tau-associated neurodegeneration if a ratio of the soluble HMW tau species to the soluble LMW tau species is the same as or above a reference level ratio; or the subject is identified to be less likely to have tau-associated neurodegeneration if the ratio of the soluble HMW tau species to the soluble LMW tau species is below the reference level ratio.
39. The method of any of paragraphs 34-38, wherein the fractionating is by size exclusion.
40. The method of any of paragraphs 34-39, wherein the detecting further comprises detecting phosphorylation of the soluble HMW tau species.
41. The method of any of paragraphs 34-40, wherein the tau-associated neurodegeneration is Alzheimer's disease, Parkinson's disease, or frontotemporal dementia.
42. A method of identifying an agent that is effective to reduce cross-synaptic spread of misfolded tau proteins comprising
   a. contacting a first neuron in a first chamber of a neuron culture device with soluble HMW tau species, wherein the first neuron is axonally connected with a second neuron in a second chamber of the neuron culture device, and wherein the second neuron is not contacted with the soluble HMW tau species;
   b. contacting the first neuron from (a) in the first chamber with a candidate agent;
   c. detecting transport of the soluble HMW tau species from the first neuron to the second neuron, thereby identifying an effective agent for reducing cross-synaptic spread of misfolded tau proteins based on detection of the presence of the soluble HMW tau species in an axon and/or soma of the second neuron.
43. The method of paragraph 42, wherein the neuron culture device is a microfluidic device.
44. The method of paragraph 43, wherein the micro fluidic device comprises a first chamber for placing a first neuron and a second chamber for placing a second neuron, wherein the first chamber and the second chamber are interconnected by at least one microchannel exclusively sized to permit axon growth.
45. A method of reducing neural damage or neurodegeneration induced by tauopathy comprising administering to the brain of a subject determined to have tauopathy an agent that inhibits at least about 50% expression level of endogenous, intracellular tau protein in the subject, thereby reducing neurotoxicity (and/or increasing neuron survival) in the presence of neurofibrillary tangles.
46. The method of paragraph 45, wherein the agent inhibits at least about 70% expression level of the endogenous, intracellular tau protein in the subject.
47. The method of paragraph 45, wherein the agent inhibits at least about 90% expression level of the endogenous, intracellular tau protein in the subject.
48. The method of paragraph 45, wherein the agent inhibits at least about 95% expression level of the endogenous, intracellular tau protein in the subject.
49. The method of any of paragraphs 45-48, wherein the agent disrupts expression of the MAPT (microtubule-associated protein tau) gene.
50. The method of any of paragraphs 45-49, wherein the agent inhibits transcription of the MAPT gene.
51. The method of any of paragraphs 45-50, wherein the agent is selected from the group consisting of an antibody, a zinc finger nuclease, a transcriptional repressor, a nucleic acid inhibitor, a small molecule, an aptamer, a gene-editing composition, and a combination thereof.
52. The method of any of paragraphs 45-51, wherein the agent is administered to the brain via a carrier.
53. The method of paragraph 52, wherein the carrier is an adeno-associated virus.
54. The method of any of paragraphs 45-53, wherein the brain of the subject is further determined to have an amyloid beta plaque and the administration reduces neurotoxicity (and/or increases neuron survival) in the presence of amyloid beta.
55. The method of any of paragraphs 45-54, wherein the tauopathy is Alzheimer's disease, Parkinson's disease, or frontotemporal dementia.
56. A solid support comprising soluble HMW tau polypeptide immobilized thereupon, said support substantially lacking LMW tau.
57. A preparation of HMW tau polypeptide comprising covalent cross-links between one or more tau polypeptide monomers.
58. A composition comprising HMW tau covalently conjugated to a carrier polypeptide or an adjuvant.

### Some selected definitions

For convenience, certain terms employed herein, in the specification, examples and appended claims are collected herein. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Further, unless otherwise required by context, singular terms shall include pluralities and plural terms shall include the singular.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

The present invention relates to the herein described aspects, as essential to the invention, yet open to the inclusion of unspecified elements, essential or not ("comprising"). In some cases, other elements to be included in the description of the composition, method or respective component thereof are limited to those that do not materially affect the basic and novel characteristic(s) of the invention ("consisting essentially of"). This applies equally to steps within a described method as well as compositions and components therein. In other cases, the inventions, compositions, methods, and respective components thereof, described herein are intended to be exclusive of any element not deemed an essential element to the component, composition or method ("consisting of").

The singular terms "a," "an," and "the" include plural referents unless context clearly indicates otherwise. Similarly, the word "or" is intended to include "and" unless the context clearly indicates otherwise.

Other than in the operating examples, or where otherwise indicated, all numbers expressing quantities of ingredients or reaction conditions used herein should be understood as modified in all instances by the term "about." The term "about" when used in connection with percentages may mean ± 1% of the value being referred to. For example, about 100 means from 99 to 101.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

The term "neurons" as used herein refers to cells that express one or more neuron-specific markers. Examples of such markers can include, but are not limited to, neurofilament, microtubule-associated protein-2, tau protein, neuron-specific Class III β-tubulin, and NeuN. In some cases, neurons can include cells that are post-mitotic and express one or more neuron-specific markers.

As used herein, the term "transcriptional repressor" refers to an agent (e.g., protein) that binds to specific sites on DNA and prevents transcription of nearby genes. In some cases, the transcriptional repressor is an agent (e.g., protein, peptide, aptamer, and/or a nucleic acid molecule) that binds to specific sites on DNA and prevents transcription of MAPT gene. In some cases, the transcriptional repressor can be regulatable.

As used herein and throughout the specification, the terms "administering," or "administration" refer to the placement of an agent (e.g., an antimicrobial agent) into a subject by a method or route which results in at least partial localization of such agents at a desired site, such as a site of infection, such that a desired effect(s) is produced. Examples of administration routes can include, but are not limited to, intracranial administration, intracortical administration, intracerebroventricular administration, and parenteral administration. The phrase "parenteral administration" as used herein refers to modes of administration other than enteral and topical administration, usually by injection. In some cases, the administration can comprise catheterization (using a catheter).

As used herein an "expression vector" refers to a DNA molecule, or a clone of such a molecule, which has been modified through human intervention to contain segments of DNA combined and juxtaposed in a manner that would not otherwise exist in nature. DNA constructs can be engineered to include a first DNA segment encoding an acetylation-resistant an anti-soluble HMW tau species antagonist or a tau antagonist described herein operably linked to additional DNA segments encoding a desired recombinant protein of interest. In addition, an expression vector can comprise additional DNA segments, such as promoters, transcription terminators, enhancers, and other elements. One or more selectable markers can also be included. DNA constructs useful for expressing cloned DNA segments in a variety of prokaryotic and eukaryotic host cells can be prepared from readily available components or purchased from commercial suppliers.

By "cell culture" or "culture" is meant the growth and propagation of cells outside of a multicellular organism or tissue. Suitable culture conditions for mammalian cells are known in the art. See e.g. Animal cell culture: A Practical Approach, D. Rickwood, ed., Oxford University Press, New York (1992). Mammalian cells can be cultured in suspension or while attached to a solid substrate. Fluidized bed bioreactors, hollow fiber bioreactors, roller bottles, shake flasks, or stirred tank bioreactors, with or without microcarriers, can be used.

As used herein, "cell culture medium" is a media suitable for growth of animal cells, such as mammalian cells, in in vitro cell culture. Cell culture media formulations are well known in the art. Typically, cell culture media are comprised of buffers, salts, carbohydrates, amino acids, vitamins and trace essential elements. "Serum-free" applies to a cell culture medium that does not contain animal sera, such as fetal bovine serum. Various tissue culture media, including defined culture media, are commercially available.

The disclosure is further illustrated by the following examples which should not be construed as limiting. The examples are illustrative only, and are not intended to limit, in any manner, any of the aspects described herein. Further, various changes and modifications to the disclosed embodiments, which will be apparent to those of skill in the art, can be made without departing from the scope of the present invention. The following examples do not in any way limit the invention.

### EXAMPLES

### Example 1. Uptake and propagation of a novel phosphorylated high molecular weight tau species derivedfrom tau-transgenic mouse and human Alzherimer's disease brain

Accumulation and aggregation of microtubule-associated protein tau (1), as intracellular inclusions known as neurofibrillary tangles (NFTs), is a pathological hallmark of neurodegenerative diseases including Alzheimer's disease (AD) (2, 3). Cognitive deficits in AD are most closely linked with progression of NFTs in a hierarchical pattern, starting in the entorhinal cortex (EC) and marching throughout the brain during disease progression (4, 5). Although the precise mechanisms for this characteristic tau pathology spread remain unknown, previous reports suggest a trans-synaptic transfer of tau proteins between neurons (6-8). By developing the rTgTauEC mouse model of early AD that overexpresses human mutant P301L tau selectively in the EC, it has been previously demonstrated that aggregated tau accumulates in synaptically connected downstream areas such as dentate gyrus (DG), indicating that NFT propagation occurs by cross-synaptic spread of pathologically misfolded tau proteins (9-12). It has been reported that tau can be secreted from intact neurons into the extracellular space in an activity-dependent manner (13, 14). However, none of the previous reports identifies a specific extracellular misfolded tau species that can be taken up by neurons, thus contributing to tau pathology spreading. Better understanding of the molecular basis of tau propagation is key to preventing progression from early mild memory impairment to full cognitive deterioration and dementia.

Previous reports showed that cellular tau uptake and trans-cellular propagation occur in various systems in vitro and in vivo; however, whether the tau species involved in neuron-to-neuron transfer is fibrillar or not, and what its specific properties are, is not discussed in any of the previous reports.

In this Example, to identify specific tau species responsible for propagation, the inventors compared the uptake and propagation properties of different tau species derived from brain extracts of tau-transgenic mouse lines rTg4510 (expressing aggregating P301L tau (0N4R)) (15) and rTg21221 (expressing non-aggregating wild-type human tau (0N4R)) (16), human sporadic AD and other tauopathy (two familial frontotemporal dementia cases with P301L or G389R tau mutation) brain extracts, and recombinant wild-type full-length human tau (2N4R, 441 aa). Distinct species of tau were fractionated via differential centrifugation and size exclusion chromatography (SEC), biochemically characterized, and neuronal uptake of each tau species was assessed in mouse primary cortical neurons. For all different sources of tau, efficient uptake was only observed for high-molecular-weight (HMW) tau that was characterized as oligomeric and non-fibrillar using high resolution atomic force microscopy (AFM).

The transfer of tau between neurons was examined using a newly developed microfluidic neuron culture platform, which comprises three distinct chambers that are connected through arrays of thin channels such that the axon growth and formation of synaptic connections are precisely controlled between neurons in different chambers. Furthermore, a unique large-pore (1,000kDa cut-off) probe in vivo microdialysis (17, 18) was used to investigate the presence of HMW tau species in brain interstitial fluid (ISF) of awake, freely-moving mice. The findings presented herein indicate that PBS-soluble phosphorylated HMW tau species, present in the brain extracellular space, are involved in neuronal uptake and propagation, and can be a target for therapeutic intervention and biomarker development.

### Results

### Identification and characterization of tau species taken up by neurons

Identification and characterization of tau species taken up by neurons is critical for understanding the mechanism of neuron-to-neuron tau propagation. The molecular weight (MW) of tau species involved in neuronal uptake was first examined. PBS-soluble brain extracts were prepared from rTg4510 mice, which overexpress human mutant P301L tau, by centrifugation either at 3,000g, 10,000g, 50,000g, or 150,000g, and the supernatant was applied to mouse primary cortical neurons. The uptake of tau was assessed by immunofluorescence labeling of intracellular human tau. After 24 hours, human tau uptake was detected in neurons treated with 3,000g and 10,000g brain extracts, which contained HMW proteins. No uptake occurred from 50,000g and 150,000g extracts **(****Fig. 1A****)** from which HMW tau was depleted by sedimentation. In neurons treated for longer incubation, robust tau uptake was observed from 3,000g extract after 2 and 5 days, however, little uptake occurred from 150,000g extract even after 5 days of incubation **(****Fig. 1B****).**

The MW size distribution of tau species contained in each brain extract was then assessed by size exclusion chromatography (SEC). The 3,000g brain extract had a small peak of HMW tau species (SEC Frc.2-4) in addition to a dominant monomer/dimer peak (SEC Frc.13-16, 50-150 kDa), while the 150,000g brain extract from the same rTg4510 mouse brain had only a monomer/dimer peak and a trace amount of HMW tau species **(****Figs. 1C** and **1D****).** The involvement of HMW tau species in neuronal uptake was validated by incubating each SEC-fraction with primary neurons **(****Fig. 1E****).** The most extensive tau uptake was observed for HMW fractions (Frc.2, 3). Essentially no detectable uptake was observed from the dramatically more abundant lower MW fractions, indicating that HMW tau species were the forms being taken up.

Tau isolated from HMW brain extract fraction by immunoprecipitation was then characterized by atomic force microscopy (AFM) **(****Figs. 1F-1G****).** HMW SEC fraction (Frc. 3) contained small oligomeric but no fibrillar tau aggregates (Fig. If). The tau oligomer size distribution revealed particle heights of 12.1 ± 1.5 (h1) and 16.8 ± 4.2 (h2) nm (height ± s.d., n = 1206) **(****Fig. 1G****).** Tau particle sizes correspond well to the height of tau fibrils assembled from different full length tau isoforms (19). Thus, in some cases, these tau-containing particles can be made exclusively of tau. In some cases, these tau-containing particles can also contain other constituents such as proteins and lipids.

Human tau species observed within primary neurons 2-5 days after exposure to rTg4510 brain extract were Alz50 positive (**Fig. 1H****,** top) but negative for Thioflavin-S (ThioS) staining (**Fig. 1H****,** bottom), indicating an early stage of pathological conformation of tau that is taken up. Furthermore, tau species taken up by primary neurons co-localized with subcellular organelle markers such as the Golgi apparatus and the lysosomes at day 3 (**Fig. 1I**)**.**

Uptake of human tau occurred in a time- and concentration-dependent manner (**Figs. 1J,** and **1K**). Colocalization of human tau with a subcellular marker (Golgi, TGN46) could be observed after 12 hours of incubation and a higher degree of colocalization was observed at day 10 (**Fig. 1J****,** white arrows). Applying different human tau concentrations, it was determined that the minimum concentration of HMW tau from rTg4510 brain extract required for detection of neuronal uptake was 10 ng/ml (**Fig. 1K**), which is lower than the ISF levels of tau in tau-transgenic mouse (approximately 250 ng/ml (20)).

### Phosphorylated HMW tau is taken up by neurons

To evaluate the relevance of tau aggregation and phosphorylation for neuronal uptake, 3,000g brain extracts were prepared from rTg21221 mice and their uptake and biochemical properties were compared to those of rTg4510 homogenate. rTg21221 mice overexpress wild-type human tau under the same promoter as rTg4510 mice and show phosphorylation but no accumulation of misfolded and aggregated tau species in the brain (16). Unlike the case for the rTg4510 mice, no uptake was observed in primary neurons from rTg21221 brain extracts at day 2 (**Fig. 2A****,** top four), although some uptake was evident after longer incubation (day 5) (**Fig. 2A** bottom four), indicating that the species formed by wild-type tau had a slower or less robust uptake kinetic compared to P301L mutant tau. Human tau and total tau levels in PBS-soluble brain extracts were comparable to those seen in rTg4510 brains (**Figs. 2B** and **2C**), although an upward shift of the tau band in western blot (**Fig. 2C****,** arrow) indicated a higher degree of tau phosphorylation in rTg4510 brain.

The degree of tau phosphorylation in rTg4510 and rTg21221 extracts was compared using 10 different phospho-tau epitope specific antibodies (**Fig. 2D**). The PBS-extractable tau species from rTg4510 brain had higher levels of phosphorylation compared to the tau species obtained from rTg21221, especially those associated with some specific phosphorylation sites such as pT205, pS262, pS400, pS404, pS409, and pS422. SEC analysis of the MW distribution of tau demonstrated that rTg21221 brain extracts (PBS-soluble, 3,000g) contained primarily LMW species and very low levels of HMW tau species, whereas rTg4510 brain extract showed both HMW and LMW peaks (**Figs. 2E** and **2F**). The degree of tau uptake into primary neurons correlated significantly with HMW (SEC Frc.2-4, > 669kDa) tau levels, but not with middle molecular weight (MMW) (SEC Frc.9-10, 200-300kDa) or LMW (SEC Frc.13-16, 50-150kDa) tau levels (**Fig. 2G**). The differences in HMW tau levels between rTg4510 and rTg21221 brain extracts were also evaluated by western blot analysis of SEC fractions (**Fig. 2H**); HMW tau from rTg4510 brain was highly phosphorylated (**Fig. 2H****,** bottom). These findings indicate that phosphorylated HMW tau species are taken up by neurons.

### Intercellular propagation of tau in a microfluidic neuron culture platform that models neuron-to-neuron interactions

The transfer of tau between neurons was then assessed using a microfluidic neuron culture platform. The design of this platform includes three distinct chambers forming layering synaptic connections between neurons, which are plated on different chambers and arrays of microgrooves, allowing an exclusive axon growth by sizes **(****Fig. 3A****).** Two sets of neurons are plated into the 1st and 2nd chambers **(****Fig. 3A****).** The axons from the 1st chamber neurons extend into the 2nd chamber within four days (**Fig. 3B****,** left), and axons from the 2nd chamber neurons extend to the 3rd chamber (**Fig. 3B****,** middle). The resulting two sets of neurons therefore have "in line" synaptic connections at the 2nd chamber (**Fig. 3B****,** right). The 1st chamber neurons were labeled with green fluorescent protein (GFP) and the 2nd chamber neurons with red fluorescent protein (RFP) using a hydrostatic pressure barrier to fluidically isolate neurons in different chambers (21). This showed that the two neuronal populations were connected to each other in the 2nd chamber **(****Fig. 3C****).**

### Neuron-to-neuron transfer of rTg4510 mouse brain-derived tau species in the microfluidic chamber

The propagation properties of rTg4510 brain-derived tau species was assessed using the 3-chamber microfluidic neuron chamber. PBS-soluble brain extracts from an rTg4510 mouse (3,000g, 500 ng/ml human tau) were added to the 1st chamber **(****Fig. 4A****).** To assure that only the 1st chamber neurons were exposed to brain extract, the diffusion-driven transport of various tau species was blocked by convective flow in the opposite direction (hydrostatic pressure barrier). After 5 days of incubation, human tau-positive neurons were detected in the axons of the neurons from the 1st chamber, as well as the soma of the neurons in the 2nd chamber **(****Fig. 4B****),** indicating that human tau species taken up by the 1st chamber neurons had been transported through their axons and transferred into the 2nd chamber neurons. Neurons that establish little or no axon-dendrite connection with the 1st chamber neurons (in the side reservoir of the 2nd chamber) remained negative for human tau staining (**Fig. 4B****,** bottom), indicating that axonal input from the 1st chamber is beneficial for tau transfer. Human tau was also detected in axons and dendrites extending from the human tau positive 2nd chamber neurons **(****Fig. 4C****),** indicating further transport of tau species into the 3rd chamber.

The propagation of tau in the microfluidic device was concentration dependent and propagation to the 2nd chamber neurons was detected over the course of a few days when 500-600 ng/ml of human tau (in the 1st chamber) was used (**Figs. 4D****,** and **4E**). These concentrations are similar to the ISF tau levels in tau-transgenic mice (20). Time-course analysis showed early uptake of human tau in the 1st chamber neurons (as early as day 1), propagation to the 2nd chamber neurons after 5 days, and progression to the 2nd chamber neuron axon terminals in the 3rd chamber after 8 days **(****Fig. 4E****).** The distance longer than 1300 µm between the 1st and 3rd chambers indicate that in order to reach the 3rd chamber at 8 days the transport of the tau cannot rely solely on diffusion.

### High axonal transport processivity and stability of internalized HMW tau

To assess the stability of tau in primary neurons after uptake, brain extract from rTg4510 (3,000g, 500 ng/ml human tau) was added into the 1st chamber of the microfluidic device and excess tau was removed before (at day 2, **Fig. 4E**) or after (at day 5, **Fig. 4E**) tau had propagated to the 2nd chamber neurons, and neurons were further cultured for 6 (day 2-8) or 9 (day 5-14) days, respectively **(****Fig. 5A****).** Surprisingly, human tau positive neurons in the 2nd chamber were detected even after removal of brain extract from the 1st chamber prior to propagation (at day 8, 6 days after excess tau removal) **(****Fig. 5B****).** Similarly, human tau positive axons were observed in the 3rd chamber at day 8 (3 days after removal of brain extract from the 1st chamber) **(****Fig. 5C****).** These findings indicate that once a certain amount of tau was taken up by the neurons, tau could propagate to the next neuron even after removal of transmissible extracellular tau species. Tau species taken up by the 1st chamber neurons or propagated to the 2nd chamber neurons could be detected for up to 6 days (day 2-8, **Fig. 5B**) or 9 days (day 5-14, **Fig. 5C**) after washing out the human tau from the medium, indicating a slow degradation of HMW tau species in cultured neurons.

### Neuronal uptake of phosphorylated HMW tau species derived from human AD brain

Uptake and propagation properties of tau species derived from human AD and control brain tissues were next examined. Like rTg4510 brain extract as shown earlier, the PBS-soluble extract from human AD brain contained tau species that could be taken up by mouse primary neurons **(****Fig. 6A****).** These tau species again were found only in the 3,000g extract **(****Figs. 6A-6B****).** No uptake was observed from human control brain extracts **(****Figs. 6A-6B****).** The tau species taken up by neurons co-localized with markers for the Golgi apparatus and the lysosomes **(****Fig. 6C****),** indicating the internalization and intracellular processing of tau. The tau species from AD brain extracts also propagated between neurons in the 3-chamber microfluidic device within 7 days **(****Fig. 6D****).**

Total tau levels in brain extracts from AD and control brains were similar, showing a trend towards decreased PBS-soluble tau levels in the AD brain **(****Fig. 6E****).** However, the AD brain extract (3,000g) contained significantly higher levels of phosphorylated HMW tau (**Figs. 6F** and **6J**) when compared to the control brain. Surprisingly, both AD and control brain extracts (PBS-soluble, 3,000g) had comparable total amounts of HMW tau species on SEC analysis (**Figs. 6G** and **6H**), despite the clear difference in tau uptake by primary neurons from the AD and control extracts **(****Fig. 6A****).** The involvement of AD brain-derived HMW tau species in neuronal uptake was confirmed by incubating each SEC fraction with primary neurons **(****Fig. 6I****).** Little uptake of the lower MW fractions occurred, even when tau was supplied at 100 times higher concentrations (5 vs. 500 ng/ml human tau in the medium).

Phosphorylation levels of tau species in each SEC fraction were then measured. The HMW tau species from the AD brain were highly phosphorylated compared to those from control brain **(****Fig. 6J****).** Notably, most of the highly phosphorylated tau species from PBS-soluble AD brain extract were detected in the HMW fractions **(****Fig. 6J****).** These findings indicate the presence of phosphorylated HMW tau species in PBS-soluble extracts from AD brain tissue and indicate that these phosphorylated forms can be at least one of the forms taken up and propagated by neurons.

### Phosphorylation of tau correlates with neuronal uptake

To examine the role of phosphorylation in uptake and propagation of tau, PBS-soluble extracts of FTD brains with P301L and G389R tau gene mutations were prepared **(****Fig. 7A****),** which also contained tau species that could be taken up by primary neurons (**Fig. 7B****,** left) but differed from AD brain extracts in their tau phosphorylation state (pS396, **Fig. 7C**). The degree of tau uptake from these brain extracts was lower than that seen with AD brain extracts (**Fig. 7B****,** right) and correlated with tau phosphorylation levels **(****Fig. 7C****).**

It was next sought to determine whether post-translational modifications or, simply, size of oligomer, was desirable for neuronal uptake. To this end, a monomer-dimer-oligomer tau mixture from recombinant human wild-type full-length tau (441 aa) was prepared and separated by SEC **(****Fig. 7D****).** Each SEC fraction of this non-phosphorylated tau mixture was then incubated **(****Fig. 7E****)** with mouse primary neurons. No uptake was observed in primary neurons even from HMW tau fractions **(****Fig. 7F****).** Taken together, these findings indicate that, without wishing to be limited by theory, phosphorylation, other post-translational modification, or complexation with molecules other than tau may be required for neuronal uptake, although endogenous tau in rTg4510 mice or human AD and FTD brains might adopt conformations different from recombinant tau under the conditions tested.

### Brain extracellular tau species can be taken up by primary neurons

It is known that soluble tau species exist in the cerebrospinal fluid (CSF) and the interstitial fluid in the brain (20). However, the soluble HMW tau species described herein in the postmortem brain has not been previously reported, due to, e.g., limitations of the microdialysis probes. As shown in **Fig. 8A****,** The inventors employed a unique large-pore (1,000kDa cut-off) probe microdialysis technique with push-pull perfusion system that allows consistent collection of HMW molecules from the brain ISF of awake, freely-moving mice (17, 18). SEC fractionation followed by human tau-specific ELISA demonstrated that brain ISF from rTg4510 mouse contained HMW tau species in addition to monomer/dimer, or truncated tau **(****Fig. 8C****).** ISF tau from rTg4510 mouse was taken up by primary neurons after 3 days of incubation **(****Fig. 8D****),** with 40 ng/ml total human tau being sufficient to detect tau uptake **(****Fig. 8E****).** These data show that secreted tau, present in the ISF of awake behaving animals, can be taken up by neurons and therefore, without wishing to be bound by theory, can account for the propagation of tau across neural systems observed in transgenic models.

### Discussion

Identifying the tau species that can be transferred between neurons is essential for understanding mechanisms by which misfolded tau propagates in AD and other tauopathies. Here, the uptake and propagation properties of tau from various sources were characterized: brain extracts and ISF from tau-transgenic mice, brain extracts from postmortem AD and FTD patients, and recombinant human tau protein. It was discovered that a rare HMW tau species, which accounts for only a small fraction of all tau in the samples, was robustly taken up by neurons, whereas uptake of monomer/dimer-size tau was very inefficient; findings from the microfluidic neuron culture platform showed that this rare species is uniquely capable of propagating between neurons. Furthermore, tau with similar biochemical characteristics can be identified in the brain ISF of rTg451 animals obtained while they were awake and behaving, indicating that it is present in the brain; this ISF can also donate tau that can be taken up by neurons in culture. Together, these data indicate that (i) a relatively rare, HMW, phosphorylated tau oligomers are released from neurons and found in brain ISF; and (ii) this species can be taken up, axonally transported, secreted, and taken up by synaptically connected neurons and thus "propagated". These results can allow further molecular characterization of the "propagating" species, and provide a technical platform for assaying propagation in vitro in days, a dramatically faster time scale than with the transgenic mouse models (∼18 months) (10) or injection of brain homogenate in mouse cortex (1-2 months) (22).

Uptake of tau from mice expressing aggregating P301L tau (rTg4510) depended on tau MW, correlated with the level of phosphorylation. These findings indicate that oligomerization and pathological phosphorylation increased the uptake efficiency of tau. Accordingly, tau from mice expressing non-aggregating wild-type human tau (rTg21221), which contained minor amount of HMW tau and less phosphorylated tau, showed slower uptake. The HMW tau species had a pathologically misfolded conformation (positive for Alz50 antibody staining) and appeared as oligomeric structures in AFM. Previous studies reported that synthetic tau fibrils (23-26) or fibrillar tau species, which are not soluble HMW tau species described herein, extracted from tau-transgenic mouse brain (22, 27) could be taken up by neurons and induce filamentous tau pathology in vitro and in vivo. The findings herein indicate that more soluble, misfolded tau oligomers are present in the extracellular spaces and also likely play a role in propagation.

The findings described herein do not entirely exclude the involvement of lower MW tau species in uptake and propagation. It may well be possible that the concentration of tau used in this study (500 ng/ml human tau) was too low for uptake of LMW tau species, or that immunostaining was not sensitive enough to detect LMW tau internalized by neurons. Michel et al. (28) demonstrated that extracellular monomeric tau (LMW tau species) enters SH-SY5Y neuroblastoma cells at concentration as high as 1 µM (approximately 55 µg/ml).

PBS-soluble extracts from human AD brains contained comparable amounts of HMW tau as control brains but showed significantly higher phosphorylation and uptake of tau, indicating a role of phosphorylation in HMW tau uptake. Further, there was little uptake of non-phosphorylated HMW tau species prepared from recombinant tau. PBS-soluble brain extracts from FTD patients with P301L and G389R mutations also contained highly phosphorylated tau and showed neuronal tau uptake. Tau uptake was highest in AD brain cortical extracts, followed by G389R and then P301L tau mutant brain extracts, which appeared to be consistent with the extent of tau phosphorylation levels observed in each brain extract. Although the P301L clearly supports NFT formation **(****Fig. 7A****),** the G389R mutation is associated with frontotemporal dementia without filamentous tau inclusions, thus indicating that soluble tau species rather than filamentous aggregates is involved in the propagation phenomenon described herein. Without wishing to be bound by theory, phosphorylation, aggregation, or both can be desirable factors for uptake and propagation of HMW tau.

Yanamandra et al. reported that anti-tau antibodies improved cognitive deficits in a tau-transgenic mouse model associated with reduction of brain insoluble tau species (70% formic acid fraction), although levels of soluble fraction tau were unchanged by immunotherapy (29). That is, Yanamandra et al. do not describe use of antibodies to specifically target soluble HMW tau species or soluble HMW phospho-tau species described herein. The amount of HMW phospho-tau species that was characterized in this Example accounted for less than 1% of the total PBS-soluble tau in AD brain extracts, and less than 10% even in mutant-tau overexpressing rTg4510 mouse brain extracts, in contrast to the much more abundant monomer/dimer-size low MW tau species. Therapeutic targeting of this low abundant HMW tau species can be a more effective therapeutic approach.

As shown in this Example, neuronal uptake of HMW tau could occur within 24 hours. Tau fibrils were shown to enter cells via macropinocytosis within a similar time scale (29). Further, it was discovered herein that uptake and propagation of HMW tau was concentration-dependent. Notably, tau uptake from HMW SEC fractions occurred at relatively low concentrations (less than 50 ng/ml of human tau), which are lower than the ISF total tau levels reported for tau-transgenic mice (20).

It should be noted that after tau uptake occurred in neurons in the 1st chamber of microfluidic devices, tau propagation to neurons in the 2nd chamber continued even after removing all excess tau from the 1st chamber. This indicates that, in some cases, interventions seeking to block tau propagation in AD need to be started during an early stage of the disease, before substantial tau accumulation occurs.

The intracellular accumulation of insoluble tau aggregates has long been considered to be toxic to neurons (30); however, a recent report discusses that insoluble tau aggregates are not sufficient to impair neuronal function (31-34). Furthermore, extracellular tau can bind and activate neuronal muscarinic receptors (35, 36), indicating extracellular functions of tau that so far have been largely neglected. Identifying normal and pathophysiological roles of extracellular tau species can enable development of novel and efficient therapeutic strategies against soluble tau species, e.g., soluble HMW tau species.

The findings presented herein, at least in part, show that PBS-soluble HMW phospho-tau species, present in the extracellular space, are involved in uptake and propagation between neurons. Intervention to deplete these specific extracellular tau species can inhibit tau propagation and hence disease progression in tauopathies.

### Exemplary materials and methods used in Example 1

**Animals.** Eleven- to 13-month-old rTg4510, rTg21221, and control animals were used in this study. The rTg4510 (P301L tau) mouse is a well-characterized model of tauopathy, which overexpresses full-length human four-repeat tau (0N4R) with the P301L frontotemporal dementia (FTD) mutation (15). rTg21221 (wild-type tau) mouse expresses wild-type human tau at levels comparable to rTg4510 mouse and does not show accumulation of tau pathology in the brain (16). Littermate animals with only the activator CK-tTA transgene, which do not overexpress tau, were used as controls. Both male and female mice were used. All experiments were performed under national (United States National Institutes of Health) and institutional guidelines.

**Human brain samples.** Frozen brain tissues from the frontal cortex of three patients with AD, three non-demented control subjects, and two familial frontotemporal dementia cases (P301L and G389R tau mutation) were obtained, e.g., from the Massachusetts Alzheimer's Disease Research Center Brain Bank. The demographic characteristics of the subjects are shown in **Table 2** below. All the study subjects or their next of kin gave informed consent for the brain donation, and the Massachusetts General Hospital Institutional Review Board approved the study protocol. All the AD subjects fulfilled the NIA-Reagan criteria for high likelihood of AD. Cortical gray matter was weighed and processed as described in the following section (Brain extraction).

**Table 2. Characteristics of the subjects with AD, FTD, and controls used in the study.**

| **Case (sample #)** | **Age at death (years)** | **Sex** | **Postmortem-interval (hours)** | **Diagnosis** | **Braak stage, CERAD score** |
|---|---|---|---|---|---|
| AD 1 (#1762) | 71 | Female | 16 | AD | VI. C |
| AD 2 (#1746) | 60 | Male | 24 | AD | VI. C |
| AD 3 (#1745) | 85 | Male | 24 | AD | VI. C |
| Control 1 (#1722) | 91 | Female | 8 | Control | I. A |
| Control 2 (#1703) | 73 | Female | 20 | Control | - |
| Control 3 (#1669) | 86 | Male | 10 | Control | |
| FTD P301L (#150) | 70 | Male | 12 | FTD | |
| FTD G389R (#1691) | 33 | Male | 33 | FTD | |

**Brain extraction.** Mice were perfused with cold PBS containing protease inhibitors (protease inhibitor mixture; Roche, Indianapolis, IN, USA), and the brain was rapidly excised and frozen in liquid nitrogen, then stored at -80°C before use. Brain tissue was homogenized in 5 volumes (wt/vol) of cold PBS using a Teflon-glass homogenizer. The homogenate was briefly sonicated (Fisher Scientific Sonic Dismembrator Model 100, output 2, 6 × 1 sec) and centrifuged at 3,000 × g for 5 min at 4°C (3,000g extract), 10,000 × g for 15 min at 4°C (10,000g extract), 50,000 × g for 30 min at 4°C (50,000g extract), or 150,000 × g for 30 min at 4°C (150,000g extract). The supernatants were collected and stored at -80°C before use.

**Primary cortical neuron culture.** Primary cortical neurons were prepared from cerebral cortices of embryonic day (E) 14 - 15 CD1 mouse embryos (Charles River Laboratories) as described previously (37) with modifications. Cortices were dissected out and mechanically dissociated in Neuro-basal (Life Technologies, Inc., Gaithersburg, MD, USA) medium supplemented with 10% fetal bovine serum, 2 mM Glutamax, 100 U/ml penicillin, and 100 g/ml streptomycin (plating medium), centrifuged at 150 g for 5 min, and resuspended in the same medium. Neurons were plated at a density of 0.6 × 10⁵ viable cells on a Lab-Tek 8-well chambered coverglass (Nalge Nunc) or microfluidic devices (see below for cell density and protocol) previously coated with poly-D-lysine (50 µg/ml, Sigma) overnight. Cultures were maintained at 37°C with 5% CO2 in Neuro-basal medium with 2% (v:v) B27 nutrient, 2 mM Glutamax, 100 U/ml penicillin, and 100 g/ml streptomycin (culture medium).

**Tau uptake in primary neurons.** Mouse primary neurons (7-8 days in vitro) were incubated with PBS-soluble brain extracts (3,000g, 10,000g, 50,000g, or 150,000g centrifugation supernatant, or SEC fractions from 3,000g extract) from mouse (rTg4510 or rTg21221 animals) or human (control, sporadic AD, or familial frontotemporal dementia cases) brain tissues, microdialysate from rTg4510/control mice, or recombinant tau oligomer mixture solution. Neurons were maintained at 37°C in 5% CO₂ in a humidified incubator. Each sample was diluted with culture medium to obtain the designated human tau concentrations (measured by human tau ELISA). Neurons were washed extensively with PBS, fixed, and immunostained with human tau-specific antibody (Tau13) to detect exogenously applied human tau in mouse primary neurons at the designated time point. For most experiments described in this Example, total (human and mouse) tau antibody (DAKO) was used as a neuronal marker. Each sample was filtered through a 0.2-µm membrane filter to remove large aggregates and fibrils before incubation.

**Atomic force microscopy (AFM).** Immunoprecipitation (IP) isolation of tau from rTg4510 brain extract for AFM analysis was performed as described previously with minor modifications (38). Briefly, tosylactivated magnetic Dynabeads (#14203, Life Technologies) were coated with human tau-specific Tau13 antibody. Beads were washed (0.2 M Tris, 0.1% bovine serum albumin, pH 8.5) and incubated with either PBS-soluble brain extract from rTg4510 mouse (10,000g, total extract) or HMW SEC fraction (Frc.3 from 10,000g extract) sample for 1 hour at R.T. Beads were washed three times with PBS and eluted using 0.1 M glycine, pH 2.8, and the pH of each eluted fraction was immediately adjusted using 1 M Tris pH 8.0. For AFM imaging, isolated tau fractions were adsorbed onto freshly cleaved muscovite mica and imaged using oscillation mode AFM (Nanoscope III, Di-Veeco, Santa Barbara, CA) and Si3N4 cantilevers (NPS series, Di-Veeco) in PBS, as described previously (19).

**In vivo microdialysis.** In vivo microdialysis sampling of brain interstitial fluid tau was performed as described previously (17, 18). The microdialysis probe had a 4 mm shaft with a 3.0 mm, 1000 kDa molecular weight cutoff (MWCO) polyethylene (PE) membrane (PEP-4-03, Eicom, Kyoto, Japan). This probe contains a ventilation hole near the top which serves to produce a reservoir of fluid within the probe that is open to the atmosphere. This structure minimizes pressure which would otherwise cause a net flow of perfusate out through the large pore membrane. Before use, the probe was conditioned by briefly dipping it in ethanol, and then washed with an artificial cerebrospinal fluid (aCSF) perfusion buffer (in mM: 122 NaCl, 1.3 CaCl₂, 1.2 MgCl₂, 3.0 KH₂PO₄, 25.0 NaHCO₃) that was filtered through a 0.2 µm pore-size membrane. The preconditioned probe's outlet and inlet were connected to a peristaltic pump (ERP-10, Eicom, Kyoto, Japan) and a microsyringe pump (ESP-32, Eicom, Kyoto, Japan), respectively, using fluorinated ethylene propylene (FEP) tubing (ϕ 250 µm i.d.).

Probe implantation was performed as previously described (17), with slight modifications. Briefly, the animals were anesthetized with isoflurane, while a guide cannula (PEG-4, Eicom, Kyoto, Japan) was stereotactically implanted in the hippocampus (bregma -3.1 mm, -2.5 mm lateral to midline, -1.0 mm ventral to dura). The guide was fixed using binary dental cement.

Three or four days after the implantation of the guide cannula, the mice were placed in a standard microdialysis cage and a probe was inserted through the guide. After insertion of the probe, in order to obtain stable recordings, the probe and connecting tubes were perfused with aCSF for 180 min at a flow rate of 10 µl/min before sample collection. Samples were collected at a flow rate of 0.5 µl/min and stored at 4 °C in polypropylene tubes. During microdialysis sample collection, mice were awake and freely-moving in the microdialysis cage designed to allow unrestricted movement of the animals without applying pressure on the probe assembly (AtmosLM microdialysis system, Eicom, Kyoto, Japan).

**Tau ELISA.** The concentrations of human tau in the samples (brain extracts, brain ISF samples, and recombinant human tau solution, and SEC-separated samples) were determined by Tau (total) Human ELISA kit (#KHB0041, Life Technologies) and Tau [pS396] Human ELISA kit (#KHB7031, Life Technologies), according to the manufacturer's instructions.

**Immunoblot analysis.** Brain extracts were electrophoresed on 4-20% Novex Tris-Glycine gels (Life Technologies, Grand Island, NY, USA) in Tris-Glycine SDS running buffer for SDS-PAGE (Life Technologies). Gels were transferred to PVDF membranes, and membranes were blocked for 60 min at R.T. in 5% (w:v) BSA / TBS-T, and then probed with primary antibodies overnight at 4°C in 2% (w:v) BSA / TBS-T. The following primary antibodies were used: mouse monoclonal antibody DA9 (total tau (aa112-129), courtesy of Peter Davies, 1:5000), mouse monoclonal antibody PHF1 (pS396/pS404 tau, courtesy of Peter Davies, 1:5000), mouse monoclonal antibody CP13 (pS202 tau, courtesy of Peter Davies, 1:1000), rabbit polyclonal anti-phospho tau antibodies (pS199 (#44734G), pT205 (#44738G), pS262 (#44750G), pS396 (#44752), pS400 (#44754G), pS404 (#44758G), pS409 (#44760G), and pS422 (#44764G)) from Life Technologies (1:2000 dilution for these antibodies), and mouse monoclonal anti-actin antibody (#A4700, Sigma-Aldrich, 1:2500). After washing three times in PBS-T, blots were incubated with HRP-conjugated goat anti-mouse (#172-1011, Bio-Rad) or anti-rabbit (#172-1019, Bio-Rad) IgG secondary antibodies (1:2000 dilution) for 1 hour at R.T. Immunoreactive proteins were developed using an ECL kit (Western Lightning, PerkinElmer, Waltham, MA, USA) and detected on Hyperfilm ECL (GE healthcare, Pittsburgh, PA, USA). 15µg protein/lane were loaded, unless indicated otherwise. Scanned images were analyzed using Image J (National Institutes of Health).

**Size-exclusion chromatography** (SEC). Brain PBS-soluble extracts, ISF microdialysate, oligomer tau (recombinant hTau-441) mixture solution were separated by size-exclusion chromatography (SEC) on single Superdex200 10/300GL columns (#17-5175-01, GE Healthcare) in phosphate buffered saline (#P3813, Sigma-Aldrich, filtered through a 0.2-µm membrane filter), at a flow rate of 0.5 ml/min, with an AKTA purifier 10 (GE Healthcare). Each brain extract was diluted with PBS to contain 6000 ng of human tau in a final volume of 350 µl, which was filtered through a 0.2-µm membrane filter and then loaded onto an SEC column. The individual fractions separated by SEC were analyzed by ELISA (Tau (total) Human ELISA kit, diluted 1:50 in kit buffer). For the ISF sample, 400 µl of microdialysate from rTg4510 mice was loaded onto the column after filtration through a 0.2-µm membrane, and SEC fractions were measured by human tau ELISA. For the oligomer tau mixture solution, 500 µl of sample (hTau-441, 3.35 mg/ml with 2 mM DTT, filtered through a 0.2-µm membrane filter) was loaded onto column and each SEC-fraction was diluted 1:200,000 in kit buffer for human tau ELISA.

**Immunocytochemistry.** Primary neurons were washed extensively with PBS (three times) and fixed with 4% paraformaldehyde (PFA) for 15 min. Neurons were washed with PBS, permeabilized with 0.2% Triton X-100 in PBS for 15 min (R.T.), blocked with 5% normal goat serum (NGS) in PBS-T (R.T.), and then incubated with the primary antibodies overnight at 4°C in 2% NGS / PBS-T. The following primary antibodies were used to detect tau: mouse monoclonal antibody Tau13 (specific for human tau (aa20-35), #MMS-520R, Covance, 1:2000), rabbit polyclonal anti-total tau antibody (recognizes both human and mouse tau, #A0024, DAKO, 1:1000), mouse monoclonal antibody Alz50 (the conformation-specific antibody, courtesy of Peter Davies, Albert Einstein College of Medicine; 1:100). Goat anti-mouse Alexa488 and anti-rabbit Alexa555 secondary antibodies (Life Technologies, 1:1,000) were applied in 2% NGS in PBS-T for 1 hour at R.T. CY3-labeled anti-mouse IgM secondary antibody (Invitrogen, 1:200) was used to detect Alz50. After washing in PBS, coverslips were mounted with aqueous mounting medium (Vectashield). For co-staining with ThioS, neurons were first immunostained with rabbit polyclonal antibody TAUY9 (specific for human tau (aa12-27), #BML-TA3119-0025, Enzo Life Sciences, 1:200) and goat anti-rabbit Alexa555 secondary antibody (Invitrogen, 1:200), and then incubated with 0.025% (w:v) ThioS in 50% ethanol for 8 min. ThioS was differentiated in 80% ethanol for 30s. Neurons were washed with water for 3 min, and coverslips were mounted using mounting medium (Vectashield).

The subcellular localization of human tau was studied by co-staining with the following primary antibodies: rabbit polyclonal anti-TGN46 antibody (the Golgi apparatus marker, #ab16059, Abcam, 1:200), rabbit polyclonal anti-GRP94 antibody (the endoplasmic reticulum marker, #ab18055, Abcam, 1:100), rabbit polyclonal anti-LAMP2a antibody (the lysosome marker, #ab18528, Abcam, 1:200), rabbit polyclonal anti-Rab5 antibody (the endosome marker, #ab13253, Abcam, 1:200), rabbit polyclonal anti-catalase antibody (the peroxisome marker, #ab1877, Abcam, 1:200). Goat anti-rabbit Alexa555 secondary antibody (Invitrogen, 1:200) was used to detect subcellular markers. Images were acquired using confocal microscope (Zeiss Axiovert 200 inverted microscope, Carl Zeiss).

**Immunostaining of brain sections.** Twelve-month-old rTg4510 mice were euthanized by CO2 asphyxiation and perfused with 4% PFA in PBS. Brains were postfixed in 4% PFA for 2 days at 4°C, incubated for 2 days in 30% sucrose in PBS, and then 40-µm-thick sagittal sections were cut on a freezing sliding microtome. Sections were permeabilized with 0.2% Triton-X100 in PBS, blocked in 5% NGS in PBS, and incubated in primary antibody (mouse monoclonal antibody Alz50 (courtesy of Peter Davies, 1:100)) in 2% NGS in PBS overnight at 4°C. CY3-labeled anti-mouse IgM secondary antibody (Invitrogen, 1:200) was applied in 2% NGS in PBS for 1 hour (R.T.). After washing in PBS, brain slices were mounted on microscope slides, and coverslips were mounted using DAPI containing mounting medium (Vectashield). NFTs were stained with 0.025% ThioS in 50% ethanol for 8 min. ThioS was differentiated in 80% ethanol for 30 sec. Sections were washed with water for 3 min, and coverslips were mounted using mounting medium.

**Tau immunostaining of human brain tissue.** Postmortem human brain tissues were fixed with 10% formalin, treated with 90% formic acid for one hour, and then embedded into paraffin. 5-µm sections were cut, deparaffinized, and hydrated in water. Sections were immunostained with ABC Vectastain kit (#PK-4001, Vector Laboratories), according to the manufacturer's instructions. Sections were blocked in normal serum (30min at R.T.), washed twice in TBS, and incubated with rabbit polyclonal anti-tau antibody (#A0024, DAKO, 1:3000 in TBS) for one hour at R.T. After washing twice in TBS, sections were incubated with biotinylated secondary antibody (Vector Laboratories) for 30 min at R.T., washed twice in TBS, and incubated with Vectastain ABC reagent for 30 min at R.T. After washing twice in TBS, sections were incubated with DAB peroxidase substrate, rinsed in water, and then counterstained with hematoxylin. Images were obtained using an Olympus BX51 microscope mounted with a DP 70 Olympus digital camera (20× objective, UPlanApo).

**Recombinant human tau expression.** Human full-length wild-type tau (2N4R, 441 aa) was expressed in E. coli BL21 DE3 using tau/pET29b plasmid (Adgene). Expression was induced at OD = 0.6 by adding 1 mM IPTG for 3.5 h at 37°C. Tau purification was performed by heat treatment and FPLC Mono S chromatography (Amersham Biosciences) as described previously (39). Cells of 300 ml culture were boiled in 3 ml buffer solution [50 mM MES pH 6.8, 500 mM NaCl, 1 mM MgCl2, 5 mM DTT] for 20 min. Whole cell lysate was ultracentrifuged at 125,000g for 45 min, and supernatant was dialyzed (MWCO 20 kDa) against 20 mM MES pH 6.8, 50 mM NaCl, 2 mM DTT. Protein and tau content was determined by BCA assay kit (Pierce), SEC, and Western Blot. The sample contained a mixture of monomeric, dimeric, and oligomeric recombinant tau.

**Microfluidic three-chamber devices.** A neuron-layering microfluidic platform was designed, where the platform is composed of three distinct chambers connected through microgroove arrays (3 × 8 × 600 µm in height, width, and length) using standard soft lithographic techniques (40). The length of the microgrooves was selected such that no MAP2-positive dendrites entered the adjacent chambers **(****Fig. 3B****).** Taylor et al. reported that a 450 µm microgrooves are sufficiently long to isolate axon terminals from soma and dendrites (21). The platform was punched on two side reservoirs of each chamber and bonded to a poly-D-lysine (50 µg/ml, Sigma) coated glass-bottom dish (#P50G-1.5-30-F, MatTek Corporation) to enhance neuronal adhesion.

First, primary cortical neurons isolated from E15 mouse embryos were plated into the 1st chamber at an approximate density of 0.6 × 10⁵ viable cells (in 10 µl plating medium) per device. After 15 min (most neurons in the 1st chamber adhered to the bottom of dish during this period), 0.3 × 10⁵ viable cells in 2 µl plating medium were loaded into the 2nd chamber via one of the side reservoirs. Microfluidic devices were set at a tilt (approximately an 80 degree angle) in an incubator (37°C in 5% CO₂) immediately after neurons were plated into the 2nd chamber, so that the neurons would settle down to surfaces close to the third chamber by gravity. Therefore, most neurons in the 2nd chamber were plated in a line along a sidewall of the 2nd chamber, which had microgrooves connecting to the 3rd chamber. We empirically established this protocol to allow most of the axons of neurons in the 2nd chamber to extend into the 3rd chamber **(****Fig. 3B****).** After 3 h, the devices were set in a normal position without tilting and maintained at 37°C in 5% CO₂ in culture medium. The medium was changed every 4-5 days.

For tau uptake and the propagation assay, PBS-soluble extracts from rTg4510 and AD brain tissue were added to the 1st chamber (10 µ in total) on 7 or 8 DIV. The 2nd and 3rd chambers were filled with 40 µl of media (20 µl in each reservoir). The volume difference between the chambers resulted in continuous convection ("hydrostatic pressure barrier"; 10 µl in the 1st chamber and 40 µl in the 2nd and 3rd chambers), which prevented diffusion of brain extract from the 1st chamber into other chambers. After incubation for the designated periods, neurons were washed, fixed, and immunostained as described above (see "Immunocytochemistry").

Transfections of GFP and RFP **(****Fig. 3C****)** were carried out inside microfluidic devices as follows: Initially, the 1st chamber neurons (7 DIV) were transfected with GFP (0.04 µg DNA + 0.1 µl of Lipofectamine 2000 (#11668-019, Life Technologies) in 10 µl ofNeuroBasal for 3 h. Diffusion of DNA from the 1st chamber to the 2nd chamber was prevented by a hydrostatic pressure barrier, as described above. After washing the DNA (GFP)-containing medium from the 1st chamber (washed three times with NeuroBasal), the 2nd chamber neurons were transfected with RFP (0.04 µg DNA + 0.1 µl of Lipofectamine 2000 in 10 µl NeuroBasal) for 3 h. Diffusion of DNA from the 2nd chamber to the 1st and 3rd chambers was prevented by the hydrostatic pressure barrier (40 µl media in the 1st and 3rd chambers). After washing the DNA (RFP)-containing medium from the 2nd chamber (washed three times with NeuroBasal), devices were maintained at 37°C in 5% CO₂ in culture medium. Expressions of GFP and RFP were examined 2 days later.

Neurons in the microfluidic device were examined using a Zeiss Axiovert 200 inverted microscope (Carl Zeiss) equipped with a Zeiss LSM 510 META (Zeiss, Jena, Germany) confocal scanhead using 488- and 543- nm lasers. All images were acquired using a 25× APO-Plan Neoflu lens or 63× 1.2 NA C-APO-Plan Neoflu lens (Carl Zeiss).

**Statistical analysis.** All data were expressed as mean ± S.E.M. Two-group comparisons were performed by Student's t test. Comparison of means among three or more groups was performed by analysis of variance (ANOVA) followed by Tukey-Kramer multiple range test. Correlations were analyzed with Spearman's rank test. P values less than 0.05 were considered significant.

### Example 2. Removing endogenous tau does not prevent tau propagation and is neuroprotective

Neurofibrillary tangle (NFT) formation is a key feature of several neurodegenerative disorders, including Alzheimer's disease and frontotemporal dementia (1, 2). Progressive tangle appearance in AD follows a highly consistent pattern: starting in the entorhinal cortex (EC), NFT pathology then "spreads" to connected regions (3, 4). In mice, restriction of human mutant P301L tau expression to the EC (ECrTgTau model) revealed trans-synaptic travel of misfolded tau (5-7). A "prion-like" progression of tau misfolding, in which aberrantly folded tau propagates trans-synaptically and then recruits naive endogenous mouse tau in downstream cells and templates toxic aggregation, is widely viewed as a possible mechanism for the spread of tau pathology (8-10). Endogenous mouse tau can co-aggregate with human tau (5, 11). Neurons expressing mutant tau ultimately die, presumably due to intracellular tau aggregate accumulation.

In this Example, the inventors sought to determine if tau spreading and toxicity rely on prion-like mechanisms of corrupting endogenous tau by misfolded "seeds." In contrast to common belief based on prion analogy, the inventors show that synaptic tau transmission surprisingly occurs independently of endogenous tau in postsynaptic cells. Comparing 18-month old mice expressing human P301L tau in the EC in presence (ECrTgTau) or absence (ECrTgTau-Mapt0/0) of endogenous mouse tau, it was found that similar amounts of human tau propagated from the EC to the hippocampus. Neuron-to-neuron transmission of transgenic tau thus can occur independently of endogenous tau and therefore differs from prion-like templated misfolding. Surprisingly, ECrTgTau-Mapt0/0 mice had significantly less tau phosphorylation, lacked pathological misfolding, and showed less gliosis than ECrTgTau mice. Furthermore, mice with P301L tau expression throughout the cortex (rTg4510 model) developed tangles both in presence (rTg4510) and absence (rTg4510-Mapt0/0) of mouse tau, but the pronounced transgenic tau induced brain atrophy and neuronal loss observed in 9 and 12 month old Tg4510 mice were rescued on the Mapt0/0 background. These data show that the lack of endogenous tau protects neurons against toxicity associated with mutant tau expression and aggregation, therefore dissociating tau propagation, aggregation and toxicity in vivo.

### Trans-synaptic propagation of transgenic tau in absence of endogenous tau

Neuron-to-neuron propagation of aggregated tau, followed by templated misfolding of naive endogenous tau, has been reported as a mechanism enabling the progression of NFT pathology in AD and other tauopathies. By analogy to the spreading of prion protein misfolding, the absence of naive tau would stop spreading of tau (12). Given that misfolded tau aggregates are generally considered to be toxic, prevention of interneuronal tau transfer would also eliminate the spread of tau-induced toxicity. To test this hypothesis, transgenic mice that expressed human P301L tau in the entorhinal cortex and lacked endogenous mouse tau (ECrTgTau-MaptO/O) were generated (**Fig. 10A** and **Fig. 15A**) and examined whether tau propagated along the perforant pathway from the EC to neurons in the dentate gyrus (DG). Surprisingly, ECrTgTau-MaptO/O mice showed robust propagation of transgenic tau to DG neurons by immunostaining of horizontal brain sections using human tau N-terminus specific antibodies (Tau13 and TauY9; **Fig. 10B**). We found that the number of human tau containing neurons in the DG was similar compared to mice expressing P301L tau in presence of mouse tau (ECrTgTau; **Fig. 10C****,** n=4, p=0.58). Levels of transgenic tau in EC (p=0.29) and HPC (p=0.14) extracts were also comparable in both ECrTgTau-MaptO/O and ECrTgTau mice **(****Fig. 10D****,** **Figs. 15B-15C****).** Co-labeling with tau C-terminus specific antibody (DAKO, recognizes mouse and human tau) in ECrTgTau-MaptO/O mice **(****Fig. 16A****)** showed the co-propagation of N- and C-terminal tau, indicating that full-length mutant tau propagation occurs in the absence of endogenous tau. In situ hybridization also showed that DG granule cells having human tau protein did not express human tau mRNA, as expected in this line **(****Fig. 16B****).** These results contrast common belief of the prion hypothesis, in which propagation of misfolding prion protein (PrPsc) calls for the presence of endogenous prion protein (PrPc). The propagation of mutant tau reported in ECrTgTau mice (5, 7) thus relies on robust trans-synaptic transmission of already misfolded tau.

### Reduced hyperphosphorylation and aggregation of tau without endogenous tau

Many cellular functions of tau, such as microtubule binding (13) and interaction with synaptic proteins (14), and its aggregation (15) are regulated by phosphorylation. To determine if phosphorylation correlates with trans-synaptic tau transmission, tau phosphorylation in tau-expressing EC neurons and in tau-receiving hippocampal neurons was compared using tau phosphorylation site specific antibodies (16). Both ECrTgTau **(****Fig. 11A****)** and ECrTgTau-MaptO/O **(****Fig. 11B****)** mice showed phosphorylated tau labeled with CP13 (pS202/pT205) and PHF1 (pS396/pS404,) in somata of EC neurons but only in a subset of human tau containing DG neurons and their processes; this finding may be, in part, explained by trans-synaptic transport of differentially phosphorylated tau or dephosphorylation/phosphorylation in post-synaptic neurons. In brain lysates, ECrTgTau mice showed consistently higher levels of different phospho-tau species **(****Figs. 11C-11D**) compared to ECrTgTau-MaptO/O mice in both EC **(****Fig. 11E**) and HPC **(****Fig. 11F****).** Surprisingly, phospho-tau levels in wildtype (WT) mice were comparable to ECrTgTau mice (**Figs. 11E-11F**; and **Figs. 17A-17C**), indicating a major contribution of mouse tau phosphorylation to overall high phospho-tau levels in ECrTgTau mice. Estimating tau propagation efficiencies in ECrTgTau versus ECrTgTau-MaptO/O mice, it was found that similar HPC:EC ratios of human and PHF1 tau but a significantly reduced HPC:EC ratio of CP13 tau in ECrTgTau-MaptO/O mice **(****Fig. 11G****).** Interestingly, although all transgenic tau expressing EC neurons were positive for PHF1 and CP13 (**Figs. 11A-11B****),** both mouse lines showed intense pre-synaptic PHF1 but not CP13 labeling of the EC axonal terminal zone in the middle molecular layer (**Figs. 11A-11B**; MML), indicating that tau phosphorylation at pS394/pS404 (PHF1) more than pS202/pT205 (CP13) may target tau to axonal terminals (17). In 18 month old ECrTgTau mice, neurons in EC and DG as well as axonal terminals in the MML contained "misfolded" (Alz50-positive) tau **(****Fig. 11A**) (5). However, no Alz50 staining could be detected in ECrTgTau-Mapt0/0 mice at 18 months in any brain region **(****Fig. 11B****).** Aggregated tau that stained with tangle dye Thiazine Red (ThiaRed) was found in EC and DG cell bodies of ECrTgTau but not ECrTgTau-MaptO/O mice **(****Figs. 18A-18B****).** The findings presented herein show that tau propagation can occur in the absence of endogenous tau and independently of overt aggregation, thus separating the phenomena of propagation from NFT formation.

### Reduced neurotoxicity in absence of endogenous tau in ECrTgTau mice

It was next sought to determine whether reduced tau aggregation in the absence of mouse tau is accompanied by reduced neurotoxicity. At 18 months of age no cell death or synaptic protein (synapsin-1) loss were detected **(****Figs. 19A-19B****).** However, gliosis and increased phosphorylation of neurofilament proteins can serve as early indirect signs for inflammation and neuronal damage (18). There were significantly higher number of microglia and increased levels of Iba1 in the EC of ECrTgTau mice compared to WT mice, which were partially rescued in ECrTgTau-MaptO/O mice **(****Fig. 12A****).** Accordingly, the levels of GFAP in EC and HPC extracts were markedly reduced in ECrTgTau-MaptO/O compared to ECrTgTau animals **(****Fig. 12B****).** Immunostaining revealed intense neurofilament protein phosphorylation (SMI312) in the EC and its axonal projections of ECrTgTau mice; in ECrTgTau-MaptO/O, SMI312 labeling was comparable to WT and Mapt0/0 mice **(****Fig. 12C****).** These data show that axonal changes and glial response precede overt neurodegeneration (19), and that the absence of mouse tau attenuates early pathological changes and can be required for tau toxicity.

### Removal of endogenous tau rescues P301L tau-induced brain atrophy

Although aggregation and toxicity are frequently viewed as linked phenomena, previous reports discuss the dissociation of tau aggregate formation from acute neuronal death (20) or functional alterations (21, 22). Here, the inventors further explored whether a mouse tau knock-out background impacts tau aggregation and/or toxicity by utilizing a mouse model with cortex-wide expression of human P301L tau (23) (rTg4510) which develops robust late-stage NFT pathology and brain atrophy, and crossed this line with mouse tau knock-out mice (rTg4510-Mapt0/0). At 9 months of age, the whole brain weight of rTg4510 mice was significantly reduced by ≈16% (n=6; p=0.0002) and cortical (CTX) thickness decreased by ≈26% (n=3 mice, p=0.009) compared to WT and Mapt0/0 mice **(****Figs. 13A-13B****).** Brain weight and cortical thickness decreased even further in rTg4510 mice at 12 month of age. Surprisingly, this major loss of brain matter was fully rescued in rTg4510-Mapt0/0 mice at 9 months (brain weight: ≈96%, n=5; CTX thickness: ≈96%, n=3), and only minor atrophy occurred in 12 month old ECrTgTau-MaptO/O mice. Neuron and volume loss in CA1 of rTg4510s were partially rescued in rTg4510-Mapt0/0 animals **(****Figs. 20A-20B****).** Accordingly, the findings described herein show that neuronal health and survival was largely improved in the absence of mouse tau.

Next, it was sought to determine, if NFT pathology was also rescued in absence of endogenous tau. The number of phospho-tau positive (PHF1) tangle-like tau inclusions was visibly reduced in CTX and CA1 **(****Fig. 13C****)** of rTg4510-Mapt0/0 mice at 9 month, and, interestingly, PHF1 tau was still present in CA1 axons in rTg4510-Mapt0/0 mice but was fully condensed into somata in rTg4510 mice. At 12 month of age, PHF1 positive tau appeared condensed into somata in both mouse lines. Gallyas silver stains (24) of 12 month-old mice **(****Fig. 13D****)** showed a similar pattern, with healthier neuropil (less axonal and dendritic staining) in rTg4510-Mapt0/0 compared to rTg4510 mice.

### Removal of endogenous tau improves neuron survival in presence of NFTs

To assess the effect of tangle pathology on neuronal loss, the number of cortical neurons (NeuN positive cells) and tangles (Thioflavine-S positive) was next determined. In rTg4510 mice, the number of cortical neurons decreased by ≈33% (n=3 mice, p=0.002) at 9 month and by ≈37% (n=3 mice, p=0.0001) at 12 month compared to WT mice. rTg4510-Mapt0/0 mice had no neuronal loss at 9 month, and minor reduction in neuron number (≈12%, n=3, not significant) at 12 month compared to Mapt0/0 mice **(****Fig. 14A****).** 9 month and 12 month old rTg4510 and rTg4510-Mapt0/0 mice had similar overall numbers of cortical tangles that increased with age (ThioS staining, n=3 mice per group; **Fig. 14B**). Because rTg4510-Mapt0/0 mice showed the same number of tangles but reduced neuronal loss, rTg4510-Mapt0/0 animals had significantly lower (p=0.029) percentage of neurons with tangles compared to rTg4510 mice **(****Fig. 14C****).** Taken together, these data intriguingly demonstrate that neuronal loss resulting from mutant tau overexpression can be attenuated by eliminating endogenous mouse tau. Furthermore, despite a similar total number of ThioS-positive tangles in the CTX of rTg4510 and rTg4510-Mapt0/0 mice, only rTg4510 animals had pronounced neuronal death and neuropil degeneration, indicating a reduced tangle toxicity in absence of endogenous tau (**Fig. 14D**, and **Fig. 20C**). These data thus reveal a dissociation between tau aggregation and toxicity in rTg4510-Mapt0/0 animals.

In sum, the data presented herein challenge two assumptions regarding tau spreading, aggregation, and toxicity. First, previous classical studies of prion protein showed that spread of PrPsc depends on the presence of endogenous PrPc, as does prion toxicity (12, 25). Tau has been reported to be "prionoid" based on observations of trans-synaptic spread of misfolded proteins and the ability to corrupt endogenous tau protein in the receiving neuron. In contrast, the findings presented herein demonstrate that, in contrast to prion proteins, tau propagates trans-synaptically in the absence of endogenous tau, indicating that tau neuron-to-neuron transfer does not obligatorily depend on templated misfolding. Thus, a modification of the prionoid hypothesis for tau propagation seems necessary.

Secondly, the commonly-assumed association between tau misfolding, aggregation, and neurotoxicity appears to be changed in the context of a tau-null phenotype as described herein: Mapt0/0 animals are protected against the earliest signs of neuronal damage, such as gliosis and axonal alterations (in 18-month ECrTgTau mice), as well as against late stage neuronal loss occurring in the setting of wide-spread NFT pathology (in 9 and 12 month old rTg4510 mice). The surprisingly robust neuroprotection and decrease in inflammation in the face of continued tau aggregation indicate that reducing tau can be highly beneficial for neuronal health in tauopathies and AD.

### Exemplary materials and methods

**Animals.** To study spreading of human mutant tau (0N4R P301Ltau), ECrTgTau mice were produced by crossing (C57BL/6J)B6.TgEC-tTA mice expressing the tetracycline-controlled transactivator (tTa) exclusively in EC-II (30) with FVB-Tg(tetO-TauP301L)4510 mice (23) as previously described (5). Mice positive for both the transactivator and responder transgenes expressed human P301L mutant 0N4R tau in the EC, while mice lacking the human tau transgene served as tau-negative controls (WT). To produce ECrTgTau-MaptO/O mice that lacked endogenous mouse tau (encoded by the Mapt gene), B6.*Mapt^{tm1(EGFP)Kit}* mice (Mapt0/0) were obtained from the Jackson Laboratory (29). To ensure mice expressing or lacking endogenous mouse tau were on the same (B6 x FVB) F1 background, a FVB.*Mapt^{tm1(EGFP)Kit}* congenic strain was produced. The TgEC-tTA transgene was transferred onto the B6.Mapt0 background and the Tg(tetO-tau_{P301L})4510 transgene was transferred onto the FVB.Mapt0 background; crossing these two lines generated ECrTgTau-MaptO/O mice with their genetic background identical to that of ECrTgTau mice that expressed mouse tau. Brains from gender-mixed 18 month-old animals of these lines were analyzed. Similarly, the CK-tTA transgene, which drives expression in forebrain excitatory neurons (31), was transferred to the B6.Mapt0 background, enabling production of rTg4510 and rTg4510-Mapt0/0 mice for atrophy, neuron loss, and tangle analyses. All animal experiments conformed with United States National Institutes of Health guidelines and were approved by the Institutional Animal Care and Use Committees of Massachusetts General Hospital and McLaughlin Research Institute.

**RT-PCR Analysis.** To verify mouse genotypes, RNA was extracted from frozen brain tissue with Trizol (Sigma-Aldrich) and RNA quality was tested using Agilent RNA 6000 Pico Kit (Agilent Technologies). For RT-PCR, 1 µg RNA extract was transcribed into cDNA with SuperScript® III Reverse Transcriptase (Life Sciences) using primers targeting the human tau transgene product (5'-CCC AAT CAC TGC CTA TAC CC-3' and 5'-CCA CGA GAA TGC GAA GGA-3') and mouse tau exon 7 (5'-CACCAAAATCCGGAGAACGA-3' and 5'-CTTTGCTCAGGTCCACCGGC-3'). Transcript number of human and mouse tau RNA was quantified with RT² qPCR Primer Assay and normalized to GAPDH (5'-TGG TGA AGC AGG CAT CTG AG-3' and 5'-TGC TGT TGA AGT CGC AGG AG-3'). Statistical significance (p≤0.05) was tested using two-tailed t-test.

**Immunohistochemistry.** Mice were killed, intracardial perfused with PBS, and brain hemispheres were drop-fixed in 4% paraformaldehyde (PFA) in PBS for 72 h at 4°C, then cryoprotected in sucrose, frozen embedded in M1 mounting medium, cut into 10 µm thick horizontal sections, mounted on glass slides, and stored at -80 °C. For immunofluorescence labeling, sections were re-fixed in PFA, permeabilized with 0.1 % Triton in PBS, blocked in 5% normal goat serum in PBS (NGS) for 1 h. Primary antibodies (as shown below) were diluted in 5% NGS and applied over night at 4°C. After washing in PBS, secondary antibodies (1:500) in 5% NGS for 1 h at room temperature. Secondary anti-mouse or anti-rabbit antibodies were fluorescently labeled with Cy3 (Jackson ImmunoResearch Laboratories) or AlexaFluor647 (Life Technologies). For tangle stain with Thiazine Red, brain sections were incubated in 0.025% Thiazine Red (Sigma Aldrich) in PBS prior to blocking with NGS. Sections were mounted using antifade mounting medium with DAPI (VectaShield) and images recorded on a Zeiss Axiolmager microscope equipped with a Coolsnap digital camera and Axio-VisionV4.8.

**Antibodies.** For immunofluroescent labeling of brain sections, the following primary antibodies were used: rabbit anti-human tau (N-terminus) TauY9 (dilution 1:500, Enzo Lifescience), mouse anti-human tau (N-terminus) Tau13 (1:1000, Covance), rabbit anti-mouse and human tau (C-terminus; 1:1000, DAKO), mouse anti-phospho tau CP13 (pS202/sT205) and PHF1 (pS396/pS404) (1:1000, courtesy of Peter Davies), rabbit anti-"misfolded" tau Alz50 (1:500, Peter Davies), mouse anti-glial fibrillary acidic protein (GFAP; 1:1000, Abcam), rabbit anti-Ibal (for ICC; 1:500, WAKO), mouse anti-phospho neurofilament proteins SMI312 (1:1000, Covance), mouse anti- NeuN (1:1000, Millipore). Additionally utilized for Western Blotting were: rabbit anti-phospho tau pT205 (1:1000, Thermo Scientific) and 12e8 (pS262/pS356; 1:1000, Elan Pharmaceuticals), rabbit anti-Ibal (for WB; 1:500, WAKO), and mouse anti-synapsin-1 (1:100, EMS).

**Fluorescence immuno/in situ hybridization (Immuno-FISH).** FISH combined with immunohistochemistry was performed as previously described (5). Digoxigenin-labeled RNA probes matching human Mapt (NM_016835; nucleotides 2773-3602) were generated by RT-PCR and detected with anti-digoxigenin horseradish peroxidase labeled antibodies (Roche) and Alexa568 labeled tyramide (Invitrogen) (32). Human tau was immunolabeled using primary antibody TauY9 and Alexa647-conjugated goat anti-rabbit secondary antibody.

**Cell counting, stereology, and cortical thickness.** To count activated astroglia (GFAP) in HPC and microglia (Iba1) in EC layer 2/3 **(****Figs. 12A-12C****)** and cell nuclei (DAPI) in layer 2/3 of EC **(****Figs. 19A-19B****),** the respective ROI was outlined for each section in the DAPI channel (3-4 10 µm thick sections of each mouse; 3 mice per group), then manually counted the respective cell/nucleus staining per ROI using ImageJ, and determined cell densities (number/mm²) for each brain structure.

For stereological counting of neurons (NeuN) and tangles (ThioS) in CTX **(****Figs. 14A-14D****)** and CA1 **(****Figs. 20A-20C****),** cells were sampled in four 10 µm thick horizontal sections of matching brain regions. Sections were spaced each by 100 µm, thus spanning a total depth of 4x100 µm = 400 µm brain tissue per mouse. As ROIs, entire CTX (layer 1, 2/3, and 4/5) or entire CA1 was defined.

**Western blotting.** EC and HPC were dissected from frozen brains and homogenated in RIPA buffer (Sigma Aldrich) including protease (Complete, Roche) and phosphatase (PhosphoStop, Roche) inhibitors, and 10 µg total protein were loaded onto 10% Bis-Tris SDS-polyacrylamide gels (Life Technologies) and separated using MOPS buffer. After blotting onto nitrocellulose membranes and blocking in Odyssey blocking buffer (LI-COR), primary antibodies in blocking buffer were applied overnight at 4°C. After washing in 0.05% (v/v) Tween-20 in TBS, blots were incubated with goat anti-rabbit-IRDye680 and anti-mouse-IRDye800 (Rockland) 1h at room temperature. Protein bands were visualized using Odyssey imaging system (LI-COR) and intensities analyzed using ImageJ (accessible online at http://rsb.info.NIH.gov/nih-image/).

**Brain weight.** Wet weight of whole brains including cerebellum and olfactory bulb from 9 and 12 month-old rTg4510, rTg4510-Mapt0/0, WT, and Mapt0/0 mice was determined after intracardial perfusion with 15 ml of room temperature PBS.

**Gallyas silver and Thioflavine-S staning of tangles.** Silver staning of tangles in 12 month old rTg4510,rTg4510-Mapt0/0, and control mice was performed on 30 µm thick free-floating coronal sections of PFA-perfused brains as described previously (24). Sections were incubated in 5% periodic acid for 5 min, rinsed with water for 5 min twice, then treated with alkaline silver iodide solution (1 M NaOH, 0.6 M KI, 0.053% (w/v) AgNO₃) for 1 min, and washed in 0.5% acetic acid for 10 min. Staining was developed combining solution A (5% (w/v) Na₂CO₃), solution B (24 mM NH₄NO₃, 12 mM AgNO₃, 3 mM tungstosilicic acid), and solution C (24 mM ammonium nitrate, 12 mM AgNO₃, 3 mM tungstosilicic acid, 0.25% (w/v) formaldehyde) in a 2:1:1 ratio, by adding B and C drop-wise to solution A and incubating for 20 min. Sections were then washed 3x in 0.5% acetic acid and 1x in water. After incubation in gold tone for 3-4 min, rinsing in water, then in 1% (w/v) Na2S2O3 for 5 min, and again in water, the sections were mounted on glass slides. Neurons (Nissl substance) were counter stained in Cresyl violet stain for 30 sec, then dipped in 50%, 70%, 95%, and 100% ethanol, then placed in 100% xylene until clear and mounted in mounting media.For Thioflavine-S (ThioS) stain of tangles (33) in 9 an 12 month old rTg4510 and rTg4510-Mapt0/0 brains, 10 µm thick cryosections of PFA-fixed brains were thawed, rinsed in de-ionized water, incubated in 0.05% (w/v) ThioS in 50% (v/v) ethanol in the dark for 8 min. ThioS staining was differentiated by two washes in 80% ethanol for 10 sec, then sections were washed briefly in de-ionized water and mounted on microscope slides using anti-fade mounting medium (without DAPI).

**Statistical analysis.** To compare cell numbers (positive for human tau, NeuN, GFAP, Iba1, ThioS) in ECrTgTau, ECrTgTau-Mapt0/0, and controls, we determined the average cell densities (cell number/µm²) per mouse from 3-4 brain sections, utilizing 3 mice per group. For western blot analyses, brain extracts of 3 mice per genotype group were analyzed. Statistical analysis of differences between groups was performed using GraphPad Prism 5 applying non-paired Student's T-tests with confidence intervals of 95%. Values are given as mean±SEM.

### Example 3. Tau enhances amyloid-β induced neuronal intrinsic apoptotic cascades

Alzheimer's disease (AD) brains are characterized by the deposition of aggregated amyloid beta (Aβ) in amyloid plaques and of abnormal phosphorylated tau in neurofibrillary tangles (NFTs). Soluble oligomeric forms of both Aβ and tau are associated with synaptic loss (Alpar et al., 2006; DaRocha-Souto et al., 2012; Hudry et al., 2012; Knafo et al., 2009; Koffie et al., 2009; Kopeikina et al., 2013a; Kopeikina et al., 2013b; Spires et al., 2005; Wu et al., 2010; Wu et al., 2012; Rapoport et al., 2002; Roberson et al., 2007; Shipton et al., 2011; Vossel et al., 2010; Zempel et al., 2013), but the mechanisms of spine loss are unknown. Moreover, Alzheimer disease is known to involve alterations of both Aβ and tau, but whether these are synergistic or parallel pathways are unknown (Ittner and Gotz, 2011). In this Example, the inventors show that tau is a critical collaborator for Aβ-induced synaptotoxicity. This molecular pathway connecting tau to Aβ-induced mitochondrial changes represents a novel apoptosis-related program of neurodegeneration which we observed in neuronal culture, transgenic mouse models, and in human brain.

To understand the mechanism of Aβ-induced spine loss and the relationship of tau to Aβ-induced spine loss, primary neuronal cultures from wild-type or tau null mice were exposed to soluble Aβ, and caspase activation and spine loss were monitored. A series of molecular and pharmacological probes were used to dissect the order in which these events occur, and the role of tau in each step. It was discovered that Aβ treatment leads to activation of the intrinsic (mitochondrial) caspase pathway, as well as tau translocation to the mitochondria. This appears to be a critical step in the initiation and progression of intrinsic mitochondrial apoptotic-related cascades ultimately marked by (subapoptotic) caspase 9 and 3 activation and spine loss. Tau was found to be associated with mitochondria in cultured neurons exposed to soluble Aβ, in APP/PS1 mice compared to wild type mice, and in human AD compared to controls. These data indicate a mechanistic link between tau translocation to the mitochondria and early steps of Aβ toxicity and, in a broader context, an unexpected collaboration of tau in caspase activation cascades.

### Results

### Aβ induces caspase 3 activation

Long-term depression synaptic depression and spine loss has been reported to be associated with nonapoptotic caspase activation (Jiao and Li, 2011; Li et al., 2010, see Spires-Jones and Hyman for review). Since young Tg2576 APP mice show non-apoptotic levels of caspase 3 activation (D'Amelio et al., 2011), it was sought herein to determine if exposure of neurons in culture to Aβ activates caspases. Conditioned media from Tg2576 cultures (TgCM) or wild-type cultures (WtCM) was harvested and applied to wild-type cortical neuron cultures for 24 hours. A 3:1 dilution of TgCM was applied to cultures so that the final concentration was 4 nM as measured by Aβ40 ELISA. Following treatment, cell lysates were prepared for analysis by Western blot. Blots were probed with an antibody specific for cleaved caspase 3 which detects the large fragment of activated caspase-3 (17/19 kDa) resulting from cleavage adjacent to Asp175, and does not recognize full-length caspase 3 or other cleaved caspases **(****Figs. 21A-21B****).** Immunoblots of neurons exposed to Aβ-containing TgCM versus WtCM showed a significant increase in caspase 3 activation after 24 hours (**Figs. 21A-21B**, p<0.0001) relative to the total levels of caspase 3, which were not altered.

To determine if the results from TgCM treatment were due to Aβ present in the medium, immunodepletion of Aβ from TgCM (TgCM-ID) was achieved by using 6E10, a high-titer mouse monoclonal Aβ antibody. Immunodepletion using 6E10 reduced Aβ40 to ∼0.1 nM as measured by ELISA. Cleaved caspase 3 levels in cultures treated with immunodepleted media were no different than in cultures treated with WtCM (**Figs. 21A-21B****,** p=0.1115) and caspase 3 activation was significantly reduced compared to cultures treated with TgCM (**Figs. 21A-21B****,** p<0.0001). This indicates that Aβ, and not another factor in the CM, is responsible for activation of caspase 3.

It was also examined whether tau, a caspase 3 substrate, was cleaved at Asp421 by using an antibody directed against the tau-Δ421 neoepitope (TauC3) (Gamblin et al., 2003) **(****Figs. 21A****,** **21C****).** Incubation with TgCM versus WtCM did not change the total levels of tau (p=0.1426; representative blot is shown in **Fig. 21A** and quantification is shown in **Fig. 28****,** but did result in increased cleavage of tau as measured by TauC3 western blot **(****Figs. 21A****,** **21C****,** p=0.0105). Additionally, compared to TgCM treated cultures, truncated tau was decreased when Aβ was immunodepleted from the media (p=0.0107).

To determine whether caspase activation is responsible for cleavage of tau in response to TgCM treatment, the pan caspase inhibitor zVAD-FMK was used. TgCM exposed neurons treated with 20 µM zVAD showed no increase in active caspase 3 (**Fig. 21A, 21B**, p<0.0001) or TauC3 levels (**Figs. 21A****,** **21C****,** p=0.0439). As a positive control for caspase activation, neuronal cultures were also incubated with staurosporine (STS, 1 µM), a potent inducer of apoptosis. In STS treated cells, caspase inhibition also reduced activation of caspase 3 (**Figs. 21A-21B****,** p<0.0001) and reduced tau cleavage (**Figs. 21A****,** **21C****,** p=0.0097). Thus, TauC3 can be used as an alternate readout for caspase activation in TgCM treated primary neurons.

No significant changes in cleaved caspase 3 and truncated tau levels were detected between neurons treated for 24 hrs with conditioned medium from wild-type neurons (WtCM) and untreated neurons (data not shown). Altogether, these data indicate that soluble Aβ species in TgCM promote activation of caspase 3, as measured by both the presence of cleaved caspase 3 and tau truncation at Asp421.

### Aβ promotes non-apoptotic caspase 3 activation

Given that caspases are activated by Aβ treatment, it was next sought to determine if neurons treated with TgCM were undergoing apoptosis. A key indicator of apoptosis is cleavage of poly (ADP-ribose) polymerase (PARP) by caspase 3 (Nicholson, 1995; Oliver, 1998). Immunoblots for cleaved PARP revealed that 24hrs of TgCM treatment did not lead to an increase in cleaved PARP, whereas STS exposure for 6 hours induced PARP cleavage **(****Fig. 29A****).** Similarly, assessment of cell toxicity using the Toxilight assay, which measures the release of adenylate kinase from damaged cells, revealed that Aβ treatment did not cause significant cell death compared to control (**Fig. 29B****,** p=n.s.). Similarly, assessment of cell toxicity using the Toxilight assay, which measures the release of adenylate kinase from damaged cells, revealed that Aβ treatment did not cause significant cell death compared to control (**Fig. 29B****,** p= n.s.). By comparison, cell death was induced by STS treatment (**Fig. 29B****,** p<0.0001) and was blocked by zVAD (p<0.0001). These results indicate that incubation with Aβ (∼4 nM) causes non-apoptotic caspase activation in neurons. Moreover, exposure to STS, an inducer of neuronal apoptosis, activated the components of the intrinsic mitochondrial pathway to a greater extent than Aβ, indicating that the activation of this pathway by Aβ is either more subtle or localized, and may not necessarily result in cell death.

### Aβ promotes caspase activation via the mitochondrial intrinsic pathway

Next, it was sought to determine whether Aβ activated caspases via the classical intrinsic pathway, which involves dephosphorylation of BAD, translocation of BAX to the mitochondria, cytochrome c (cyt c) release, activation of caspase 9 and caspase 3 (Chipuk et al., 2006; Goping et al., 1998; Wang et al., 1999; Wolter et al., 1997). Caspase 9 activation was detected using an antibody against cleaved caspase 9 (Asp353) which detects endogenous levels of the 37 kDa subunit of mouse caspase 9 only after cleavage at aspartic acid 353 and does not cross-react with full-length caspase 9 or with other caspases (**Figs. 21D** and **21E**). There was significant increase in the levels of cleaved caspase 9 relative to total caspase 9 after TgCM treatment versus WtCM (**Fig. 21E****,** p<0.0001). Aβ-induced caspase 9 activation was blocked by zVAD-FMK treatment (p<0.0001) or Aβ immunodepletion (p<0.0001). Other markers of the intrinsic pathway were also activated. Phosphorylated-BAD (pBAD) was significantly decreased after TgCM treatment compared to WtCM (**Fig**. **21D****,** **21F****,** p<0.0001) and this effect was blocked by Aβ immunodepletion (p=0.0455). Caspase inhibition by zVAD-FMK did not prevent dephosphorylation of BAD (**Fig. 21D****,** **21F**, p=0.7051).

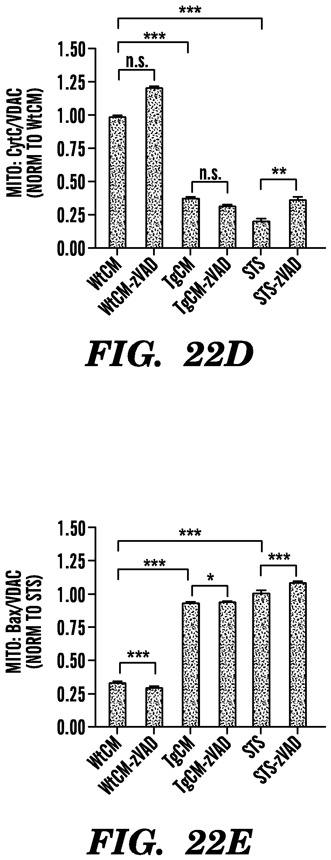

To further investigate the activation of the intrinsic pathway after Aβ treatment, mitochondria and cytosolic fractions from neurons were isolated and the levels of VDAC, Cyt C, and BAX were analyzed both in the cytosolic fraction and the mitochondrial pellet. The mitochondrial outer membrane protein VDAC was absent in the cytosolic fraction (**Fig. 22A**) and enriched in the mitochondria pellet (**Fig. 22B**). TgCM treatment induced cytochrome c release from the mitochondria compared to WtCM, which is shown by increased levels in the cytosol (**Figs. 22A, 22C****,** p<0.0001) and decreased levels in the mitochondria pellet (**Figs. 22B****,** **22D****,** p<0.0001). In TgCM treated cultures, caspase inhibitor zVAD-FMK did not alter levels of cytochrome c in the cytosol (**Figs. 22A, 22C**, p=0.3968) or mitochondrial pellet (**Figs. 22B****,** **22D****,** p=n.s.) indicating that cytochrome c release is upstream of caspase activation following Aβ treatment. Moreover, in TgCM treated cultures versus WtCM treated cultures, an increased in total levels of BAX was detected in the mitochondrial pellet (**Figs. 22B****,** **22E****,** p<0.0001). Altogether, the release of cytochrome c and translocation of BAX to the mitochondria after Aβ treatment is indicative of activation of the intrinsic pathway of apoptosis.

### Calcineurin activation is an early step of Aβ induced activation of mitochondrial intrinsic pathway

Caspase inhibition did not prevent dephosphorylation of BAD, BAX translocation to the mitochondria or cytochrome c release, indicating that these events are upstream of caspase activation. Calcineurin activation and BAD dephosphorylation are upstream pre-mitochondrial signaling events leading to caspase-9 activation, an initiator caspase that activates caspase 3. It was speculated that calcineurin activation is the upstream event leading to the activation of the mitochondria intrinsic pathway in response to Aβ in accord with recent observations (Hudry et al., 2012; Wu et al., 2010; Wu et al., 2012). Calcineurin activation was inhibited by FK506, which was applied 20 minutes prior to the addition of WtCM, TgCM, or STS. Compared to TgCM alone, TgCM treated cultures incubated with FK506 had reduced caspase 3 activation (**Figs. 23A, 23B****,** p<0.0001), tau cleavage (**Figs. 23A****,** **23C****,** p<0.0444), caspase 9 activation (**Figs. 23D****,** **23E****,** p<0.0001), and dephosphorylation of BAD (**Figs. 23D****,** **23F****,** p<0.0001). In WtCM treated cultures, FK506 had no effect. These data indicate that calcineurin activation is an early event in Aβ-mediated caspase activation and toxicity.

### Aβ induced spine loss is blocked by caspase inhibition

It was next examined the relationship of these biochemical markers to spine loss. The spine density in GFP transfected neurons treated with either WtCM or TgCM for 24 hrs was assessed. TgCM treated neurons showed decreased spine density versus WtCM treated neurons (**Figs. 24** **A, 24C,** p<0.0001). This spine loss in TgCM treated cultures was reduced by Aβ immunodepletion (p=0.0005). Treatment with Aβ immunodepleted media was not different than with WtCM (p=n.s.). To examine if pharmacological blockade of caspase activation is sufficient to prevent Aβ induced spine density reduction, the effect of zVAD-FMK in TgCM treated neurons was explored and it was found that it fully blocked the spine loss triggered by TgCM (p<0.0001). However, treatment of WtCM exposed neurons with zVAD-FMK also promoted a significant increase in spine density (p=0.0088).

As a second measure of synaptic alterations, the postsynaptic density protein PSD95, which is highly enriched in dendritic spines and has been associated with spine stability, was measured. The levels of PSD95 following TgCM treatment was determined and a significant decrease in PSD95 levels compared to WtCM cultures was detected, which is consistent with spine loss (**Figs. 24B****,** **24D****,** p<0.0001). PSD95 levels were preserved when neurons exposed to TgCM were co-treated with zVAD-FMK (p<0.0001) or when Aβ was immunodepleted from TgCM (p<0.0001). There were no changes in spine density and PSD95 levels between neurons treated for 24 hours with WtCM and untreated neurons. These data indicate that Aβ induces synaptic toxicity shown by reduced PSD95 and spine density through caspase activation. Further, this indicates that, without wishing to be bound by theory, spine turnover in wild-type neurons can be regulated by endogenous, non-apoptotic caspase activation.

Surprisingly, synaptoneurosomes isolated from human brain AD cases showed a significant increase in the levels of cleaved caspase 3 compared to those isolated from non-demented controls (**Figs. 30A-30B****,** p=0.0046). Concurrently, an increase in tau in the synaptoneurosome fraction from AD brain compared to controls was detected (**Figs. 30C-30D****,** p=0.0019). Altogether, this can be indicative of the role of Aβ and tau in activating caspases locally in synapses to induce synaptic toxicity in AD.

### Reduction of endogenous tau levels protects neurons from Aβ-triggered caspase activation and spine loss

Tau has been reported to be associated with Aβ-mediated spine loss (Roberson et al., 2007). To explore where tau fits in the cascade of caspase activation induced spine loss, neurons from tau null (Tau-/-) mice were exposed to Aβ and the set of intermediate read-outs developed above was monitored for non-apoptotic caspase activation and spine loss **(****Figs. 25A-25H****).** When treated with TgCM, tau null neurons (Tau-/-) showed a significant reduction in caspase 3 activation (**Figs. 25A****,** **25C****,** p<0.0001) while caspase 9 activation and pBAD dephosphorylation were unchanged (**Figs. 25B****,** **25D****,** p=n.s. and **Figs 25B****,** **25E****,** p=0.9694 respectively), indicating a block of non-apoptotic caspase pathway activation in the absence of tau. By comparison, heterozygous (Tau+/-) and wild-type tau (Tau+/+) neurons both exhibited an increase in Aβ-induced cleaved caspase 3(p<0.0001). Tau+/+ neurons, but not Tau+/- neurons, also had significantly increased cleaved caspase 9 (p<0.0001) and exhibited pBAD dephosphorylation (p<0.0001).

To determine if the absence of tau was the critical variable in preventing induction of the mitochondrial intrinsic pathway, full-length wild-type tau (Tau4R) was introduced to Tau-/- neurons using adeno-associated virus (AAV) mediated gene delivery. Subsequent to TgCM application, neurons transduced with Tau4R showed significant caspase 3 activation (**Figs. 25F****,** **25I****,** p<0.001) compared to neurons expressing GFP only. No difference was seen in caspase 3 activation between Tau4R and GFP expressing neurons treated with WtCM (p=0.6186). Additionally, both tau knockout (**Fig. 30C****,** p<0.0001) and heterozygous (p<0.0001) neurons were also less susceptible to STS induced cell death compared to wild-type (Tau+/+) neurons. This is consistent with the possibility that endogenous levels of tau facilitate caspase activation.

In accord with these biochemical observations, only Tau+/+ expressing neurons (**Figs. 25G, 25H****,** p=0.001) but not Tau-/- (p=n.s.) or Tau+/- (p=n.s.) were observed to have reduced spine density after treatment with Aβ containing media. These results indicate that reduced expression of tau is protective against the intermediate biochemical consequences of Aβ exposure and Aβ-induced spine loss, and indicate that tau is central to the initiation of mitochondrial caspase cascades in neurons.

### The mitochondrial intrinsic pathway is activated in APP/PS1 mice and is dependent on tau

To investigate if Aβ also leads to activation of the intrinsic caspase cascade in vivo, the levels of cyt c were measured in the brain cytosolic fraction and mitochondrial pellet obtained from APPswePS1d9 bi-transgenic mice (APP/PS1), a mouse model of Alzheimer's disease with abundant extracellular amyloid plaque pathology **(****Figs. 26A****,** **26D****,** **26B****,** **26F****).** Increased levels of cyt c in the cytosol (**Figs. 26A****,** **26D****,** p=0.0001) and decreased levels in the mitochondria pellet (**Figs. 26B****,** **26F****,** p=0.006) were found in 6-month-old APP/P1 mice compared to age-matched controls. In addition, an increase in BAX mitochondrial translocation was detected (**Figs. 26A****,** **26C****,** **26B****,** **26E**). BAX was significantly reduced in cytosol (**Fig. 26C****,** p=0.011) and significantly increased in mitochondria (**Fig. 26E****,** p=0.0341). This is consistent with the speculation that Aβ can cause activation of the intrinsic mitochondrial pathway in vivo.

To investigate if tau reduction was protective against Aβ-induced changes in vivo, the levels of cytochrome c were measured in the brain cytosolic fraction obtained from APP/PS1 mice and APP/PS1-Tau+/- mice and Tau+/- mice. It was found that reducing tau levels by crossing APP/PS1 mice with Tau+/- mice, cytochrome c release into cytosol was reduced compared to APP/PS1 mice alone (**Figs. 26G****,** **26I****,** p=0.016), and was not significantly different compared to control mice (p=0.4755). Synaptoneurosomes from APP/PS1-Tau+/- mice also had reduced cleaved caspase 3 compared to APP/PS1 mice alone (**Figs. 31A, 31B****,** p=0.0322). Moreover, to assess if synaptic alterations were associated with caspase 3 activation, PSD95 in synaptoneurosomes was measured and it was found that there was a significant decrease in PSD95 levels in APP/PS1 mice compared to controls (**Fig. 26H****,** **26J****,** p=0.0022) whereas PSD95 synaptic levels were preserved in APP/PS1-Tau+/- and did not differ from non-transgenic mice levels (p=0.4901). Taken together, these data indicate that tau reduction prevents Aβ-induced activation of the intrinsic mitochondrial pathway and can be protective against Aβ-induced synapse dysfunction in vivo.

### Tau is localized to the mitochondria following Aβ treatment in vitro and in vivo

Mitochondria were next isolated from Aβ-treated wild-type neurons and it was found that tau levels were increased in the mitochondrial pellet of TgCM treated neurons compared to levels in neurons exposed to WtCM (**Figs. 27A****,** **27D****,** p=0.0104). To determine if tau is also localized to mitochondria in vivo, mitochondria were isolated from APP/PS1 mice and from human frontal cortex **(****Figs. 27B****,** **27E****).** Tau localization is increased in mitochondria from APP/PS1 mouse brains compared to age-matched controls (**Figs. 27B****,** **27E****,** p=0.0087). Importantly, the same result was observed in humans, with increased levels of tau in mitochondria from AD brain compared to age-matched controls (**Figs. 27C****,** **27F****,** p<0.0001; characteristics of human samples in **Table 3.** This can indicate that localization of tau to mitochondria is a feature of AD disease pathobiology.

**Table 3. Characteristics of control and AD-affected brains used in quantitative studies. Samples designated as controls (C) and as Alzheimer's disease affected (AD) are indicated along with sex and age of death. Clinical diagnosis and post-mortem Braak stage of neurofibrillary tangles (0, none; I-II, entorhinal; III-IV, limbic; V-VI, isocortical) are also indicated. Samples were used for Fig. 27C and Figs. 30A-30D.**

| **Group** | **Sex** | **Age (year)** | **Clinical Diagnosis** | **Braak Stage** |
|---|---|---|---|---|
| C | M | 82 | Non-demented | I |
| C | F | 76 | Non-demented | I |
| C | M | 86 | Non-demented | II |
| C | M | 85 | Non-demented | II |
| C | M | 63 | Non-demented | 0 |
| C | M | 98 | Non-demented | I |
| C | M | 86 | Non-demented | I |
| C | F | 91 | Non-dem ented | I-II |
| C | M | 92 | Non-demented | II |
| AD | F | 91 | Demented | V |
| AD | F | 69 | Demented | VI |
| AD | M | 84 | Demented | V |
| AD | M | 89 | Demented | VI |
| AD | M | 87 | Demented | VI |
| AD | F | 91 | Demented | V |
| AD | F | 71 | Demented | VI |
| AD | M | 87 | Demented | VI |
| AD | M | 73 | Demented | VI |

### Discussion

Here, the inventors examined the molecular pathways leading from Aβ exposure to spine loss, and discovered (1) clear evidence for sub-apoptotic activation for the intrinsic (mitochondrial) caspase cascade and (2) an unexpected role for tau as a critical mediator of caspase activation via the intrinsic mitochondrial pathway. In this Example described herein, TgCM containing Aβ engaged key mediators of a calcineurin-dependent mitochondrial intrinsic pathway, led to non-apoptotic caspase activation and ultimately, resulted in spine loss. Genetic knockout of tau protected neurons from non-apoptotic caspase activation and prevented spine loss after Aβ exposure. As presented herein, evidence of this non-apoptotic caspase activation was also found in a transgenic mouse model of Alzheimer's disease (AD) and in human AD cases. Further, it was found that tau associates with mitochondria under disease conditions both in vitro and in vivo.

The findings described herein are significant because they describe the relationship between Aβ, tau, and synaptotoxicity. Previous reports have discussed that the neuroprotective effects of tau reduction can be a consequence of the apparent involvement of tau in molecular signaling cascades involving both normal LTD and Aβ-induced synaptic changes (Ittner et al., 2010; Rapoport et al., 2002; Roberson et al., 2007; Shipton et al., 2011; Vossel et al., 2010). However, the data presented herein indicate a broader role for tau in caspase cascades mediated by the mitochondrial intrinsic pathway in neurons, as tau translocation to the mitochondria accompanies cytochrome c release and synapse loss.

Suitably, the caspase activation can be limited to prevent full expression of the apoptotic cascade and neuronal death. In some cases, tau localization to mitochondria can be desirable or necessary or sufficient to enhance intrinsic cascade initiation. In some cases, there can be specific signals that lead to tau association with mitochondria. In some cases, tau cleavage and the formation of a truncated tau product can be a critical intermediate or a consequence of caspase activation. The novel tau-mitochondria interaction can promote understanding the role of tau in Alzheimer's pathobiology. For instance, tau appears to play a role in actin stabilization, where misregulation of actin by phosphorylated tau can lead to altered mitochondrial dynamics (DuBoff et al., 2012). Without wishing to be bound by theory, in some cases, this tau-actin interaction can induce activation of the mitochondrial intrinsic pathway remains to be investigated. Also, it was previously reported that in addition to caspase 3, caspase 2 may play a critical role in dendritic spine turn-over via the RhoA pathway in APP transgenic mice (Pozueta et al., 2013). This suggests that neurons have alternative mechanisms for Aβ-mediated spine removal. Moreover, caspase 6 was also reported to cleave tau in addition to other proteases such as asparagine endopeptidase (Zhang et al., 2014). Thus, it is contemplated that other caspases or proteases can be capable of truncating tau and one or more of these forms of truncated tau can also regulate apoptotic pathways and spine density.

This Example indicates that tau localization to the mitochondria is a critical intermediate in non-apoptotic synaptotoxicity in primary neurons, mouse models of tauopathy, and in human AD brain where it appears to accelerate intrinsic apoptotic cascades. Without wishing to be bound by theory, this is one mechanistic explanation for the apparent neuroprotective effects of tau suppression in multiple circumstances. Thus, the findings described herein show that tau can be an effective therapeutic target in neurodegenerative disease, and provide a link between tau and Aβ synaptotoxicity.

### Exemplary experimental procedures

**Animals.** For these experiments, the following mouse lines were used. (1) Tg2576 (Tg) male mice (Charles River Laboratories), transgenic mice overexpressing human amyloid precursor protein APP containing the double Swedish mutation K670N, M671L (Hsiao et al., 1996). B6SJLF1 female mice (Charles River Laboratories). (2) C57BL/6 (Tau+/+) (Charles River Laboratories). (3) tau knock-out (Tau-/-) mice (Jackson Laboratory) that have a targeted disruption of exon 1 of tau (Tucker et al., 2001). (4) heterozygous tau mice (Tau+/- generated from crossing tau knockout and C57BL/6 mice). (5) APPswe; PS1d9 bi-transgenic mice (APP/PS1) were obtained from Jackson laboratories (Bar Harbor, ME) were used for experiments at 4 or 6 months of age (Jankowsky et al., 2001). APP/PS1 mice were crossed to Tau-/- mice to produce APP/PS1-Tau+/- mice which were used at 6 months of age. (6) CD1 mice (Charles River Laboratories). All experiments were performed in accordance with animal protocols approved by the Institutional Animal Care and Use Committee and conform to NIH guidelines.

**Primary Neuronal Cultures.** Primary neuronal cultures were derived from the cerebral cortex of mouse embryos at 16 days of gestation, as described previously (DaRocha-Souto et al., 2012; Wu et al., 2010) with modifications. Cortices were dissected, gently minced, and washed with Neurobasal medium. Neurons were seeded to a density of 6 × 10⁵ viable cells/35-mm culture dishes previously coated with poly-D-lysine (100 µg/ml) for at least 2 hrs at room temperature. Cultures were maintained at 37 °C with 5% CO₂, supplemented with neurobasal medium with 2% B27 nutrient, 2 mM L-glutamine, penicillin (100 units/ml) and streptomycin (100 µg/ml) (all culture media components were obtained from Invitrogen). The cultures were used at 14 or 15 days in vitro (DIV).

To produce conditioned media, timed pregnant females were bred by crossing hemizygous Tg2576 males with B6SJLF1 females. Neurons from embryos obtained from this cross were plated individually and genotyped separately. Tg2576 (Tg) and B6SJLF1, or wild-type (Wt) neuronal were cultured for 14 days in vitro (DIV). Tg2576 cultures were used to produce (conditioned media, CM) which contains high levels of Aβ oligomers species from dimer to hexamer (previously characterized (DaRocha-Souto et al., 2012; Wu et al., 2010)), similar to the Aβ found in the TBS fraction obtained from AD patient brains (DaRocha-Souto et al., 2012; Shankar et al., 2008; Wu et al., 2010). CM from 14 DIV B6SJLF1, or non-transgenic littermates referred here as wild-type (Wt) neurons, was used as control. To maintain elevated levels of extracellular Aβ, the media of these cultures was not changed during the 14 days of culture, and then was collected to treat 14 DIV WT neurons obtained from CD1 mice embryos, Tau-/-, Tau+/, and Tau+/+ neurons.

Tau-/-, Tau+/-, and Tau+/+ neurons were obtained from embryos and plated individually and genotyped separately from timed pregnant females generated by crossing tau heterozygous mice (Tau+/-). The genotype of the animals was determined by PCR on DNA extracted from sample tail taken after dissection of the cerebral cortex of each embryo.

**Aβ ELISA assay.** Aβ levels were assayed from the culture media by sandwich ELISA kit (Wako). The concentration of Aβ40 in TgCM was approximately 12 nM total (human and murine) and WtCM had 0.5 nM of murine Aβ40.

**Treatment of neuronal cultures.** Wild-type (from CD1 mice), Tau-/-, Tau+/-, and Tau+/+ cortical neurons were cultured in standard NB/B27 serum-free medium, and, at 14 DIV, the medium was replaced with diluted 1:3 CM from TgCM, corresponding to 4 nM of Aβ, or WtCM (0.16nM of murine Aβ40) and further incubated for 24 hrs. To induce apoptosis, cultures were treated with staurosporine (Enzo Life Sciences) at a final concentration of 1 µM and were harvested 4 hours after treatment. In zVAD-FMK (Sigma Aldrich) experiments, 20 µM of the inhibitor was applied to cultures 20 minutes prior to exposure to WtCM, TgCM, or STS. To inhibit calcineurin, 1 µM FK506 (Enzo Life Sciences) was applied 20 minutes prior to exposure to WtCM, TgCM or STS. Media was then aspirated and RIPA buffer with phosphatase and protease inhibitors was added to each well. Cells were scraped to prepare lysates for Western blots.

**Immunodepletion.** Immunodepletion of Aβ from TgCM (TgCM-ID) was achieved by using 6E10 (Signet), a high-titer mouse monoclonal Aβ antibody reactive to amino acid residue 1-16 of beta amyloid. The epitope lies within amino acids 3-8 of beta amyloid (EFRHDS). Protein G-Sepharose (150µl) 4 Fast Flow beads (Pharmacia Biotech, Uppsala, Sweden) were washed three times in 1ml of Neurobasal by 30 min centrifugations at 10,000 rpm. 1ml of TgCM was pre-cleaned in 50 µl of washed beads were added to 1 ml of TgCM and centrifuged at 1,000 rpm for 30 min at 4°C. The pre-cleaned TgCM was centrifuged at 10,000 rpm for 10 min and transferred into a clean tube, then 9 µg of the 6E10 antibody and 100 µl of the previously cleaned beads was added and centrifuged at 1,000 rpm for 6hrs at 4°C and centrifuge at 10,000 rpm for 10 min. Immunodepleted TgCM supernatants were collected for subsequent analysis by ELISA for Aβ quantification and application to neuronal cultures.

**Viral vectors construction and production.** Plasmids containing Tau4R-441 or GFP were subcloned into an AAV backbone containing the chicken β-actin (CBA) promoter and a Woodchuck Hepatitis Virus Post-Transcriptional Regulatory Element (WPRE). High titer of AAV serotype 2/5 vectors were produced after triple-transfection of HEK293 cells. AAV-Tau4R-441 and the control AAV-GFP constructs were verified by sequencing. Neurons were transduced by adding viruses at the same multiplicity of infection (MOI). Three days after transduction neurons were harvested and analyzed by western blot.

**Mitochondria Isolations.** Mitochondria were isolated from neurons plated in 10 cm dishes treated with WtCM and TgCM and purified by centrifugation as previously described (Ofengeim et al., 2012). For example, for neuronal cultures, 10 cm plates (each experiment was repeated three times, for each experiment an n=3 plates was used per condition) were scraped in cold PBS and centrifuged at 2,000 x g for 5 min and pellet was homogenized using ice-cold isolation buffer (250 mM sucrose, 20 mM HEPES pH 7.2, 1 mM EDTA, 0.5% BSA wt/vol, and both protease and phosphatase inhibitors). Cells were homogenized (seven times with a homogenizer; Wheaton), an aliquot was collected (whole cell fraction), the remaining material was centrifuged at 1,300 x g to pellet nuclear material (nuclear fraction). The supernatant was centrifuged at high speed (13,000 x g for 10 min at 4 °C); the supernatant (cytosol fraction) and the pellet (mitochondria fraction) were frozen on dry ice and stored at -80 °C until use. Following addition of RIPA buffer (Sigma) containing protease and phosphatase inhibitors (Roche) all of the fractions were sonicated and centrifuged at 5,000 x g for 5 min to remove insoluble debris. The protein concentration was determined by BCA assay (Thermo Scientific) in all fractions and this concentration used for loading gels for western blot analysis. Mitochondria isolated from mouse or human brain tissue was prepared using the same method homogenizing 0.2 g tissue in 1.5 mL if ice-cold isolation buffer.

**Western Blot.** Protein lysates were boiled in sample buffer consisting of LDS Sample Buffer and reducing Agent, resolved on 4%-12% Bis-Tris polyacrylamide precast gels in a MES-SDS running buffer containing antioxidant. For most analyses, 20 µg/lane were loaded. Gels were transferred onto nitrocellulose membranes (Whatman) in transfer buffer containing 20% methanol. Blots were blocked in Odyssey blocking buffer (Li-Cor biosciences), followed by incubation with primary antibodies: mouse β-actin (Sigma; 1:10,000); rabbit cleaved caspase 3 (Cell Signaling; 1:1,000); rabbit caspase 3 (Cell Signaling; 1:1,000); mouse TauC3 (courtesy of Dr Lester Binder, 1:300); rabbit polyclonal Total Tau (Dako; 1:10,000), rabbit PSD95 (1:1,000; Cell Signaling), rabbit cleaved caspase 9 (1:1,000; Cell Signaling), mouse caspase 9 (1:1,000; Cell Signaling), rabbit pBAD (1:1,000; Cell Signaling), rabbit BAD (1:1,000; Cell Signaling), VDAC (1:1,000; Cell Signaling), mouse cytochrome c (1:1,000; BD Pharmingen), mouse BAX (1:1,000; Santa Cruz), rabbit cleaved PARP (1:1,000; Cell Signaling), rabbit PARP (1:1,000; Cell Signaling). Anti-mouse or anti-rabbit IgG secondary antibodies conjugated to IRDye 680 or 800 (1:10,000, Li-Cor biosciences) were applied and blots were scanned with a Li-Cor Odyssey Infrared Imaging System. Densitometric analysis was performed using Image J software. Neurons used for western blot analysis were plated in 6 well dishes and experiments were performed using 3 wells per condition across three separate culture experiments. Triplicates were averaged to yield an n=3 for each experiment.

**Analysis of spine density and spine morphology.** Cultured neurons plated in 35 mm glass-bottomed dishes (MatTek) were transfected with green fluorescent protein (GFP) (Clontech) at 7 DIV and imaged at 15 DIV. High-resolution digital images were taken using a Zeiss LSM510 confocal microscope with a 25× water-immersion objective lens (digital zoom = 3-5), and excitation laser at 488nm. Images were analyzed with NeuronStudio software (CNIC, Mount Sinai School of Medicine). Spine density was defined as the number of spines per micrometer of dendrite length according previously published protocols (Wu et al., 2010) with modifications. Dendritic spine densities were calculated from 30 to 40 neurons per condition across four separate culture experiments. For each condition, measurements of spine densities were averaged per neuron; the means from multiple neurons were then averaged to obtain the mean ± s.e.m. for the population of neurons.

**ToxiLight BioAssay.** Toxicity assays were conducted in medium collected from wild-type, Tau+/- and Tau-/- neurons plated in 96 well plates at 15 DIV using the ToxiLight BioAssay kit (Lonza, Rockland, ME). Preparation of cell extracts and the cytotoxicity assay were performed according to manufacturer protocols. After centrifugation, 50 µl samples per well were transferred on an opaque white microtitre plate. To each well, 100 µl of the adenylate kinase detection reagent was added and the plate was incubated for 5 min at room temperature. After 5 min, luminescence was measured in the Wallac plate reader. Results are expressed as arbitrary relative light units (AU). To achieve 100% cell lysis (positive control) neurons were treated with 10% triton-X-100. Readings obtained for 100% lysis were comparable to readings obtained with 6 hrs of 1µM staurosporine treatment (data not shown). Neurons for Toxilight assays were plated in 96 well plates using 3 wells per condition in duplicate across four separate culture experiments.

**Statistical analyses.** Statistical analysis was performed using the GraphPad Prism software version 4.03 for Windows (GraphPad Software, San Diego). Data are reported as mean ± the standard error of the mean. Statistical significance was defined at p<0.05. With the exception of spine density measurements (**Fig. 24B**), all data were normally distributed as assessed by Shapiro-Wilk test. Normally distributed data with two groups were analyzed by two-tailed Student's t tests. Normally distributed data with more than two groups were analyzed by one-way or two-way ANOVAs (tau knockout and APP/PS1 experiments). In all cases, overall ANOVA statistical significance was <0.05 and planned comparisons between groups were performed. Post-hoc comparison p values are reported with Bonferroni's correction for multiple comparisons. For nonparametric data, a Kruskal-Wallis ANOVA was performed and p values are reported with Dunn's correction for multiple comparisons.

### References for Example 1

1. Mandelkow EM, et al. (1995) Tau domains, phosphorylation, and interactions with microtubules. Neurobiology of aging 16(3):355-362; discussion 362-353.
2. Iqbal K, Liu F, Gong CX, & Grundke-Iqbal I (2010) Tau in Alzheimer disease and related tauopathies. Current Alzheimer research 7(8):656-664.
3. Braak H & Braak E (1991) Neuropathological stageing of Alzheimer-related changes. Acta neuropathologica 82(4):239-259.
4. Hyman BT, Van Hoesen GW, Damasio AR, & Barnes CL (1984) Alzheimer's disease: cell-specific pathology isolates the hippocampal formation. Science (New York, N.Y.) 225(4667):1168-1170.
5. Serrano-Pozo A, et al. (2013) Examination of the clinicopathologic continuum of Alzheimer disease in the autopsy cohort of the National Alzheimer Coordinating Center. Journal of neuropathology and experimental neurology 72(12):1182-1192.
6. Pooler AM, et al. (2013) Propagation of tau pathology in Alzheimer's disease: identification of novel therapeutic targets. Alzheimer's research & therapy 5(5):49.
7. Medina M & Avila J (2014) The role of extracellular Tau in the spreading of neurofibrillary pathology. Frontiers in cellular neuroscience 8:113.
8. Walker LC, Diamond MI, Duff KE, & Hyman BT (2013) Mechanisms of protein seeding in neurodegenerative diseases. JAMA neurology 70(3):304-310.
9. Polydoro M, et al. (2013) Reversal of neurofibrillary tangles and tau-associated phenotype in the rTgTauEC model of early Alzheimer's disease. The Journal of neuroscience : the official journal of the Society for Neuroscience 33(33):13300-13311.
10. de Calignon A, et al. (2012) Propagation of tau pathology in a model of early Alzheimer's disease. Neuron 73(4):685-697.
11. Liu L, et al. (2012) Trans-synaptic spread of tau pathology in vivo. PloS one 7(2):e31302.
12. Harris JA, et al. (2012) Human P301L-mutant tau expression in mouse entorhinal-hippocampal network causes tau aggregation and presynaptic pathology but no cognitive deficits. PloS one 7(9):e45881.
13. Pooler AM, Phillips EC, Lau DH, Noble W, & Hanger DP (2013) Physiological release of endogenous tau is stimulated by neuronal activity. EMBO reports 14(4):389-394.
14. Yamada K, et al. (2014) Neuronal activity regulates extracellular tau in vivo. The Journal of experimental medicine 211(3):387-393.
15. Santacruz K, et al. (2005) Tau suppression in a neurodegenerative mouse model improves memory function. Science (New York, N.Y.) 309(5733):476-481.
16. Hoover BR, et al. (2010) Tau mislocalization to dendritic spines mediates synaptic dysfunction independently of neurodegeneration. Neuron 68(6):1067-1081.
17. Takeda S, et al. (2013) Brain interstitial oligomeric amyloid beta increases with age and is resistant to clearance from brain in a mouse model of Alzheimer's disease. FASEB journal: official publication of the Federation of American Societies for Experimental Biology 27(8):3239-3248.
18. Takeda S, et al. (2011) Novel microdialysis method to assess neuropeptides and large molecules in free-moving mouse. Neuroscience 186:110-119.
19. Wegmann S, et al. (2010) Human Tau isoforms assemble into ribbon-like fibrils that display polymorphic structure and stability. The Journal of biological chemistry 285(35):27302-27313.
20. Yamada K, et al. (2011) In vivo microdialysis reveals age-dependent decrease of brain interstitial fluid tau levels in P301S human tau transgenic mice. The Journal of neuroscience : the official journal of the Society for Neuroscience 31(37):13110-13117.
21. Taylor AM, et al. (2005) A microfluidic culture platform for CNS axonal injury, regeneration and transport. Nature methods 2(8):599-605.
22. Ahmed Z, et al. (2014) A novel in vivo model of tau propagation with rapid and progressive neurofibrillary tangle pathology: the pattern of spread is determined by connectivity, not proximity. Acta neuropathologica 127(5):667-683.
23. Guo JL & Lee VM (2011) Seeding of normal Tau by pathological Tau conformers drives pathogenesis of Alzheimer-like tangles. The Journal of biological chemistry 286(17):15317-15331.
24. Wu JW, et al. (2013) Small misfolded Tau species are internalized via bulk endocytosis and anterogradely and retrogradely transported in neurons. The Journal of biological chemistry 288(3):1856-1870.
25. Frost B, Jacks RL, & Diamond MI (2009) Propagation of tau misfolding from the outside to the inside of a cell. The Journal of biological chemistry 284(19):12845-12852.
26. Iba M, et al. (2013) Synthetic tau fibrils mediate transmission of neurofibrillary tangles in a transgenic mouse model of Alzheimer's-like tauopathy. The Journal of neuroscience : the official journal of the Society for Neuroscience 33(3):1024-1037.
27. Clavaguera F, et al. (2009) Transmission and spreading of tauopathy in transgenic mouse brain. Nature cell biology 11(7):909-913.
28. Michel CH, et al. (2014) Extracellular monomeric tau protein is sufficient to initiate the spread of tau protein pathology. The Journal of biological chemistry 289(2):956-967.
29. Holmes BB, et al. (2013) Heparan sulfate proteoglycans mediate internalization and propagation of specific proteopathic seeds. Proceedings of the National Academy of Sciences of the United States of America 110(33):E3138-3147.
30. Ghoshal N, et al. (2002) Tau conformational changes correspond to impairments of episodic memory in mild cognitive impairment and Alzheimer's disease. Experimental neurology 177(2):475-493.
31. Kuchibhotla KV, et al. (2014) Neurofibrillary tangle-bearing neurons are functionally integrated in cortical circuits in vivo. Proceedings of the National Academy of Sciences of the United States of America 111(1):510-514.
32. Kopeikina KJ, Hyman BT, & Spires-Jones TL (2012) Soluble forms of tau are toxic in Alzheimer's disease. Translational neuroscience 3(3):223-233.
33. Polydoro M, et al. (2014) Soluble pathological tau in the entorhinal cortex leads to presynaptic deficits in an early Alzheimer's disease model. Acta neuropathologica 127(2):257-270.
34. Fox LM, et al. (2011) Soluble tau species, not neurofibrillary aggregates, disrupt neural system integration in a tau transgenic model. Journal of neuropathology and experimental neurology 70(7):588-595.
35. Gomez-Ramos A, et al. (2009) Characteristics and consequences of muscarinic receptor activation by tau protein. European neuropsychopharmacology : the journal of the European College of Neuropsychopharmacology 19(10):708-717.
36. Gomez-Ramos A, Diaz-Hernandez M, Rubio A, Miras-Portugal MT, & Avila J (2008) Extracellular tau promotes intracellular calcium increase through M1 and M3 muscarinic receptors in neuronal cells. Molecular and cellular neurosciences 37(4):673-681.
37. Danzer KM, et al. (2011) Heat-shock protein 70 modulates toxic extracellular alpha-synuclein oligomers and rescues trans-synaptic toxicity. FASEB journal: official publication of the Federation of American Societies for Experimental Biology 25(1):326-336.
38. Lasagna-Reeves CA, et al. (2012) Alzheimer brain-derived tau oligomers propagate pathology from endogenous tau. Scientific reports 2:700.
39. Barghorn S, Biernat J, & Mandelkow E (2005) Purification of recombinant tau protein and preparation of Alzheimer-paired helical filaments in vitro. Methods in molecular biology (Clifton, N.J.) 299:35-51.
40. Cho H, Hamza B, Wong EA, & Irimia D (2014) On-demand, competing gradient arrays for neutrophil chemotaxis. Lab on a chip 14(5):972-978.

### References for Example 2

1. P. V. Arriagada, J. H. Growdon, E. T. Hedley-Whyte, B. T. Hyman, Neurofibrillary tangles but not senile plaques parallel duration and severity of Alzheimer's disease. Neurology 42, 631 (Mar, 1992).
2. V. M. Lee, M. Goedert, J. Q. Trojanowski, Neurodegenerative tauopathies. Annu Rev Neurosci 24, 1121 (2001).
3. H. Braak, E. Braak, Neuropathological stageing of Alzheimer-related changes. Acta neuropathologica 82, 239 (1991).
4. B. T. Hyman, G. W. Van Hoesen, A. R. Damasio, C. L. Barnes, Alzheimer's disease: cell-specific pathology isolates the hippocampal formation. Science 225, 1168 (Sep 14, 1984).
5. A. de Calignon et al., Propagation of tau pathology in a model of early Alzheimer's disease. Neuron 73, 685 (Feb 23, 2012).
6. J. A. Harris et al., Human P301L-mutant tau expression in mouse entorhinal-hippocampal network causes tau aggregation and presynaptic pathology but no cognitive deficits. PLoS One 7, e45881 (2012).
7. L. Liu et al., Trans-synaptic spread of tau pathology in vivo. PLoS One 7, e31302 (2012).
8. F. Clavaguera, M. Goedert, M. Tolnay, [Induction and spreading of tau pathology in a mouse model of Alzheimer's disease]. Medecine sciences : M/S 26, 121 (Feb, 2010).
9. D. W. Sanders et al., Distinct tau prion strains propagate in cells and mice and define different tauopathies. Neuron 82, 1271 (Jun 18, 2014).
10. L. C. Walker, M. I. Diamond, K. E. Duff, B. T. Hyman, Mechanisms of protein seeding in neurodegenerative diseases. JAMA neurology 70, 304 (Mar 1, 2013).
11. A. Van der Jeugd et al., Cognitive defects are reversible in inducible mice expressing pro-aggregant full-length human Tau. Acta neuropathologica 123, 787 (Jun, 2012).
12. S. Brandner et al., Normal host prion protein necessary for scrapie-induced neurotoxicity. Nature 379, 339 (Jan 25, 1996).
13. T. Timm, A. Marx, S. Panneerselvam, E. Mandelkow, E. M. Mandelkow, Structure and regulation of MARK, a kinase involved in abnormal phosphorylation of Tau protein. BMC neuroscience 9 Suppl 2, S9 (2008).
14. L. M. Ittner et al., Dendritic function of tau mediates amyloid-beta toxicity in Alzheimer's disease mouse models. Cell 142, 387 (Aug 6, 2010).
15. A. C. Alonso, I. Grundke-Iqbal, K. Iqbal, Alzheimer's disease hyperphosphorylated tau sequesters normal tau into tangles of filaments and disassembles microtubules. Nature medicine 2, 783 (Jul, 1996).
16. J. C. Augustinack, A. Schneider, E. M. Mandelkow, B. T. Hyman, Specific tau phosphorylation sites correlate with severity of neuronal cytopathology in Alzheimer's disease. Acta neuropathologica 103, 26 (Jan, 2002).
17. H. C. Tai et al., The synaptic accumulation of hyperphosphorylated tau oligomers in Alzheimer disease is associated with dysfunction of the ubiquitin-proteasome system. Am J Pathol 181, 1426 (Oct).
18. A. Serrano-Pozo, M. P. Frosch, E. Masliah, B. T. Hyman, Neuropathological alterations in Alzheimer disease. Cold Spring Harbor perspectives in medicine 1, a006189 (Sep, 2011).
19. Y. Yoshiyama et al., Synapse loss and microglial activation precede tangles in a P301S tauopathy mouse model. Neuron 53, 337 (Feb 1, 2007).
20. A. de Calignon et al., Caspase activation precedes and leads to tangles. Nature 464, 1201 (Apr 22, 2010).
21. K. V. Kuchibhotla et al., Neurofibrillary tangle-bearing neurons are functionally integrated in cortical circuits in vivo. Proc Natl Acad Sci U S A 111, 510 (Jan 7, 2014).
22. N. Rudinskiy et al., Tau pathology does not affect experience-driven single-neuron and network-wide Arc/Arg3.1 responses. Acta neuropathologica communications 2, 63 (2014).
23. K. SantaCruz et al., Tau suppression in a neurodegenerative mouse model improves memory function. Science 309, 476 (Jul 15, 2005).
24. F. Gallyas, Silver staining of Alzheimer's neurofibrillary changes by means of physical development. Acta Morphol Acad Sci Hung 19, 1 (1971).
25. P. E. Fraser, Prions and Prion-like Proteins. J Biol Chem 289, 19839 (Jul 18, 2014).
26. S. Kumar et al., Stages and Conformations of the Tau Repeat Domain during Aggregation and Its Effect on Neuronal Toxicity. J Biol Chem 289, 20318 (Jul 18, 2014).
27. M. Rapoport, H. N. Dawson, L. I. Binder, M. P. Vitek, A. Ferreira, Tau is essential to beta -amyloid-induced neurotoxicity. Proc Natl Acad Sci U S A 99, 6364 (Apr 30, 2002).
28. E. D. Roberson et al., Reducing endogenous tau ameliorates amyloid beta-induced deficits in an Alzheimer's disease mouse model. Science 316, 750 (May 4, 2007).
29. K. L. Tucker, M. Meyer, Y. A. Barde, Neurotrophins are required for nerve growth during development. Nat Neurosci 4, 29 (Jan, 2001).
30. M. Yasuda, M. R. Mayford, CaMKII activation in the entorhinal cortex disrupts previously encoded spatial memory. Neuron 50, 309 (Apr 20, 2006).
31. M. Mayford, J. Wang, E. R. Kandel, T. J. O'Dell, CaMKII regulates the frequency-response function of hippocampal synapses for the production of both LTD and LTP. Cell 81, 891 (Jun 16, 1995).
32. N. Schaeren-Wiemers, A. Gerfin-Moser, A single protocol to detect transcripts of various types and expression levels in neural tissue and cultured cells: in situ hybridization using digoxigenin-labelled cRNA probes. Histochemistry 100, 431 (Dec, 1993).
33. A. Sun, X. V. Nguyen, G. Bing, Comparative analysis of an improved thioflavin-s stain, Gallyas silver stain, and immunohistochemistry for neurofibrillary tangle demonstration on the same sections. The journal of histochemistry and cytochemistry : official journal of the Histochemistry Society 50, 463 (Apr, 2002).

### References for Example 3

Alpar, A., Ueberham, U., Bruckner, M.K., Seeger, G., Arendt, T., and Gartner, U. (2006). Different dendrite and dendritic spine alterations in basal and apical arbors in mutant human amyloid precursor protein transgenic mice. Brain Res 1099, 189-198.
Chipuk, J.E., Bouchier-Hayes, L., and Green, D.R. (2006). Mitochondrial outer membrane permeabilization during apoptosis: the innocent bystander scenario. Cell death and differentiation 13, 1396-1402.
D'Amelio, M., Cavallucci, V., Middei, S., Marchetti, C., Pacioni, S., Ferri, A., Diamantini, A., De Zio, D., Carrara, P., Battistini, L., et al. (2011). Caspase-3 triggers early synaptic dysfunction in a mouse model of Alzheimer's disease. Nat Neurosci 14, 69-76.
D'Amelio, M., Sheng, M., and Cecconi, F. (2012). Caspase-3 in the central nervous system: beyond apoptosis. Trends Neurosci 35, 700-709.
DaRocha-Souto, B., Coma, M., Perez-Nievas, B.G., Scotton, T.C., Siao, M., Sanchez-Ferrer, P., Hashimoto, T., Fan, Z., Hudry, E., Barroeta, I., et al. (2012). Activation of glycogen synthase kinase-3 beta mediates beta-amyloid induced neuritic damage in Alzheimer's disease. Neurobiol Dis 45, 425-437.
DuBoff, B., Gotz, J., and Feany, M.B. (2012). Tau promotes neurodegeneration via DRP1 mislocalization in vivo. Neuron 75, 618-632.
Gamblin, T.C., Chen, F., Zambrano, A., Abraha, A., Lagalwar, S., Guillozet, A.L., Lu, M., Fu, Y., Garcia-Sierra, F., LaPointe, N., et al. (2003). Caspase cleavage of tau: linking amyloid and neurofibrillary tangles in Alzheimer's disease. Proc Natl Acad Sci U S A 100, 10032-10037.
Goping, I.S., Gross, A., Lavoie, J.N., Nguyen, M., Jemmerson, R., Roth, K., Korsmeyer, S.J., and Shore, G.C. (1998). Regulated targeting of BAX to mitochondria. The Journal of cell biology 143, 207-215.
Hsieh, H., Boehm, J., Sato, C., Iwatsubo, T., Tomita, T., Sisodia, S., and Malinow, R. (2006). AMPAR removal underlies Abeta-induced synaptic depression and dendritic spine loss. Neuron 52, 831-843.
Hudry, E., Wu, H.Y., Arbel-Ornath, M., Hashimoto, T., Matsouaka, R., Fan, Z., Spires-Jones, T.L., Betensky, R.A., Bacskai, B.J., and Hyman, B.T. (2012). Inhibition of the NFAT pathway alleviates amyloid beta neurotoxicity in a mouse model of Alzheimer's disease. J Neurosci 32, 3176-3192.
Ittner, L.M., and Gotz, J. (2011). Amyloid-beta and tau--a toxic pas de deux in Alzheimer's disease. Nature reviews Neuroscience 12, 65-72.
Ittner, L.M., Ke, Y.D., Delerue, F., Bi, M., Gladbach, A., van Eersel, J., Wolfing, H., Chieng, B.C., Christie, M.J., Napier, I.A., et al. (2010). Dendritic function of tau mediates amyloid-beta toxicity in Alzheimer's disease mouse models. Cell 142, 387-397.
Jankowsky, J.L., Slunt, H.H., Ratovitski, T., Jenkins, N.A., Copeland, N.G., and Borchelt, D.R. (2001). Co-expression of multiple transgenes in mouse CNS: a comparison of strategies. Biomolecular engineering 17, 157-165.
Jiao, S., and Li, Z. (2011). Nonapoptotic function of BAD and BAX in long-term depression of synaptic transmission. Neuron 70, 758-772.
Johnson-Wood, K., Lee, M., Motter, R., Hu, K., Gordon, G., Barbour, R., Khan, K., Gordon, M., Tan, H., Games, D., et al. (1997). Amyloid precursor protein processing and A beta42 deposition in a transgenic mouse model of Alzheimer disease. Proc Natl Acad Sci U S A 94, 1550-1555.
Knafo, S., Alonso-Nanclares, L., Gonzalez-Soriano, J., Merino-Serrais, P., Fernaud-Espinosa, I., Ferrer, I., and DeFelipe, J. (2009). Widespread changes in dendritic spines in a model of Alzheimer's disease. Cereb Cortex 19, 586-592.
Koffie, R.M., Meyer-Luehmann, M., Hashimoto, T., Adams, K.W., Mielke, M.L., Garcia-Alloza, M., Micheva, K.D., Smith, S.J., Kim, M.L., Lee, V.M., et al. (2009). Oligomeric amyloid beta associates with postsynaptic densities and correlates with excitatory synapse loss near senile plaques. Proc Natl Acad Sci U S A 106, 4012-4017.
Kopeikina, K.J., Polydoro, M., Tai, H.C., Yaeger, E., Carlson, G.A., Pitstick, R., Hyman, B.T., and Spires-Jones, T.L. (2013a). Synaptic alterations in the rTg4510 mouse model of tauopathy. The Journal of comparative neurology 521, 1334-1353.
Kopeikina, K.J., Wegmann, S., Pitstick, R., Carlson, G.A., Bacskai, B.J., Betensky, R.A., Hyman, B.T., and Spires-Jones, T.L. (2013b). Tau causes synapse loss without disrupting calcium homeostasis in the rTg4510 model of tauopathy. PloS one 8, e80834.
Li, Z., Jo, J., Jia, J.M., Lo, S.C., Whitcomb, D.J., Jiao, S., Cho, K., and Sheng, M. (2010). Caspase-3 activation via mitochondria is required for long-term depression and AMPA receptor internalization. Cell 141, 859-871.
Li, Z., Okamoto, K., Hayashi, Y., and Sheng, M. (2004). The importance of dendritic mitochondria in the morphogenesis and plasticity of spines and synapses. Cell 119, 873-887.
Rapoport, M., Dawson, H.N., Binder, L.I., Vitek, M.P., and Ferreira, A. (2002). Tau is essential to beta -amyloid-induced neurotoxicity. Proc Natl Acad Sci U S A 99, 6364-6369.
Roberson, E.D., Scearce-Levie, K., Palop, J.J., Yan, F., Cheng, I.H., Wu, T., Gerstein, H., Yu, G.Q., and Mucke, L. (2007). Reducing endogenous tau ameliorates amyloid beta-induced deficits in an Alzheimer's disease mouse model. Science 316, 750-754.
Shankar, G.M., Li, S., Mehta, T.H., Garcia-Munoz, A., Shepardson, N.E., Smith, I., Brett, F.M., Farrell, M.A., Rowan, M.J., Lemere, C.A., et al. (2008). Amyloid-beta protein dimers isolated directly from Alzheimer's brains impair synaptic plasticity and memory. Nat Med 14, 837-842.
Shipton, O.A., Leitz, J.R., Dworzak, J., Acton, C.E., Tunbridge, E.M., Denk, F., Dawson, H.N., Vitek, M.P., Wade-Martins, R., Paulsen, O., et al. (2011). Tau protein is required for amyloid {beta}-induced impairment of hippocampal long-term potentiation. J Neurosci 31, 1688-1692.
Spires, T.L., Meyer-Luehmann, M., Stern, E.A., McLean, P.J., Skoch, J., Nguyen, P.T., Bacskai, B.J., and Hyman, B.T. (2005). Dendritic spine abnormalities in amyloid precursor protein transgenic mice demonstrated by gene transfer and intravital multiphoton microscopy. J Neurosci 25, 7278-7287.
Spires-Jones, T.L., and Hyman, B.T. (2014). The intersection of amyloid beta and tau at synapses in Alzheimer's disease. Neuron 82, 756-771.
Tucker, K.L., Meyer, M., and Barde, Y.A. (2001). Neurotrophins are required for nerve growth during development. Nat Neurosci 4, 29-37.
Vossel, K.A., Zhang, K., Brodbeck, J., Daub, A.C., Sharma, P., Finkbeiner, S., Cui, B., and Mucke, L. (2010). Tau reduction prevents Abeta-induced defects in axonal transport. Science 330, 198.
Wang, H.G., Pathan, N., Ethell, I.M., Krajewski, S., Yamaguchi, Y., Shibasaki, F., McKeon, F., Bobo, T., Franke, T.F., and Reed, J.C. (1999). Ca2+-induced apoptosis through calcineurin dephosphorylation of BAD. Science 284, 339-343.
Wolter, K.G., Hsu, Y.T., Smith, C.L., Nechushtan, A., Xi, X.G., and Youle, R.J. (1997). Movement of Bax from the cytosol to mitochondria during apoptosis. The Journal of cell biology 139, 1281-1292.
Wu, H.Y., Hudry, E., Hashimoto, T., Kuchibhotla, K., Rozkalne, A., Fan, Z., Spires-Jones, T., Xie, H., Arbel-Ornath, M., Grosskreutz, C.L., et al. (2010). Amyloid beta induces the morphological neurodegenerative triad of spine loss, dendritic simplification, and neuritic dystrophies through calcineurin activation. J Neurosci 30, 2636-2649.
Wu, H.Y., Hudry, E., Hashimoto, T., Uemura, K., Fan, Z.Y., Berezovska, O., Grosskreutz, C.L., Bacskai, B.J., and Hyman, B.T. (2012). Distinct dendritic spine and nuclear phases of calcineurin activation after exposure to amyloid-beta revealed by a novel fluorescence resonance energy transfer assay. J Neurosci 32, 5298-5309.
Zempel, H., Luedtke, J., Kumar, Y., Biernat, J., Dawson, H., Mandelkow, E., and Mandelkow, E.M. (2013). Amyloid-beta oligomers induce synaptic damage via Tau-dependent microtubule severing by TTLL6 and spastin. The EMBO journal 32, 2920-2937.

To the extent not already indicated, it will be understood by those of ordinary skill in the art that any one of the various embodiments herein described and illustrated can be further modified to incorporate features shown in any of the other embodiments disclosed herein. Thus, other embodiments are within the scope of the invention.

All patents and other publications identified herein are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

## Claims

1. An isolated antibody or antigen-binding portion thereof that specifically binds soluble high molecular weight (HMW) tau species and does not bind soluble low molecular weight (LMW) tau species, wherein the HMW tau species is non-fibrillar, with a molecular weight of at least 500 kDa as determined by size exclusion chromatography, and wherein the LMW tau species has a molecular weight of no more than 200 kDa as determined by size exclusion chromatography.

2. The isolated antibody or antigen-binding portion thereof of claim 1, which reduces the soluble HMW tau species being taken up by a neuron.

3. The isolated antibody or antigen-binding portion thereof of claim 1 or 2, wherein the soluble HMW tau species has a molecular weight of 669 kDa to 1000 kDa as determined by size exclusion chromatography.

4. The isolated antibody or antigen-binding portion thereof of claim 1, 2, or 3, wherein the soluble HMW tau species is phosphorylated.

5. An antagonist of a soluble HMW tau species for use in the treatment of neurodegenerative diseases by preventing propagation of pathological tau protein between synaptically-connected neurons comprising selectively reducing the extracellular level of soluble HMW tau species in contact with a synaptically-connected neuron, wherein the soluble HMW tau species is non-fibrillar, with a molecular weight of at least 500 kDa as determined using size exclusion chromatography, wherein a reduced level of the soluble HMW tau species results in reduced propagation of pathological tau protein between synaptically-connected neurons.

6. The antagonist for use in the treatment of neurodegenerative diseases according to claim 5, wherein the extracellular level of soluble LMW tau species is not substantially reduced during said selective reduction.

7. The antagonist for use in the treatment of neurodegenerative diseases according to claim 5 or 6, wherein the soluble HMW tau species is selectively reduced by contacting the extracellular space or fluid in contact with the synaptically-connected neurons with an antagonist of the soluble HMW tau species selected from the group consisting of an antibody, a zinc finger nuclease, a transcriptional repressor, a nucleic acid inhibitor, a small molecule, an aptamer, a gene-editing composition, and a combination thereof.

8. A method of diagnosing tau-associated neurodegeneration comprising
a. fractionating a sample of brain interstitial fluid or cerebrospinal fluid from a subject;
b. detecting soluble HMW tau species in the sample such that the presence and amount of the soluble HMW tau species is determined, wherein the soluble HMW tau species is non-fibrillar, with a molecular weight of at least 500 kDa as determined using size exclusion chromatography; and
c. identifying the subject to have, or be at risk for tau-associated neurodegeneration when the level of the soluble HMW tau species in the sample is the same as or above a reference level; or
identifying the subject to be less likely to have tau-associated neurodegeneration when the level of the soluble HMW tau species is below a reference level.

9. The method of claim 8, wherein the sample is substantially free of soluble LMW tau species, wherein the soluble LMW tau species has a molecular weight of no more than 200 kDa as determined using size exclusion chromatography.

10. The method of claim 8 or 9, further comprising detecting the amount of the soluble LMW tau species in the sample.

11. The method of claim 10, wherein the subject is identified to have, or be at risk for tau-associated neurodegeneration if a ratio of the soluble HMW tau species to the soluble LMW tau species is the same as or above a reference level ratio; or the subject is identified to be less likely to have tau-associated neurodegeneration if the ratio of the soluble HMW tau species to the soluble LMW tau species is below the reference level ratio.

12. The method of claim 10 or 11, wherein the detecting further comprises detecting phosphorylation of the soluble HMW tau species.

13. The method of any of claims 10-12, wherein the tau-associated neurodegeneration is Alzheimer's disease, Parkinson's disease, or frontotemporal dementia.

14. A method of identifying an agent that is effective to reduce cross-synaptic spread of misfolded tau proteins comprising
a. contacting a first neuron in a first chamber of a neuron culture device with soluble HMW tau species, wherein the HMW tau species is non-fibrillar, with a molecular weight of at least 500 kDa as determined by size exclusion chromatography and wherein the first neuron is axonally connected with a second neuron in a second chamber of the neuron culture device, and wherein the second neuron is not contacted with the soluble HMW tau species;
b. contacting the first neuron from (a) in the first chamber with a candidate agent;
c. detecting transport of the soluble HMW tau species from the first neuron to the second neuron, thereby identifying an effective agent for reducing cross-synaptic spread of misfolded tau proteins based on detection of the presence of the soluble HMW tau species in an axon and/or soma of the second neuron.

15. The method of claim 14, wherein the neuron culture device is a microfluidic device comprising a first chamber for placing a first neuron and a second chamber for placing a second neuron, wherein the first chamber and the second chamber are interconnected by at least one microchannel exclusively sized to permit axon growth.

## Patentansprüche

1. Isolierter Antikörper oder Antigenbindungsabschnitt davon, der eine lösliche hochmolekulare (HMW) Tau-Spezies spezifisch bindet und keine lösliche niedrigmolekulare (LMW) Tau-Spezies bindet, wobei die HMW-Tau-Spezies nicht fibrillär ist, mit einem Molekulargewicht von mindestens 500 kDa, wie mittels Größenausschlusschromatography bestimmt, und wobei die LMW-Tau-Spezies ein Molekulargewicht von nicht mehr als 200 kDa aufweist, wie mittels Größenausschlusschromatografie bestimmt.

2. Isolierter Antikörper oder Antigenbindungsabschnitt davon nach Anspruch 1, der die lösliche HMW-Tau-Spezies, die von einem Neuron aufgenommen wird, reduziert.

3. Isolierter Antikörper oder Antigenbindungsabschnitt davon nach Anspruch 1 oder 2, wobei die lösliche HMW-Tau-Spezies ein Molekulargewicht von 669 kDa bis 1000 kDa aufweist, wie mittels Größenausschlusschromatografie bestimmt.

4. Isolierter Antikörper oder Antigenbindungsabschnitt davon nach Anspruch 1, 2 oder 3, wobei die lösliche HMW-Tau-Spezies phosphoryliert ist.

5. Antagonist einer löslichen HMW-Tau-Spezies zur Verwendung in der Behandlung neurodegenerativer Erkrankungen durch Verhindern der Ausbreitung von pathologischem Tau-Protein zwischen synaptisch verbundenen Neuronen, umfassend das selektive Reduzieren der extrazellulären Spiegel an löslicher HMW-Tau-Spezies in Kontakt mit einem synaptisch verbundenen Neuron, wobei die lösliche HMW-Tau-Spezies nicht fibrillär ist, mit einem Molekulargewicht von mindestens 500 kDa, wie mittels Größenausschlusschromatografie bestimmt, wobei ein reduzierter Spiegel an löslicher HMW-Tau-Spezies in einer reduzierten Ausbreitung von pathologischem Tau-Protein zwischen synaptisch verbundenen Neuronen resultiert.

6. Antagonist zur Verwendung in der Behandlung neurodegenerativer Erkrankungen nach Anspruch 5, wobei der extrazelluläre Spiegel an löslicher LMW-Tau-Spezies während der selektiven Reduktion nicht wesentlich reduziert wird.

7. Antagonist zur Verwendung in der Behandlung neurodegenerativer Erkrankungen nach Anspruch 5 oder 6, wobei die lösliche HMW-Tau-Spezies selektiv reduziert wird durch Kontaktieren des extrazellulären Raums oder Fluids in Kontakt mit den synaptisch verbundenen Neuronen mit einem Antagonisten der löslichen HMW-Tau-Spezies, die aus der Gruppe bestehend aus einem Antikörper, einer Zinkfingernuklease, einem transkriptionalen Repressor, einem Nukleinsäurehemmer, einem kleinen Molekül, einem Aptamer, einer genbearbeitenden Zusammensetzung und einer Kombination davon ausgewählt ist.

8. Verfahren zum Diagnostizieren Tau-verbundener Neurodegeneration, umfassend
a. Fraktionieren einer Probe interstitieller Gehirnflüssigkeit oder Zerebrospinalflüssigkeit von einem Subjekt;
b. Erfassen löslicher HMW-Tau-Spezies in der Probe derart, dass das Vorhandensein und die Menge der löslichen HMW-Tau-Spezies bestimmt werden, wobei die lösliche HMW-Tau-Spezies nicht fibrillär ist, mit einem Molekulargewicht von mindestens 500 kDa, wie mittels Größenausschlusschromatografie bestimmt; und
c. Identifizieren, dass das Subjekt ein Risiko für Tau-verbundene Neurodegeneration aufweist oder diesbezüglich gefährdet ist, wenn der Spiegel der löslichen HMW-Tau-Spezies in der Probe einem Referenzspiegel entspricht oder darüber liegt; oder
Identifizieren, dass die Wahrscheinlichkeit, dass das Subjekt eine Tau-verbundene Neurodegeneration haben wird, gering ist, wenn der Spiegel der löslichen HMW-Tau-Spezies unter einem Referenzspiegel liegt.

9. Verfahren nach Anspruch 8, wobei die Probe im Wesentlichen frei von löslicher LMW-Tau-Spezies ist, wobei die lösliche LMW-Tau-Spezies ein Molekulargewicht von nicht mehr als 200 kDa aufweist, wie mittels Größenausschlusschromatografie bestimmt.

10. Verfahren nach Anspruch 8 oder 9, ferner umfassend das Erfassen der Menge an löslicher LMW-Tau-Spezies in der Probe.

11. Verfahren nach Anspruch 10, wobei identifiziert wird, dass das Subjekt ein Risiko für Tau-verbundene Neurodegeneration hat oder diesbezüglich gefährdet ist, wenn ein Verhältnis der löslichen HMW-Tau-Spezies zu der löslichen LMW-Tau-Spezies einem Referenzspiegelverhältnis entspricht oder darüber liegt; oder identifiziert wird, dass die Wahrscheinlichkeit gering ist, dass das Subjekt Tau-verbundene Neurodegeneration hat, wenn das Verhältnis der löslichen HMW-Tau-Spezies zu der löslichen LMW-Tau-Spezies unter dem Referenzspiegelverhältnis liegt.

12. Verfahren nach Anspruch 10 oder 11, wobei das Erfassen ferner das Erfassen der Phosphorylierung der löslichen HMW-Tau-Spezies umfasst.

13. Verfahren nach einem der Ansprüche 10-12, wobei die Tau-verbundene Neurodegeration Alzheimer-Krankheit, Parkinson-Krankheit oder frontotemporale Demenz ist.

14. Verfahren zum Identifizieren eines Stoffes, der wirksam ist, die quersynaptische Ausbreitung fehlgefalteter Tau-Proteinen zu reduzieren, umfassend
a. Kontaktieren eines ersten Neurons in einer ersten Kammer einer Neuronenkulturvorrichtung mit einer löslichen HMW-Tau-Spezies, wobei die HMW-Tau-Spezies nicht fibrillär ist, mit einem Molekulargewicht von mindestens 500 kDa, wie mittels Größenausschlusschromatografie bestimmt, und wobei das erste Neuron axonal mit einem zweiten Neuron in einer zweiten Kammer der Neuronenkulturvorrichtung verbunden ist, und wobei das zweite Neuron nicht mit der löslichen HMW-Tau-Spezies kontaktiert ist;
b. Kontaktieren des ersten Neurons aus (a) in der ersten Kammer mit einem möglichen Mittel;
c. Erfassen des Transports der löslichen HMW-Tau-Spezies von dem ersten Neuron zu dem zweiten Neuron, dadurch Identifizieren eines wirksamen Mittels zum Reduzieren quersynaptischer Verbreitung fehlgefalteter Tau-Proteine basierend auf der Erfassung des Vorhandenseins der löslichen HMW-Tau-Spezies in einem Axon und/oder Soma des zweiten Neurons.

15. Verfahren nach Anspruch 14, wobei die Neuronenkulturvorrichtung eine Mikrofluidikvorrichtung ist, umfassend eine erste Kammer zum Platzieren eines ersten Neurons und eine zweite Kammer zum Platzieren eines zweiten Neurons, wobei die erste Kammer und die zweite Kammer durch mindestens einen Mikrokanal miteinander verbunden sind, der ausschließlich dazu bemessen ist, Axonwachstum zu erlauben.

## Revendications

1. Anticorps isolé ou fragment de liaison à l'antigène de celui-ci qui se lie spécifiquement à une forme soluble de Tau de haut poids moléculaire (HMW) et qui ne se lie pas à une forme soluble de Tau de bas poids moléculaire (LMW), où la forme de Tau de HMW est non fibrillaire et a un poids moléculaire, tel que déterminé par chromatographie d'exclusion stérique, de 500 kDa au moins et où la forme de Tau de LMW a un poids moléculaire, tel que déterminé par chromatographie d'exclusion stérique, qui ne dépasse pas 200 kDa.

2. Anticorps isolé ou fragment de liaison à l'antigène de celui-ci de la revendication 1 qui diminue la forme soluble de Tau de HMW captée par un neurone.

3. Anticorps isolé ou fragment de liaison à l'antigène de celui-ci de la revendication 1 ou 2, où la forme soluble de Tau de HMW a un poids moléculaire, tel que déterminé par chromatographie d'exclusion stérique, qui va de 669 kDa à 1 000 kDa.

4. Anticorps isolé ou fragment de liaison à l'antigène de celui-ci de la revendication 1, 2 ou 3, où la forme soluble de Tau de HMW est phosphorylée.

5. Antagoniste d'une forme soluble de Tau de HMW pour une utilisation dans le traitement de maladies neurodégénératives par inhibition de la propagation de la forme pathologique de la protéine Tau entre des neurones connectés par des synapses, qui comprend une diminution sélective du taux extracellulaire de la forme soluble de Tau de HMW en contact avec un neurone connecté par des synapses, où la forme soluble de Tau de HMW est non fibrillaire et a un poids moléculaire, tel que déterminé par chromatographie d'exclusion stérique, de 500 kDa au moins et où une diminution du taux de la forme soluble de Tau de HMW résulte en une diminution de la propagation de la forme pathologique de la protéine Tau entre des neurones connectés par des synapses.

6. Antagoniste pour une utilisation dans le traitement de maladies neurodégénératives selon la revendication 5, où le taux extracellulaire de la forme soluble de Tau de LMW n'est essentiellement pas diminué lors de ladite diminution sélective.

7. Antagoniste pour une utilisation dans le traitement de maladies neurodégénératives selon la revendication 5 ou 6, où la forme soluble de Tau de HMW est sélectivement diminuée par mise en contact de l'espace ou du fluide extracellulaire en contact avec des neurones connectés par des synapses avec un antagoniste de la forme soluble de Tau de HMW sélectionné dans le groupe consistant en un anticorps, une nucléase à doigt de zinc, un répresseur de la transcription, un inhibiteur d'acides nucléiques, une petite molécule, un aptamère, une composition d'édition de gènes et une combinaison de ceux-ci.

8. Méthode de diagnostic d'une neurodégénérescence associée à Tau comprenant :
a. le fractionnement d'un échantillon du liquide interstitiel cérébral ou du liquide céphalorachidien d'un sujet ;
b. la détection de la forme soluble de Tau de HMW dans l'échantillon de sorte que la présence et la quantité de la forme soluble de Tau de HMW sont déterminées, où la forme soluble de Tau de HMW est non fibrillaire et a un poids moléculaire, tel que déterminé par chromatographie d'exclusion stérique, de 500 kDa au moins ; et
c. l'identification du sujet comme présentant ou étant à risque de présenter une neurodégénérescence associée à Tau quand le taux de la forme soluble de Tau de HMW dans l'échantillon est égal ou supérieur à un taux de référence ; ou
l'identification du sujet comme étant moins à risque de présenter une neurodégénérescence associée à Tau quand le taux de la forme soluble de Tau de HMW est inférieur à un taux de référence.

9. Méthode de la revendication 8, où l'échantillon est essentiellement exempt de la forme soluble de Tau de LMW, où la forme soluble de Tau de LMW a un poids moléculaire, tel que déterminé par chromatographie d'exclusion stérique, qui ne dépasse pas 200 kDa.

10. Méthode de la revendication 8 ou 9 comprenant en outre la détection de la quantité de la forme soluble de Tau de LMW dans l'échantillon.

11. Méthode de la revendication 10, où le sujet est identifié comme présentant ou comme étant à risque de présenter une neurodégénérescence associée à Tau si un rapport entre la forme soluble de Tau de HMW et la forme soluble de Tau de LMW est égal ou supérieur à un rapport de référence des taux ; ou le sujet est identifié comme étant moins à risque de présenter une neurodégénérescence associée à Tau si le rapport entre la forme soluble de Tau de HMW et la forme soluble de Tau de LMW est inférieur à un rapport de référence des taux.

12. Méthode de la revendication 10 ou 11, où la détection comprend en outre une détection de la phosphorylation de la forme soluble de Tau de HMW.

13. Méthode de l'une quelconque des revendications 10-12, où la neurodégénérescence associée à Tau est la maladie d'Alzheimer, la maladie de Parkinson ou la démence fronto-temporale.

14. Méthode d'identification d'un agent qui est efficace dans la diminution du transfert, d'une synapse à une autre, de formes mal repliées de la protéine Tau comprenant
a. la mise en contact d'un premier neurone, placé dans un premier compartiment d'un système de culture de neurones, avec la forme soluble de Tau de HMW, où la forme soluble de Tau de HMW est non fibrillaire et a un poids moléculaire, tel que déterminé par chromatographie d'exclusion stérique, de 500 kDa au moins et où le premier neurone est en connexion axonale avec un deuxième neurone placé dans un deuxième compartiment du système de culture de neurone, le deuxième neurone n'étant pas mis en contact avec la forme soluble de Tau de HMW ;
b. la mise en contact du premier neurone de (a) placé dans le premier compartiment avec un agent candidat ;
c. la détection du transfert de la forme soluble de Tau de HMW du premier neurone au deuxième neurone, conduisant ainsi à l'identification d'un agent qui est efficace dans la diminution du transfert, d'une synapse à une autre, de formes mal repliées de la protéine Tau sur la base de la détection de la présence de la forme soluble de Tau de HMW dans un axone et/ou un corps du deuxième neurone.

15. Méthode de la revendication 14, où le système de culture de neurones est un dispositif microfluidique comprenant un premier compartiment dans lequel est placé un premier neurone et un deuxième compartiment dans lequel est placé un deuxième neurone, où le premier compartiment et le deuxième compartiment sont reliés ensemble par au moins un microcanal dimensionné de manière exclusive pour permettre une croissance axonale.
